(19) Europäisches Patentamt
European Patent Office
Office européen des brevets

(11) **EP 3 287 450 B1**

(12) **EUROPÄISCHE PATENTSCHRIFT**

(45) Veröffentlichungstag und Bekanntmachung des Hinweises auf die Patenterteilung:
**20.03.2019 Patentblatt 2019/12**

(21) Anmeldenummer: **17187662.6**

(22) Anmeldetag: **24.08.2017**

(51) Int Cl.:
***C07D 401/14*** (2006.01) ***C07D 405/14*** (2006.01)
***C07D 403/14*** (2006.01) ***C07D 519/00*** (2006.01)
***C07D 209/86*** (2006.01) ***C07D 209/88*** (2006.01)
***H01B 1/12*** (2006.01) ***H01L 51/50*** (2006.01)

(54) **ORGANISCHE MOLEKÜLE, INSBESONDERE ZUR VERWENDUNG IN ORGANISCHEN OPTOELEKTRONISCHEN VORRICHTUNGEN**

ORGANIC MOLECULES, IN PARTICULAR FOR USE IN ORGANIC OPTOELECTRONIC DEVICES

MOLÉCULES ORGANIQUES, EN PARTICULIER DESTINÉES À ÊTRE UTILISÉES DANS DES COMPOSANTS OPTOÉLECTRONIQUES ORGANIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.08.2016 DE 102016115854**
**15.09.2016 DE 102016117390**
**24.02.2017 DE 102017103942**
**16.03.2017 DE 102017105691**

(43) Veröffentlichungstag der Anmeldung:
**28.02.2018 Patentblatt 2018/09**

(73) Patentinhaber: **cynora GmbH**
**76646 Bruchsal (DE)**

(72) Erfinder:
- **FABIO, Martina**
**38440 Wolfsburg (DE)**
- **BERGMANN, Larissa**
**76199 Karlsruhe (DE)**
- **ZINK, Daniel**
**76646 Bruchsal (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**WO-A1-2014/183080 WO-A1-2015/175678**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 287 450 B1

**Beschreibung**

Die Erfindung betrifft rein organische Moleküle und deren Verwendung in organischen lichtemittierenden Dioden (OLEDs) und in anderen organischen optoelektronischen Vorrichtungen.

**Beschreibung**

Der vorliegenden Erfindung lag die Aufgabe zu Grunde, Moleküle bereitzustellen, die sich zur Verwendung in optoelektronischen Vorrichtungen eignen.

Diese Aufgabe wird durch die Erfindung gelöst, die eine neue Klasse von organischen Molekülen bereitstellt.

Die erfindungsgemäßen organischen Moleküle sind rein organische Moleküle, weisen also keine Metallionen auf und grenzen sich so von den zur Verwendung in organischen optoelektronischen Vorrichtungen bekannten Metallkomplexverbindungen ab.

Die erfindungsgemäßen organischen Moleküle zeichnen sich durch Emissionen im blauen, himmelblauen oder grünen Spektralbereich aus. Die Photolumineszenzquantenausbeuten der erfindungsgemäßen organischen Moleküle betragen insbesondere 20 % und mehr. Die erfindungsgemäßen Moleküle zeigen insbesondere thermisch aktivierte verzögerte Fluoreszenz (TADF). Die Verwendung der erfindungsgemäßen Moleküle in einer optoelektronischen Vorrichtung, beispielsweise einer organischen lichtemittierenden Diode (OLED), führt zu höheren Effizienzen der Vorrichtung. Entsprechende OLEDs weisen eine höhere Stabilität auf als OLEDs mit bekannten Emittermaterialien und vergleichbarer Farbe. Unter dem blauen Spektralbereich wird hier der sichtbare Bereich von 420 nm bis 470 nm verstanden. Unter dem himmelblauen Spektralbereich wird hier der Bereich von 470 nm bis 499 nm verstanden. Unter dem grünen Spektralbereich wird hier der Bereich von 500 nm bis 599 nm verstanden. Dabei liegt das Emissionsmaximum im jeweiligen Bereich. WO2015175678 und WO2014183080 offenbaren heterozyklische Verbindungen und deren Verwendung in OLEDs.

Die organischen Moleküle enthalten eine erste chemische Einheit aufweisend eine oder bestehend aus einer Struktur gemäß Formel I:

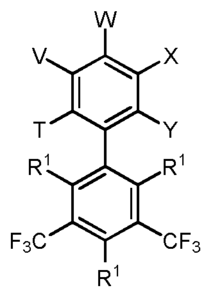

Formel I

und

- zwei zweite chemische Einheiten D jeweils bei jedem Auftreten gleich oder verschieden aufweisend eine oder bestehend aus einer Struktur gemäß Formel II,

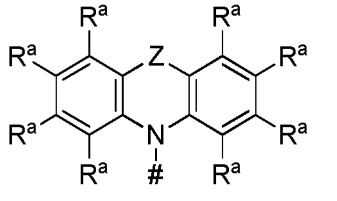

Formel II

Hierbei ist die erste chemische Einheit jeweils über eine Einfachbindung mit den zwei zweiten chemischen Einheiten D verknüpft.

T ist Anknüpfungspunkt der Einfachbindung zwischen der chemischen Einheit gemäß Formel I und einer chemischen Einheit D oder H.

V ist Anknüpfungspunkt der Einfachbindung zwischen der chemischen Einheit gemäß Formel I und einer

chemischen Einheit D oder H.

W ist Anknüpfungspunkt der Einfachbindung zwischen der chemischen Einheit gemäß Formel I und einer chemischen Einheit D oder ausgewählt aus der Gruppe bestehend aus H, CN und $CF_3$.

X ist Anknüpfungspunkt der Einfachbindung zwischen der chemischen Einheit gemäß Formel I und einer chemischen Einheit D oder ausgewählt aus der Gruppe bestehend aus H, CN und $CF_3$.

Y ist Anknüpfungspunkt der Einfachbindung zwischen der chemischen Einheit gemäß Formel I und einer chemischen Einheit D oder ausgewählt aus der Gruppe bestehend aus H, CN und $CF_3$;
# ist Anknüpfungspunkt der Einfachbindung zwischen der jeweiligen chemischen Einheit D und der chemischen Einheit gemäß Formel I.

Z ist bei jedem Auftreten gleich oder verschieden eine direkte Bindung oder ausgewählt aus der Gruppe bestehend aus $CR^3R^4$, $C=CR^3R^4$, C=O, $C=NR^3$, $NR^3$, O, $SiR^3R^4$, S, S(O) und $S(O)_2$.

$R^1$ ist bei jedem Auftreten gleich oder verschieden H, Deuterium, eine lineare Alkylgruppe mit 1 bis 5 C-Atomen, eine lineare Alkenyl- oder Alkinylgruppe mit 2 bis 8 C-Atomen, eine verzweigte oder cyclische Alkyl-, Alkenyl- oder Alkinylgruppe mit 3 bis 10 C-Atomen, wobei ein oder mehrere H-Atome durch Deuterium ersetzt sein können, oder ein aromatisches oder heteroaromatisches Ringsystem mit 5 bis 15 aromatischen Ringatomen, das jeweils durch einen oder mehrere Reste $R^6$ substituiert sein kann.

$R^a$, $R^3$ und $R^4$ ist bei jedem Auftreten gleich oder verschieden H, Deuterium, $N(R^5)_2$, OH, $Si(R^5)_3$, $B(OR^5)_2$, $OSO_2R^5$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils durch einen oder mehrere Reste $R^5$ substituiert sein kann, oder eine Aryloxy- oder Heteroaryloxygruppe mit 5 bis 60 aromatischen Ringatomen, die durch einen oder mehrere Reste $R^5$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^5$ substituiert sein kann.

$R^5$ ist bei jedem Auftreten gleich oder verschieden H, Deuterium, $N(R^6)_2$, OH, $Si(R^6)_3$, $B(OR^6)_2$, $OSO_2R^6$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils durch einen oder mehrere Reste $R^6$ substituiert sein kann, oder eine Aryloxy- oder Heteroaryloxygruppe mit 5 bis 60 aromatischen Ringatomen, die durch einen oder mehrere Reste $R^6$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^6$ substituiert sein kann.

$R^6$ ist bei jedem Auftreten gleich oder verschieden H, Deuterium, OH, $CF_3$, CN, F, eine lineare Alkyl-, Alkoxy- oder Thioalkoxygruppe mit 1 bis 5 C-Atomen oder eine lineare Alkenyl- oder Alkinylgruppe mit 2 bis 5 C-Atomen oder eine verzweigte oder cyclische Alkyl-, Alkenyl-, Alkinyl-, Alkoxy- oder Thioalkoxygruppe mit 3 bis 5 C-Atomen, wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen oder eine Aryloxy- oder Heteroaryloxygruppe mit 5 bis 60 aromatischen Ringatomen oder eine Diarylaminogruppe, Diheteroarylaminogruppe oder Arylheteroarylaminogruppe mit 10 bis 40 aromatischen Ringatomen.

Erfindungsgemäß kann jeder der Reste $R^a$, $R^3$, $R^4$ oder $R^5$ auch mit einem oder mehreren weiteren Resten $R^a$, $R^3$, $R^4$ oder $R^5$ ein mono- oder polycyclisches, aliphatisches, aromatisches und/oder benzoanelliertes Ringsystem bilden.

Erfindungsgemäß ist genau ein Rest ausgewählt aus W, X und Y gleich CN oder $CF_3$ und genau zwei Reste ausgewählt aus der Gruppe bestehend aus T, V, W, X und Y sind gleich einem Anknüpfungspunkt einer Einfachbindung zwischen der chemischen Einheit gemäß Formel I und einer chemischen Einheit D.

In einer Ausführungsform ist $R^1$ bei jedem Auftreten gleich oder verschieden H, Methyl oder Phenyl.

In einer Ausführungsform ist W gleich CN.

In einer weiteren Ausführungsform der organischen Moleküle weist die chemische Gruppe D bei jedem Auftreten gleich oder verschieden eine Struktur der Formel IIa auf bzw. besteht aus einer Struktur der Formel IIa:

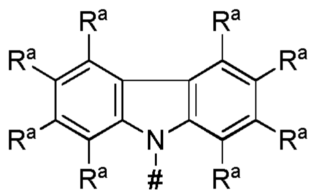

Formel IIa

wobei für # und $R^a$ die für Formel I und II genannten Definitionen gelten.

In einer weiteren Ausführungsform der erfindungsgemäßen organischen Moleküle weist die chemische Einheit D bei jedem Auftreten gleich oder verschieden eine Struktur der Formel IIb, der Formel IIb-2,der Formel IIb-3 oder der der Formel IIb-4 auf oder besteht daraus:

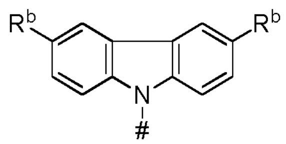

Formel IIb

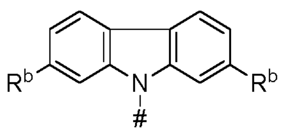

Formel IIb-2

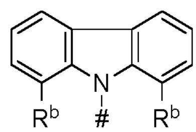

Formel IIb-3

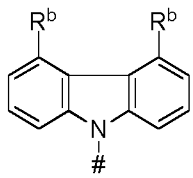

Formel IIb-4

wobei gilt

$R^b$ ist bei jedem Auftreten gleich oder verschieden $N(R^5)_2$, OH, $Si(R^5)_3$, $B(OR^5)_2$, $OSO_2R^5$, $CF_3$, CN, F, Br, I, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C{=}CR^5$, C≡C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils durch einen oder mehrere Reste $R^5$ substituiert sein kann, oder eine Aryloxy- oder Heteroaryloxygruppe mit 5 bis 60 aromatischen Ringatomen, die durch einen oder mehrere Reste $R^5$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^5$ substituiert sein kann. Ansonsten gelten die oben genannten Definitionen.

In einer weiteren Ausführungsform der erfindungsgemäßen organischen Moleküle weist die chemische Einheit D bei jedem Auftreten gleich oder verschieden eine Struktur der Formel IIc, der Formel IIc-2, der Formel IIc-3 oder der Formel IIc-4 auf oder besteht daraus:

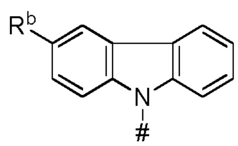

Formel IIc

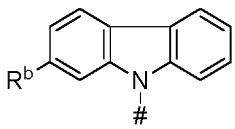

Formel IIc-2

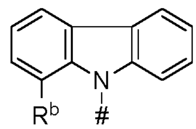

Formel IIc-3

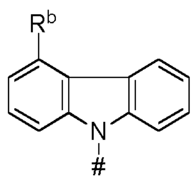

Formel IIc-4

wobei die oben genannten Definitionen gelten.

In einer weiteren Ausführungsform der erfindungsgemäßen organischen Moleküle ist $R^b$ bei jedem Auftreten unabhängig voneinander ausgewählt aus der Gruppe bestehend aus Me, $^i$Pr, $^t$Bu, CN, $CF_3$, Ph, das jeweils durch einen oder mehrere Reste ausgewählt aus Me, $^i$Pr, $^t$Bu, CN, $CF_3$ oder Ph substituiert sein kann, Pyridinyl, das jeweils durch einen oder mehrere Reste ausgewählt aus Me, $^i$Pr, $^t$Bu, CN, $CF_3$ oder Ph substituiert sein kann, Pyrimidinyl, das jeweils durch einen oder mehrere Reste ausgewählt aus Me, $^i$Pr, $^t$Bu, CN, $CF_3$ oder Ph substituiert sein kann, Carbazolyl, das jeweils durch einen oder mehrere Reste ausgewählt aus Me, $^i$Pr, $^t$Bu, CN, $CF_3$ oder Ph substituiert sein kann, Triazinyl, das jeweils durch einen oder mehrere Reste ausgewählt aus Me, $^i$Pr, $^t$Bu, CN, $CF_3$ oder Ph substituiert sein kann, und $N(Ph)_2$.

Im Folgenden sind beispielhaft Ausführungsformen der chemischen Gruppe D gezeigt:

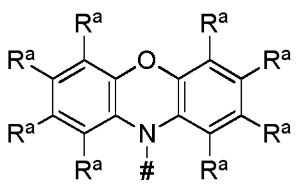
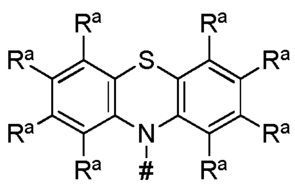
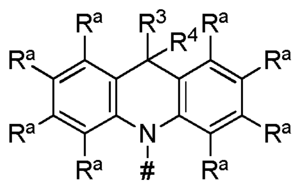
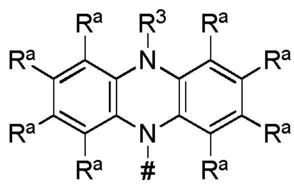

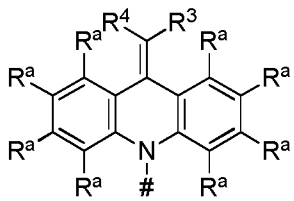
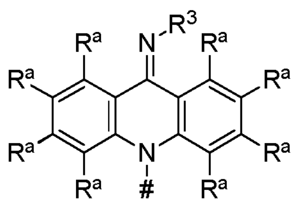
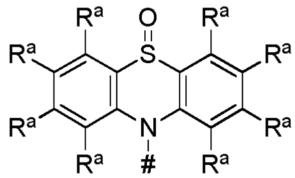

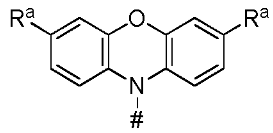
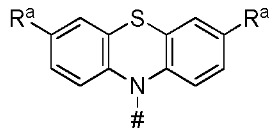
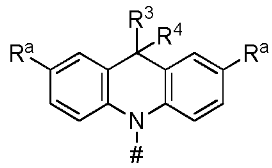
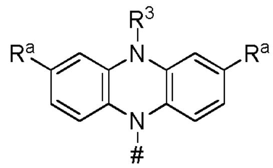

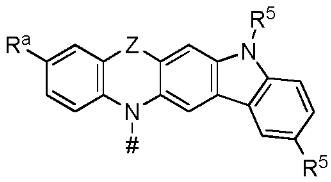
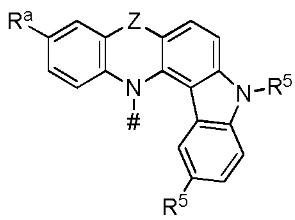
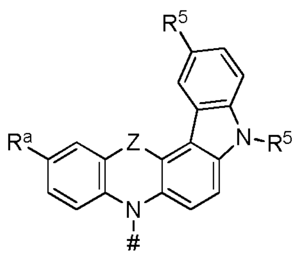

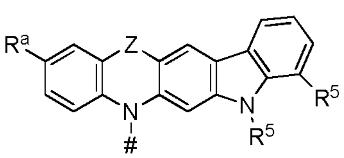
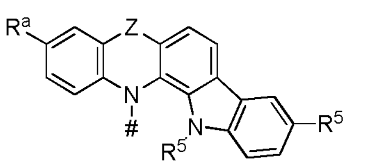
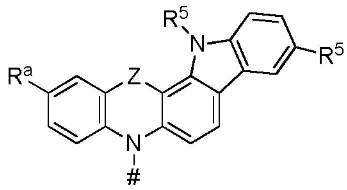

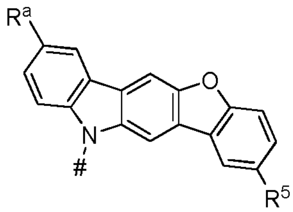
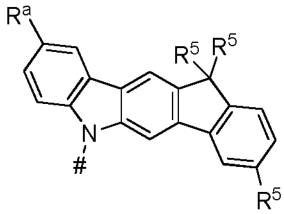
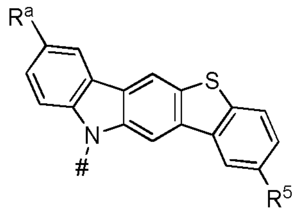

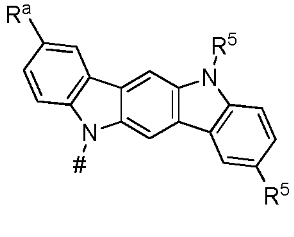

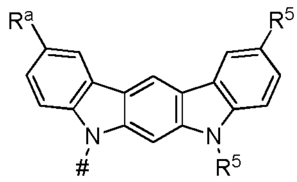

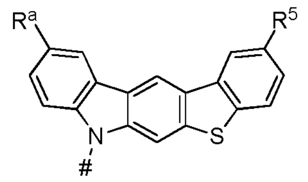

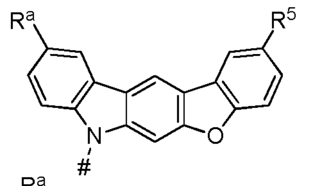

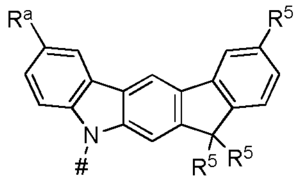

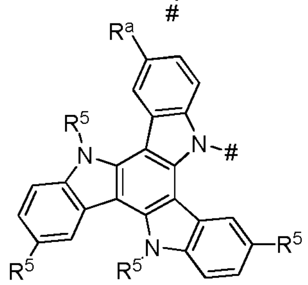

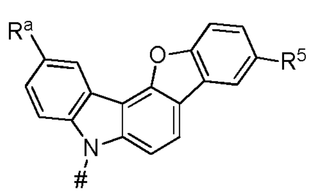

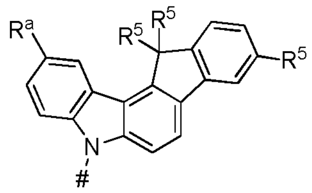

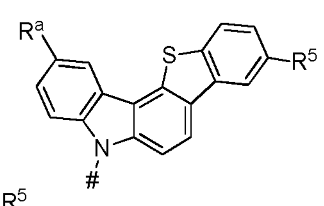

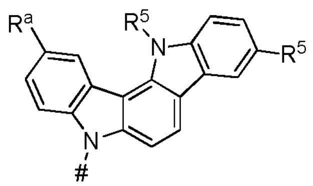

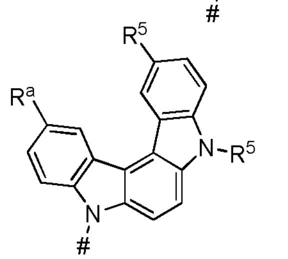

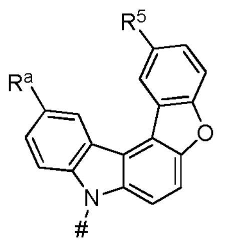

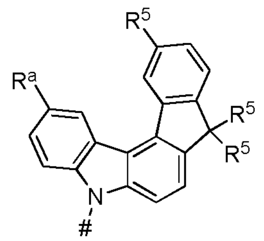

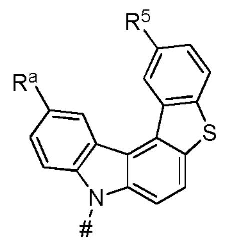

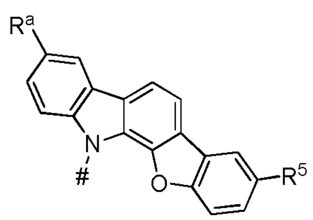

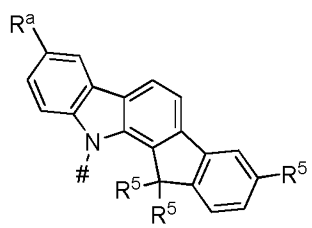

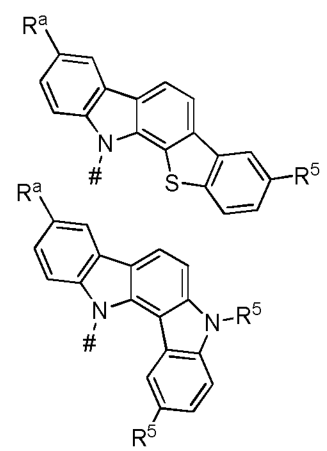

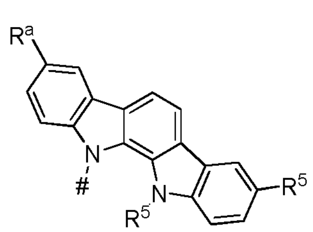

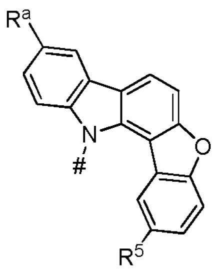
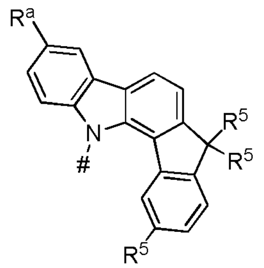
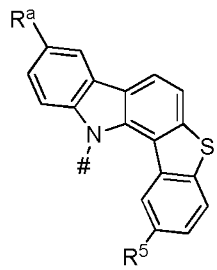

wobei für #, Z, $R^a$, $R^3$, $R^4$ und $R^5$ die oben genannten Definitionen gelten. In einer Ausführungsform ist der Rest $R^5$ bei jedem Auftreten gleich oder verschieden ausgewählt aus der Gruppe bestehend aus H, Methyl, Ethyl, Phenyl und Mesityl. In einer Ausführungsform ist $R^a$ bei jedem Auftreten gleich oder verschieden ausgewählt aus der Gruppe bestehend aus H, Methyl (Me), i-Propyl ($CH(CH_3)_2$) ($^iPr$), t-Butyl ($^tBu$), Phenyl (Ph), CN, $CF_3$ und Diphenylamin ($NPh_2$).

In einer Ausführungsform weisen die erfindungsgemäßen organischen Moleküle eine Struktur der Formel III auf:

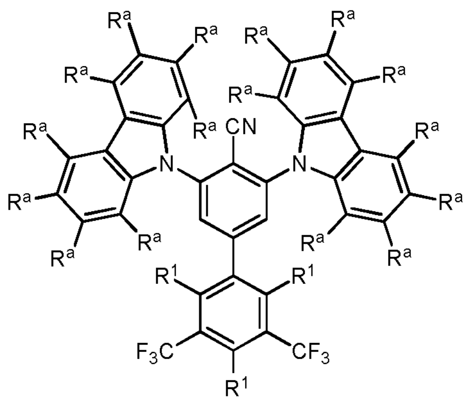

Formel III

wobei die oben genannten Definitionen gelten.

In einer weiteren Ausführungsform weisen die erfindungsgemäßen organischen Moleküle eine Struktur der Formel IIIa auf:

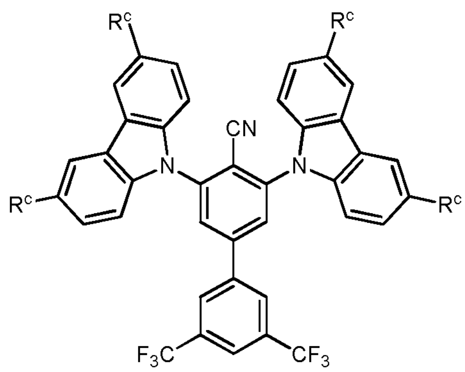

Formel IIIa

wobei

$R^c$ bei jedem Auftreten unabhängig voneinander ausgewählt aus der Gruppe bestehend aus Me, $^iPr$, $^tBu$, CN, $CF_3$, Ph, das jeweils durch einen oder mehrere Reste ausgewählt aus Me, $^iPr$, $^tBu$, CN, $CF_3$ oder Ph substituiert sein kann, Pyridinyl, das jeweils durch einen oder mehrere Reste ausgewählt aus Me, $^iPr$, $^tBu$, CN, $CF_3$ oder Ph substituiert sein kann, Pyrimidinyl, das jeweils durch einen oder mehrere Reste ausgewählt aus Me, $^iPr$, $^tBu$, CN, $CF_3$ oder Ph substituiert sein kann, Carbazolyl, das jeweils durch einen oder mehrere Reste ausgewählt aus Me, $^iPr$, $^tBu$, CN, $CF_3$ oder Ph substituiert sein kann, Triazinyl, das jeweils durch einen oder mehrere Reste ausgewählt aus Me, $^iPr$, $^tBu$, CN, $CF_3$ oder

Ph substituiert sein kann, und $N(Ph)_2$ ist.

In einer weiteren Ausführungsform weisen die erfindungsgemäßen organischen Moleküle eine Struktur der Formel IIIb auf:

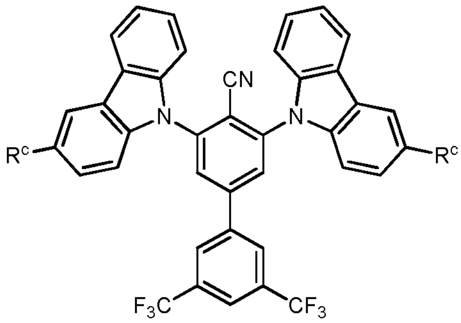

Formel IIIb

wobei die oben genannten Definitionen gelten.

In einer weiteren Ausführungsform weisen die erfindungsgemäßen organischen Moleküle eine Struktur der Formel IIIc auf:

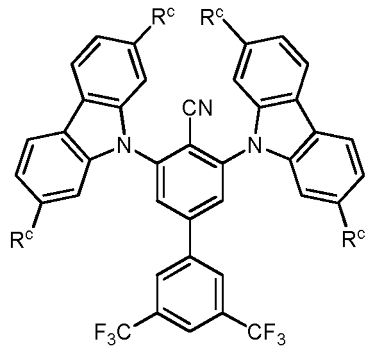

Formel IIIc

wobei die oben genannten Definitionen gelten.

In einer weiteren Ausführungsform weisen die erfindungsgemäßen organischen Moleküle eine Struktur der Formel IIId auf:

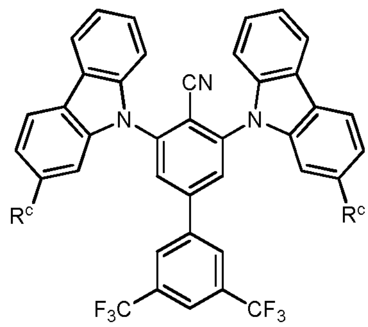

Formel IIId

wobei die oben genannten Definitionen gelten.

In einer weiteren Ausführungsform weisen die erfindungsgemäßen organischen Moleküle eine Struktur der Formel IIIe auf:

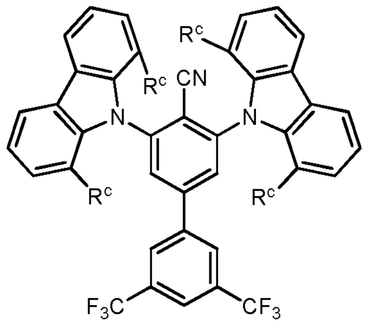

Formel IIIe

wobei die oben genannten Definitionen gelten.

In einer weiteren Ausführungsform weisen die erfindungsgemäßen organischen Moleküle eine Struktur der Formel IIIf auf:

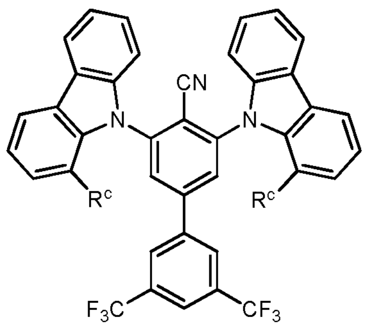

Formel IIIf

wobei die oben genannten Definitionen gelten.

In einer weiteren Ausführungsform weisen die erfindungsgemäßen organischen Moleküle eine Struktur der Formel IIIg auf:

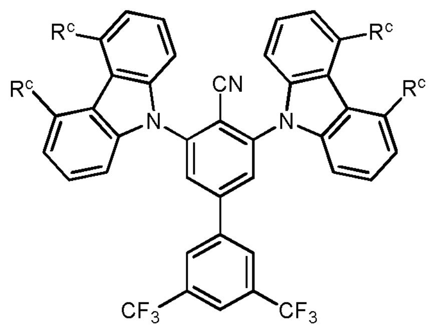

Formel IIIg

wobei die oben genannten Definitionen gelten.

In einer weiteren Ausführungsform weisen die erfindungsgemäßen organischen Moleküle eine Struktur der Formel IIIh auf:

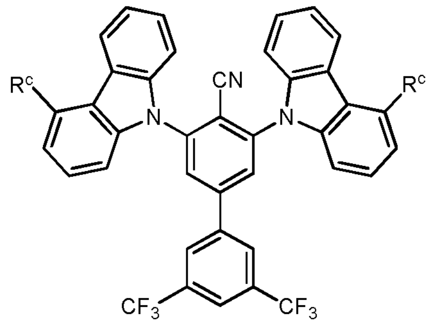

Formel IIIh

wobei die oben genannten Definitionen gelten.

In einer Ausführungsform weisen die erfindungsgemäßen organischen Moleküle eine Struktur der Formel IV auf:

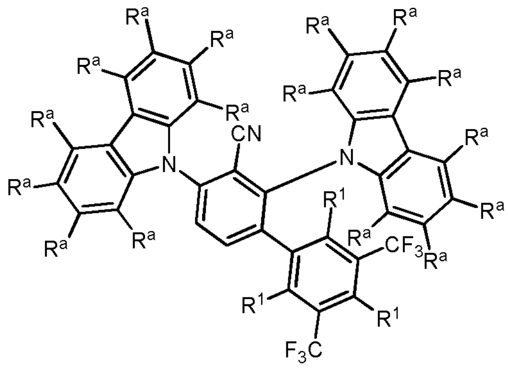

Formel IV

wobei die oben genannten Definitionen gelten.

In einer weiteren Ausführungsform weisen die erfindungsgemäßen organischen Moleküle eine Struktur der Formel IVa auf:

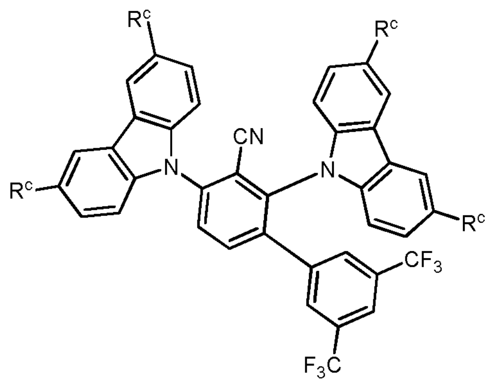

Formel IVa

wobei die oben genannten Definitionen gelten.

In einer weiteren Ausführungsform weisen die erfindungsgemäßen organischen Moleküle eine Struktur der Formel IVb auf:

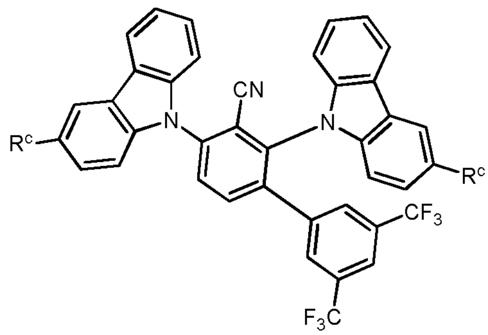

Formel IVb

wobei die oben genannten Definitionen gelten.

In einer weiteren Ausführungsform weisen die erfindungsgemäßen organischen Moleküle eine Struktur der Formel IVc auf:

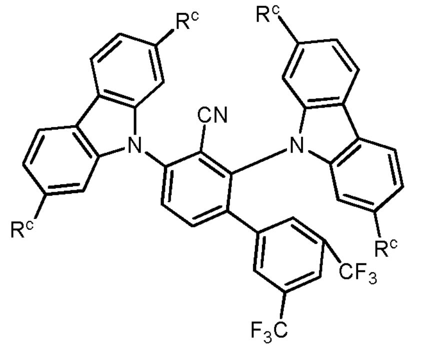

Formel IVc

wobei die oben genannten Definitionen gelten.

In einer weiteren Ausführungsform weisen die erfindungsgemäßen organischen Moleküle eine Struktur der Formel IVd auf:

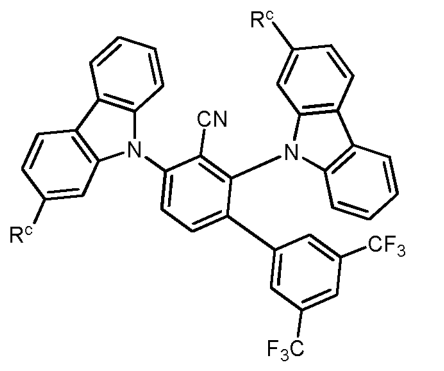

Formel IVd

wobei die oben genannten Definitionen gelten.

In einer weiteren Ausführungsform weisen die erfindungsgemäßen organischen Moleküle eine Struktur der Formel IVe auf:

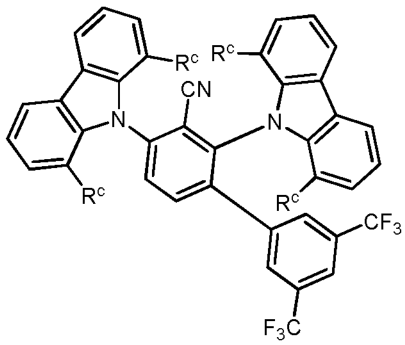

Formel IVe

wobei die oben genannten Definitionen gelten.

In einer weiteren Ausführungsform weisen die erfindungsgemäßen organischen Moleküle eine Struktur der Formel IVf auf:

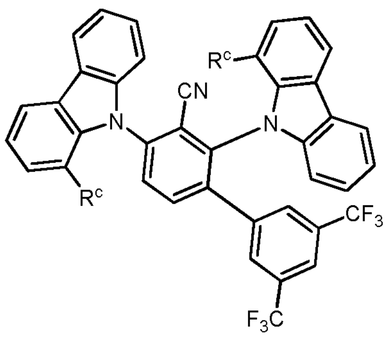

Formel IVf

wobei die oben genannten Definitionen gelten.

In einer weiteren Ausführungsform weisen die erfindungsgemäßen organischen Moleküle eine Struktur der Formel IVg auf:

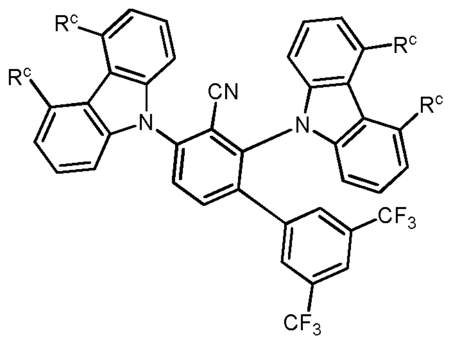

Formel IVg

wobei die oben genannten Definitionen gelten.

In einer weiteren Ausführungsform weisen die erfindungsgemäßen organischen Moleküle eine Struktur der Formel IVh auf:

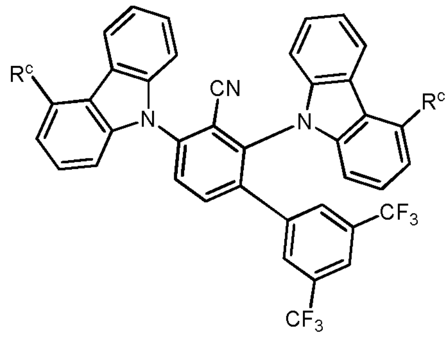

Formel IVh

wobei die oben genannten Definitionen gelten.

In einer Ausführungsform weisen die erfindungsgemäßen organischen Moleküle eine Struktur der Formel V auf:

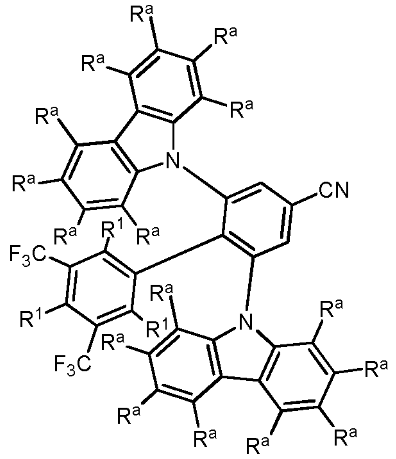

Formel V

wobei die oben genannten Definitionen gelten.

In einer weiteren Ausführungsform weisen die erfindungsgemäßen organischen Moleküle eine Struktur der Formel Va auf:

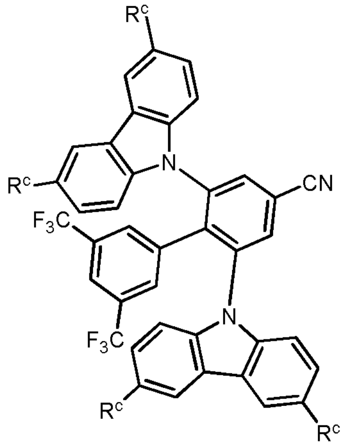

Formel Va

wobei die oben genannten Definitionen gelten.

In einer weiteren Ausführungsform weisen die erfindungsgemäßen organischen Moleküle eine Struktur der

Formel Vb auf:

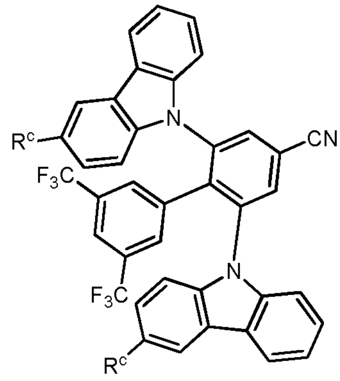

Formel Vb

wobei die oben genannten Definitionen gelten.

In einer weiteren Ausführungsform weisen die erfindungsgemäßen organischen Moleküle eine Struktur der Formel Vc auf:

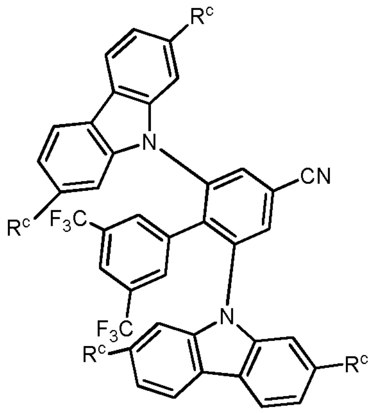

Formel Vc

wobei die oben genannten Definitionen gelten.

In einer weiteren Ausführungsform weisen die erfindungsgemäßen organischen Moleküle eine Struktur der Formel Vd auf:

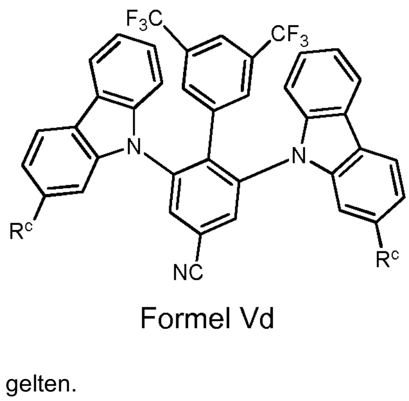

Formel Vd

wobei die oben genannten Definitionen gelten.

In einer weiteren Ausführungsform weisen die erfindungsgemäßen organischen Moleküle eine Struktur der Formel Ve auf:

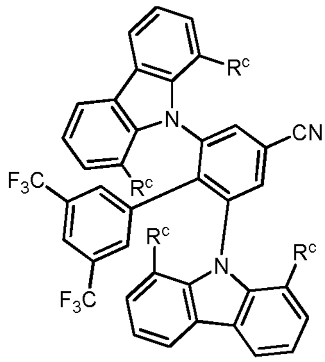

Formel Ve

wobei die oben genannten Definitionen gelten.

In einer weiteren Ausführungsform weisen die erfindungsgemäßen organischen Moleküle eine Struktur der Formel Vf auf:

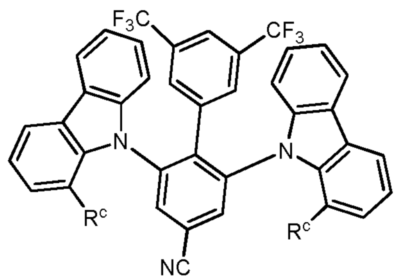

Formel Vf

wobei die oben genannten Definitionen gelten.

In einer weiteren Ausführungsform weisen die erfindungsgemäßen organischen Moleküle eine Struktur der Formel Vg auf:

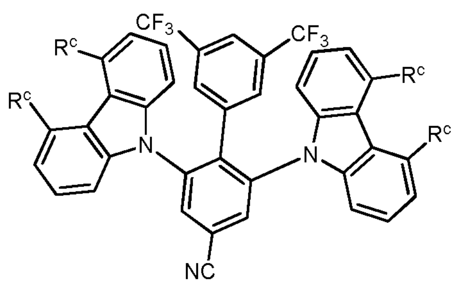

Formel Vg

wobei die oben genannten Definitionen gelten.

In einer weiteren Ausführungsform weisen die erfindungsgemäßen organischen Moleküle eine Struktur der Formel Vh auf:

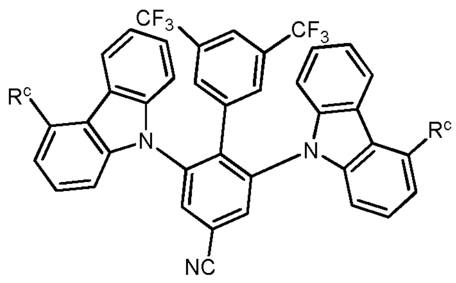

Formel Vh

wobei die oben genannten Definitionen gelten.

In einer Ausführungsform weisen die erfindungsgemäßen organischen Moleküle eine Struktur der Formel VI auf:

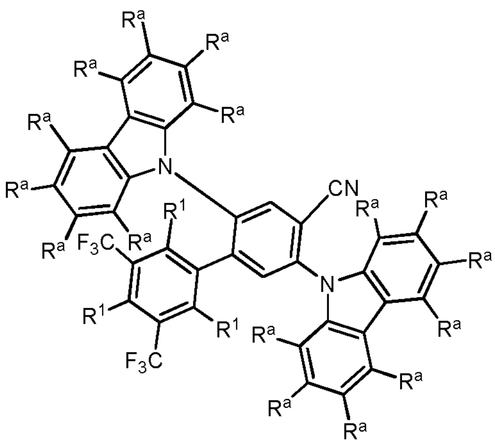

Formel VI

wobei die oben genannten Definitionen gelten.

In einer weiteren Ausführungsform weisen die erfindungsgemäßen organischen Moleküle eine Struktur der Formel VIa auf:

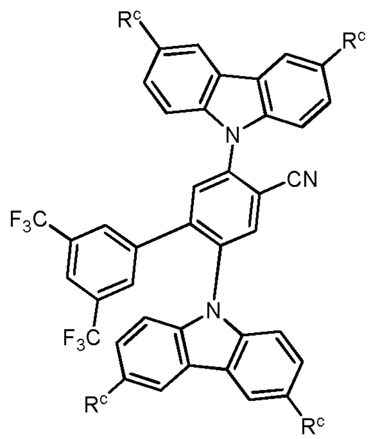

Formel VIa

wobei die oben genannten Definitionen gelten.

In einer weiteren Ausführungsform weisen die erfindungsgemäßen organischen Moleküle eine Struktur der Formel VIb auf:

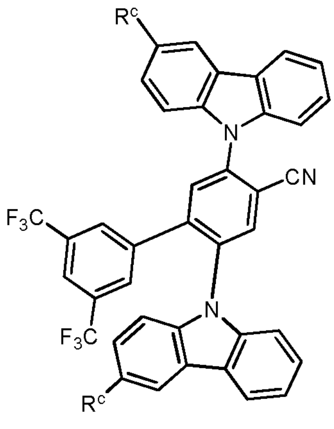

Formel VIb

wobei die oben genannten Definitionen gelten.

In einer weiteren Ausführungsform weisen die erfindungsgemäßen organischen Moleküle eine Struktur der Formel VIc auf:

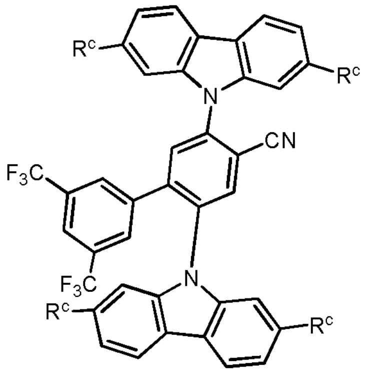

Formel VIc

wobei die oben genannten Definitionen gelten.

In einer weiteren Ausführungsform weisen die erfindungsgemäßen organischen Moleküle eine Struktur der Formel VId auf:

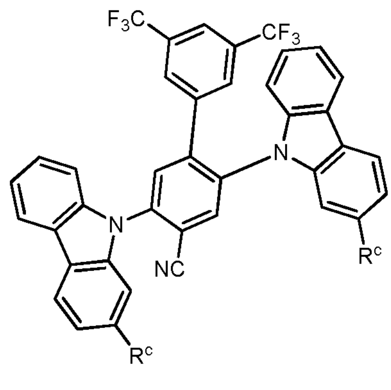

Formel VId

wobei die oben genannten Definitionen gelten.

In einer weiteren Ausführungsform weisen die erfindungsgemäßen organischen Moleküle eine Struktur der Formel VIe auf:

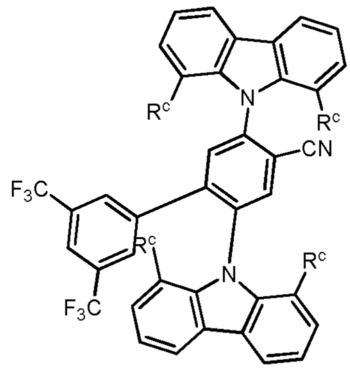

Formel VIe

wobei die oben genannten Definitionen gelten.

In einer weiteren Ausführungsform weisen die erfindungsgemäßen organischen Moleküle eine Struktur der Formel VIf auf:

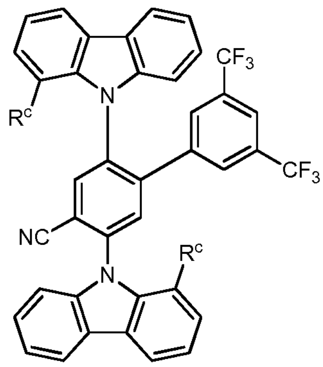

Formel VIf

wobei die oben genannten Definitionen gelten.

In einer weiteren Ausführungsform weisen die erfindungsgemäßen organischen Moleküle eine Struktur der Formel VIg auf:

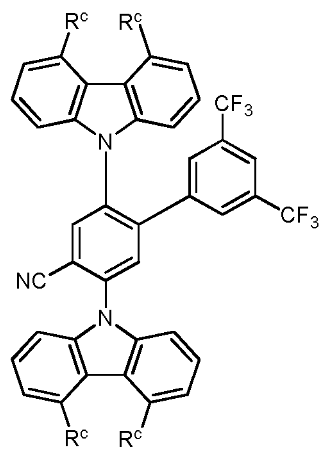

Formel VIg

wobei die oben genannten Definitionen gelten.

In einer weiteren Ausführungsform weisen die erfindungsgemäßen organischen Moleküle eine Struktur der Formel VIh auf:

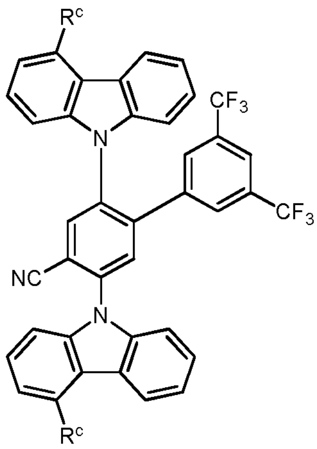

Formel VIh

wobei die oben genannten Definitionen gelten.

In einer Ausführungsform weisen die erfindungsgemäßen organischen Moleküle eine Struktur der Formel VII auf:

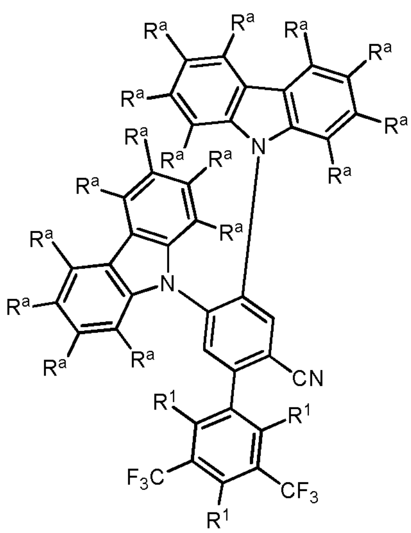

Formel VII

wobei die oben genannten Definitionen gelten.

In einer weiteren Ausführungsform weisen die erfindungsgemäßen organischen Moleküle eine Struktur der Formel VIIa auf:

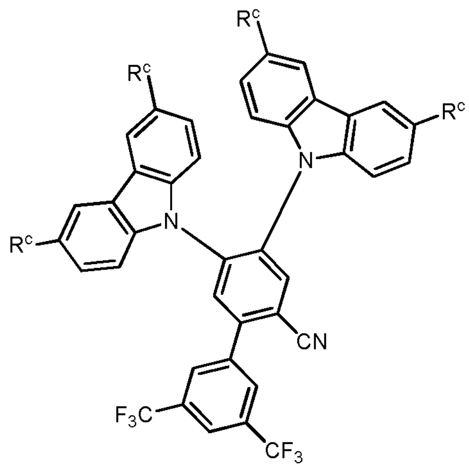

Formel VIIa

wobei die oben genannten Definitionen gelten.

In einer weiteren Ausführungsform weisen die erfindungsgemäßen organischen Moleküle eine Struktur der Formel VIIb auf:

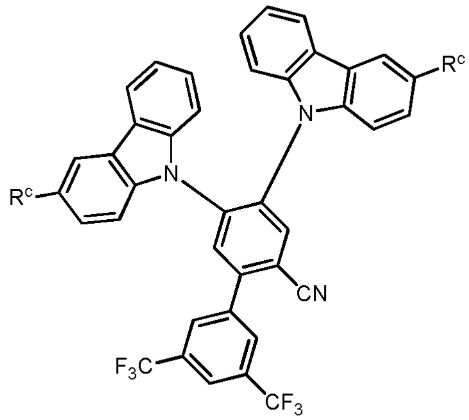

Formel VIIb

wobei die oben genannten Definitionen gelten.

In einer weiteren Ausführungsform weisen die erfindungsgemäßen organischen Moleküle eine Struktur der Formel VIIc auf:

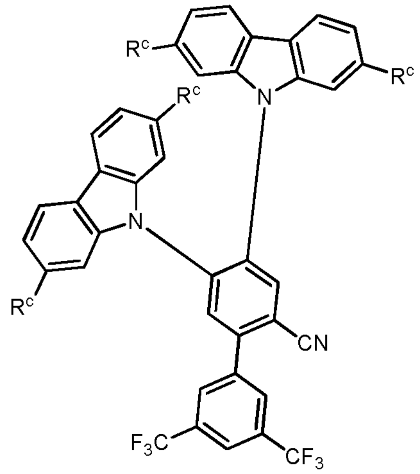

Formel VIIc

wobei die oben genannten Definitionen gelten.

In einer weiteren Ausführungsform weisen die erfindungsgemäßen organischen Moleküle eine Struktur der Formel VIId auf:

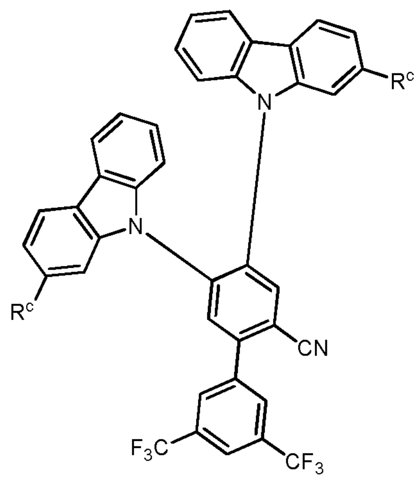

Formel VIId

wobei die oben genannten Definitionen gelten.

In einer weiteren Ausführungsform weisen die erfindungsgemäßen organischen Moleküle eine Struktur der Formel VIIe auf:

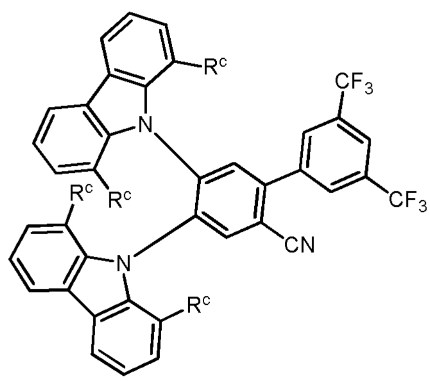

Formel VIIe

wobei die oben genannten Definitionen gelten.

In einer weiteren Ausführungsform weisen die erfindungsgemäßen organischen Moleküle eine Struktur der Formel VIIf auf:

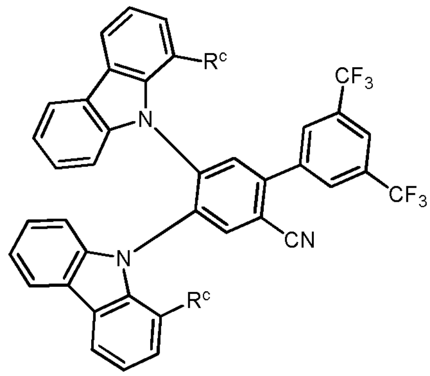

Formel VIIf

wobei die oben genannten Definitionen gelten.

In einer weiteren Ausführungsform weisen die erfindungsgemäßen organischen Moleküle eine Struktur der Formel VIIg auf:

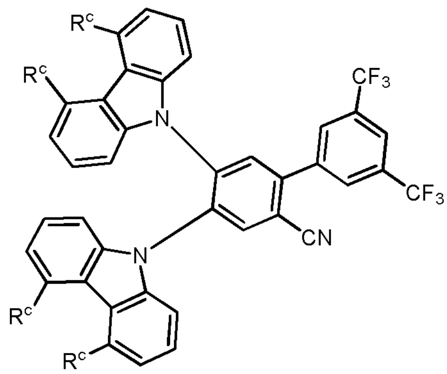

Formel VIIg

wobei die oben genannten Definitionen gelten.

In einer weiteren Ausführungsform weisen die erfindungsgemäßen organischen Moleküle eine Struktur der Formel VIIh auf:

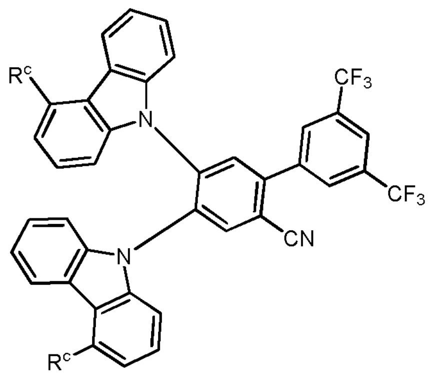

Formel VIIh

wobei die oben genannten Definitionen gelten.

In einer Ausführungsform weisen die erfindungsgemäßen organischen Moleküle eine Struktur der Formel VIII auf:

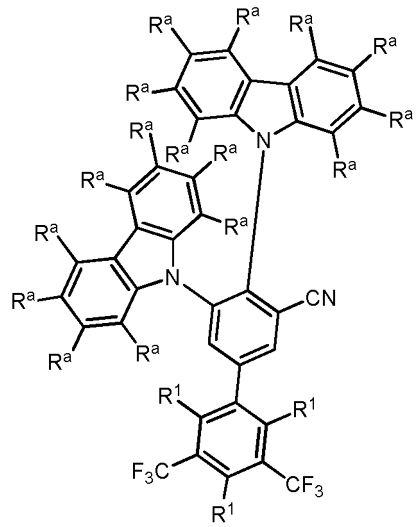

Formel VIII

wobei die oben genannten Definitionen gelten.

In einer Ausführungsform ist $R^c$ ist bei jedem Auftreten unabhängig voneinander ausgewählt aus der Gruppe bestehend aus Me, $^iPr$, $^tBu$, Ph, das jeweils durch einen oder mehrere Reste ausgewählt aus Me, $^iPr$, $^tBu$, CN, $CF_3$ oder Ph substituiert sein kann, und Carbazolyl, das jeweils durch einen oder mehrere Reste ausgewählt aus Me, $^iPr$, $^tBu$, oder Ph substituiert sein kann.

Im Sinne dieser Erfindung enthält eine Arylgruppe 6 bis 60 aromatische Ringatome; eine Heteroarylgruppe enthält 5 bis 60 aromatische Ringatome, von denen mindestens eines ein Heteroatom darstellt. Die Heteroatome sind insbesondere N, O und/oder S. Werden in der Beschreibung bestimmter Ausführungsformen der Erfindung andere, von der genannten Definition abweichende Definitionen angegeben, beispielsweise bezüglich der Zahl der aromatischen Ringatome oder der enthaltenen Heteroatome, so gelten diese.

Unter einer Arylgruppe bzw. Heteroarylgruppe wird ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol oder Kombinationen der genannten Gruppen.

Unter einer cyclischen Alkyl-, Alkoxy- oder Thioalkoxygruppe wird hier eine monocyclische, eine bicyclische oder eine polycyclische Gruppe verstanden.

Im Rahmen der vorliegenden Erfindung werden unter einer $C_1$- bis $C_{40}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neoPentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl,

Trifluor-methyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-0ctyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Eine Ausführungsform der Erfindung betrifft organische Moleküle, welche einen $\Delta E(S_1-T_1)$-Wert zwischen dem untersten angeregten Singulett ($S_1$)- und dem darunter liegenden Triplett ($T_1$)-Zustand von nicht höher als 5000 $cm^{-1}$, insbesondere nicht höher als 3000 $cm^{-1}$, oder nicht höher als 1500 $cm^{-1}$ oder 1000 $cm^{-1}$ aufweisen und/oder eine Emissionslebensdauer von höchstens 150 $\mu$s, insbesondere von höchstens 100 $\mu$s, von höchsten 50 $\mu$s, oder von höchstens 10 $\mu$s aufweisen und/oder eine Hauptemissionsbande mit einer Halbwertsbreite kleiner als 0,55 eV, insbesondere kleiner als 0,50 eV, kleiner als 0,48 eV, oder kleiner als 0,45 eV aufweisen.

Die organischen Moleküle zeigen insbesondere ein Emissionsmaximum zwischen 420 und 500 nm, zwischen 430 und 480 nm, insbesondere zwischen 450 und 470 nm.

Die Moleküle weisen insbesondere einen "blue material index" (BMI), den Quotienten aus der PLQY (in %) und ihrer $CIE_y$-Farbkoordinate des von dem erfindungsgemäßen Molekül emittierten Lichts, von größer 150, insbesondere von größer 200, von größer 250 oder von größer 300 auf.

In einem weiteren Aspekt betrifft die Erfindung ein Verfahren zur Herstellung eines erfindungsgemäßen organischen Moleküls der hier beschriebenen Art (mit einer eventuellen Folgeumsetzung), wobei eine in 2,4,6-Position $R^1$-substituierte 3,5-Bis(trifluormethyl)-phenylboronsäure oder ein entsprechender in 2,4,6-Position $R^1$-substituierter 3,5-Bis(trifluormethyl)-phenylboronsäureester als Edukt eingesetzt wird

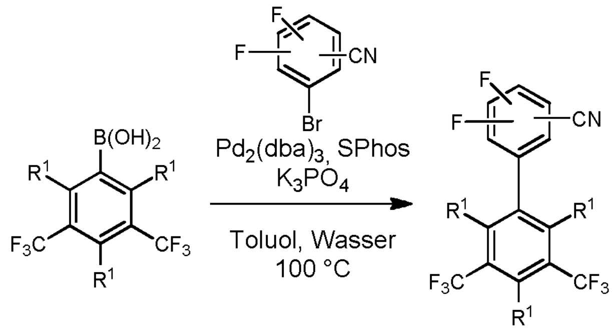


E1

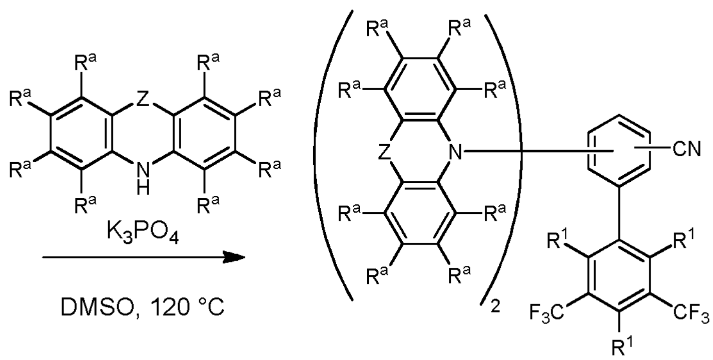


Im obigen Schema ist in einer Ausführungsform die chemische Gruppe CN durch $CF_3$ ersetzt.

In einer Ausführungsform wird eine in 2,4,6-Position $R^1$-substituierte 3,5-Bis(trifluormethyl)-phenylboronsäure oder ein entsprechender in 2,4,6-Position $R^1$-substituierter 3,5-Bis(trifluormethyl)-phenylboronsäureester als Edukt mit einem Brom-difluorbenzonitril in einer Palladium-katalysierten Kreuzkupplungsreaktion umgesetzt. Hierbei können erfindungsgemäß beispielhaft 4-Brom-2,6-difluorbenzonitril, 4-Brom-2,5-difluorbenzonitril, 4-Brom-3,5-difluorbenzonitril, 3-Brom-2,6-difluorbenzonitril, 3-Brom-5,6-difluorbenzonitril und 2-Brom-4,5-difluorbenzonitril eingesetzt werden. Das

Produkt wird durch Deprotonierung des entsprechenden Amins und anschließender nukleophiler Substitution der zwei Fluorgruppen erhalten. Hierbei wird ein Stickstoffheterozyklus im Sinne einer nukleophilen aromatischen Substitution mit einem Edukt E1 umgesetzt. Typische Bedingungen beinhalten die Verwendung einer Base wie beispielweise tribasisches Kaliumphosphat oder Natriumhydrid in einem aprotischen polaren Lösungsmittel wie beispielweise Dimetylsulfoxid (DMSO) oder N,N-Dimethylformamid (DMF).

In einem weiteren Aspekt betrifft die Erfindung die Verwendung der organischen Moleküle als lumineszierender Emitter oder als Hostmaterial in einer organischen optoelektronischen Vorrichtung, insbesondere wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

In einem weiteren Aspekt betrifft die Erfindung eine Zusammensetzung aufweisend oder bestehend aus:

(a) mindestens einem erfindungsgemäßen organischen Molekül, insbesondere als Emitter und/oder Host, und
(b) mindestens ein, d. h. ein oder mehrere Emitter- und/oder Hostmaterialien, die von dem erfindungsgemäßen organischen Molekül verschiedenen ist bzw. sind und
(c) optional eine oder mehreren Farbstoffen und/ oder einem oder mehreren organischen Lösungsmitteln.

In einer Ausführungsform besteht die erfindungsgemäße Zusammensetzung aus einem erfindungsgemäßen organischen Molekül und einem oder mehreren Hostmaterialien. Das oder die Hostmaterialen weisen insbesondere Triplett ($T_1$)- und Singulett ($S_1$)- Energieniveaus auf, die energetisch höher liegen als die Triplett ($T_1$)- und Singulett ($S_1$)-Energieniveaus des erfindungsgemäßen organischen Moleküls. In einer Ausführungsform weist die Zusammensetzung neben dem erfindungsgemäßen organischen Molekül ein elektronendominantes und ein lochdominantes Hostmaterial auf. Das höchste besetzte Orbital (HOMO) und das niedrigste unbesetzte Orbital (LUMO) des lochdominanten Hostmaterials liegen energetisch insbesondere höher als das des elektronendominanten Hostmaterials. Das HOMO des lochdominanten Hostmaterials liegt energetisch unter dem HOMO des erfindungsgemäßen organischen Moleküls, während das LUMO des elektronendominanten Hostmaterials energetisch über dem LUMO des erfindungsgemäßen organischen Moleküls liegt. Um Exciplex-Formation zwischen Emitter und Hostmaterial oder Hostmaterialien zu vermeiden, sollten die Materialien so gewählt sein, dass die Energieabstände zwischen den jeweiligen Orbitalen gering sind. Der Abstand zwischen dem LUMO des elektronendominanten Hostmaterials und dem LUMO des erfindungsgemäßen organischen Moleküls beträgt insbesondere weniger als 0,5 eV, bevorzugt weniger als 0,3 eV, noch bevorzugter weniger als 0,2 eV. Der Abstand zwischen dem HOMO des lochdominanten Hostmaterials und dem HOMO des erfindungsgemäßen organischen Moleküls beträgt insbesondere weniger als 0,5 eV, bevorzugt weniger als 0,3 eV, noch bevorzugter weniger als 0,2 eV.

In einem weiteren Aspekt betrifft die Erfindung eine organische optoelektronische Vorrichtung, die ein erfindungsgemäßes organisches Molekül oder eine erfindungsgemäße Zusammensetzung aufweist. Die organische optoelektronische Vorrichtung ist insbesondere ausgeformt als eine Vorrichtung ausgewählt aus der Gruppe bestehend aus organischer lichtemittierender Diode (OLED); lichtemittierender elektrochemischer Zelle; OLED-Sensor, insbesondere nicht hermetisch nach außen abgeschirmten Gas- und Dampf-Sensoren; organischer Diode; organischer Solarzelle; organischem Transistor; organischem Feldeffekttransistor; organischem Laser und Down-Konversion-Element.

Eine organische optoelektronische Vorrichtung aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein erfindungsgemäßes organisches Molekül aufweist, stellt einen weitere Ausführungsform der Erfindung dar.

In einer Ausführungsform handelt es sich bei der optoelektronischen Vorrichtung um eine OLED. Eine typische OLED weist beispielsweise folgenden Schichtaufbau auf:

1. Substrat (Trägermaterial)
2. Anode
3. Lochinjektionsschicht (hole injection layer, HIL)
4. Lochtransportschicht (hole transport layer, HTL)
5. Elektronenblockierschicht (electron blocking layer, EBL)
6. Emitterschicht (emitting layer, EML)
7. Lochblockierschicht (hole blocking layer, HBL)
8. Elektronenleitschicht (electron transport layer, ETL)
9. Elektroneninjektionsschicht (electron injection layer, EIL)
10. Kathode.

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

Gemäß einer Ausführungsform ist mindestens eine Elektrode des organischen Bauelements transluzent ausgebildet. Hier wird mit "transluzent" eine Schicht bezeichnet, die durchlässig für sichtbares Licht ist. Dabei kann die transluzente Schicht klar durchscheinend, also transparent, oder zumindest teilweise Licht absorbierend und/oder teilweise Licht streuend sein, so dass die transluzente Schicht beispielsweise auch diffus oder milchig durchscheinend sein kann. Insbesondere ist eine hier als transluzent bezeichnete Schicht möglichst transparent ausgebildet, so dass insbesondere die Absorption von Licht so gering wie möglich ist.

Gemäß einer weiteren Ausführungsform weist das organische Bauelement, insbesondere eine OLED, einen invertierten Aufbau auf. Der invertierte Aufbau zeichnet sich dadurch aus, dass sich die Kathode auf dem Substrat befindet und die anderen Schichten entsprechend invertiert aufgebracht werden:

1. Substrat (Trägermaterial)
2. Kathode
3. Elektroneninjektionsschicht (electron injection layer, EIL)
4. Elektronenleitschicht (electron transport layer, ETL)
5. Lochblockierschicht (hole blocking layer, HBL)
6. Emissionsschicht bzw. Emitterschicht (emitting layer, EML)
7. Elektronenblockierschicht (electron blocking layer, EBL)
8. Lochtransportschicht (hole transport layer, HTL)
9. Lochinjektionsschicht (hole injection layer, HIL)
10. Anode

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

In einer Ausführungsform wird bei der invertierten OLED die Anodenschicht des typischen Aufbaus, z.B. eine ITO-Schicht (Indium-Zinn-Oxid), als Kathode geschaltet.

Gemäß einer weiteren Ausführungsform weist das organische Bauelement, insbesondere eine OLED, einen gestapelten Aufbau auf. Hierbei werden die einzelnen OLEDs übereinander und nicht wie üblich nebeneinander angeordnet. Durch einen gestapelten Aufbau kann die Erzeugung von Mischlicht ermöglicht werden. Beispielsweise kann dieser Aufbau bei der Erzeugung von weißem Licht eingesetzt werden, für dessen Erzeugung das gesamte sichtbare Spektrum typischerweise durch die Kombination des emittierten Lichts von blauen, grünen und roten Emittern abgebildet wird. Weiterhin können bei praktisch gleicher Effizienz und identischer Leuchtdichte signifikant längere Lebensdauern im Vergleich zu üblichen OLEDs erzielt werden. Für den gestapelten Aufbau wird optional eine sogenannte Ladungserzeugungsschicht (charge generation layer, CGL) zwischen zwei OLEDs eingesetzt. Diese besteht aus einer n-dotierten und einem p-dotierten Schicht, wobei die n-dotierte Schicht typischerweise näher an der Anode aufgebracht wird.

In einer Ausführungsform - einer sogenannten Tandem-OLED - treten zwei oder mehr Emissionsschichten zwischen Anode und Kathode auf. In einer Ausführungsform sind drei Emissionsschichten übereinander angeordnet, wobei eine Emissionsschicht rotes Licht emittiert, eine Emissionsschicht grünes Licht emittiert und eine Emissionsschicht blaues Licht emittiert und optional weitere Ladungserzeugungs-, Blockier- oder Transportschichten zwischen den einzelnen Emissionsschichten aufgebracht sind. In einer weiteren Ausführungsform werden die jeweiligen Emissionsschichten direkt angrenzend aufgebracht. In einer weiteren Ausführungsform befindet sich jeweils eine Ladungserzeugungsschicht zwischen den Emissionsschichten. Weiterhin können in einer OLED direkt angrenzende und durch Ladungserzeugungsschichten getrennte Emissionsschichten kombiniert werden.

Über den Elektroden und den organischen Schichten kann weiterhin noch eine Verkapselung angeordnet sein. Die Verkapselung kann beispielsweise in Form eines Glasdeckels oder in Form einer Dünnschichtverkapselung ausgeführt sein.

Als Trägermaterial der optoelektronischen Vorrichtung kann beispielsweise Glas, Quarz, Kunststoff, Metall, ein Siliziumwafer oder jedes andere geeignete feste oder flexible, optional durchsichtige Material dienen. Das Trägermaterial kann beispielsweise ein oder mehrere Materialien in Form einer Schicht, einer Folie, einer Platte oder einem Laminat aufweisen.

Als Anode der optoelektronischen Vorrichtung können beispielsweise transparente leitende Metalloxide wie beispielsweise ITO (Indium-Zinn-Oxid), Zinkoxid, Zinnoxid, Cadmiumoxid, Titanoxid, Indiumoxid oder Aluminiumzinkoxid (AZO), $Zn_2SnO_4$, $CdSnO_3$, $ZnSnO_3$, $MgIn_2O_4$, $GaInO_3$, $Zn_2In_2O_5$ oder $In_4Sn_3O_{12}$ oder Mischungen unterschiedlicher transparenter leitender Oxide dienen.

Als Materialien einer HIL können beispielsweise PEDOT:PSS (Poly-3,4-ethylendioxythiophen:Polystyrolsulfonsäure), PEDOT (Poly-3,4-ethylendioxythiophen), m-MTDATA (4,4',4"-Tris[phenyl(m-tolyl)amino]triphenylamin), Spiro-TAD (2,2',7,7'-Tetrakis(N,N-diphenylamino)-9,9-spirobifluoren), DNTPD (4,4'-Bis[N-[4-{N,N-bis(3-methylphenyl)amino}phenyl]-N-phenylamino]biphenyl), NPB (N,N'-Bis-(1-naphthalenyl)-N,N'-bis-phenyl-(1,1'-biphenyl)-4,4'-diamin), NPNPB (N,N'-Diphenyl-N,N'-di-[4-(N,N-diphenyl-amino)phenyl]benzol), MeO-TPD (N,N,N',N'-Tetrakis(4-methoxyphenyl)benzol), HAT-CN (1,4,5,8,9,11-Hexaazatriphenylen-hexacarbonitril) oder Spiro-NPD (N,N'-diphenyl-N,N'-Bis-(1-naphthyl)-9,9'-spirobifluorene-2,7-diamin) dienen. Beispielhaft ist die Schichtdicke 10-80 nm. Desweiteren können kleine Moleküle können verwendet werden (z. B. Kupfer-Phthalocyanin (CuPc z. B. 10 nm dick)) oder Metalloxide wie beispielhaft $MoO_3$, $V_2O_5$.

Als Materialien einer HTL können tertiäre Amine, Carbazolderivate, mit Polystyrolsulfonsäure dotiertes Polyethylendioxythiophen, mit Camphersulfonsäure dotiertes Polyanilin poly-TPD (Poly(4-butylphenyl-diphenyl-amin)), [alpha]-NPD (Poly(4-butylphenyl-diphenyl-amin)), TAPC (4,4'-Cyclohexyliden-bis[*N,N*-bis(4-methylphenyl)benzenamin]), TCTA (Tris(4-carbazoyl-9-ylphenyl)amin), 2-TNATA (4,4',4"-Tris[2-naphthyl(phenyl)amino]triphenylamin), Spiro-TAD, DNTPD, NPB, NPNPB, MeO-TPD, HAT-CN oder TrisPcz (9,9'-Diphenyl-6-(9-phenyl-9H-carbazol-3-yl)-9H,9'H-3,3'-bicarbazol) dienen. Beispielhaft ist die Schichtdicke 10 nm bis 100 nm.

Die HTL kann eine p-dotierte Schicht aufweisen, die einen anorganischen oder organischen Dotierstoff in einer organischen löcherleitenden Matrix aufweist. Als anorganischer Dotierstoff können beispielsweise Übergangsmetalloxide wie etwa Vanadiumoxid, Molybdänoxid oder Wolframoxid genutzt werden. Als organische Dotierstoffe können beispielsweise Tetrafluorotetracyanoquinodimethan (F4-TCNQ), Kupfer-Pentafluorobenzoat (Cu(I)pFBz) oder Übergangsmetallkomplexe verwendet werden. Beispielhaft ist die Schichtdicke 10 nm bis 100 nm.

Als Materialien einer Elektronenblockierschicht können beispielsweise mCP (1,3-Bis(carbazol-9-yl)benzol), TCTA, 2-TNATA, mCBP (3,3-Di(9H-carbazol-9-yl)biphenyl), tris-Pcz (9,9'-Diphenyl-6-(9-phenyl-9H-carbazol-3-yl)-9H,9'H-3,3'-bicarbazol), CzSi (9-(4-tert-Butylphenyl)-3,6-bis(triphenylsilyl)-9H-carbazol) oder DCB (N,N'-Dicarbazolyl-1,4-dimethylbenzol) dienen. Beispielhaft ist die Schichtdicke 10nm bis 50 nm.

Die Emitter-Schicht EML oder Emissionsschicht besteht aus oder enthält Emittermaterial oder eine Mischung aufweisend mindestens zwei Emittermaterialien und optional ein oder mehreren Hostmaterialien. Geeignete Hostmaterialien sind beispielsweise mCP, TCTA, 2-TNATA, mCBP, CBP (4,4'-Bis-(N-carbazolyl)-biphenyl), Sif87 (Dibenzo[b,d]thiophen-2-yltriphenylsilan), Sif88 (Dibenzo[b,d]thiophen-2-yl)diphenylsilan), 9-[3-(Dibenzofuran-2-yl)phenyl]-9H-carbazol, 9-[3-(Dibenzofuran-2-yl)phenyl]-9H-carbazol, 9-[3-(Dibenzothiophen-2-yl)phenyl]-9H-carbazol, 9-[3,5-Bis(2-dibenzofuranyl)phenyl]-9H-carbazol, 9-[3,5-Bis(2-dibenzothiophenyl)phenyl]-9H-carbazol, T2T (2,4,6-Tris(biphenyl-3-yl)-1,3,5-triazin), T3T (2,4,6-Tris(triphenyl-3-yl)-1,3,5-triazin) TST (2,4,6-Tris(9,9'-spirobifluorene-2-yl)-1,3,5-triazin) und/oderDPEPO (Bis[2-((oxo)diphenylphosphino)phenyl]ether). In einer Ausführungsform enthält die EML 50-80 Gewichts-%, bevorzugt 60-75 Gewichts-% eines Hostmaterials ausgewählt aus der Gruppe bestehend aus CBP, mCP, mCBP, 9-[3-(Dibenzofuran-2-yl)phenyl]-9H-carbazol, 9-[3-(Dibenzofuran-2-yl)phenyl]-9H-carbazol, 9-[3-(Dibenzothiophen-2-yl)phenyl]-9H-carbazol, 9-[3,5-Bis(2-dibenzofuranyl)phenyl]-9H-carbazol und 9-[3,5-bis(2-Dibenzothiophenyl)phenyl]-9H-carbazol; 10-45 Gewichts-%, bevorzugt 15-30 Gewichts-% T2T und 5-40 Gewichts-%, bevorzugt 10-30 Gewichts-% einer erfindungsgemäßen organischen Moleküls als Emitter. Für im Grünen oder im Roten emittierendes Emittermaterial oder einer Mischung aufweisend mindestens zwei Emittermaterialien eignen sich die gängigen Matrixmaterialien wie CBP. Für im Blauen emittierendes Emittermaterial oder einer Mischung aufweisend mindestens zwei Emittermaterialien können UHG-Matrixmaterialien (Ultra-High energy Gap Materialien) (siehe z. B. M.E. Thompson et al., Chem. Mater. 2004, 16, 4743) oder andere sogenannten Wide-Gap-Matrixmaterialien eingesetzt werden. Beispielhaft ist die Schichtdicke 10 nm bis 250 nm.

Die Lochblockierschicht HBL kann beispielsweise BCP (2,9-Dimethyl-4,7-diphenyl-1,10-phenanthrolin = Bathocuproin), Bis-(2-methyl-8-hydroxychinolinato)-(4-phenylphenolato)-aluminium(III) (BAlq), NBphen (2,9-Bis(naphthalen-2-yl)-4,7-diphenyl-1,10-phenanthrolin), Alq3 (Aluminium-tris(8-hydroxychinolin)), T2T, TSPO1 (Diphenyl-4-triphenylsilylphenyl-phosphinoxid) oder TCB/TCP (1,3,5-Tris(N-carbazolyl)benzol/ 1,3,5-tris(carbazol)-9-yl) benzol) aufweisen. Beispielhaft ist die Schichtdicke 10 nm bis 50 nm.

Die Elektronentransportschicht ETL kann beispielsweise Materialien auf Basis von $AlQ_3$, TSPO1, BPyTP2 (2,7-Di(2,2'-bipyridin-5-yl)triphenyl), NBPhen, Sif87, Sif88, BmPyPhB (1,3-Bis[3,5-di(pyridin-3-yl)phenyl]benzol) oder BTB (4,4'-Bis-[2-(4,6-diphenyl-1,3,5-triazinyl)]-1,1'-biphenyl) aufweisen. Beispielhaft ist die Schichtdicke 10 nm bis 200 nm.

Als Materialien einer dünnen Elektroneninjektionsschicht EIL können beispielsweise CsF, LiF, 8-Hydroxyquinolinolatolithium (Liq), $Li_2O$, $BaF_2$, MgO oder NaF verwendet werden.

Als Materialien der Kathodenschicht können Metalle oder Legierungen dienen, beispielsweise Al, Al > AIF, Ag, Pt, Au, Mg, Ag:Mg. Typische Schichtdicken betragen 100 nm bis 200 nm. Insbesondere werden ein oder mehrere Metalle verwendet, die stabil an der Luft sind und/oder die selbstpassivierend, beispielsweise durch Ausbildung einer dünnen schützenden Oxidschicht, sind.

Als Materialien zu Verkapselung sind beispielsweise Aluminiumoxid, Vanadiumoxid, Zinkoxid, Zirkoniumoxid, Titanoxid, Hafniumoxid, Lanthanoxid, Tantaloxid geeignet.

In einer Ausführungsform der erfindungsgemäßen organischen optoelektronischen Vorrichtung ist das erfindungsgemäße organische Molekül als Emissionsmaterial in einer lichtemittierenden Schicht EML eingesetzt, wobei es entweder als Reinschicht oder in Kombination mit einem oder mehreren Hostmaterialien eingesetzt ist.

Eine Ausführungsform der Erfindung betrifft organische optoelektronische Vorrichtungen, welche eine externe Quanteneffizienz (EQE) bei 1000 cd/$m^2$ von größer 5 %, insbesondere von größer 8 %, insbesondere von größer 10 %, oder von größer 13 %, oder von größer 16 % und insbesondere von größer 20 % und/oder ein Emissionsmaximum bei einer Wellenlänge zwischen 420 nm und 500 nm, insbesondere zwischen 430 nm und 490 nm, oder zwischen 440 nm und 480 nm und insbesondere zwischen 450 nm und 470 nm und/oder einen LT80 Wert bei 500 cd/$m^2$ von größer 30 h, insbesondere von größer 70 h, oder von größer 100 h, oder von größer 150 h und insbesondere von größer 200 h aufweisen.

Der Massenanteil des erfindungsgemäßen organischen Moleküls an der Emitter-Schicht EML beträgt in einer weiteren Ausführungsform in einer lichtemittierenden Schicht in optischen Licht emittierenden Vorrichtungen, insbesondere in OLEDs, zwischen 1 % und 80 %. In einer Ausführungsform der erfindungsgemäßen organischen optoelektronischen Vorrichtung ist die lichtemittierende Schicht auf ein Substrat aufgebracht, wobei bevorzugt eine Anode und eine Kathode auf das Substrat aufgebracht sind und die lichtemittierende Schicht zwischen Anode und Kathode aufgebracht ist.

Die lichtemittierende Schicht kann in einer Ausführungsform ausschließlich ein erfindungsgemäßes organisches Molekül in 100 % Konzentration aufweisen, wobei die Anode und die Kathode auf das Substrat aufgebracht sind, und die lichtemittierende Schicht zwischen Anode und Kathode aufgebracht ist.

In einer Ausführungsform der erfindungsgemäßen organischen optoelektronischen Vorrichtung sind eine löcher- und elektroneninjizierende Schicht zwischen Anode und Kathode, und eine löcher- und elektronentransportierende Schicht zwischen löcher- und elektroneninjizierender Schicht, und die lichtemittierende Schicht zwischen löcher- und elektronentransportierender Schicht aufgebracht.

Die organische optoelektronische Vorrichtung weist in einer weiteren Ausführungsform der Erfindung auf: ein Substrat, eine Anode, eine Kathode und mindestens je eine löcher- und elektroneninjizierende Schicht, und mindestens je eine löcher- und elektronentransportierende Schicht, und mindestens eine lichtemittierende Schicht, die erfindungsgemäßes organisches Molekül und ein oder mehrere Hostmaterialen aufweist, deren Triplett ($T_1$)- und Singulett ($S_1$)-Energieniveaus energetisch höher liegen als die Triplett ($T_1$)- und Singulett ($S_1$)-Energieniveaus des organischen Moleküls, wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierender Schicht aufgebracht ist, und die lichtemittierende Schicht zwischen löcher- und elektronentransportierender Schicht aufgebracht ist.

In einem weiteren Aspekt betrifft die Erfindung ein Verfahren zur Herstellung eines optoelektronischen Bauelements. Dabei wird ein erfindungsgemäßes organisches Molekül verwendet.

In einer Ausführungsform umfasst das Herstellungsverfahren die Verarbeitung des erfindungsgemäßen organischen Moleküls mittels eines Vakuumverdampfungsverfahrens oder aus einer Lösung.

Erfindungsgemäß ist auch ein Verfahren zur Herstellung einer erfindungsgemäßen optoelektronischen Vorrichtung, bei dem mindestens eine Schicht der optoelektronischen Vorrichtung

- mit einem Sublimationsverfahren beschichtet wird,
- mit einem OVPD (Organic Vapor Phase Deposition) Verfahren beschichtet wird,
- mit einer Trägergassublimation beschichtet wird, und/oder
- aus Lösung oder mit einem Druckverfahren hergestellt wird.

Bei der Herstellung der erfindungsgemäßen optoelektronischen Vorrichtung werden bekannte Verfahren eingesetzt. Generell werden die Schichten einzeln auf ein geeignetes Substrat in aufeinanderfolgenden Abscheideverfahrensschritten aufgebracht. Bei der Gasphasenabscheidung können die gebräuchlichen Methoden, wie thermische Verdampfung, chemische Gasphasenabscheidung (CVD), physikalische Gasphasenabscheidung (PVD) angewendet werden. Für active matrix OLED (AMOLED) Displays erfolgt die Abscheidung auf eine AMOLED Backplane als Substrat.

Alternativ können Schichten aus Lösungen oder Dispersionen in geeigneten Lösungsmitteln aufgebracht werden. Beispielhafte geeignete Beschichtungsverfahren sind Rotationsbeschichtung (spin-coating), Tauchbeschichtung (dip-coating) und Strahldruckmethoden. Die einzelnen Schichten können erfindungsgemäß sowohl über dasselbe als auch über jeweils verschiedene Beschichtungsmethoden hergestellt werden.

**Beispiele**

Allgemeines Syntheseschema I

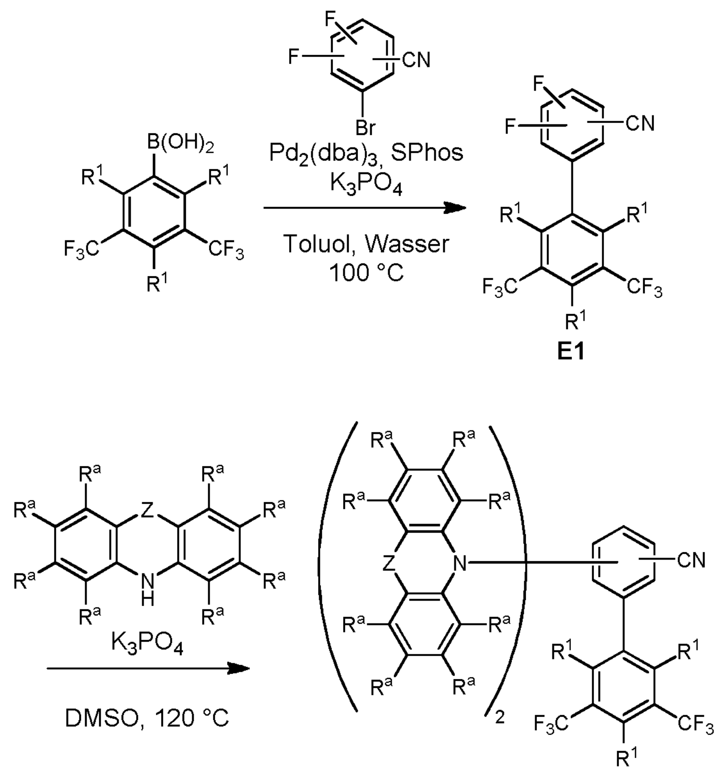


*Allgemeine Synthesevorschrift **AAV1:***

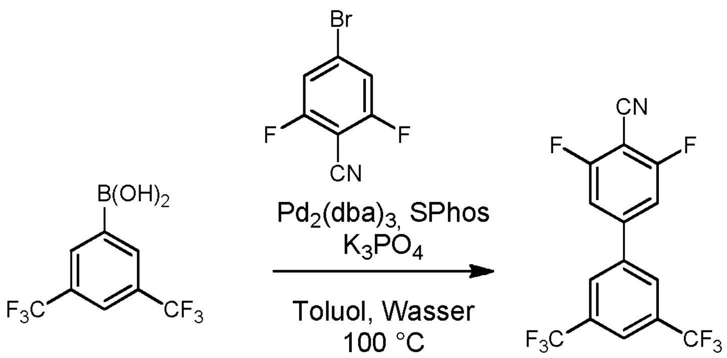


**Z1**

3,5-Bis(trifluormethyl)-phenylboronsäure, (1,50 Äquivalente), 4-Brom-2,6-difluorbenzonitril (1,00 Äquivalente), $Pd_2(dba)_3$ (0,02 Äquivalente), 2-Dicyclohexylphosphino-2',6'-dimethoxybiphenyl (SPhos) (0,08 Äquivalente) und tribasisches Kaliumphosphat (2,50 Äquivalente) werden unter Stickstoff in einem Toluol/Wasser-Gemisch (Verhältnis 20:1) bei 100 °C für 16 h gerührt. Anschließend wird die Reaktionsmischung in gesättigte Natriumchlorid-Lösung gegeben und zweimal mit Ethylacetat extrahiert. Die vereinigten organischen Phasen werden mit gesättigter Natriumchlorid-Lösung gewaschen, über $MgSO_4$ getrocknet und das Lösemittel entfernt. Das Produkt wird über wenig Kieselgel filtriert und danach umkristallisiert. Das Produkt wird als Feststoff erhalten.

Erfindungsgemäß kann statt einer Boronsäure auch ein entsprechender Boronsäureester verwendet werden.

*Allgemeine Synthesevorschrift **AAV2**:*

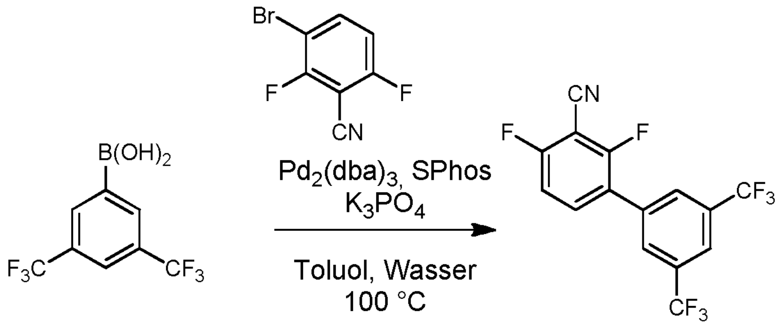


**Z2**

Die Synthese von **Z2** erfolgt analog ***AAV1***, wobei 3,5-Bis(trifluormethyl)-phenylboronsäure mit 3-Brom-2,6-difluorbenzonitril umgesetzt wird.

*Allgemeine Synthesevorschrift **AAV3**:*

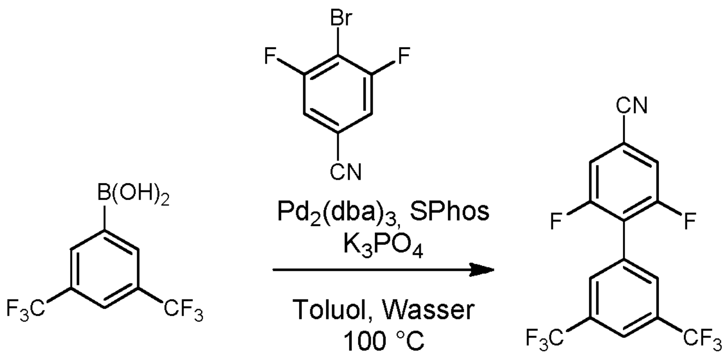


**Z3**

Die Synthese von **Z3** erfolgt analog ***AAV1***, wobei 3,5-Bis(trifluormethyl)-phenylboronsäure mit 4-Brom-3,5-difluorbenzonitril umgesetzt wird.

*Allgemeine Synthesevorschrift **AAV4**:*

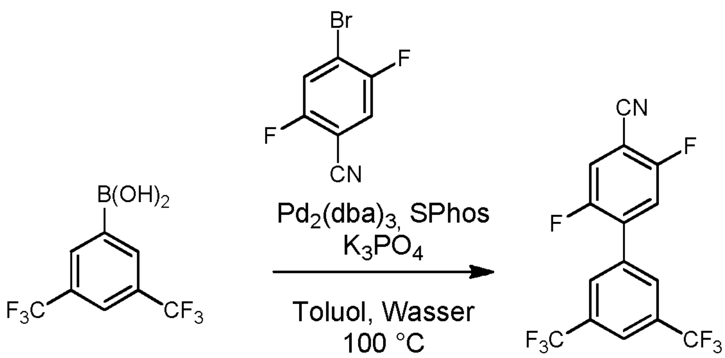


**Z4**

Die Synthese von **Z4** erfolgt analog ***AAV1,*** wobei 3,5-Bis(trifluormethyl)-phenylboronsäure mit 4-Brom-2,5-difluorbenzonitril umgesetzt wird.

*Allgemeine Synthesevorschrift **AAV5:***

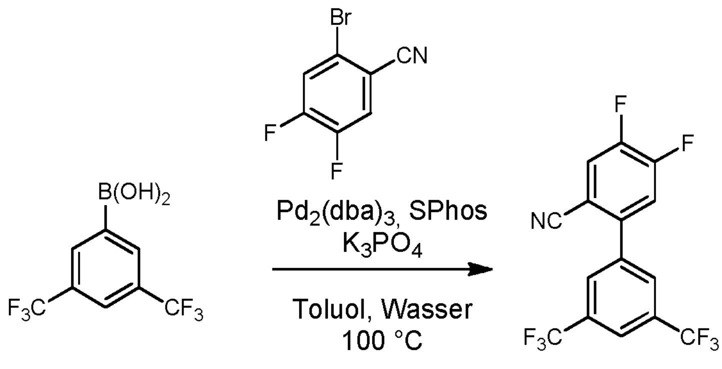


**Z5**

Die Synthese von **Z5** erfolgt analog ***AAV1***, wobei 3,5-Bis(trifluormethyl)-phenylboronsäure mit 2-Brom-4,5-difluorbenzonitril umgesetzt wird.

*Allgemeine Synthesevorschrift **AAV6:***

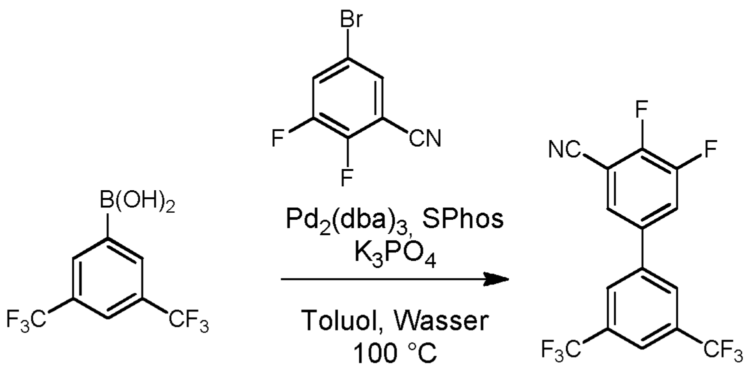


**Z6**

Die Synthese von **Z6** erfolgt analog ***AAV1,*** wobei 3,5-Bis(trifluormethyl)-phenylboronsäure mit 3-Brom-5,6-difluorbenzonitril umgesetzt wird.

*Allgemeine Synthesevorschrift **AAV7:***

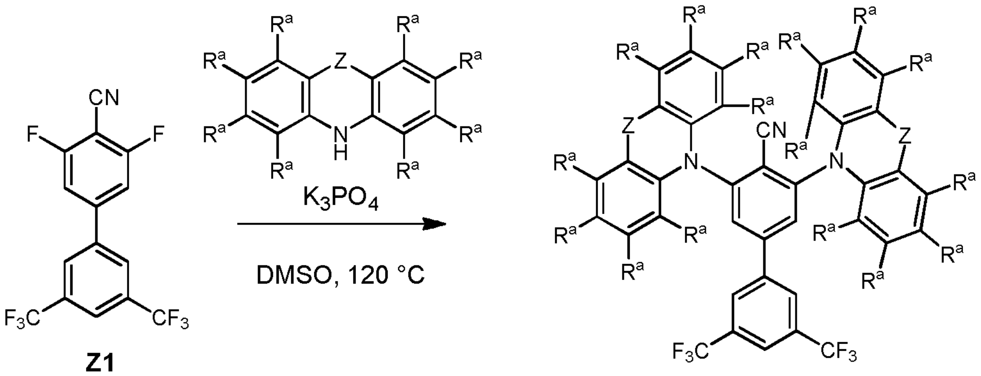
K3PO4
DMSO, 120 °C

**Z1**

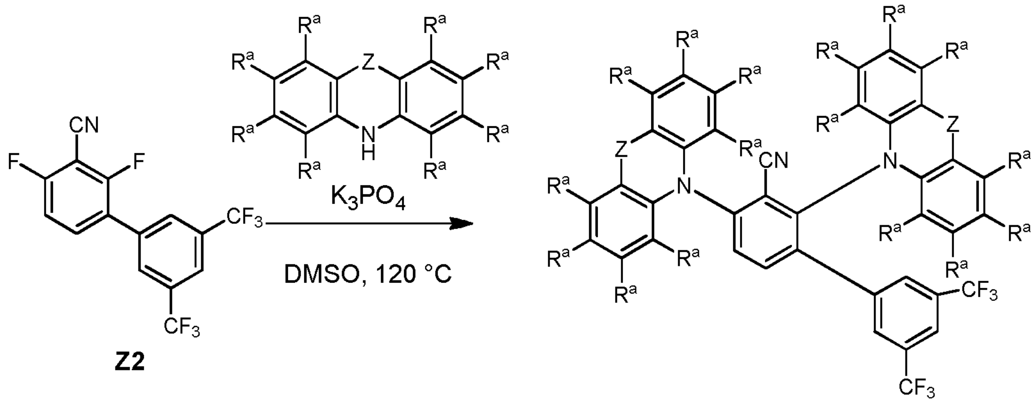
K3PO4
DMSO, 120 °C

**Z2**

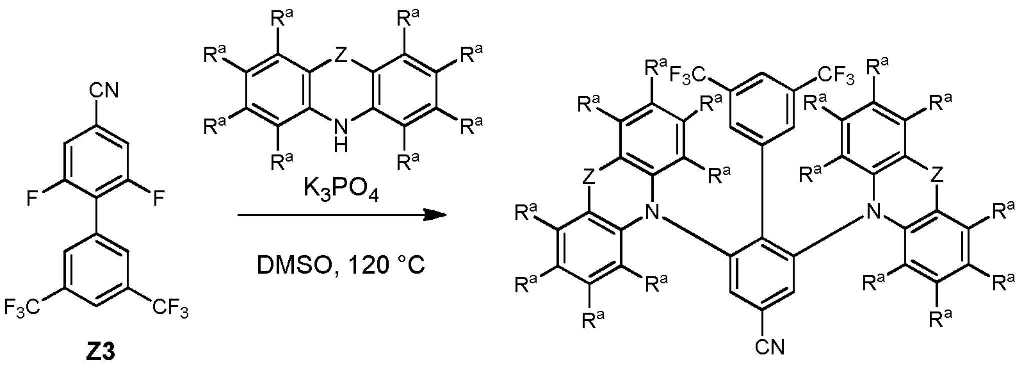
K3PO4
DMSO, 120 °C

**Z3**

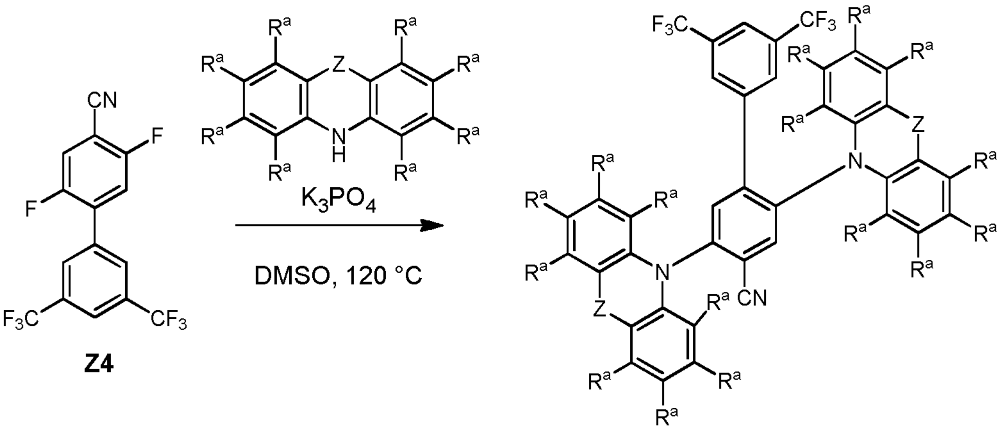
K3PO4
DMSO, 120 °C

**Z4**

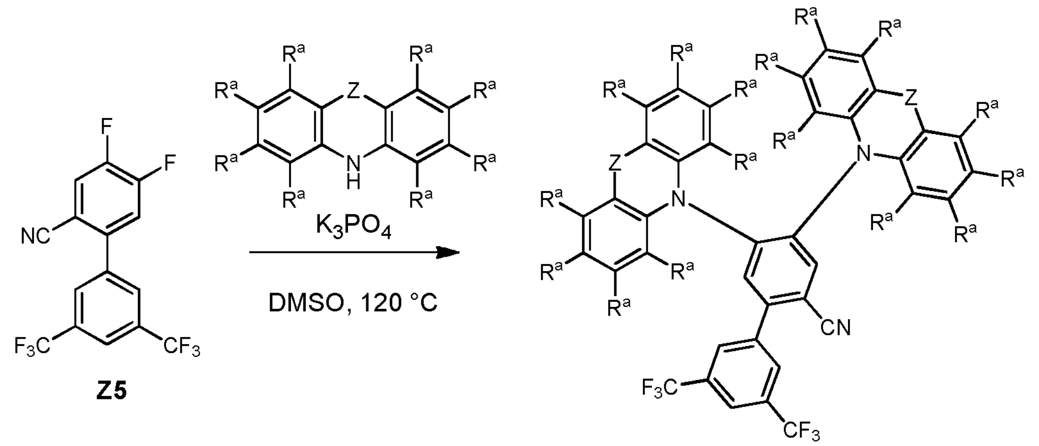


Z5

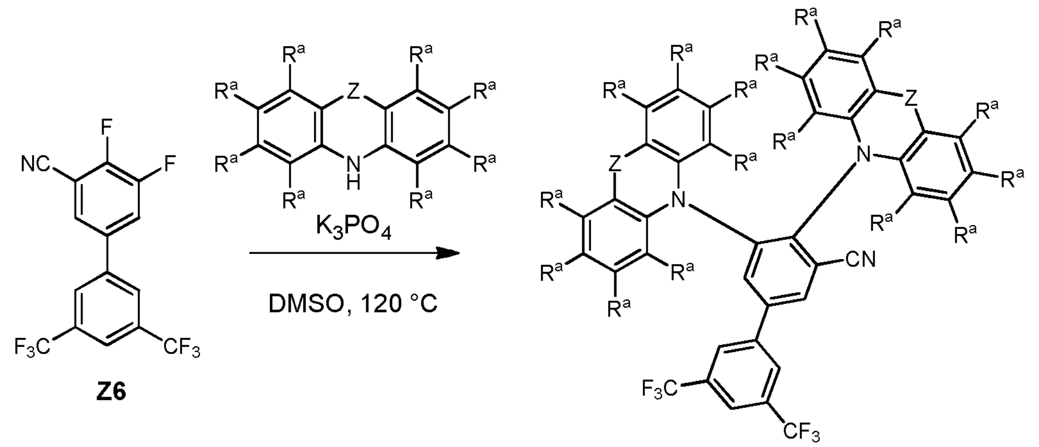


Z6

**Z1, Z2, Z3, Z4, Z5** oder **Z6** (jeweils 1,00 Äquivalente), das entsprechende Donor-Molekül D-H (2,00 Äquivalente) und tribasisches Kaliumphosphat (4,00 Äquivalente) werden unter Stickstoff in DMSO suspendiert und bei 110 °C gerührt (16 h). Anschließend wird die Reaktionsmischung in gesättigte Natriumchlorid-Lösung gegeben und dreimal mit Dichlormethan extrahiert. Die vereinigten organischen Phasen werden zweimal mit gesättigter Natriumchlorid-Lösung gewaschen, getrocknet über Magnesiumsulfat und das Lösemittel anschließend entfernt. Das Rohprodukt wurde schließlich durch Umkristallisation aus Toluol oder über Flashchromatographie gereinigt. Das Produkt wird als Feststoff erhalten.

Im speziellen entspricht D-H einem 3,6-substituierten Carbazol (z. B. 3,6-Dimethylcarbazol, 3,6-Diphenylcarbazol, 3,6-Di-tert-butylcarbazol), einem 2,7-substituierten Carbazol (z. B. 2,7-Dimethylcarbazol, 2,7-Diphenylcarbazol, 2,7-Di-tert-butylcarbazol), einem 1,8-substituierten Carbazol (z. B. 1,8-Dimethylcarbazol, 1,8-Diphenylcarbazol, 1,8-Di-tert-butylcarbazol), einem 1-substituierten Carbazol (z. B. 1-Methylcarbazol, 1-Phenylcarbazol, 1-tert-Butylcarbazol), einem 2-substituierten Carbazol (z. B. 2-Methylcarbazol, 2-Phenylcarbazol, 2-tert-Butylcarbazol) oder einem 3-substituierten Carbazol (z. B. 3-Methylcarbazol, 3-Phenylcarbazol, 3-tert-Butylcarbazol).

**Photophysikalische Messungen**

*Vorbehandlung von optischen Gläsern*

Alle Gläser (Küvetten und Substrate aus Quarzglas, Durchmesser: 1 cm) wurden nach jeder Benutzung gereinigt: Je dreimaliges Spülen mit Dichlormethan, Aceton, Ethanol, demineralisiertem Wasser, Einlegen in 5 % Hellmanex-Lösung für 24 h, gründliches Ausspülen mit demineralisiertem Wasser. Zum Trocknen wurden die optischen Gläser mit Stickstoff abgeblasen.

*Probenvorbereitung, Film: Spin-Coating*

Gerät: Spin150, SPS euro.

Die Probenkonzentration entsprach 10 mg/ml, angesetzt in Toluol oder Chlorbenzol. Programm: 1) 3 s bei 400

U/min; 2) 20 s bei 1000 U/min bei 1000 Upm/ s. 3) 10 s bei 4000 U/min bei 1000 Upm/s. Die Filme wurden nach dem Beschichten für 1 min bei 70 °C an Luft auf einer Präzisionsheizplatte von LHG getrocknet.

Photolumineszenzspektroskopie und TCSPC

Steady-state Emissionsspektroskopie wurde mit einem Fluoreszenzspektrometer der Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer "zeit-korrelierten Einphotonzähl" (*Time-correlated single-photon counting,* TCSPC)-Option. Emissions- und Anregungsspektren wurden korrigiert durch Standardkorrekturkurven.

Die Emissionsabklingzeiten wurden ebenfalls auf diesem System gemessen unter Verwendung der TCSPC-Methode mit dem FM-2013 Zubehör und einem TCSPC-Hub von Horiba Yvon Jobin. Anregungsquellen:

NanoLED 370 (Wellenlänge: 371 nm, Pulsdauer: 1,1 ns)
NanoLED 290 (Wellenlänge: 294 nm, Pulsdauer: <1 ns)
SpectraLED 310 (Wellenlänge: 314 nm)
SpectraLED 355 (Wellenlänge: 355 nm).

Die Auswertung (exponentielles Fitten) erfolgte mit dem Softwarepaket DataStation und der DAS 6 Auswertungssoftware. Der Fit wurde über die Chi-Quadrat-Methode angegeben

$$c^2 = \sum_{k=1}^{i} \frac{(e_i - o_i)^2}{e_i}$$

mit $e_i$: Durch den Fit vorhergesagte Größe und $o_i$: gemessene Größe.

Quanteneffizienzbestimmung

Die Messung der Photolumineszenzquantenausbeute (PLQY) erfolgte mittels eines *Absolute PL Quantum Yield Measurement* C9920-03G-Systems der *Hamamatsu Photonics.* Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- *(back thinned-)* CCD-Chip mit 1024 x 122 Pixeln (Größe 24 x 24 μm) verbunden ist. Die Auswertung der Quanteneffizienz und der CIE-Koordinaten erfolgte mit Hilfe der Software U6039-05 Version 3.6.0. Das Emissionsmaximum wird in nm, die Quantenausbeute Φ in % und die CIE-Farbkoordinaten als x,y-Werte angegeben.

Die Photolumineszenzquantenausbeute wurde nach folgendem Protokoll bestimmt:

1) Durchführung der Qualitätssicherung: Als Referenzmaterial dient Anthracene in Ethanol mit bekannter Konzentration.
2) Ermitteln der Anregungswellenlänge: Es wurde zuerst das Absorbtionsmaximum des organischen Moleküls bestimmt und mit diesem angeregt.
3) Durchführung der Probenmessung:
Es wurde von entgasten Lösungen und Filmen unter Stickstoff-Atmosphäre die absolute Quantenausbeute bestimmt.

Die Berechnung erfolgte systemintern nach folgender Gleichung:

$$\Phi_{PL} = \frac{n_{photon}, emittiert}{n_{photon}, absorbiert} = \frac{\int \frac{\lambda}{hc}\left[Int_{emittiert}^{Probe}(\lambda) - Int_{absorbiert}^{Probe}(\lambda)\right]d\lambda}{\int \frac{\lambda}{hc}\left[Int_{emittiert}^{Referenz}(\lambda) - Int_{absorbiert}^{Referenz}(\lambda)\right]d\lambda}$$

mit der Photonenzahl $n_{photon}$ und der Intensität Int.

Herstellung und Charakterisierung von organischen Elektrolumineszenzvorrichtungen aus der Gasphase
Mit den erfindungsgemäßen organischen Molekülen können OLED-Devices mittels Vakuum-Sublimationstechnik erstellt werden. Enthält eine Schicht mehrere Komponenten, so ist das Verhältnis dieser in Massenprozent angegeben.
Diese noch nicht optimierten OLEDs können standardmäßig charakterisiert werden; hierfür werden die Elektrolumineszenzspektren, die externe Quanteneffizienz (gemessen in %) in Abhängigkeit von der Helligkeit, berechnet aus dem von der Fotodiode detektiertem Licht, und dem Strom aufgenommen. Aus dem zeitlichen Verlauf der Elektrolumineszenzspektren kann die Lebensdauer der OLEDs bestimmt werden. Der angegebene LT50-Wert entspricht hierbei der Zeit, bei der die Leuchtdichte auf 50 % des Startwertes abgefallen ist. Analog entspricht der LT70-Wert der Zeit, bei der die Leuchtdichte auf 70 % des Startwertes abgefallen ist.
Die angegebenen Werte ergeben sich aus dem Durchschnitt der verschiedenen Pixel einer OLED. Die abgebildeten Spektren zeigen jeweils eine Messreihe eines Pixels.

**HPLC-MS:**

HPLC-MS Spektroskopie wurde mit einer HPLC-Anlage der Firma Agilent (1100er Serie) mit einem angeschlossenen MS-Detektor (Thermo LTQ XL) gemessen. Für die HPLC wurde eine Eclipse Plus C18 Säule von Agilent mit einer Partikelgröße von 3,5 µm, einer Länge von 150 mm und einem Innendurchmesser von 4,6 mm eingesetzt. Es wurde ohne Vorsäule und bei Raumtemperatur mit den Lösemitteln Acetonitril, Wasser und Tetrahydrofuran in diesen Konzentrationen gearbeitet:

| | | |
|---|---|---|
| Lösemittel A: | $H_2O$ (90%) | MeCN (10%) |
| Lösemittel B: | $H_2O$ (10%) | MeCN (90%) |
| Lösemittel C: | THF (100%) | |

Es wurde mit einem Einspritzvolumen von 15 µL und einer Konzentration von 10 µg/mL mit diesem Gradienten gearbeitet:

| Fluss [ml/min] | Zeit [min] | A[%] | B[%] | C[%] | Druck [Bar] |
|---|---|---|---|---|---|
| 0,3 | 0 | 80 | 20 | - | 115 |
| 0,3 | 5 | 80 | 20 | - | 115 |
| 0,3 | 14 | 0 | 90 | 10 | 65 |
| 0,3 | 25 | 0 | 90 | 10 | 65 |
| 0,3 | 26 | 80 | 20 | - | 115 |
| 0,3 | 33 | 80 | 20 | - | 115 |

Die Ionisation der Probe erfolgt durch APCI (atmospheric pressure chemical ionization).

**Beispiel 1**

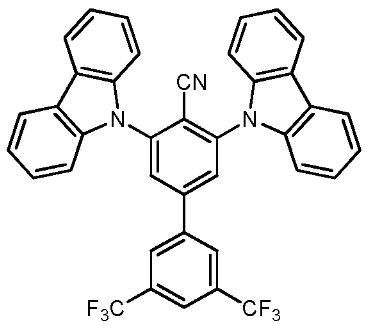

Beispiel **1** wurde nach AAV1 (Ausbeute 99%) und AAV7 hergestellt (Ausbeute 40%).
MS (HPLC-MS), m/z (Retentionszeit): 643, (18,60 min)
$R_f$ = 0,68 (Cyclohexan/Ethylacetat 5:1).
Figur 1 zeigt das Emissionsspektrum von Beispiel **1** (10 % in PMMA). Das Emissionsmaximum liegt bei 461 nm. Die Photolumineszenzquantenausbeute (PLQY) beträgt 84% und die Halbwertsbreite beträgt 0,43 eV.

**Beispiel 2**

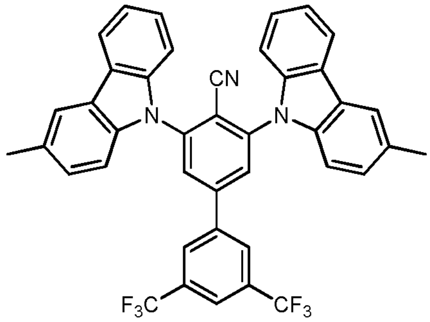

Beispiel **2** wurde nach AAV1 (Ausbeute 99%) und AAV7 (Ausbeute 47%) hergestellt.
MS (HPLC-MS), m/z (Retentionszeit): 673, (20,05 min)
$R_f$ = 0,71 (Cyclohexan/Ethylacetat 5:1).
Figur 2 zeigt das Emissionsspektrum von Beispiel **2** (10 % in PMMA). Das Emissionsmaximum liegt bei 476 nm. Die Photolumineszenzquantenausbeute (PLQY) beträgt 84% und die Halbwertsbreite beträgt 0,44 eV. Die Emissionsabklingzeit beträgt 63 μs.

**Beispiel 3**

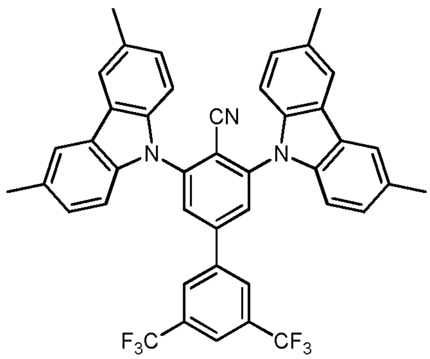

Beispiel **3** wurde nach AAV1 (Ausbeute 99%) und AAV7 (Ausbeute 77%) hergestellt. MS (HPLC-MS), m/z (Retentionszeit): 702, (21,51 min)
$R_f$ = 0,83 (Cyclohexan/Ethylacetat 5:1)
Figur 3 zeigt das Emissionsspektrum von Beispiel **3** (10 % in PMMA). Das Emissionsmaximum liegt bei 486 nm. Die Photolumineszenzquantenausbeute (PLQY) beträgt 74% und die Halbwertsbreite beträgt 0,43 eV. Die Emissionsabklingzeit beträgt 12 μs.

**Beispiel 4**

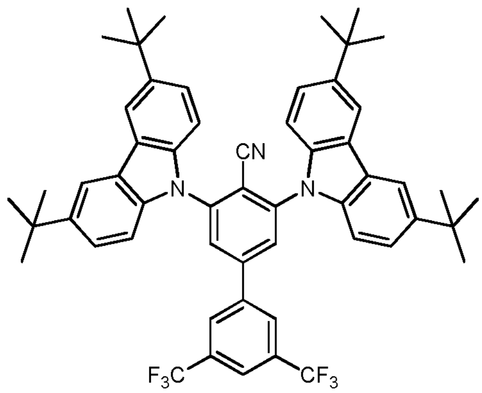

Beispiel **4** wurde nach AAV1 (Ausbeute 99%) und AAV7 hergestellt (Ausbeute 89%).
$R_f$ = 0,84 (Cyclohexan/Ethylacetat 5:1).
Figur 4 zeigt das Emissionsspektrum von Beispiel **4** (10 % in PMMA). Das Emissionsmaximum liegt bei 478 nm. Die Photolumineszenzquantenausbeute (PLQY) beträgt 78% und die Halbwertsbreite beträgt 0,43 eV. Die Emissionsabklingzeit beträgt 14 µs.

**Beispiel 5**

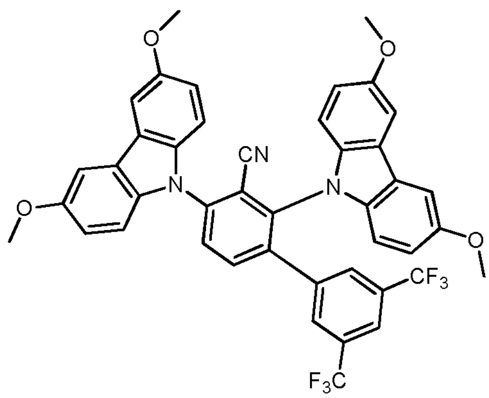

Beispiel **5** wurde nach AAV2 (Ausbeute 52%) und AAV7 hergestellt (Ausbeute 70%).
MS (HPLC-MS), m/z (Retentionszeit): 765, (14,45 min)
$R_f$ = 0,13 (Cyclohexan/Ethylacetat 5:1).
Figur 5 zeigt das Emissionsspektrum von Beispiel **5** (10 % in PMMA). Das Emissionsmaximum liegt bei 483 nm. Die Photolumineszenzquantenausbeute (PLQY) beträgt 68% und die Halbwertsbreite beträgt 0,46 eV. Die Emissionsabklingzeit beträgt 34 µs.

**Beispiel 6**

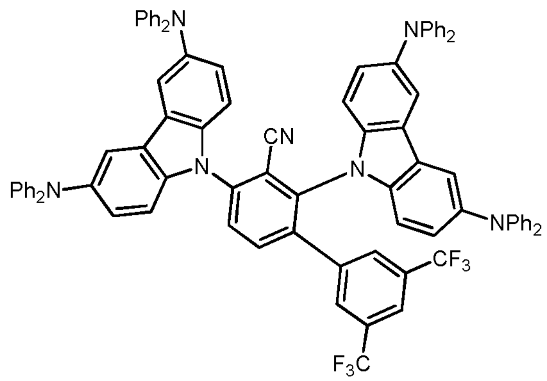

Beispiel **6** wurde nach AAV2 (Ausbeute 52%) und AAV7 hergestellt (Ausbeute 69%).
MS (HPLC-MS), m/z (Retentionszeit): 1304, (31,17 min)
$R_f$ = 0,69 (Cyclohexan/Ethylacetat 5:1).
Figur 6 zeigt das Emissionsspektrum von Beispiel **6** (10 % in PMMA). Das Emissionsmaximum liegt bei 528 nm. Die Photolumineszenzquantenausbeute (PLQY) beträgt 45% und die Halbwertsbreite beträgt 0,46 eV. Die Emissionsabklingzeit beträgt 3 µs.

Beispiel 7

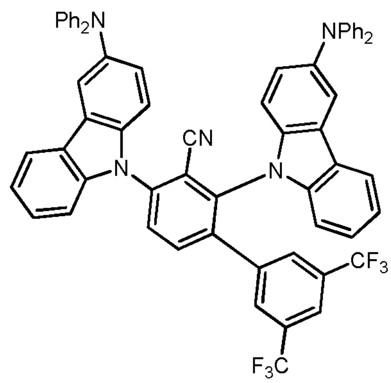

Beispiel 7 wurde nach AAV2 (Ausbeute 52%) und AAV7 hergestellt (Ausbeute 74%).
MS (HPLC-MS), m/z (Retentionszeit): 980, (22,38 min)
$R_f$ = 0,56 (Cyclohexan/Ethylacetat 5:1).
Figur 7 zeigt das Emissionsspektrum von Beispiel **7** (10 % in PMMA). Das Emissionsmaximum liegt bei 516 nm. Die Photolumineszenzquantenausbeute (PLQY) beträgt 44% und die Halbwertsbreite beträgt 0,49 eV. Die Emissionsabklingzeit beträgt 3 µs.

**Beispiel 8**

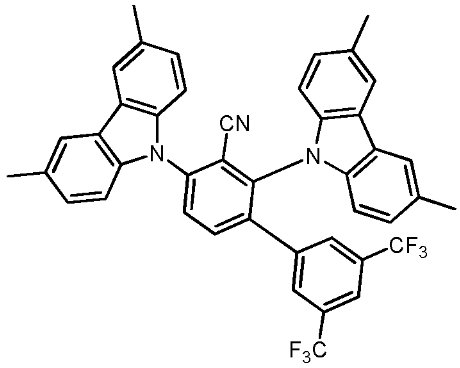

Beispiel **8** wurde nach AAV2 (Ausbeute 52%) und AAV7 hergestellt (Ausbeute 38%).
MS (HPLC-MS), m/z (Retentionszeit): 702, (19,55 min)
$R_f$ = 0,70 (Cyclohexan/Ethylacetat 5:1).
Figur 8 zeigt das Emissionsspektrum von Beispiel **8** (10 % in PMMA). Das Emissionsmaximum liegt bei 442 nm. Die Photolumineszenzquantenausbeute (PLQY) beträgt 71% und die Halbwertsbreite beträgt 0,44 eV.

Beispiel 9

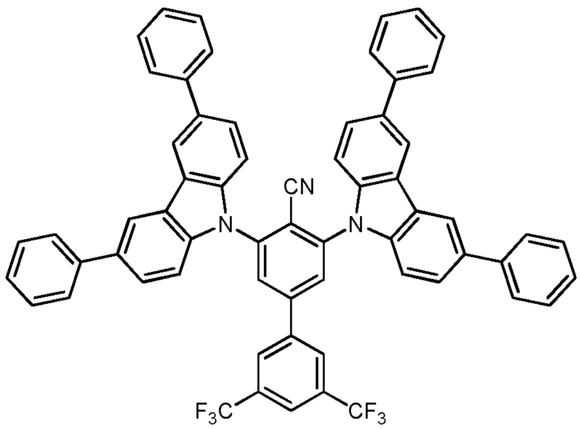

Beispiel **9** wurde nach AAV1 (Ausbeute 99%) und AAV7 (Ausbeute 47%) hergestellt.
MS (HPLC-MS), m/z (Retentionszeit): 593, (10,67 min)
Figur 9 zeigt das Emissionsspektrum von Beispiel **9** (10 % in PMMA). Das Emissionsmaximum liegt bei 492 nm. Die Photolumineszenzquantenausbeute (PLQY) beträgt 84% und die Halbwertsbreite beträgt 0,41 eV. Die Emissionsabklingzeit beträgt 7 µs.

**Beispiel 10**

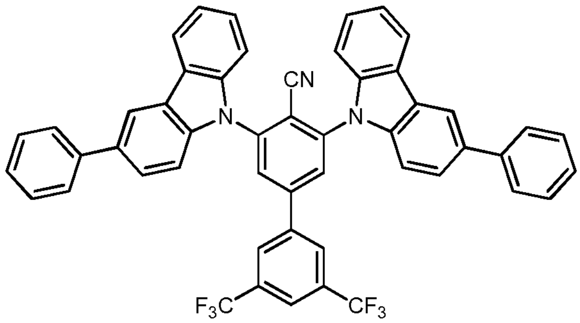

Beispiel **10** wurde nach AAV1 (Ausbeute 99%) und AAV7 (Ausbeute 74%) hergestellt.
MS (HPLC-MS), m/z (Retentionszeit): 797, (21,35 min)
$R_f$ = 0,48 (Cyclohexan/Ethylacetat 5:1)
Figur 10 zeigt das Emissionsspektrum von Beispiel **10** (10 % in PMMA). Das Emissionsmaximum liegt bei 481 nm. Die Photolumineszenzquantenausbeute (PLQY) beträgt 80% und die Halbwertsbreite beträgt 0,45 eV. Die Emissionsabklingzeit beträgt 41 µs.

**Beispiel 11**

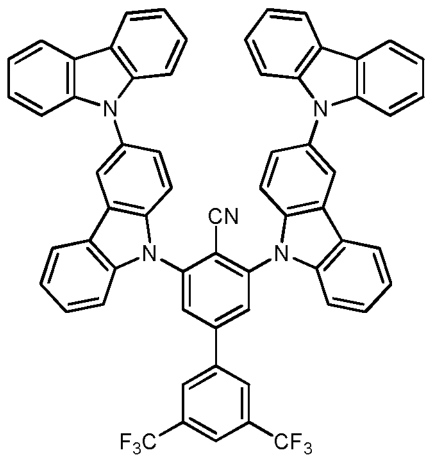

Beispiel **11** wurde nach AAV1 (Ausbeute 99%) und AAV7 (Ausbeute 81%) hergestellt.
MS (HPLC-MS), m/z (Retentionszeit): 975, (25,15 min)
$R_f$ = 0,65 (Cyclohexan/Ethylacetat 5:1)
Figur 11 zeigt das Emissionsspektrum von Beispiel **11** (10% in PMMA). Das Emissionsmaximum liegt bei 496 nm. Die Photolumineszenzquantenausbeute (PLQY) beträgt 59% und die Halbwertsbreite beträgt 0,50 eV. Die Emissionsabklingzeit beträgt 22 µs.

**Beispiel 12**

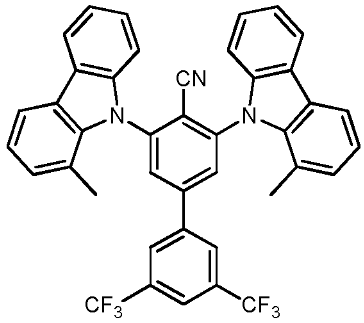

Beispiel **12** wurde nach AAV1 (Ausbeute 99%) und AAV7 hergestellt (Ausbeute 16%).
MS (HPLC-MS), m/z (Retentionszeit): 673, (19,01 min)
$R_f$ = 0,67 (Cyclohexan/Ethylacetat 5:1).
Figur 12 zeigt das Emissionsspektrum von Beispiel **12** (10 % in PMMA). Das Emissionsmaximum liegt bei 476 nm. Die Photolumineszenzquantenausbeute (PLQY) beträgt 76% und die Halbwertsbreite beträgt 0,46 eV.

**Beispiel 13**

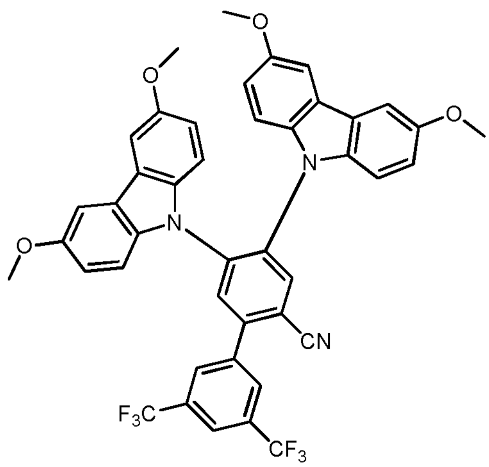

Beispiel **13** wurde nach AAV5 (Ausbeute 94 %) und AAV7 hergestellt (Ausbeute 53 %).
$R_f$ = 0,2 (Cyclohexan/Ethylacetat 5:1).
$^1$H-NMR (500 MHz, $CDCl_3$): d = 8.28 (s, 1 H), 8.18 (s, 1 H), 8.07 (s, 1 H), 8.00 (s, 1 H), 7.29-7.31 (m, 4 H), 7.04 (dd, 4 H), 6.75 (td, 4 H), 3.85 (s, 6 H), 3.87 (s, 6 H) ppm.
Figur 13 zeigt das Emissionsspektrum von Beispiel **13** (10 % in PMMA). Das Emissionsmaximum liegt bei 502 nm. Die Photolumineszenzquantenausbeute (PLQY) beträgt 75% und die Halbwertsbreite beträgt 0,49 eV.

**Beispiel 14**

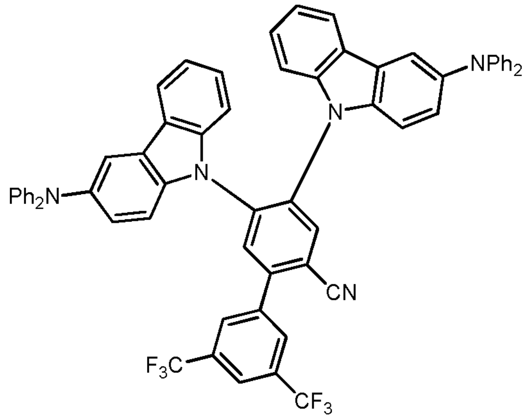

Beispiel **14** wurde nach AAV5 (Ausbeute 94 %) und AAV7 hergestellt (Ausbeute 71%).
$R_f$ = 0,7 (Cyclohexan/Ethylacetat 5:1).
$^1$H-NMR (500 MHz, $CDCl_3$): d = 8.41 (d, 1 H), 8.26 (s, 2 H), 8.12 (d, 2 H), 7.72 (q, 2 H), 7.61 (dd, 1 H), 7.56 (dd, 1 H), 7.40 (t, 1 H), 7.27-7.34 (m, 1 H), 7.14-7.22 (m, 9 H), 7.01-7.16 (m, 4 H), 6.92-6.97 (m, 12 H), 6.85 (td, 1 H), 6.60 (dd, 1 H), 6.55 (td, 1 H) ppm.
Figur 14 zeigt das Emissionsspektrum von Beispiel **14** (10 % in PMMA). Das Emissionsmaximum liegt bei 527 nm. Die Photolumineszenzquantenausbeute (PLQY) beträgt 49% und die Halbwertsbreite beträgt 0,50 eV. Die Emissionsabklingzeit beträgt 9 µs.

**Beispiel 15**

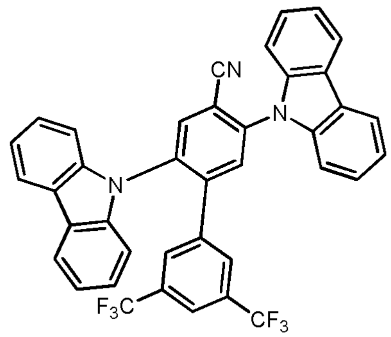

Beispiel **15** wurde nach AAV4 (Ausbeute 88%) und AAV7 hergestellt (Ausbeute 79%).
MS (HPLC-MS), m/z (Retentionszeit): 645, (18,61 min)
$R_f$ = 0,55 (Cyclohexan/Ethylacetat 5:1).
Figur 15 zeigt das Emissionsspektrum von Beispiel **15** (10 % in PMMA). Das Emissionsmaximum liegt bei 450 nm. Die Photolumineszenzquantenausbeute (PLQY) beträgt 78% und die Halbwertsbreite beträgt 0,42 eV.

**Beispiel 16**

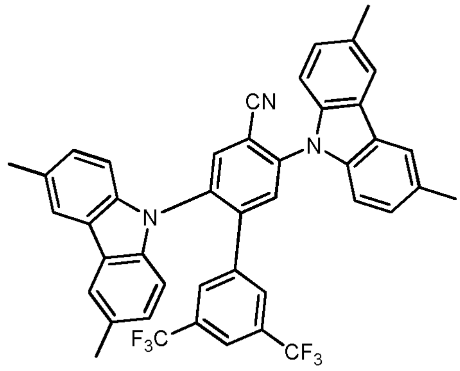

Beispiel **16** wurde nach AAV4 (Ausbeute 88%) und AAV7 hergestellt (Ausbeute 99%). MS (HPLC-MS), m/z (Retentionszeit): 702, (21,47 min)
$R_f$ = 0,74 (Cyclohexan/Ethylacetat 5:1).
Figur 16 zeigt das Emissionsspektrum von Beispiel **16** (10 % in PMMA). Das Emissionsmaximum liegt bei 471 nm. Die Photolumineszenzquantenausbeute (PLQY) beträgt 92% und die Halbwertsbreite beträgt 0,41 eV.

**Beispiel 17**

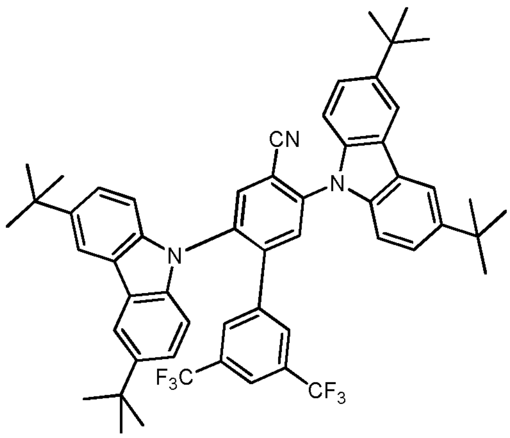

Beispiel **17** wurde nach AAV4 (Ausbeute 88%) und AAV7 hergestellt (Ausbeute 99%). $^1$H-NMR (500 MHz, $CDCl_3$): d = 8.26 (s, 1H, Ar-H), 8.19 (d, 2H, Ar-H), 8.02 (d, 2H, Ar-H), 7.87 (s, 1H, Ar-H), 7.56 (dd, 2H, Ar-H), 7.45 (bs, 1H, Ar-H), 7.38-7.35 (m, 4H, Ar-H), 7.22-7.21 (m, 2H, Ar-H), 6.98 (d, 2H, Ar-H), 1.43 (s, 18H, $CH_3$), 1.41 (s, 18H, $CH_3$) ppm.
Figur 17 zeigt das Emissionsspektrum von Beispiel **17** (10 % in PMMA). Das Emissionsmaximum liegt bei 466 nm. Die Photolumineszenzquantenausbeute (PLQY) beträgt 94% und die Halbwertsbreite beträgt 0,41 eV.

**Beispiel 18**

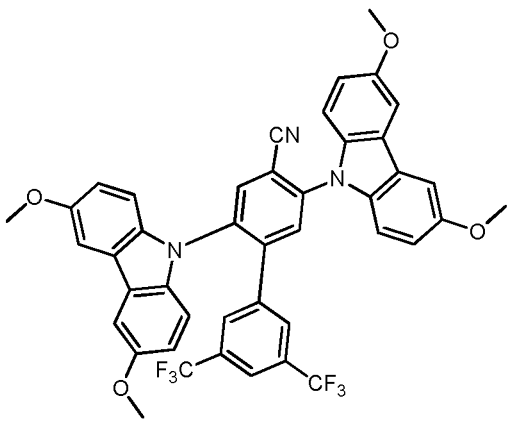

Beispiel **18** wurde nach AAV4 (Ausbeute 88%) und AAV7 hergestellt (Ausbeute 90%).
MS (HPLC-MS), m/z (Retentionszeit): 765, (16,18 min)
$R_f$ = 0,38 (Cyclohexan/Ethylacetat 5:1).
Figur 18 zeigt das Emissionsspektrum von Beispiel **18** (10 % in PMMA). Das Emissionsmaximum liegt bei 513 nm. Die Photolumineszenzquantenausbeute (PLQY) beträgt 52% und die Halbwertsbreite beträgt 0,45 eV. Die Emissionsabklingzeit beträgt 34 μs.

Beispiel 19

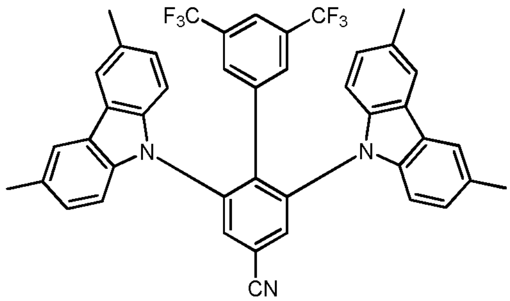

Beispiel **19** wurde nach AAV3 (Ausbeute 71%) und AAV7 hergestellt (Ausbeute 31%).
MS (HPLC-MS), m/z (Retentionszeit): 702, (20,85 min)
$R_f$ = 0,86 (Cyclohexan/Ethylacetat 5:1).
Figur 19 zeigt das Emissionsspektrum von Beispiel **19** (10 % in PMMA). Das Emissionsmaximum liegt bei 463 nm. Die Photolumineszenzquantenausbeute (PLQY) beträgt 72% und die Halbwertsbreite beträgt 0,45 eV.

**Beispiel 20**

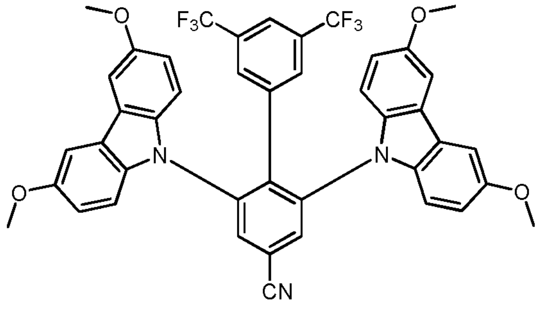

Beispiel **20** wurde nach AAV3 (Ausbeute 71%) und AAV7 hergestellt (Ausbeute 54%).
MS (HPLC-MS), m/z (Retentionszeit): 765, (15,05 min)
$R_f$ = 0,36 (Cyclohexan/Ethylacetat 5:1).
Figur 20 zeigt das Emissionsspektrum von Beispiel **20** (10 % in PMMA). Das Emissionsmaximum liegt bei 500 nm. Die Photolumineszenzquantenausbeute (PLQY) beträgt 61% und die Halbwertsbreite beträgt 0,47 eV. Die Emissionsabklingzeit beträgt 11 µs.

**Beispiel 21**

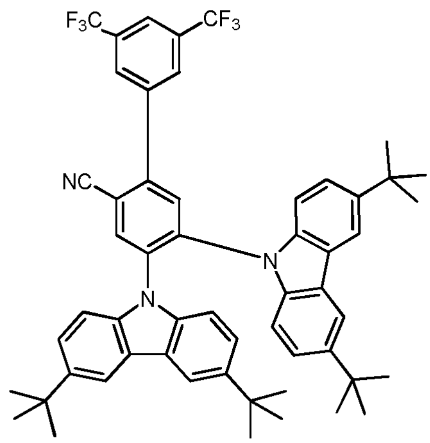

Beispiel **21** wurde nach AAV5 (Ausbeute 94%) und AAV7 hergestellt (Ausbeute 31%).
Figur 21 zeigt das Emissionsspektrum von Beispiel **21** (10 % in PMMA). Das Emissionsmaximum liegt bei 468 nm. Die Photolumineszenzquantenausbeute (PLQY) beträgt 92% und die Halbwertsbreite beträgt 0,46 eV.

**Beispiel 22**

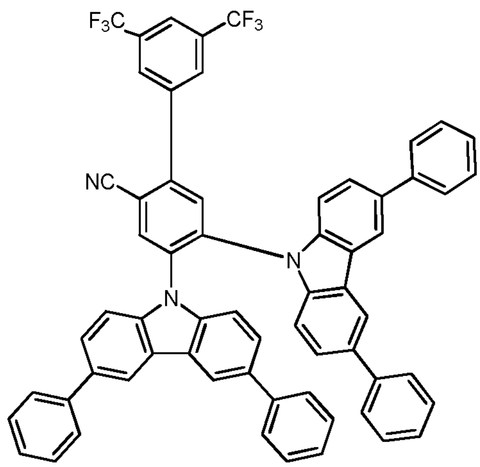

Beispiel **22** wurde nach AAV5 (Ausbeute 94%) und AAV7 hergestellt (Ausbeute 68%).
Figur 22 zeigt das Emissionsspektrum von Beispiel **22** (10 % in PMMA). Das Emissionsmaximum liegt bei 470 nm. Die Photolumineszenzquantenausbeute (PLQY) beträgt 84% und die Halbwertsbreite beträgt 0,47 eV.

**Beispiel 23**

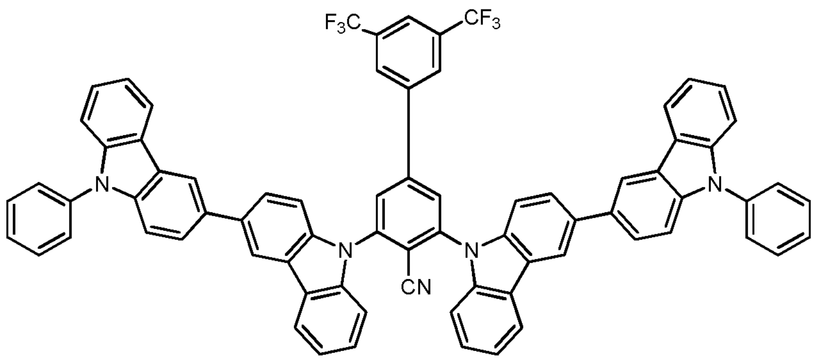

Beispiel **23** wurde nach AAV1 (Ausbeute 99%) und AAV7 hergestellt (Ausbeute 81%). Figur 23 zeigt das Emissionsspektrum von Beispiel **23** (10 % in PMMA). Das Emissionsmaximum liegt bei 470 nm. Die Photolumineszenzquantenausbeute (PLQY) beträgt 47% und die Halbwertsbreite beträgt 0,50 eV. Die Emissionsabklingzeit beträgt 3 μs.

**Beispiel 24**

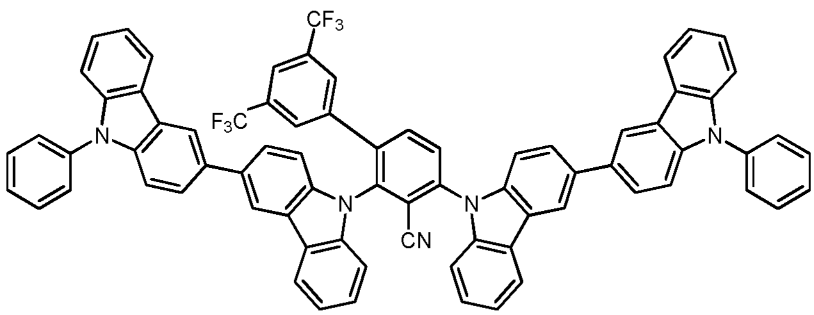

Beispiel **24** wurde nach AAV2 (Ausbeute 52%) und AAV7 hergestellt (Ausbeute 66%). Figur 24 zeigt das Emissionsspektrum von Beispiel **24** (10 % in PMMA). Das Emissionsmaximum liegt bei 475 nm. Die Photolumineszenzquantenausbeute (PLQY) beträgt 62% und die Halbwertsbreite beträgt 0,51 eV.

**Beispiel 25**

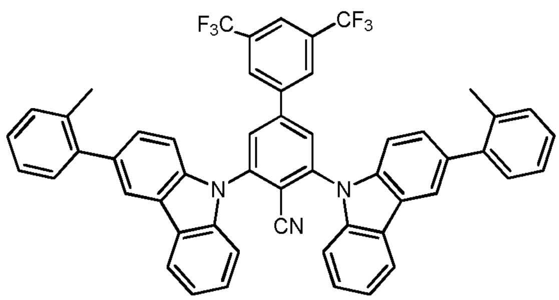

Beispiel **25** wurde nach AAV1 (Ausbeute 99%) und AAV7 hergestellt (Ausbeute 64%). Figur 25 zeigt das Emissionsspektrum von Beispiel **25** (10 % in PMMA). Das Emissionsmaximum liegt bei 473 nm. Die Photolumineszenzquantenausbeute (PLQY) beträgt 84% und die Halbwertsbreite beträgt 0,45 eV.

**Beispiel 26**

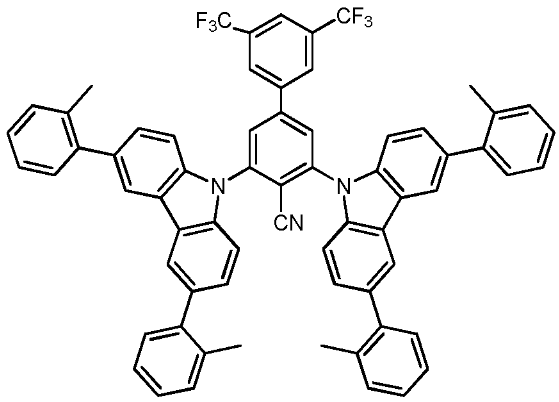

Beispiel **26** wurde nach AAV1 (Ausbeute 99%) und AAV7 hergestellt. Figur 26 zeigt das Emissionsspektrum von Beispiel **26** (10 % in PMMA). Das Emissionsmaximum liegt bei 473 nm. Die Photolumineszenzquantenausbeute (PLQY) beträgt 87% und die Halbwertsbreite beträgt 0,43 eV. Die Emissionsabklingzeit beträgt 31 µs.

**Beispiel 27**

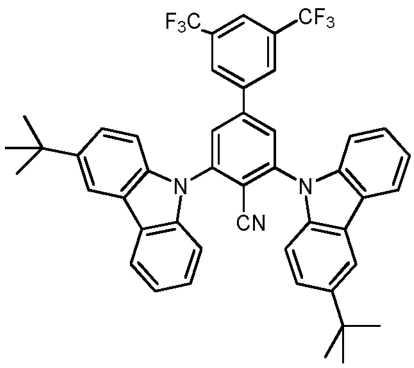

Beispiel **27** wurde nach AAV1 (Ausbeute 99%) und AAV7 hergestellt (Ausbeute 66%). Figur 27 zeigt das Emissionsspektrum von Beispiel **27** (10 % in PMMA). Das Emissionsmaximum liegt bei 473 nm. Die Photolumineszenzquantenausbeute (PLQY) beträgt 82% und die Halbwertsbreite beträgt 0,43 eV. Die Emissionsabklingzeit beträgt 79 µs.

**Beispiel 28**

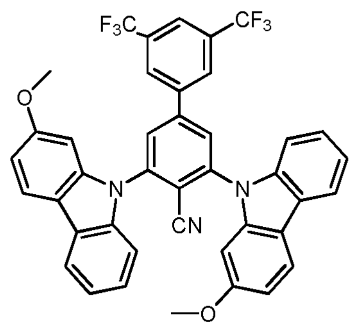

Beispiel **28** wurde nach AAV1 (Ausbeute 99%) und AAV7 hergestellt (Ausbeute 53%). Figur 28 zeigt das Emissionsspektrum von Beispiel **28** (10 % in PMMA). Das Emissionsmaximum liegt bei 473 nm. Die Photolumineszenzquantenausbeute (PLQY) beträgt 61% und die Halbwertsbreite beträgt 0,52 eV.

**Beispiel 29**

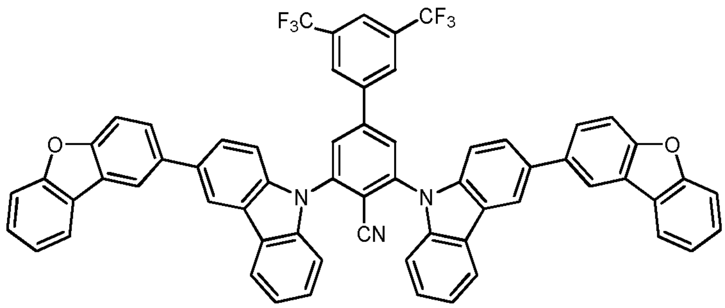

Beispiel **29** wurde nach AAV1 (Ausbeute 99%) und AAV7 hergestellt (Ausbeute 65%). Figur 29 zeigt das Emissionsspektrum von Beispiel **29** (10 % in PMMA). Das Emissionsmaximum liegt bei 489 nm. Die Photolumineszenzquantenausbeute (PLQY) beträgt 73% und die Halbwertsbreite beträgt 0,47 eV. Die Emissionsabklingzeit beträgt 10 µs.

**Beispiel 30**

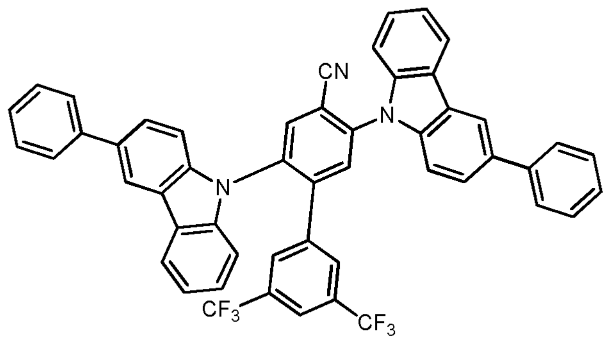

Beispiel **30** wurde nach AAV4 (Ausbeute 88%) und AAV7 hergestellt (Ausbeute 38%). Figur 30 zeigt das Emissionsspektrum von Beispiel **30** (10 % in PMMA). Das Emissionsmaximum liegt bei 464 nm. Die Photolumineszenzquantenausbeute (PLQY) beträgt 86% und die Halbwertsbreite beträgt 0,43 eV.

**Beispiel 31**

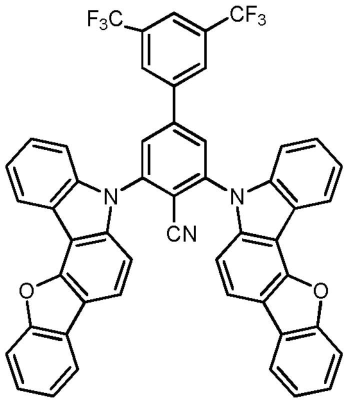

Beispiel **31** wurde nach AAV1 (Ausbeute 99%) und AAV7 hergestellt.
Figur 31 zeigt das Emissionsspektrum von Beispiel **31** (10 % in PMMA). Das Emissionsmaximum liegt bei 492 nm. Die Photolumineszenzquantenausbeute (PLQY) beträgt 78% und die Halbwertsbreite beträgt 0,46 eV. Die Emissionsabklingzeit beträgt 9 μs.

**Beispiel 32**

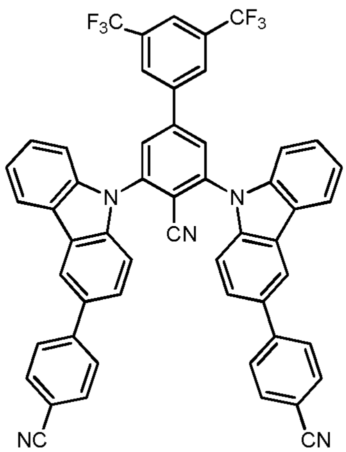

Beispiel **32** wurde nach AAV1 (Ausbeute 99%) und AAV7 hergestellt (Ausbeute 92%).
Figur 32 zeigt das Emissionsspektrum von Beispiel **32** (10 % in PMMA). Das Emissionsmaximum liegt bei 476 nm. Die Photolumineszenzquantenausbeute (PLQY) beträgt 72% und die Halbwertsbreite beträgt 0,44 eV.

**Beispiel 33**

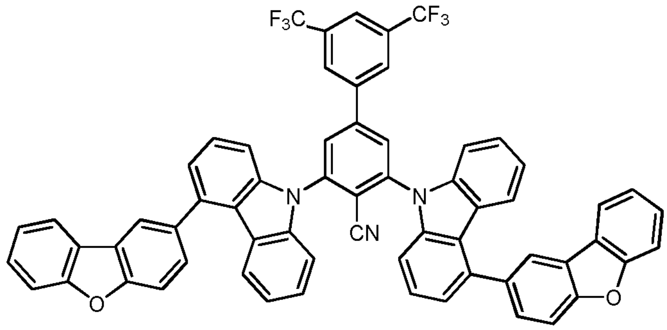

Beispiel **33** wurde nach AAV1 (Ausbeute 99%) und AAV7 hergestellt (Ausbeute 78%). Figur 33 zeigt das Emissionsspektrum von Beispiel **33** (10 % in PMMA). Das Emissionsmaximum liegt bei 465 nm. Die Photolumineszenzquantenausbeute (PLQY) beträgt 93% und die Halbwertsbreite beträgt 0,44 eV.

**Beispiel 34**

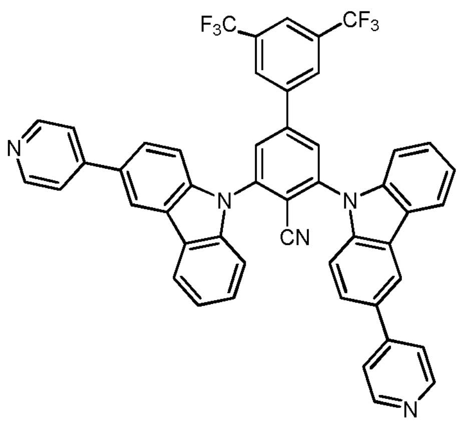

Beispiel **34** wurde nach AAV1 (Ausbeute 99%) und AAV7 hergestellt (Ausbeute 92%). Figur 34 zeigt das Emissionsspektrum von Beispiel **34** (10 % in PMMA). Das Emissionsmaximum liegt bei 467 nm. Die Photolumineszenzquantenausbeute (PLQY) beträgt 83% und die Halbwertsbreite beträgt 0,43 eV.

**Beispiel 35**

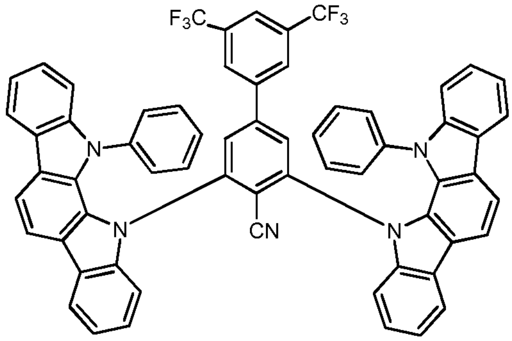

Beispiel **35** wurde nach AAV1 (Ausbeute 99%) und AAV7 hergestellt (Ausbeute 54%). Figur 35 zeigt das Emissionsspektrum von Beispiel **35** (10 % in PMMA). Das Emissionsmaximum liegt bei 501 nm. Die Photolumineszenzquantenausbeute (PLQY) beträgt 62% und die Halbwertsbreite beträgt 0,47 eV. Die Emissionsab-

klingzeit beträgt 4 μs.

**Beispiel 36**

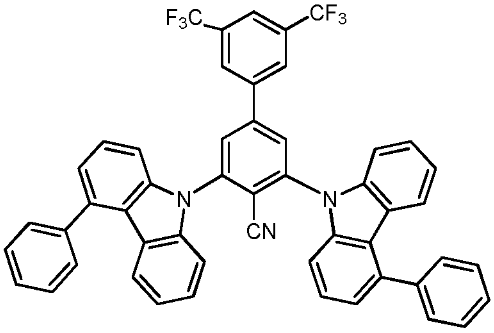

Beispiel **36** wurde nach AAV1 (Ausbeute 99%) und AAV7 hergestellt (Ausbeute 84%). Figur 36 zeigt das Emissionsspektrum von Beispiel **36** (10 % in PMMA). Das Emissionsmaximum liegt bei 466 nm. Die Photolumineszenzquantenausbeute (PLQY) beträgt 76% und die Halbwertsbreite beträgt 0,43 eV.

**Beispiel 37**

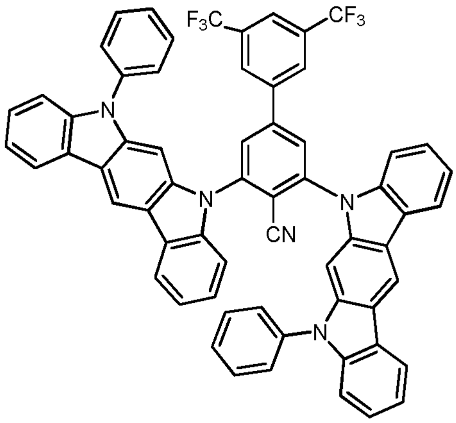

Beispiel **37** wurde nach AAV1 (Ausbeute 99%) und AAV7 hergestellt (Ausbeute 43%). Figur 37 zeigt das Emissionsspektrum von Beispiel **37** (10 % in PMMA). Das Emissionsmaximum liegt bei 509 nm. Die Photolumineszenzquantenausbeute (PLQY) beträgt 59% und die Halbwertsbreite beträgt 0,44 eV. Die Emissionsabklingzeit beträgt 3 μs.

**Beispiel 38**

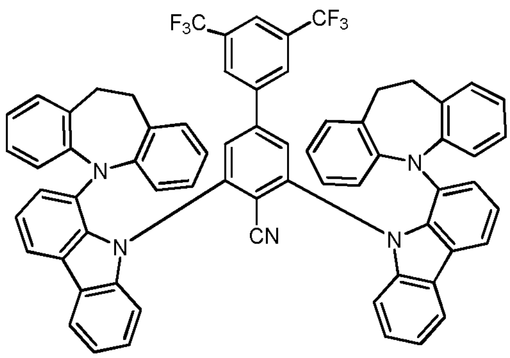

Beispiel **38** wurde nach AAV1 (Ausbeute 99%) und AAV7 hergestellt (Ausbeute 3%).
Figur 38 zeigt das Emissionsspektrum von Beispiel **38** (10 % in PMMA). Das Emissionsmaximum liegt bei 521 nm. Die Photolumineszenzquantenausbeute (PLQY) beträgt 72% und die Halbwertsbreite beträgt 0,45 eV. Die Emissionsabklingzeit beträgt 37 μs.

**Beispiel 39**

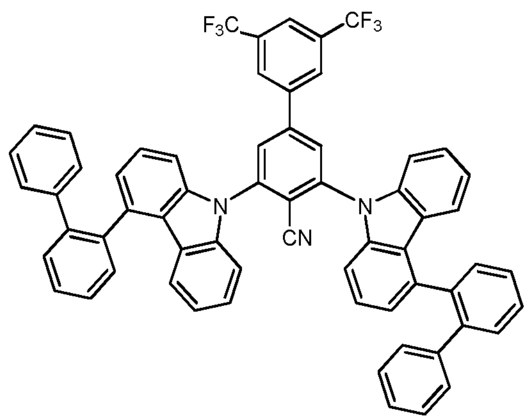

Beispiel **39** wurde nach AAV1 (Ausbeute 99%) und AAV7 hergestellt (Ausbeute 14%).
Figur 39 zeigt das Emissionsspektrum von Beispiel **39** (10 % in PMMA). Das Emissionsmaximum liegt bei 463 nm. Die Photolumineszenzquantenausbeute (PLQY) beträgt 60% und die Halbwertsbreite beträgt 0,43 eV.

**Beispiel 40**

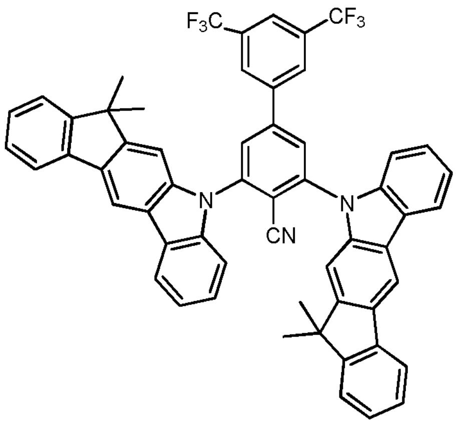

Beispiel **40** wurde nach AAV1 (Ausbeute 99%) und AAV7 hergestellt (Ausbeute 38%). Figur 40 zeigt das Emissionsspektrum von Beispiel **40** (10 % in PMMA). Das Emissionsmaximum liegt bei 504 nm. Die Photolumineszenzquantenausbeute (PLQY) beträgt 57% und die Halbwertsbreite beträgt 0,47 eV. Die Emissionsabklingzeit beträgt 4 µs.

**Beispiel 41**

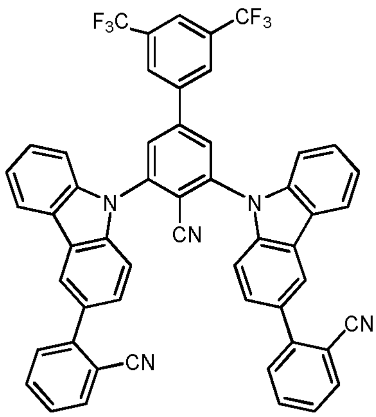

Beispiel **41** wurde nach AAV1 (Ausbeute 99%) und AAV7 hergestellt (Ausbeute 74%). Figur 41 zeigt das Emissionsspektrum von Beispiel **41** (10 % in PMMA). Das Emissionsmaximum liegt bei 462 nm. Die Photolumineszenzquantenausbeute (PLQY) beträgt 78% und die Halbwertsbreite beträgt 0,44 eV.

**Beispiel 42**

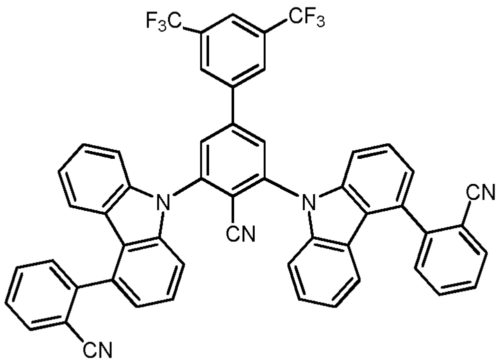

Beispiel **42** wurde nach AAV1 (Ausbeute 99%) und AAV7 hergestellt (Ausbeute 75%). Figur 42 zeigt das Emissionsspektrum von Beispiel **42** (10 % in PMMA). Das Emissionsmaximum liegt bei 455 nm. Die Photolumineszenzquantenausbeute (PLQY) beträgt 77% und die Halbwertsbreite beträgt 0,44 eV.

**Beispiel D1**

Beispiel **4** wurde in einem OLED-Bauteil ("Bauteil D1) mit folgendem Aufbau getestet (der Anteil des erfindungsgemäßen Moleküls an der Emissionsschicht ist in Massenprozent angegeben):

| **Schicht** | **Dicke** | **D1** |
|---|---|---|
| **7** | 100 nm | Al |
| **6** | 2 nm | Liq |
| **5** | 40 nm | NBPhen |
| **4** | 20 nm | **4** (20%):mCBP |
| **3** | 10 nm | TCTA |
| **2** | 80 nm | NPB |
| **1** | 130 nm | ITO |
| **Substrat** | | Glass |

| | Maximalwerte | bei 1000 cd/m$^2$ |
|---|---|---|
| Externe Quanteneffizienz (EQE): | 14,2 ± 0,7 % | 10,9 ± 0,3 % |

Das Emissionsmaximum liegt bei 480 nm, CIEx wurde mit 0,19 und die CIEy mit 0,36 bei 6 V bestimmt.

**Beispiel D2**

Beispiel **9** wurde in einem OLED-Bauteil ("Bauteil D2") mit folgendem Aufbau getestet (der Anteil des erfindungsgemäßen Moleküls an der Emissionsschicht ist in Massenprozent angegeben):

| **Schicht** | **Dicke** | **D2** |
|---|---|---|
| **7** | 100 nm | Al |
| **6** | 2 nm | Liq |
| **5** | 40 nm | NBPhen |
| **4** | 20 nm | **9** (10%):mCBP |
| **3** | 10 nm | TCTA |

(fortgesetzt)

| Schicht | Dicke | D2 |
|---|---|---|
| **2** | 80 nm | NPB |
| **1** | 130 nm | ITO |
| **Substrat** | | Glass |

| | Maximalwerte | bei 1000 cd/m$^2$ | LT80 (bei 500 cd/m$^2$) |
|---|---|---|---|
| Externe Quanteneffizienz (EQE): | -- | 14,7 ± 0,1 % | 325 h |

Das Emissionsmaximum liegt bei 486 nm, CIEx wurde mit 0,21 und die CIEy mit 0,39 bei 6 V bestimmt.

**Beispiel D3**

Beispiel **9** wurde in dem OLED-Bauteil **D3** mit folgendem Aufbau getestet (Anteil des erfindungsgemäßen Moleküls und der beiden Hostmoleküle an der Emissionsschicht ist jeweils in Massenprozent angegeben):

| Schicht | Dicke | Material |
|---|---|---|
| **8** | 100 nm | Al |
| **7** | 2 nm | Liq |
| **6** | 30 nm | NBPhen |
| **5** | 10 nm | T2T |
| **4** | 20 nm | **Beispiel 9** (10%):mCBP (75%):T2T(15%) |
| **3** | 10 nm | TCTA |
| **2** | 80 nm | NPB |
| **1** | 130 nm | ITO |
| **Substrat** | | Glas |

Das Emissionsmaximum liegt bei 493 nm, CIEx wurde mit 0,22 und die CIEy mit 0,46 bei 6 V bestimmt. Die EQE bei 1000 cd/m$^2$ beträgt 23,6 ± 0,4 % und die LT80 bei 500 cd/m$^2$ beträgt 416 h.

**Beispiel D4**

Beispiel **9** wurde in dem OLED-Bauteil **D4** mit folgendem Aufbau getestet (Anteil des erfindungsgemäßen Moleküls und der beiden Hostmoleküle an der Emissionsschicht ist jeweils in Massenprozent angegeben):

| Schicht | Dicke | Material |
|---|---|---|
| **7** | 100 nm | Al |
| **6** | 2 nm | Liq |
| **5** | 40 nm | NBPhen |
| **4** | 20 nm | **Beispiel 9** (10%):9-[3,5-Bis(2-dibenzofuranyl)phenyl]-9H-carbazol (60%):T2T(30%) |
| **3** | 10 nm | TCTA |
| **2** | 80 nm | NPB |
| **1** | 130 nm | ITO |
| **Substrat** | | Glas |

Das Emissionsmaximum liegt bei 487 nm, CIEx wurde mit 0,21 und die CIEy mit 0,42 bei 6 V bestimmt. Die EQE bei 1000 cd/$m^2$ beträgt 15,1 ± 0,6 % und die LT80 bei 500 cd/$m^2$ beträgt 1017 h.

**Beispiel D5**

Beispiel **31** wurde in dem OLED-Bauteil **D5** mit folgendem Aufbau getestet (Anteil des erfindungsgemäßen Moleküls und der beiden Hostmoleküle an der Emissionsschicht ist jeweils in Massenprozent angegeben):

| **Schicht** | **Dicke** | **Material** |
|---|---|---|
| **8** | 100 nm | Al |
| **7** | 2 nm | Liq |
| **6** | 30 nm | NBPhen |
| **5** | 10 nm | T2T |
| **4** | 20 nm | **Beispiel 31** (10%):9-[3,5-Bis(2-dibenzofuranyl)phenyl]-9H-carbazol (75%):T2T(15%) |
| **3** | 10 nm | TCTA |
| **2** | 80 nm | NPB |
| **1** | 130 nm | ITO |
| **Substrat** | | Glas |

Das Emissionsmaximum liegt bei 485 nm, CIEx wurde mit 0,21 und die CIEy mit 0,41 bei 6 V bestimmt. Die EQE bei 1000 cd/$m^2$ beträgt 16,1 ± 0,5 % und die LT80 bei 500 cd/$m^2$ beträgt 260 h.

**Beispiel D6**

Beispiel 35 wurde in dem OLED-Bauteil D6 getestet, das den gleichen Aufbau wie OLED-Bauteil D1 besitzt, wobei Beispiel 4 durch Beispiel 35 ersetzt wurde. Das Emissionsmaximum liegt bei 504 nm, CIEx wurde mit 0,27 und die CIEy mit 0,48 bei 6 V bestimmt. Die EQE bei 1000 cd/$m^2$ beträgt 9,5 ± 0,3 % und die LT80 bei 500 cd/$m^2$ beträgt 162 h.

**Beispiel D7**

Beispiel **1** wurde in dem OLED-Bauteil **D7** mit folgendem Aufbau getestet (Anteil des erfindungsgemäßen Moleküls und der beiden Hostmoleküle an der Emissionsschicht ist jeweils in Massenprozent angegeben):

| **Schicht** | **Dicke** | **Material** |
|---|---|---|
| **8** | 100 nm | Al |
| **7** | 2 nm | Liq |
| **6** | 30 nm | TPBi |
| **5** | 10 nm | DPEPO |
| **4** | 20 nm | **Beispiel 1** (20%):DPEPO (80%) |
| **3** | 10 nm | CzSi |
| **2** | 20 nm | TCTA |
| **2** | 70 nm | NPB |
| **2** | 20 nm | m-MTDATA |
| **1** | 130 nm | ITO |
| **Substrat** | | Glas |

Das Emissionsmaximum liegt bei 460 nm, CIEx wurde mit 0,16 und die CIEy mit 0,19 bei 6 V bestimmt. Die

EQE bei 1000 cd/$m^2$ beträgt 8,0 ± 0,4 %.

**Beispiel D8**

Beispiel 27 wurde in dem OLED-Bauteil D8 getestet, das den gleichen Aufbau wie OLED-Bauteil D7 besitzt, wobei Beispiel 1 durch Beispiel 27 ersetzt wurde. Das Emissionsmaximum liegt bei 473 nm, CIEx wurde mit 0,18 und die CIEy mit 0,29 bei 6 V bestimmt. Die EQE bei 1000 cd/$m^2$ beträgt 10,6 ± 0,4 %.

Ein weiteres exemplarisches OLED-Bauteil beinhaltet folgenden Aufbau:

| **Schicht** | **Dicke** | **Material** |
|---|---|---|
| **9** | 100 nm | Al |
| **8** | 2 nm | Liq |
| **7** | 30 nm | NBPhen |
| **6** | 10 nm | T2T |
| **5** | 20 nm | **Beispiel 9** (20%):mCBP (60%):T2T(20%) |
| **4** | 10 nm | mCBP |
| **3** | 10 nm | TCTA |
| **2** | 80 nm | NPB |
| **1** | 130 nm | ITO |
| **Substrat** | | Glas |

**Weitere Beispiele erfindungsgemäßer organischer Moleküle:**

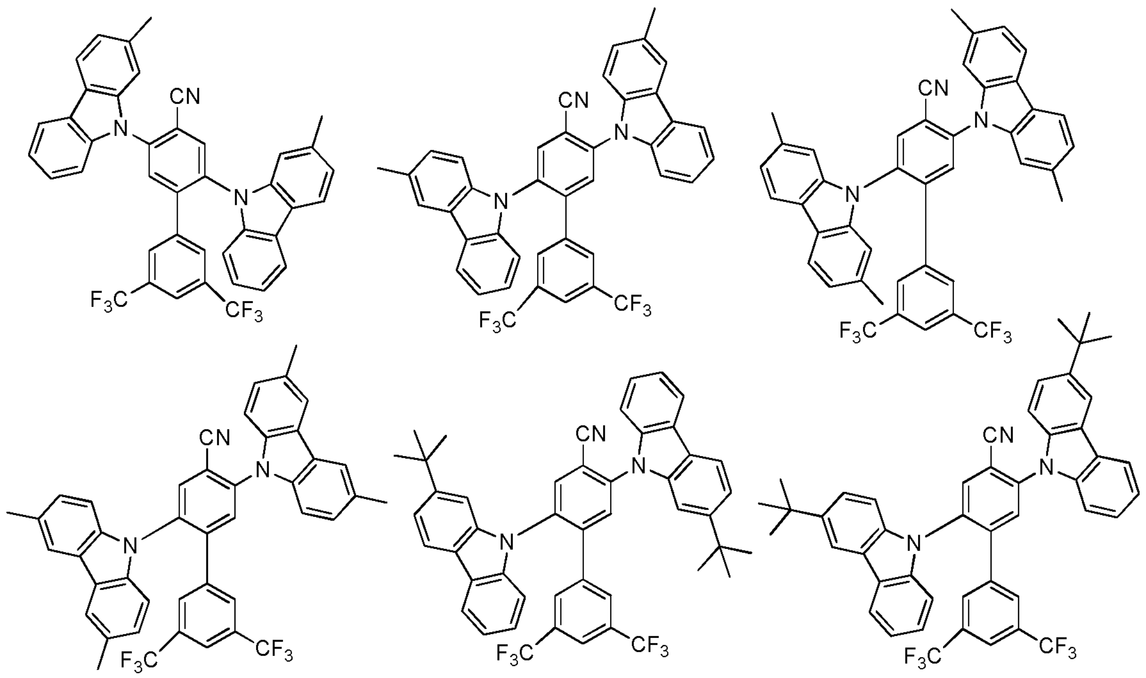

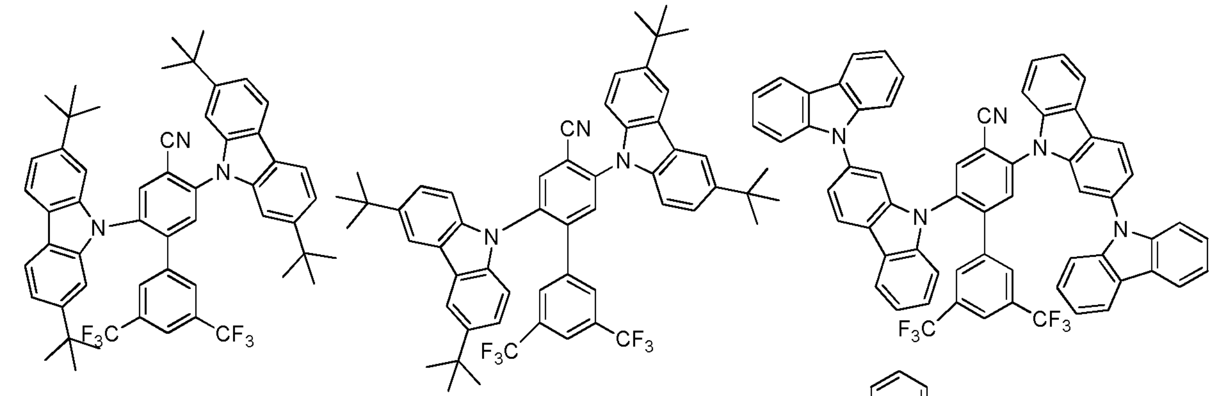

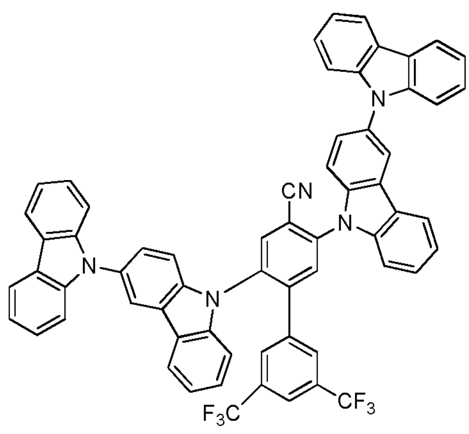

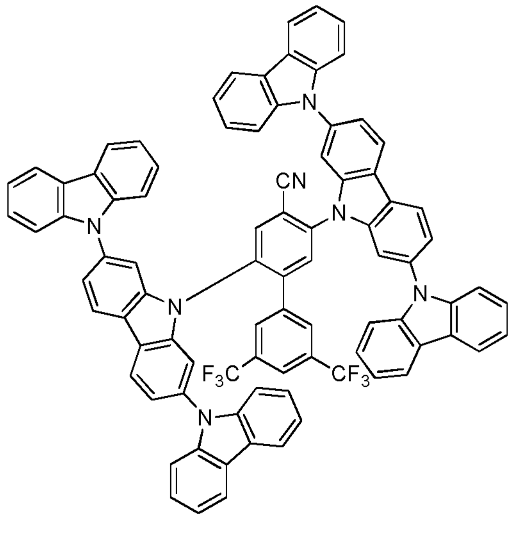

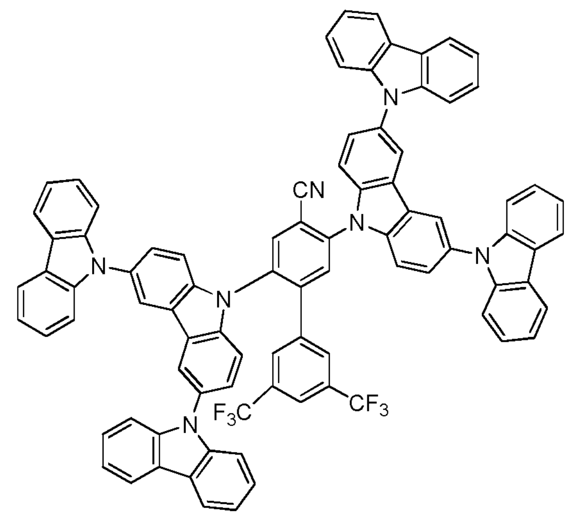

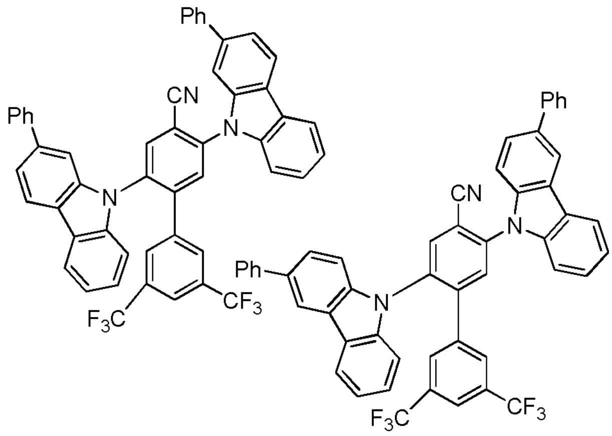

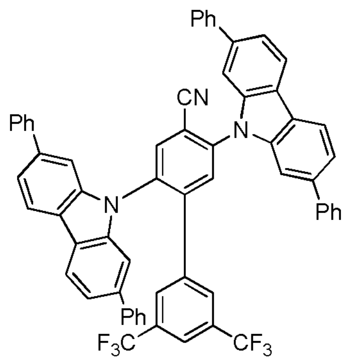

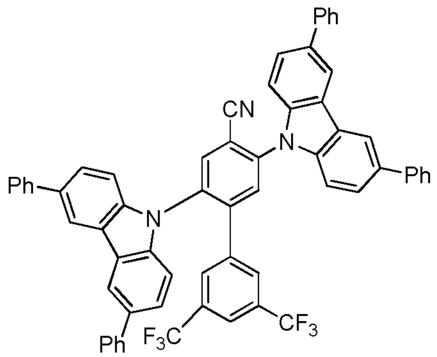

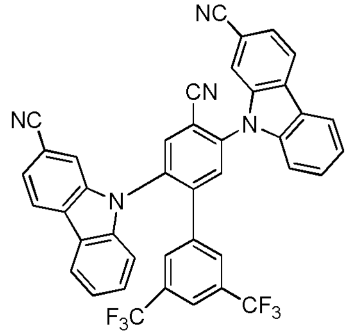

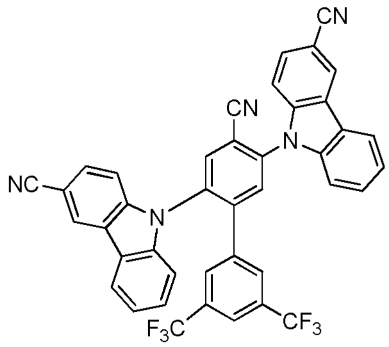

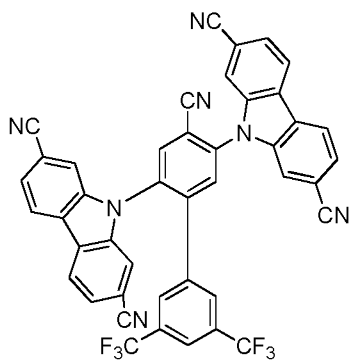

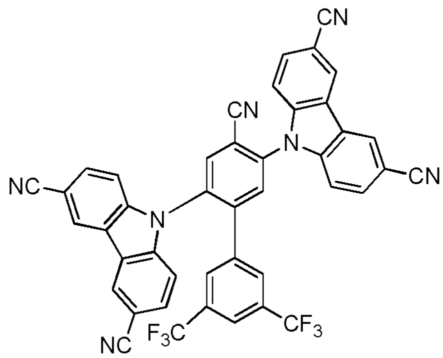

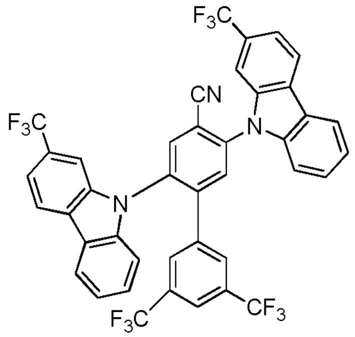

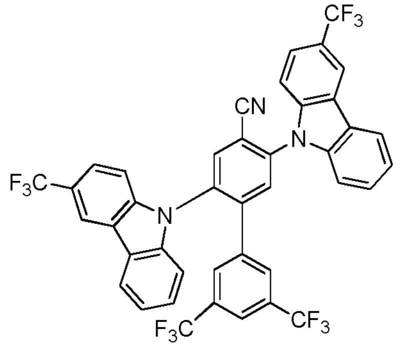

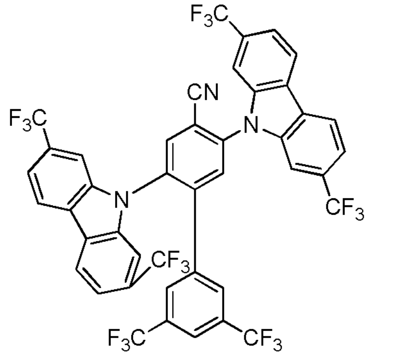

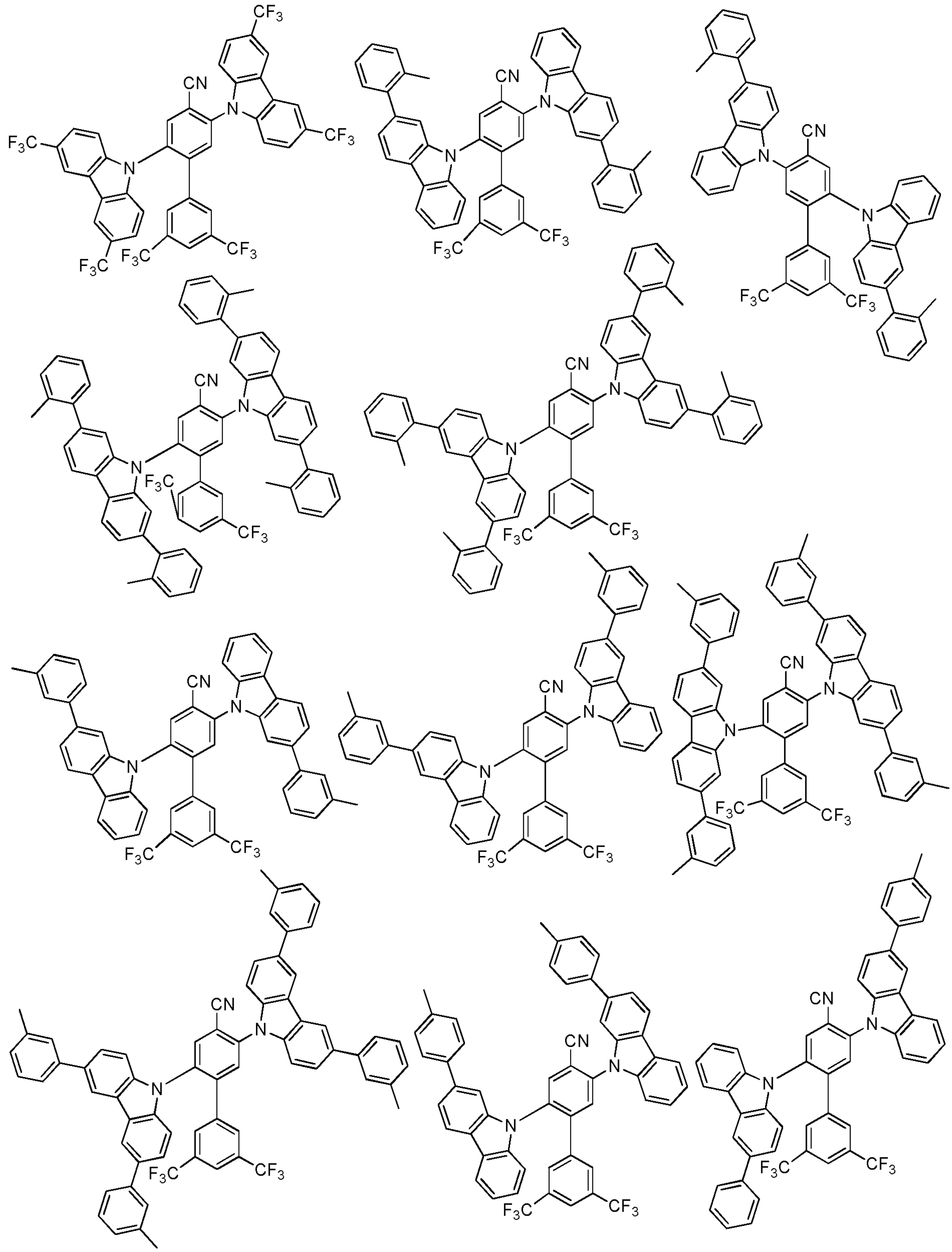

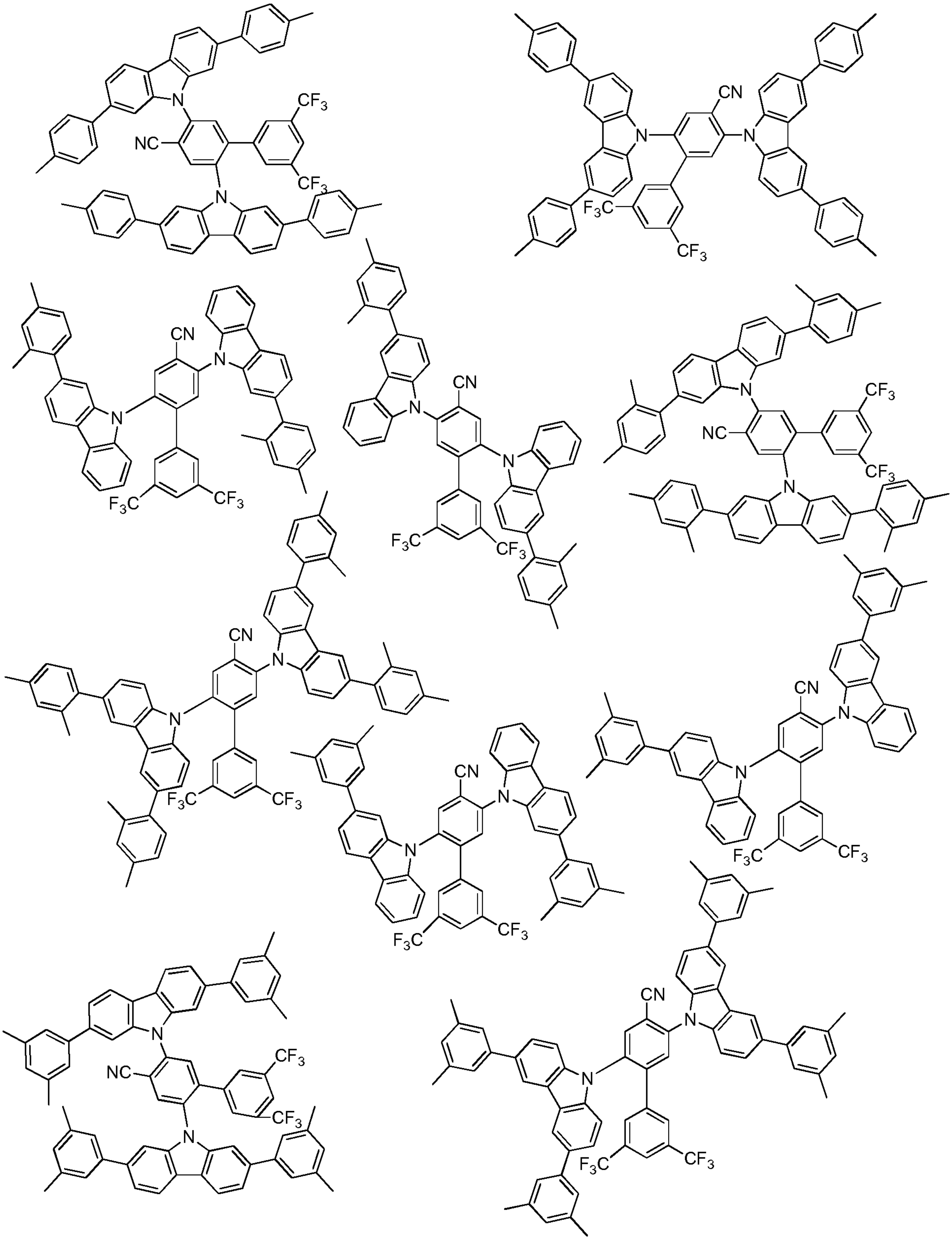

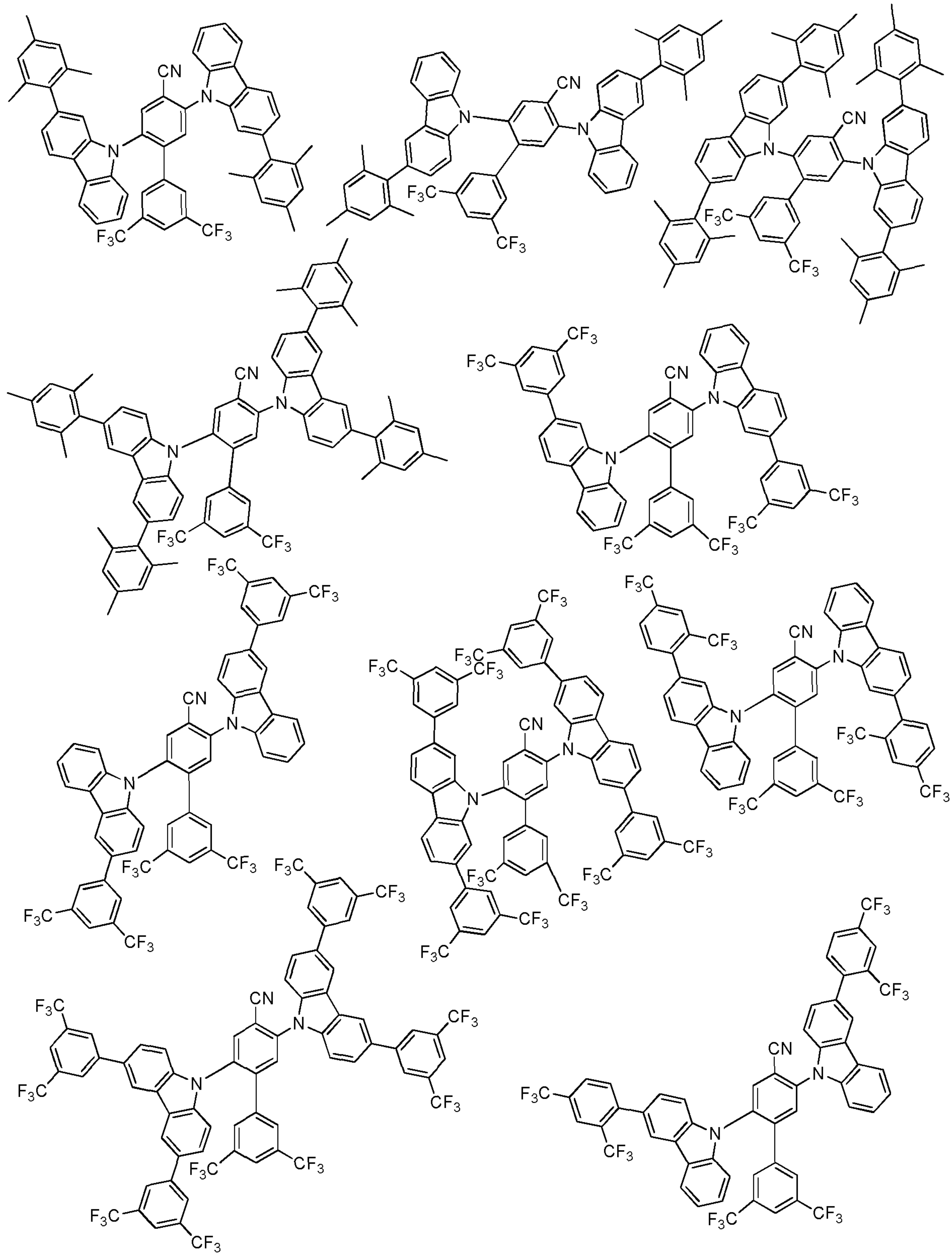

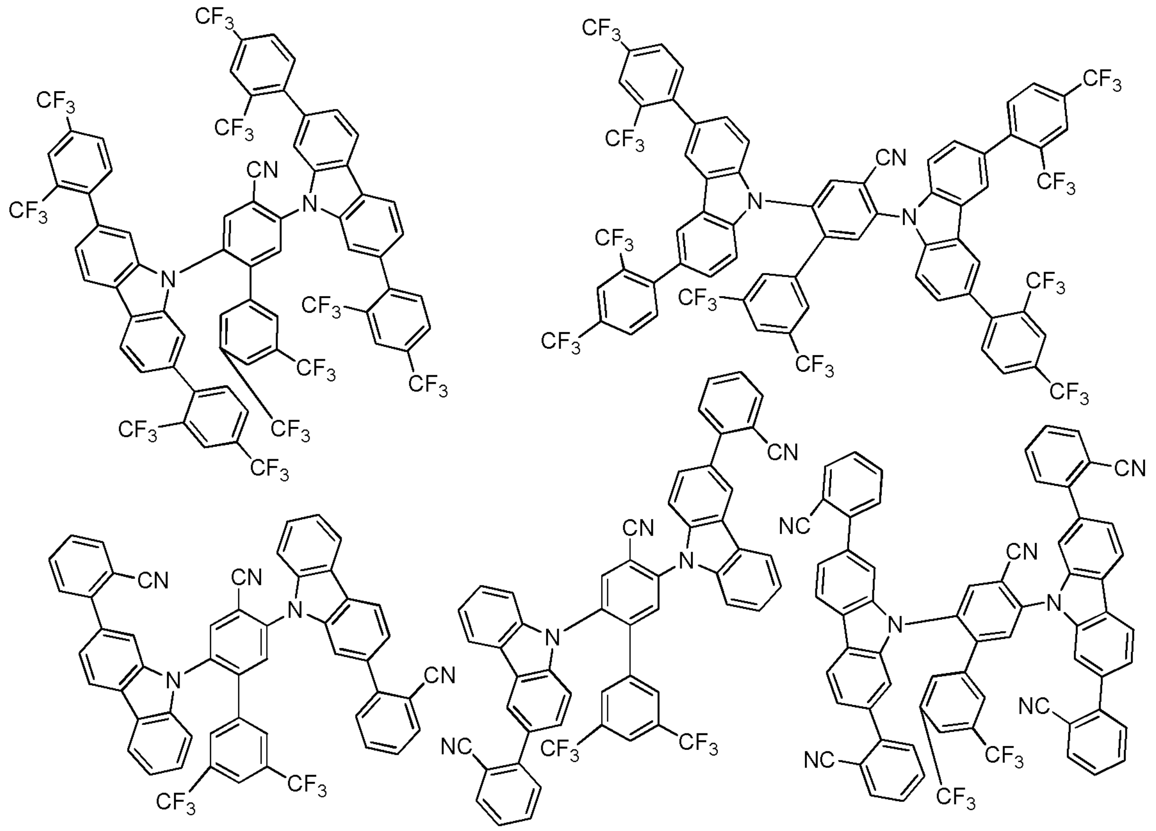

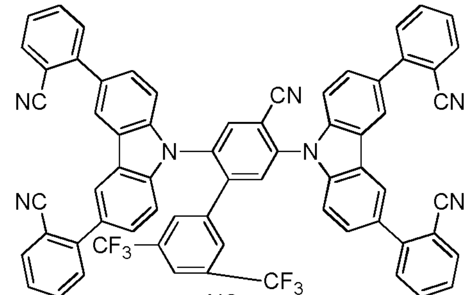

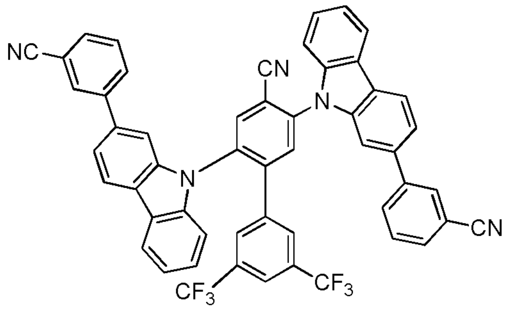

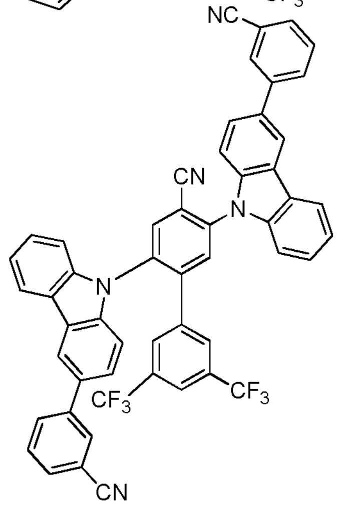

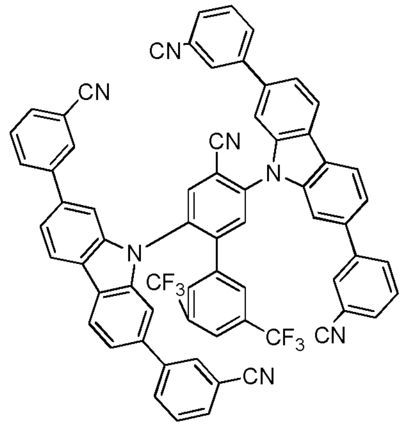

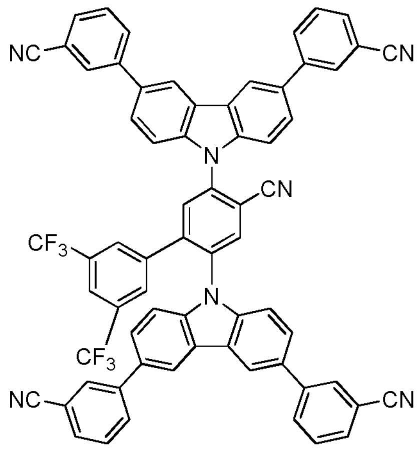

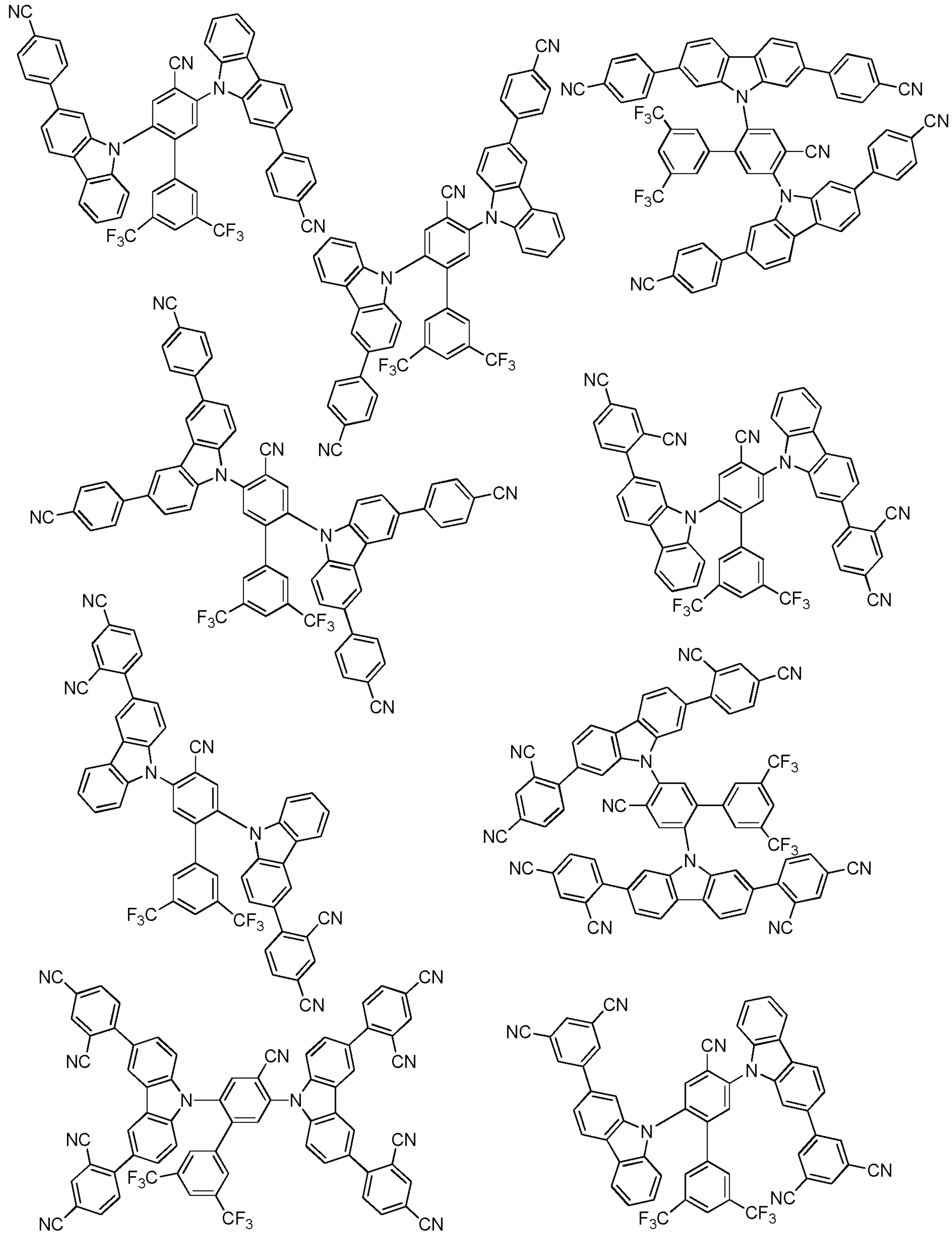

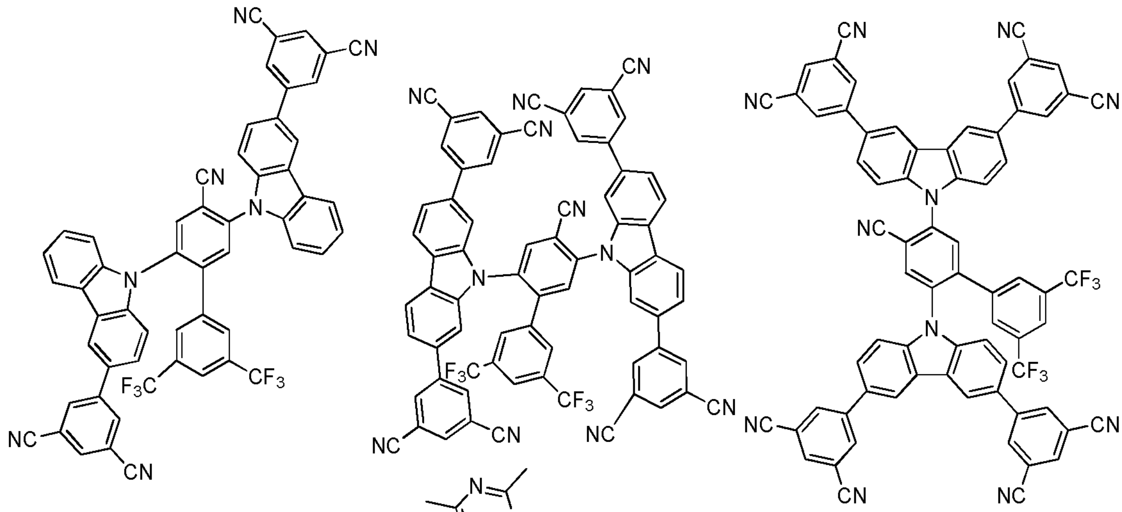

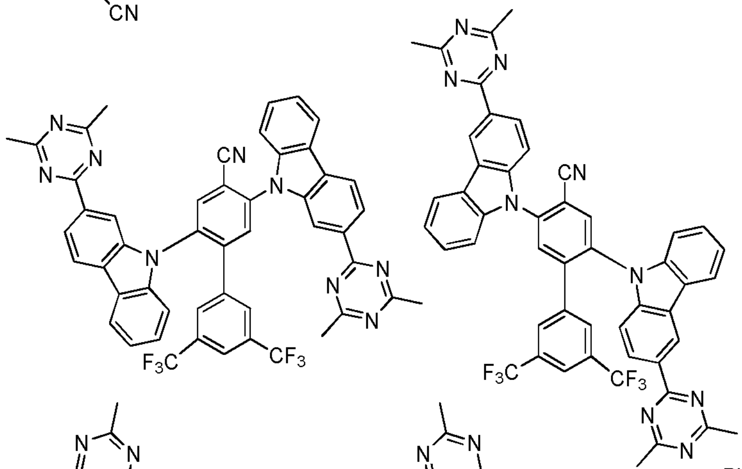

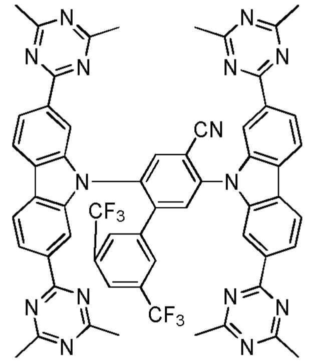

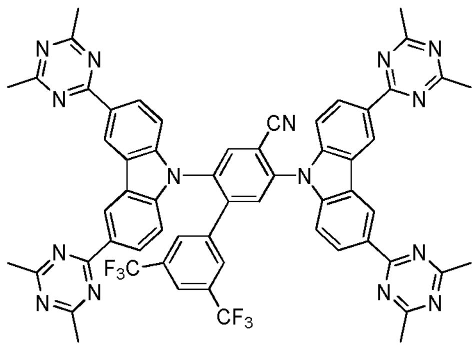

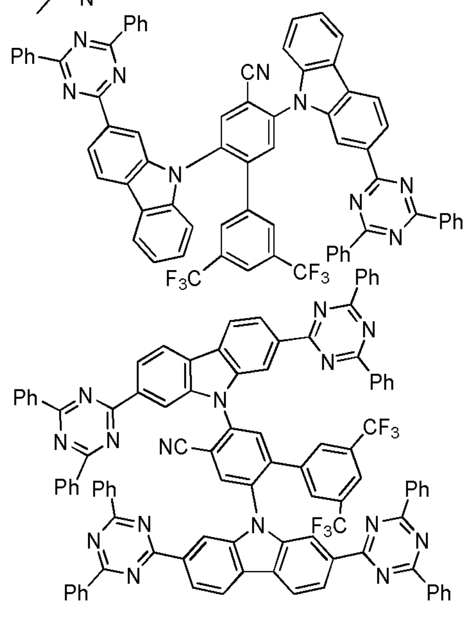

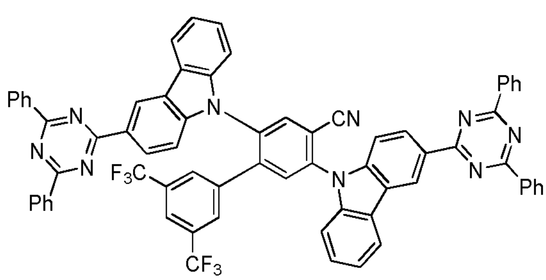

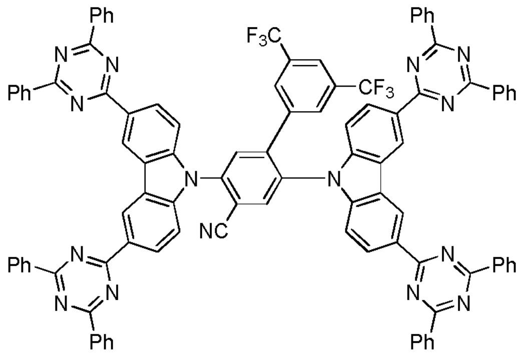

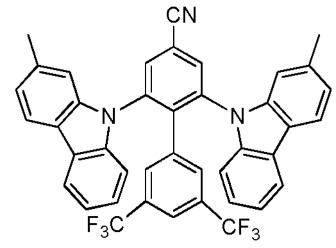
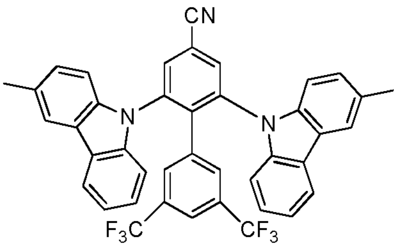
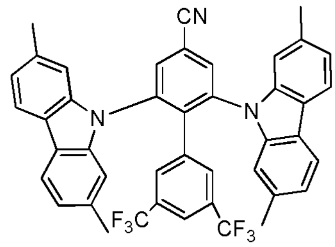
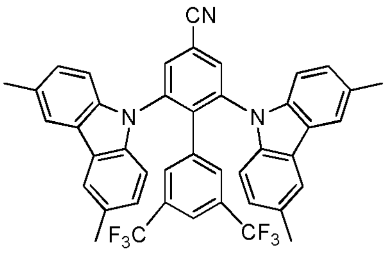
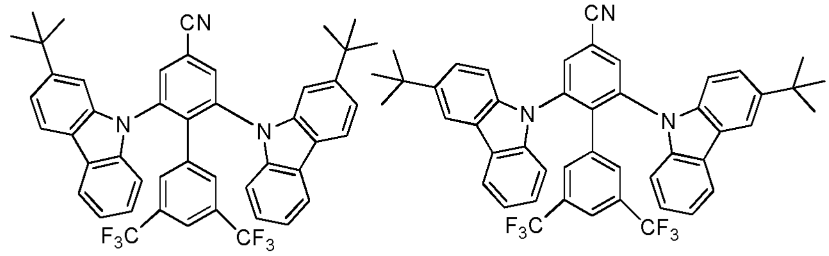
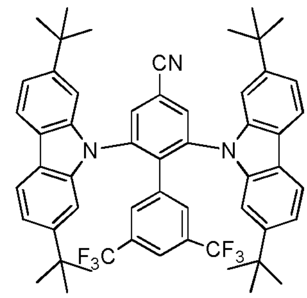
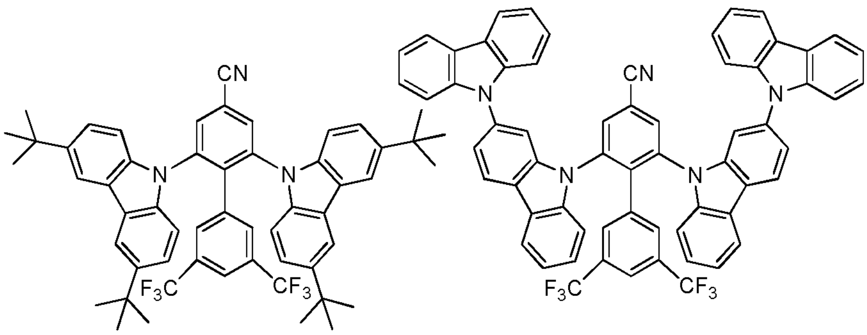
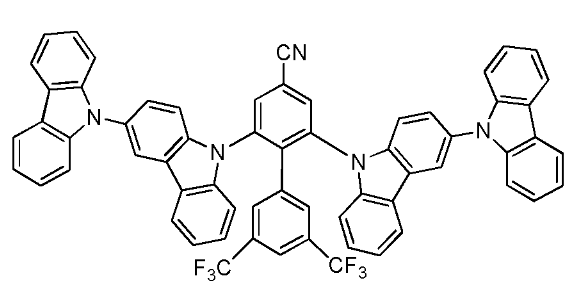
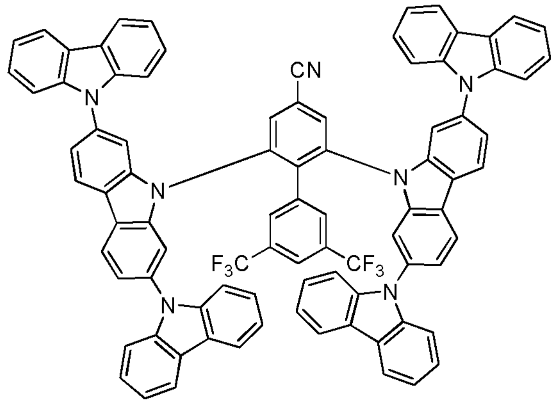
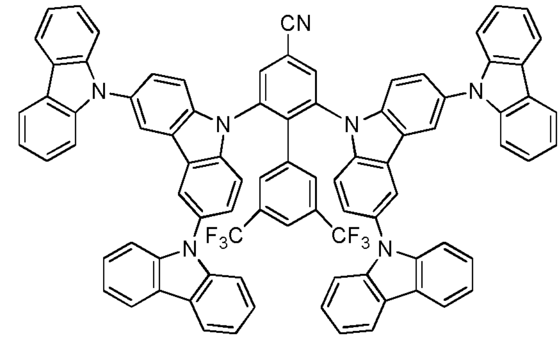
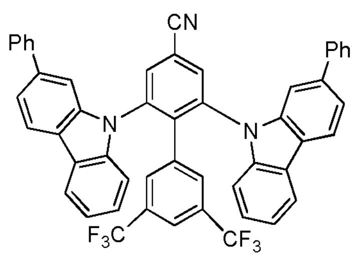

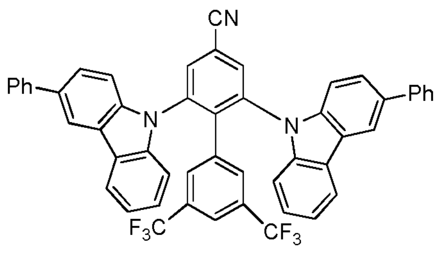

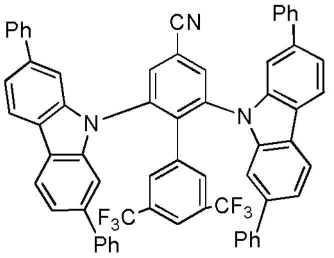

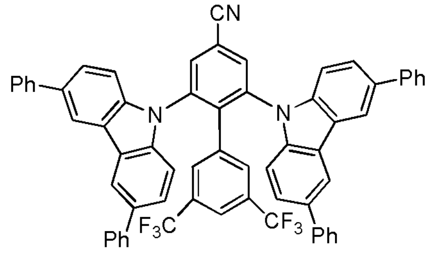

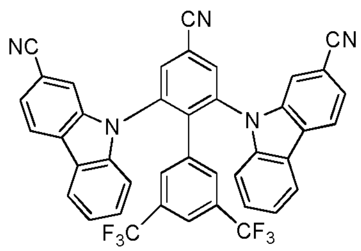

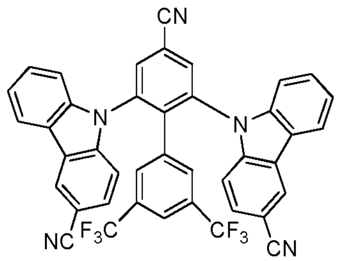

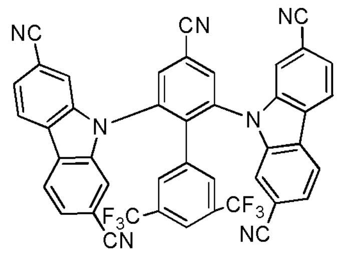

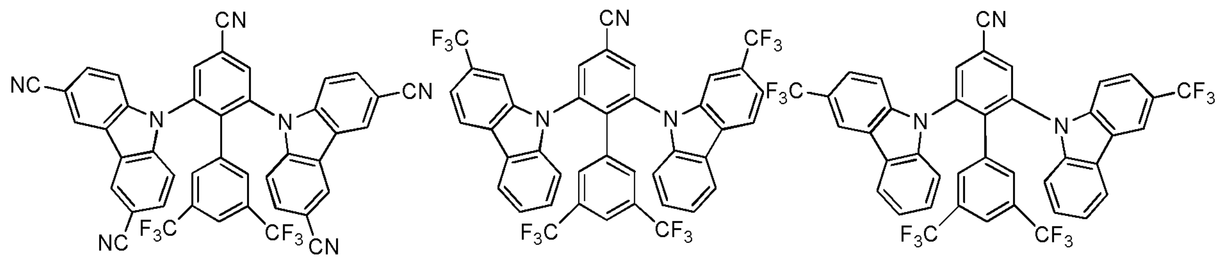

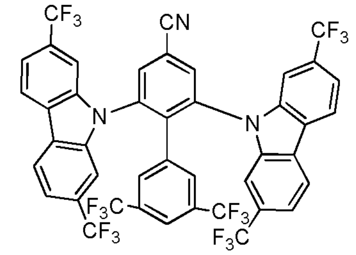

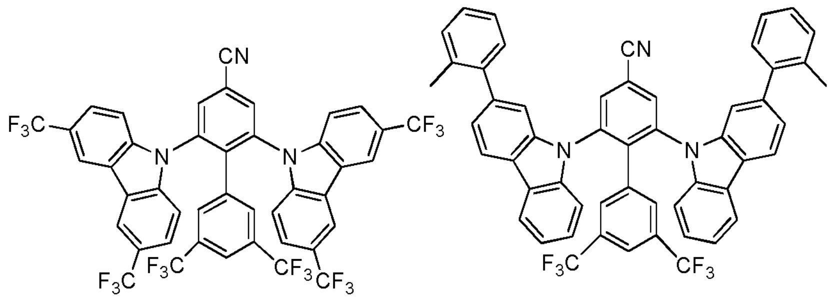

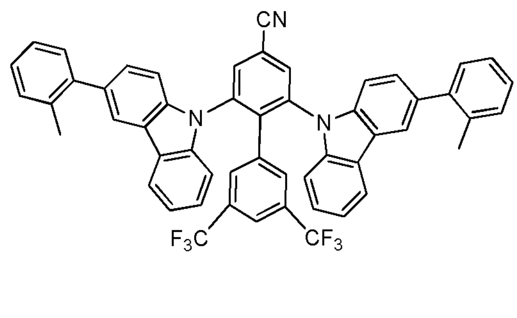

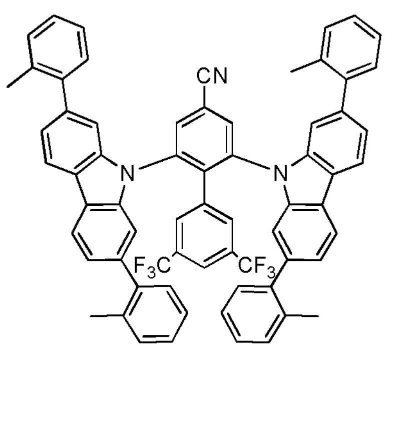

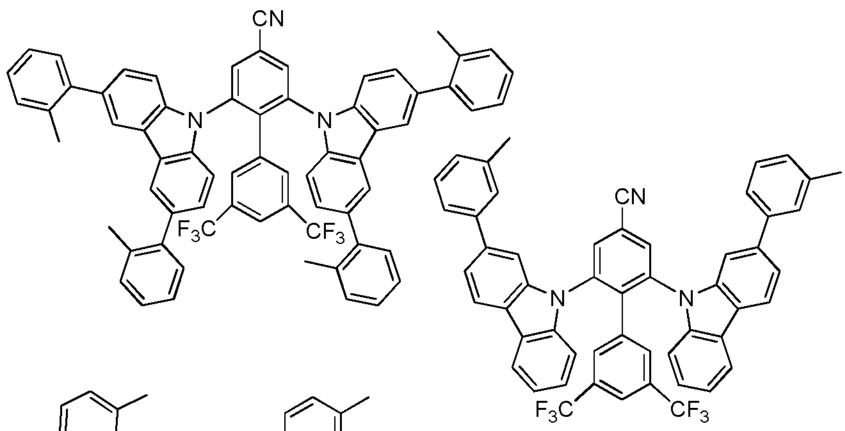

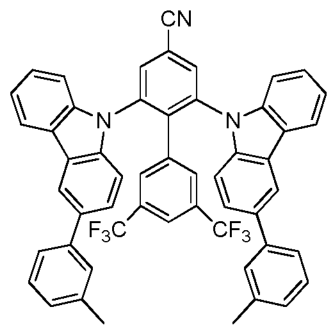

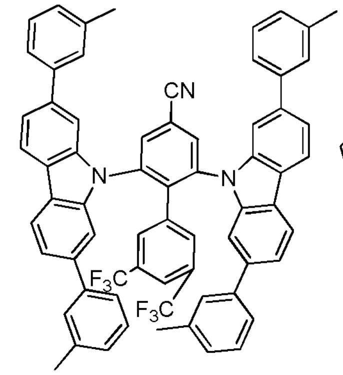

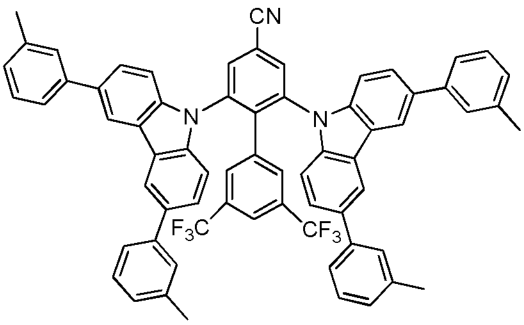

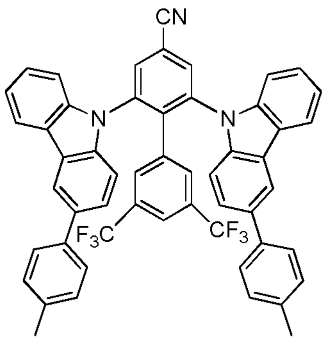

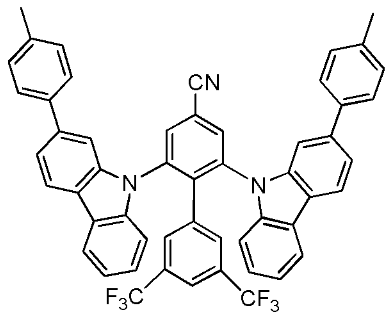

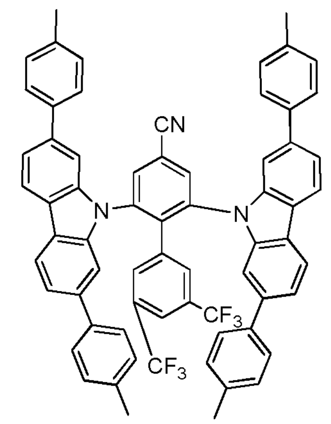

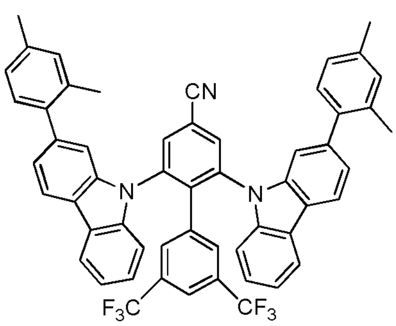

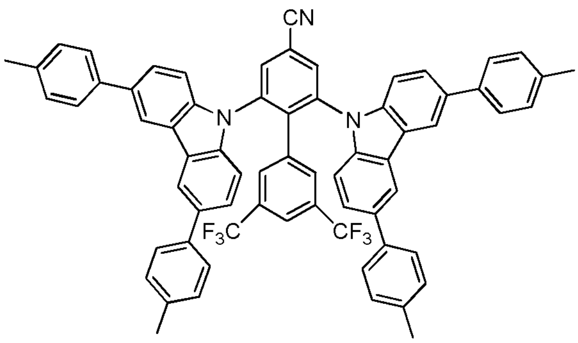

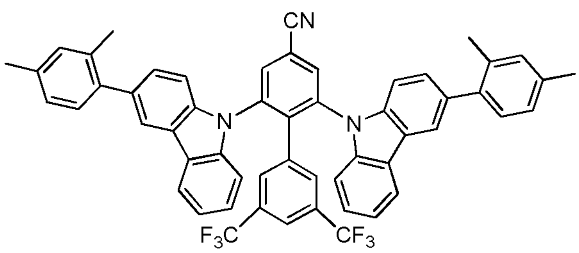

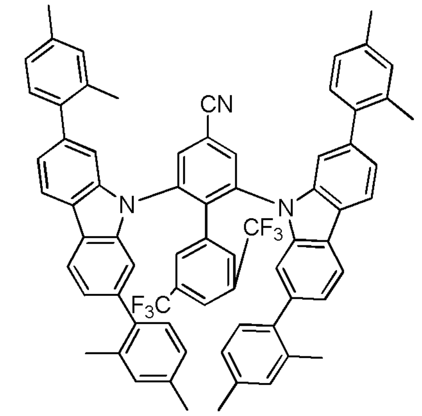

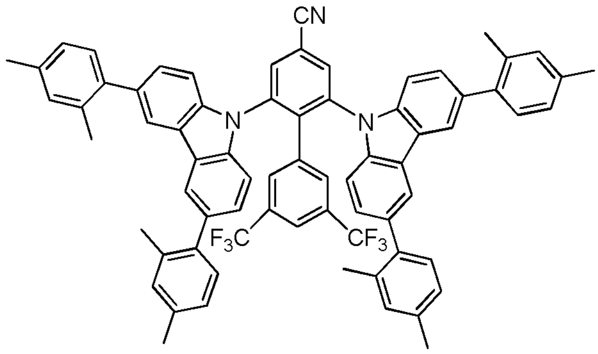

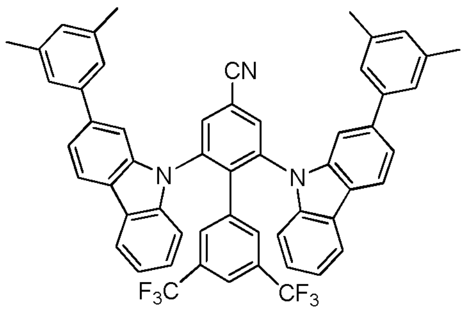

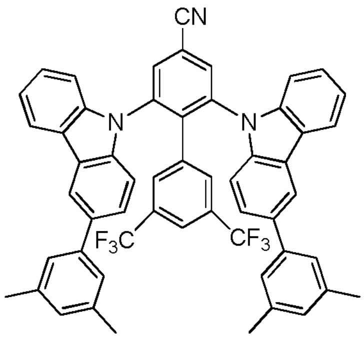

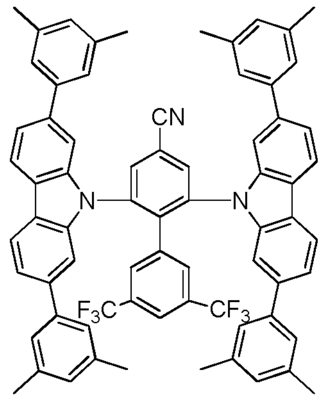

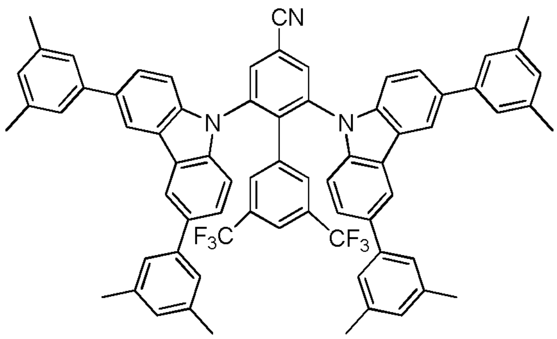

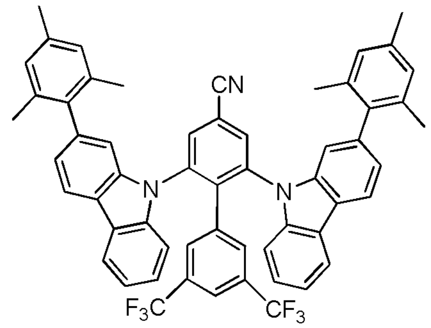

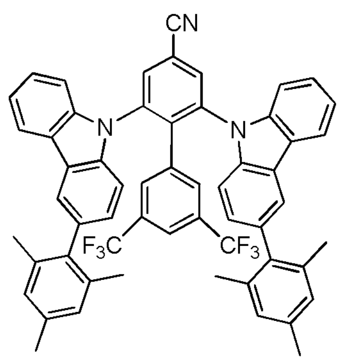

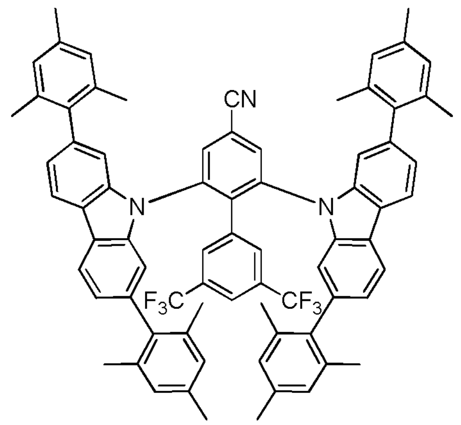

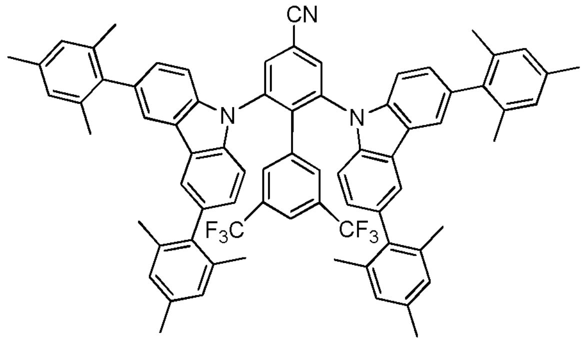

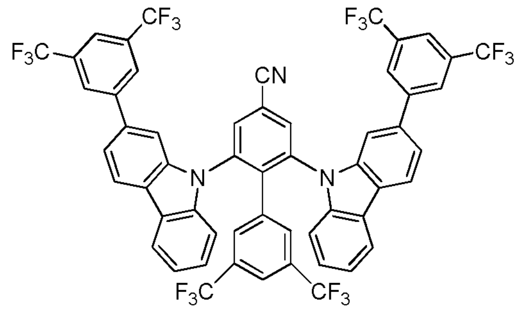

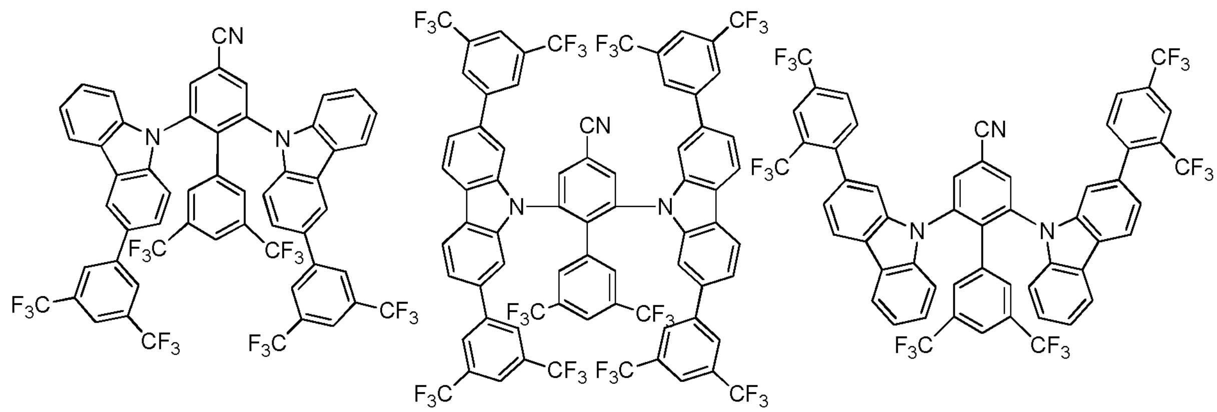

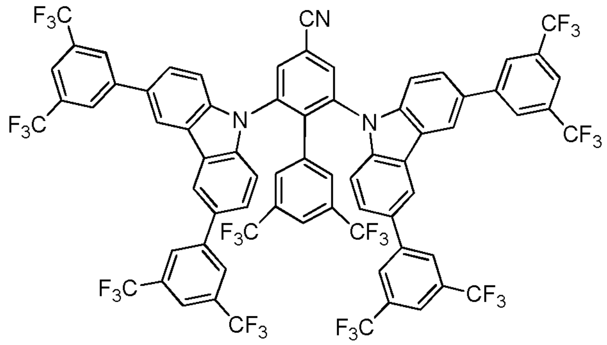

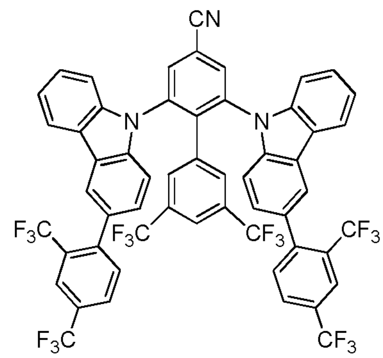

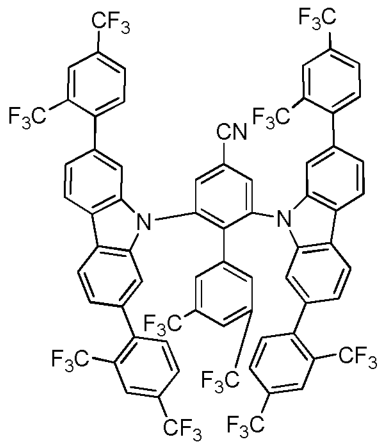

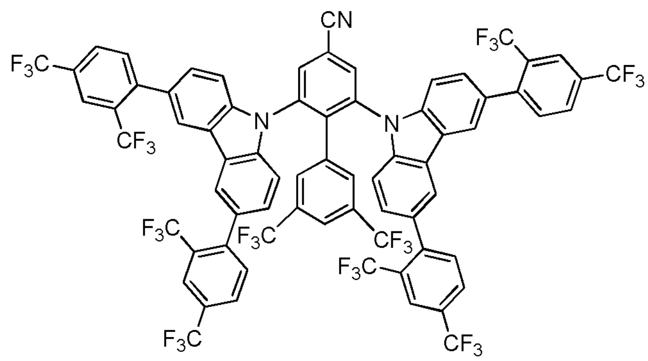

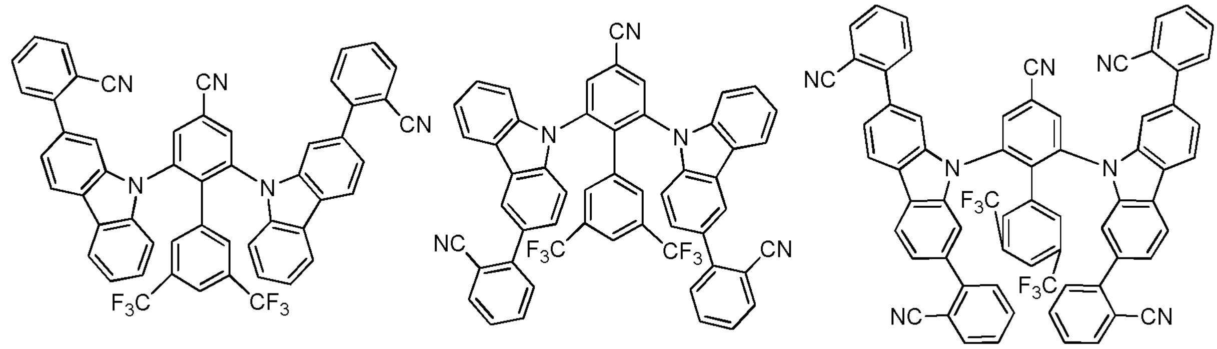

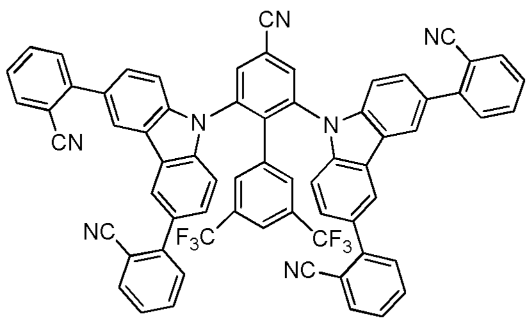

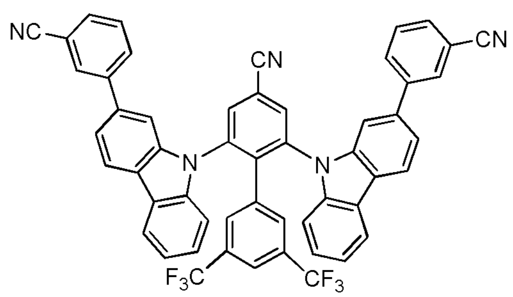

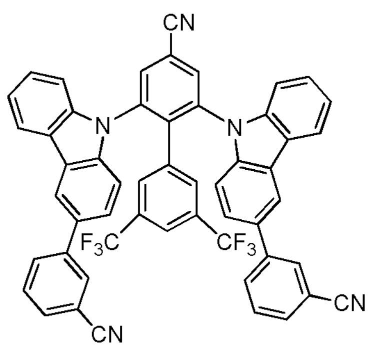

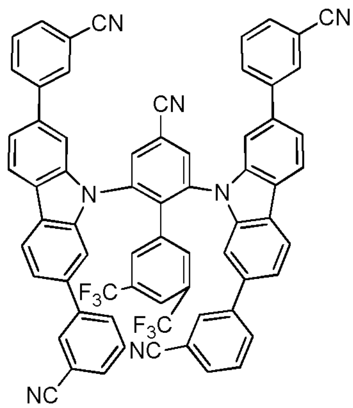

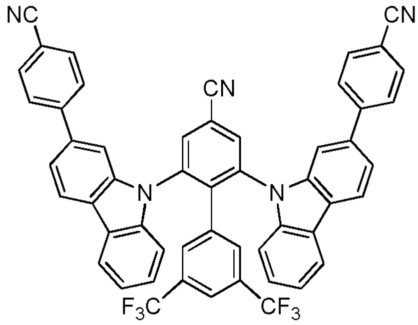

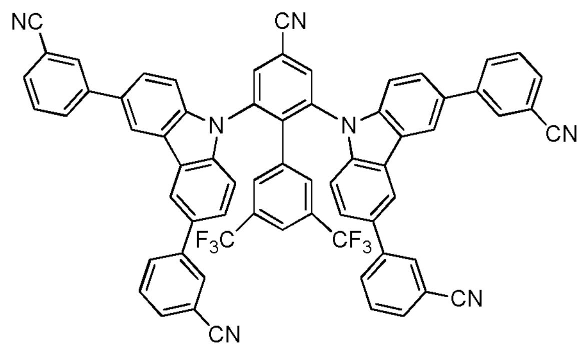

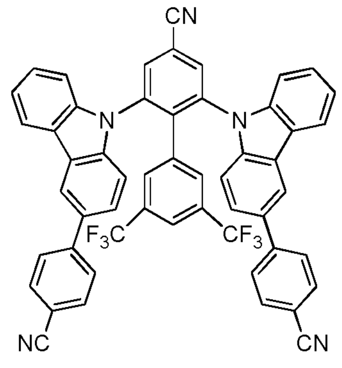

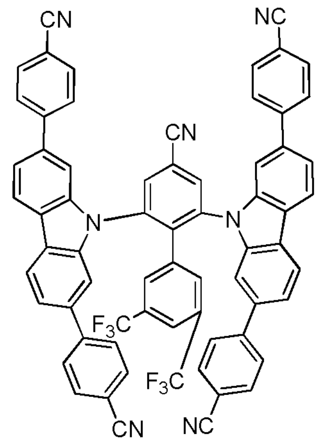

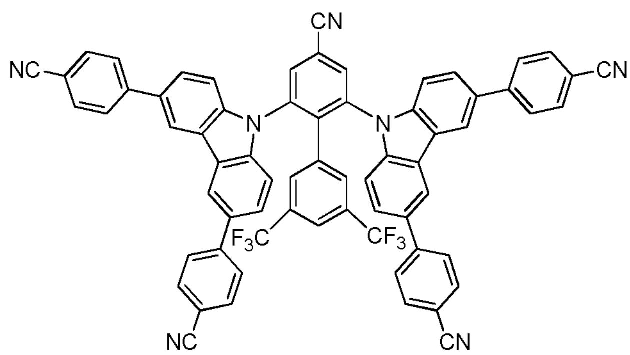

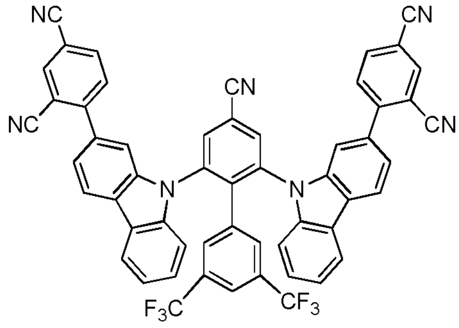

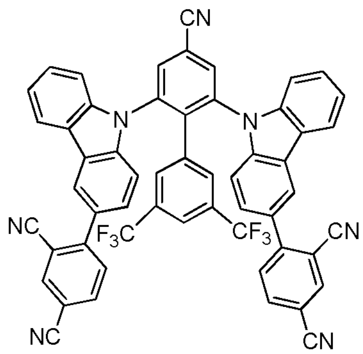

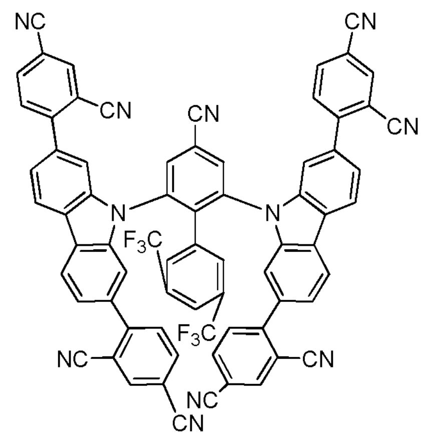

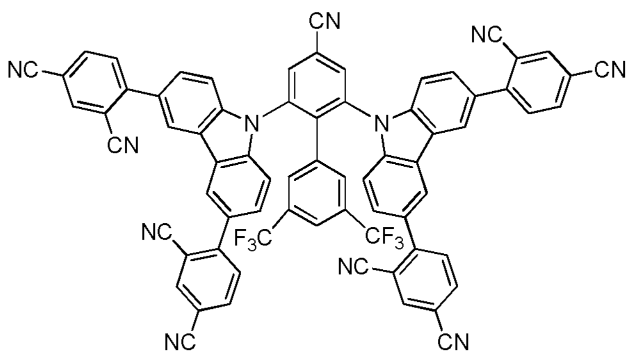

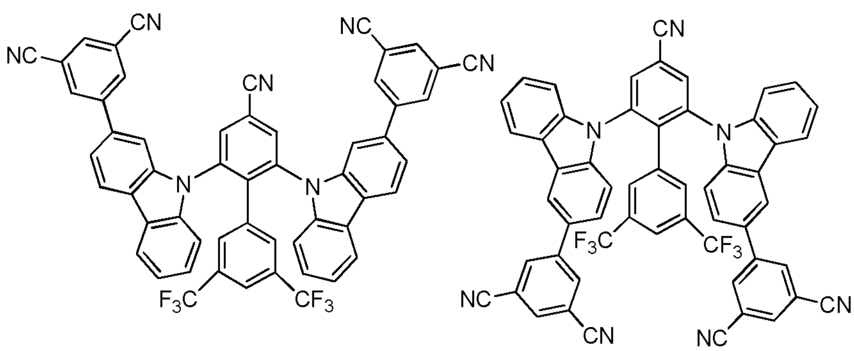

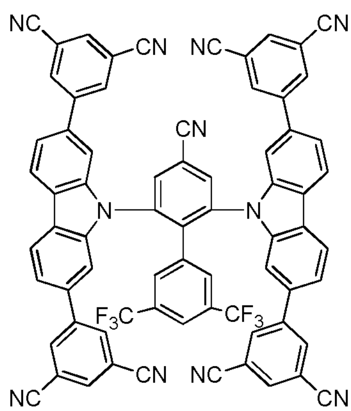

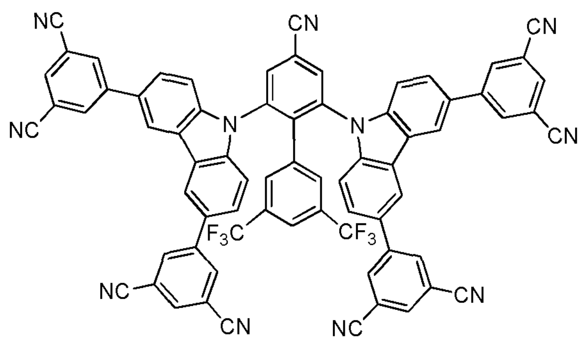

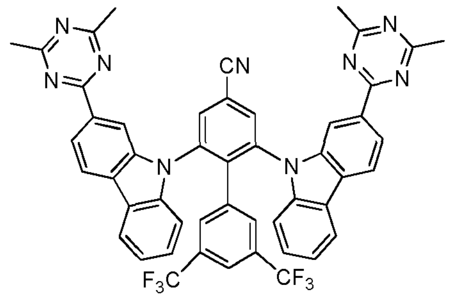

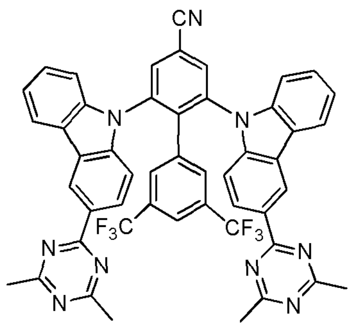

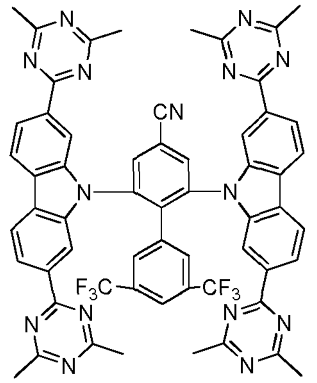

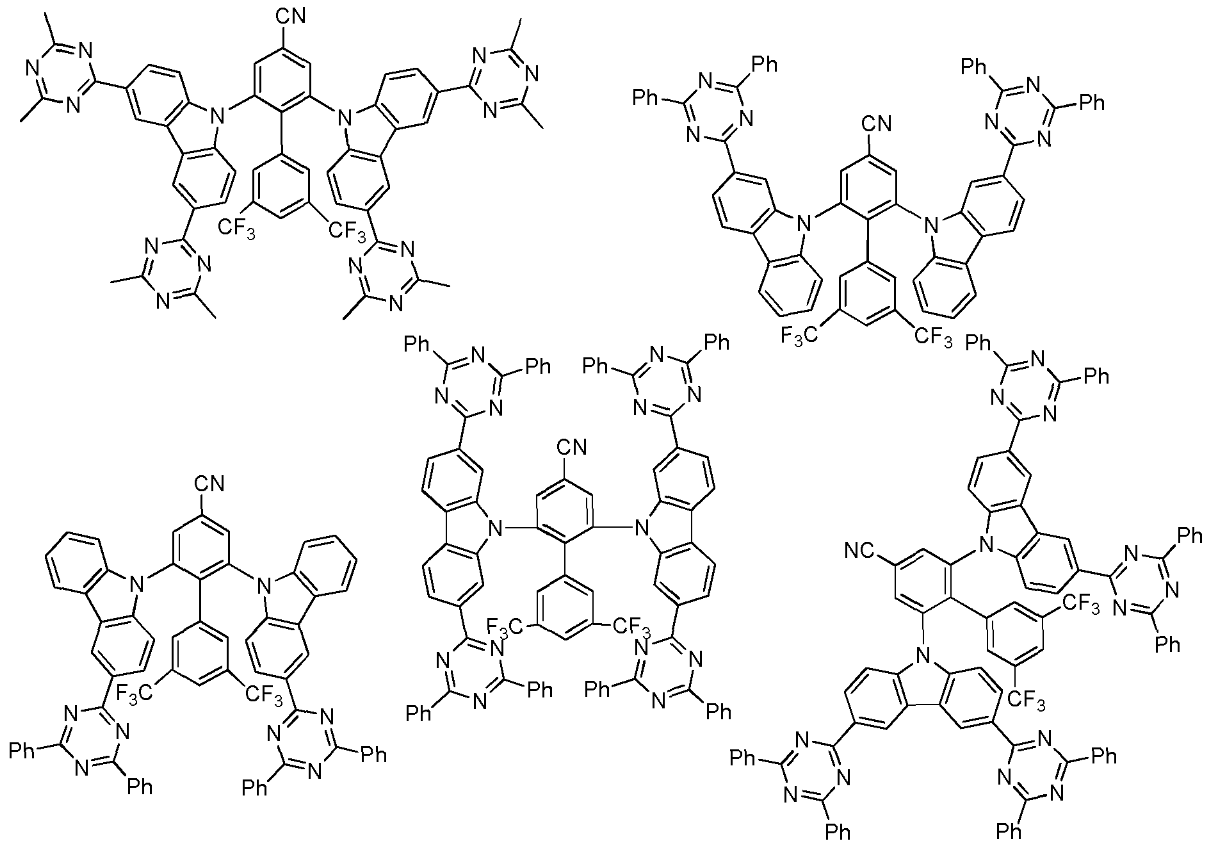

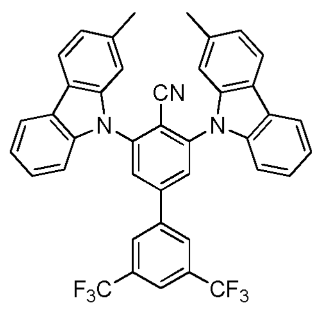
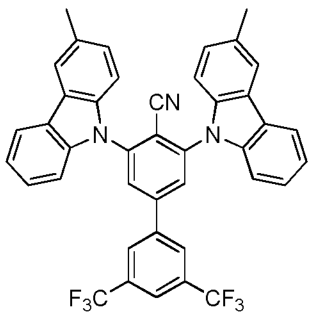
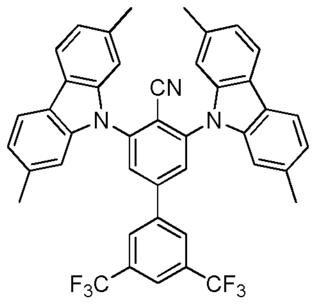

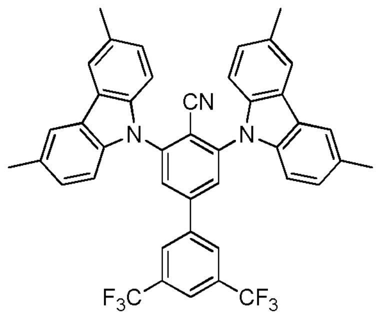
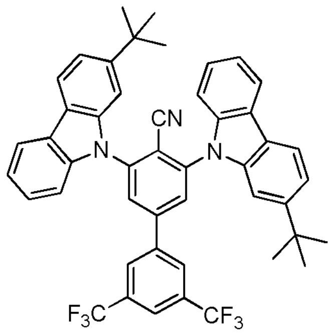
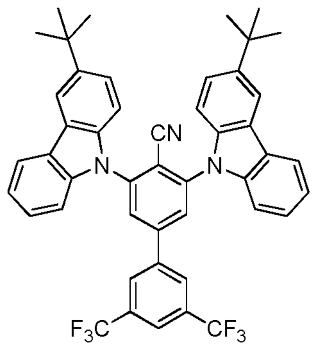

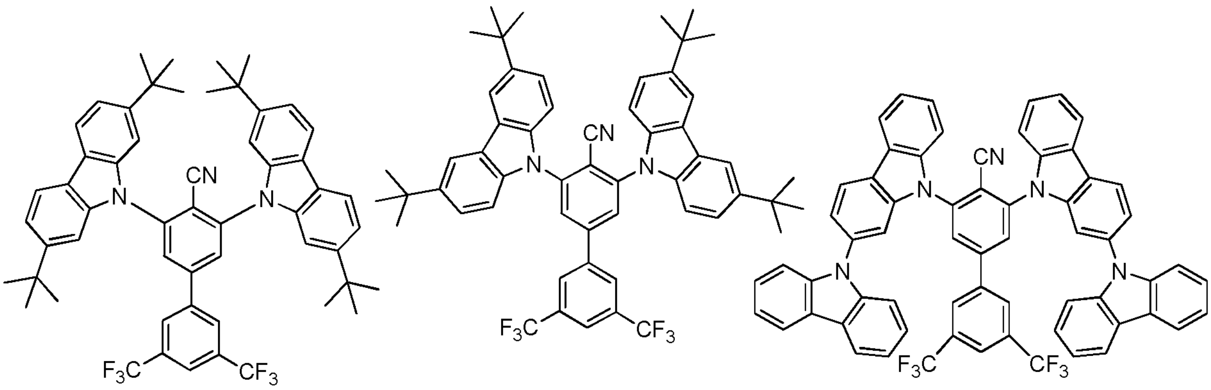

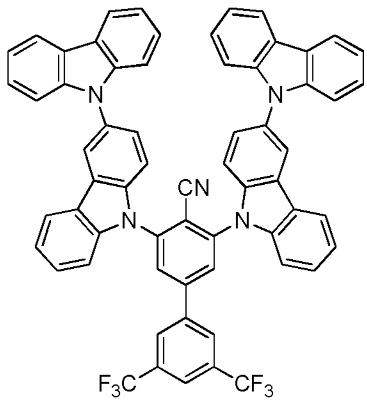
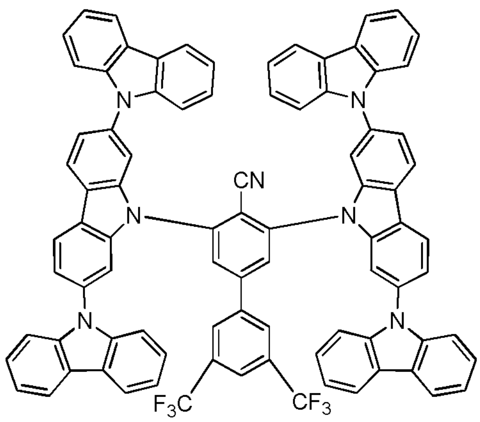

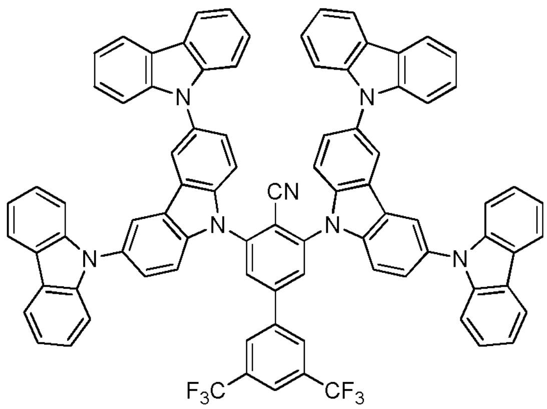

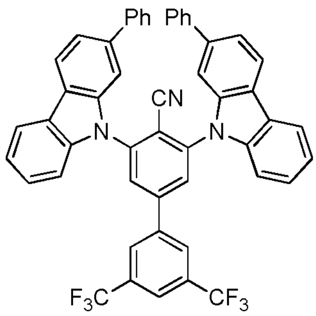

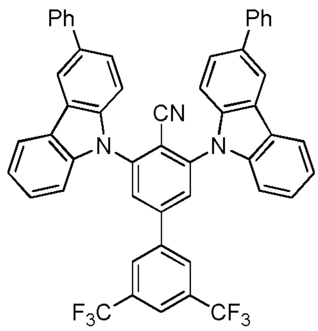

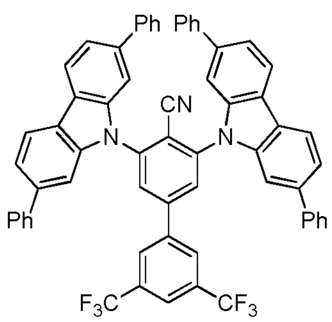

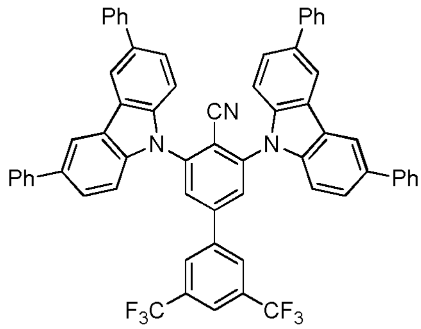

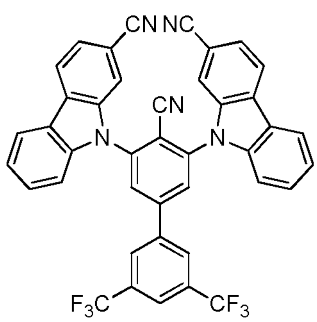

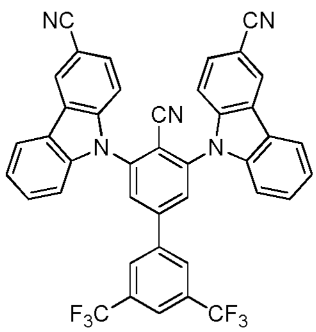

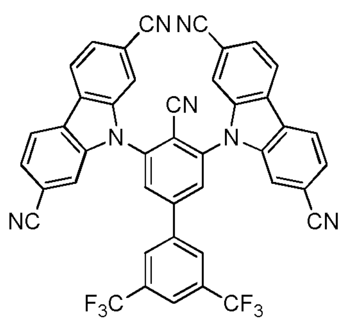

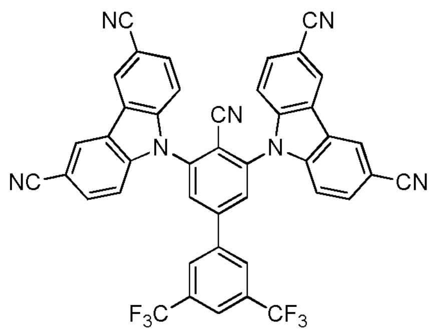

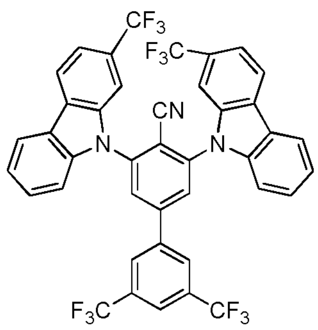

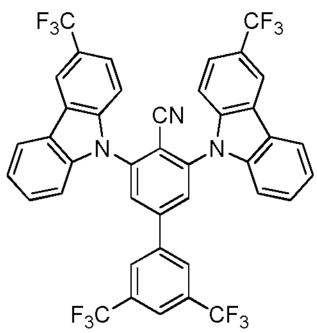

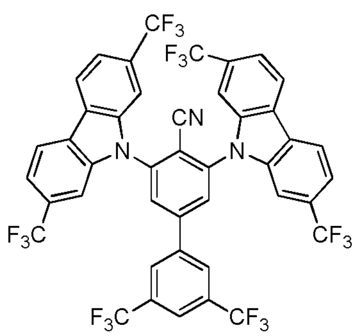

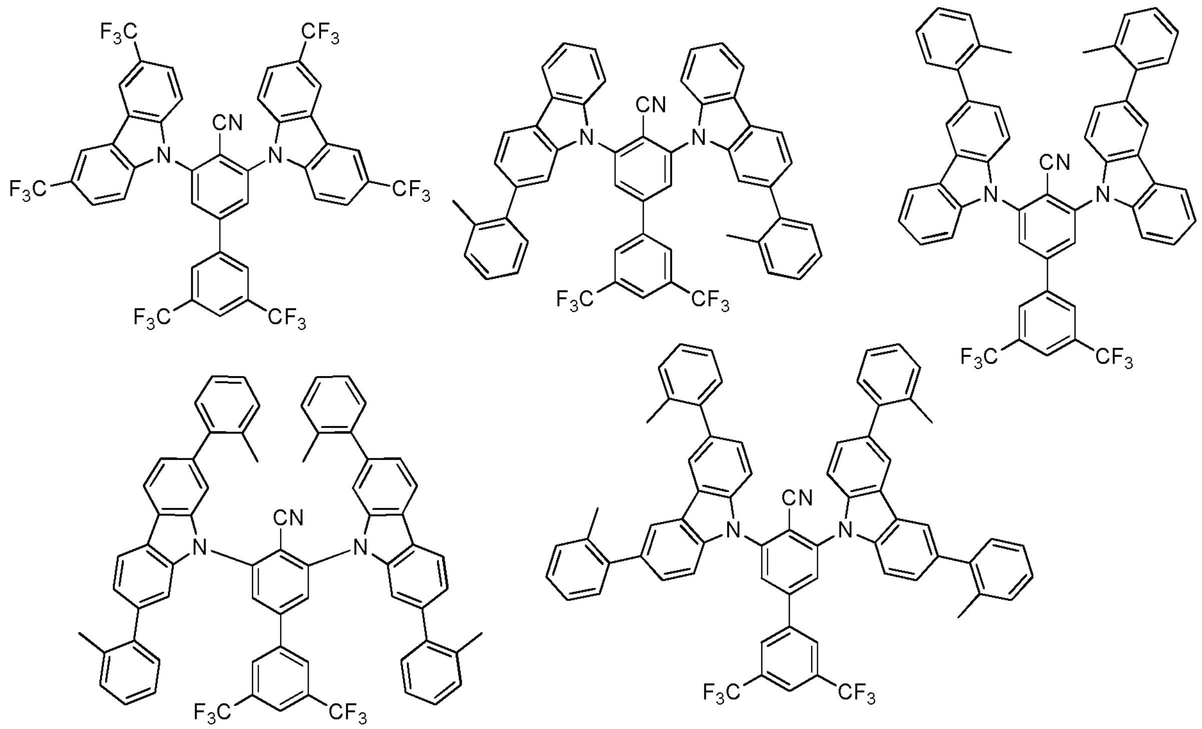

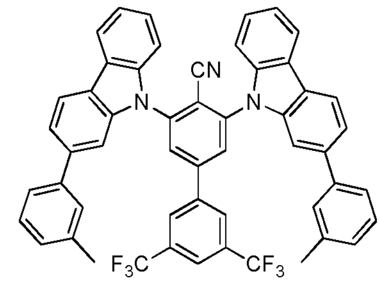

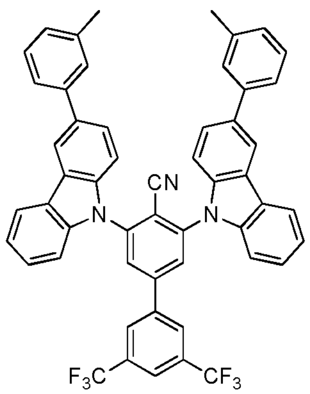

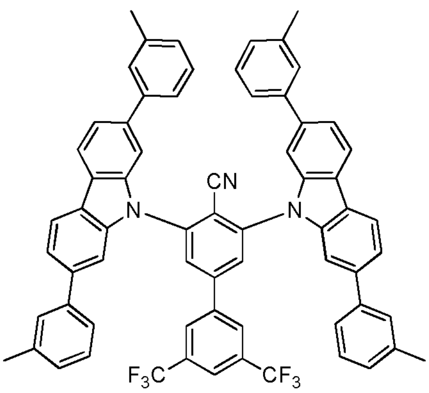

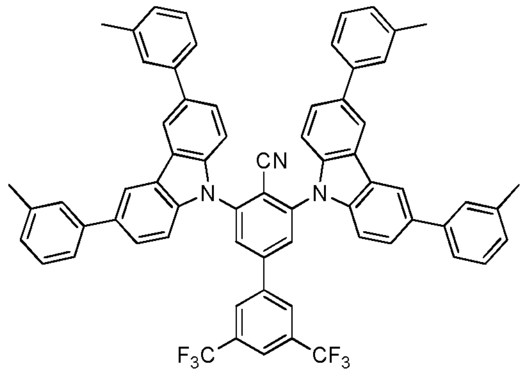

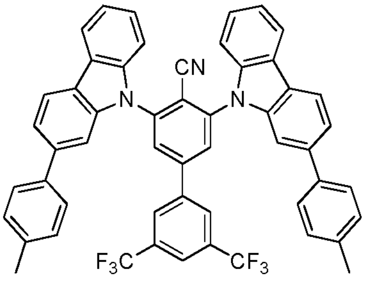

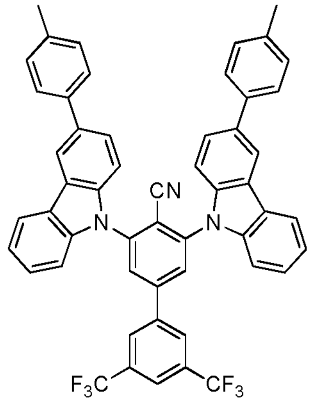

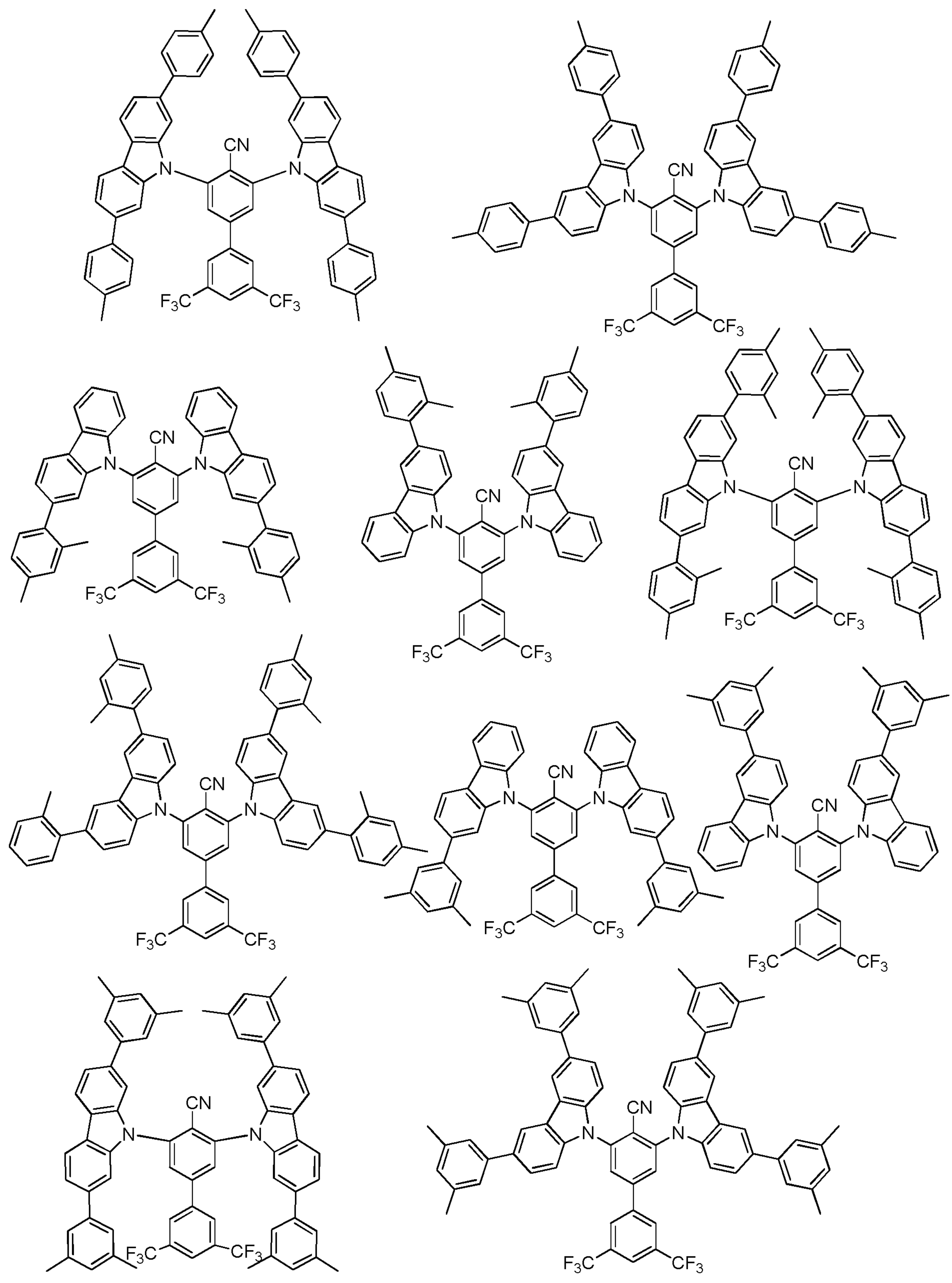

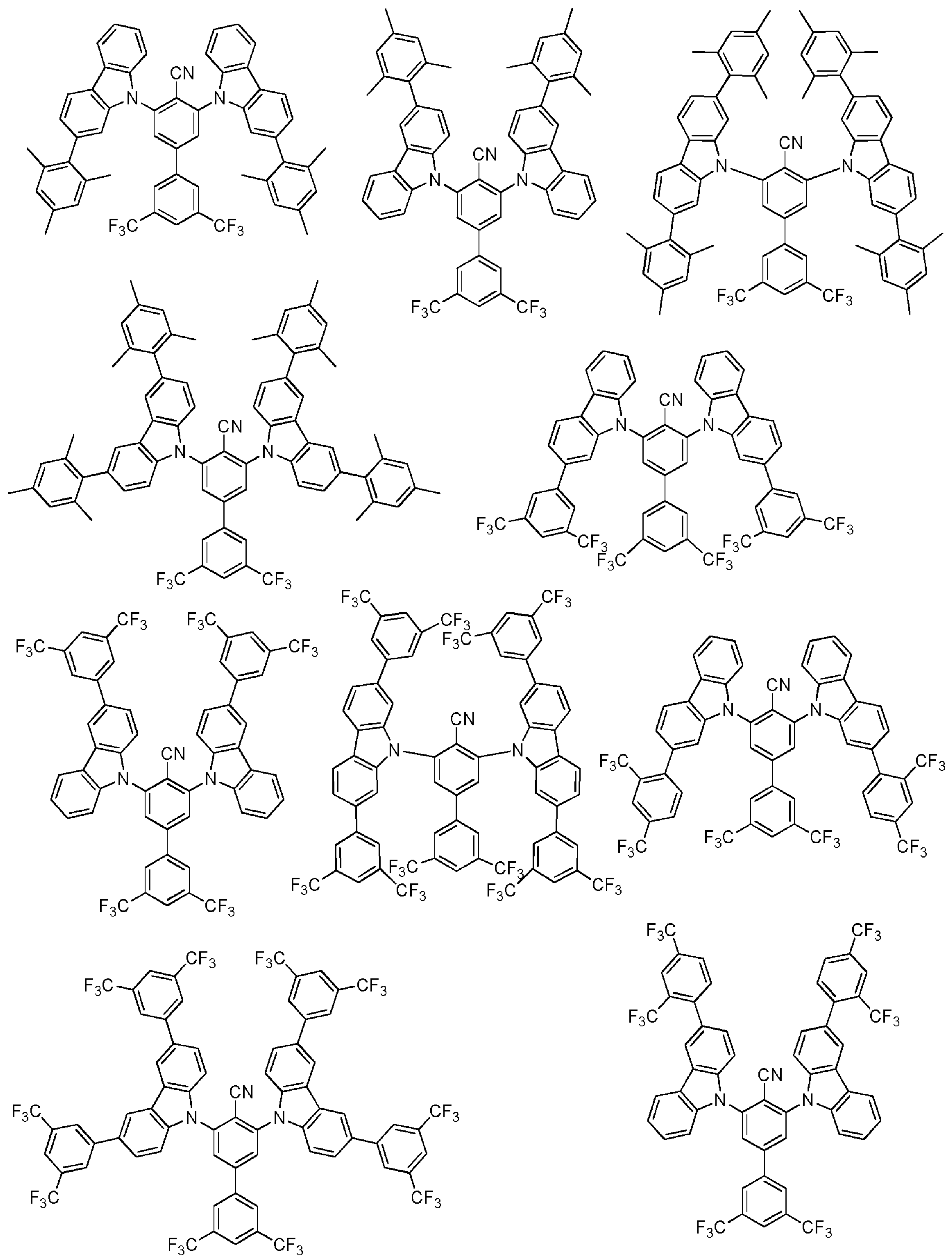

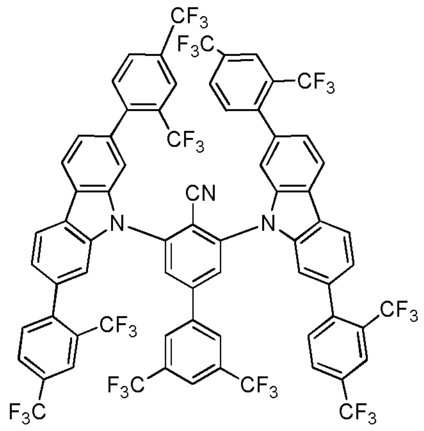

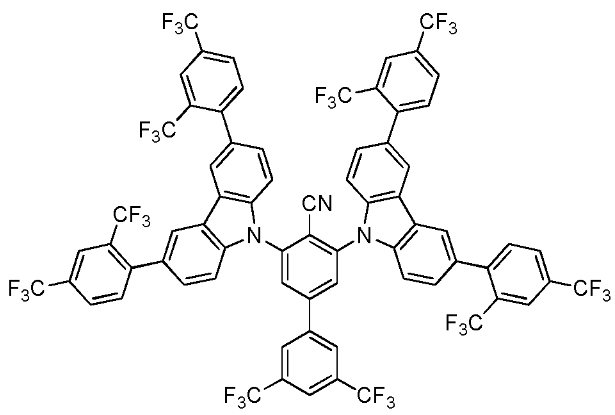

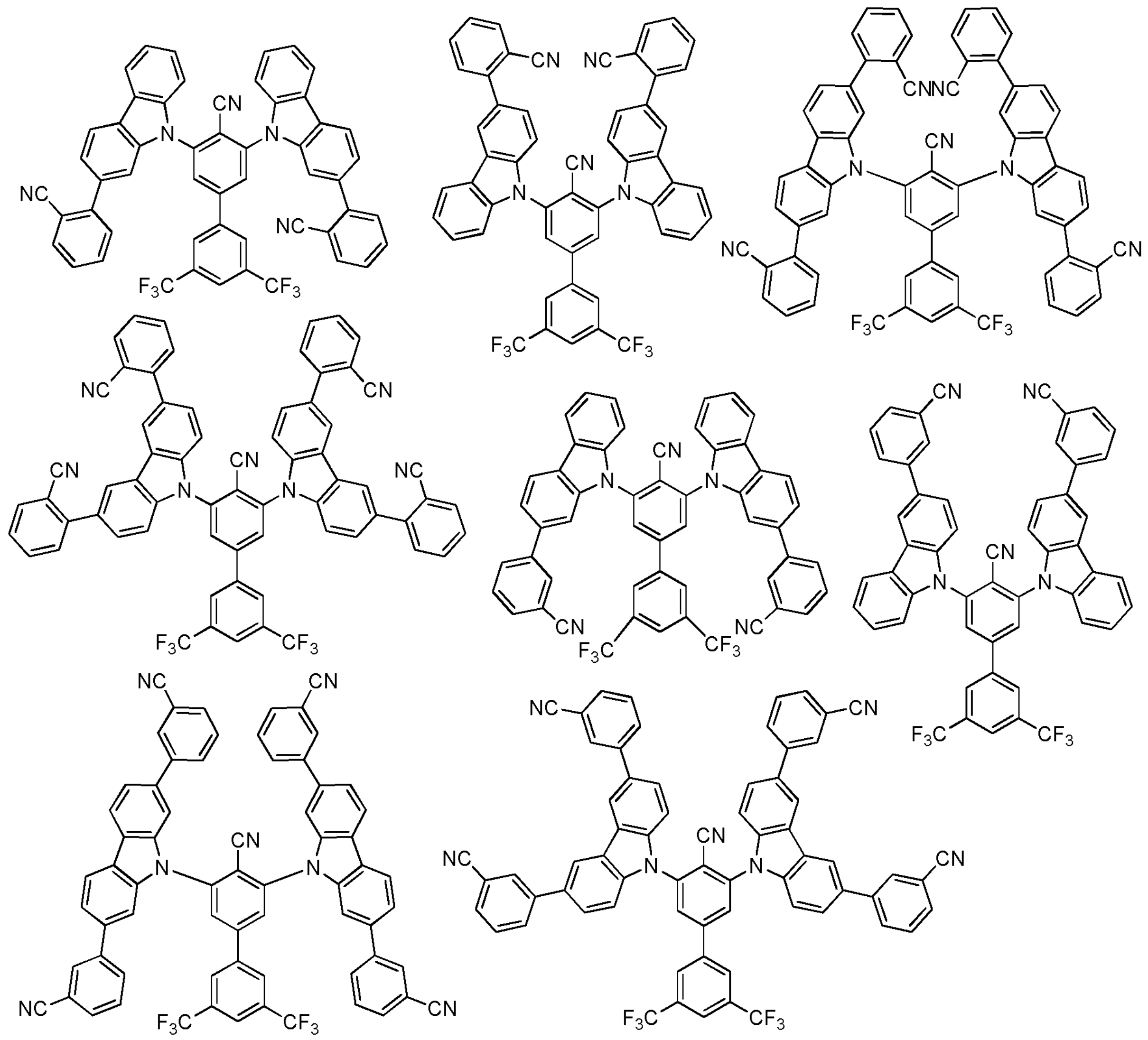

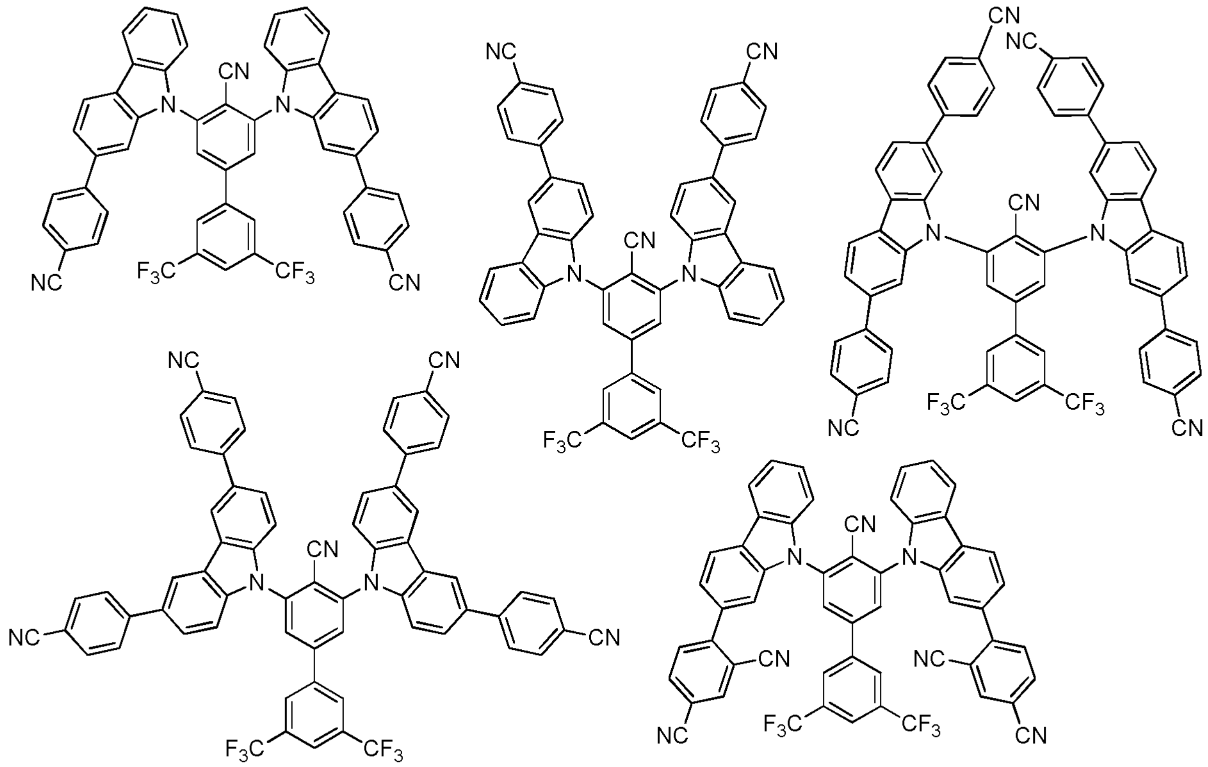

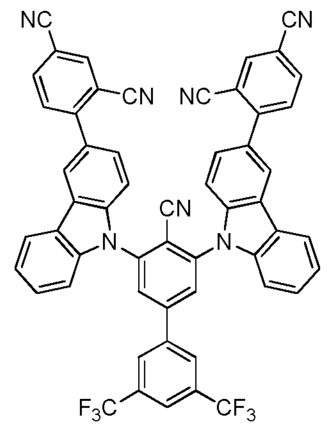

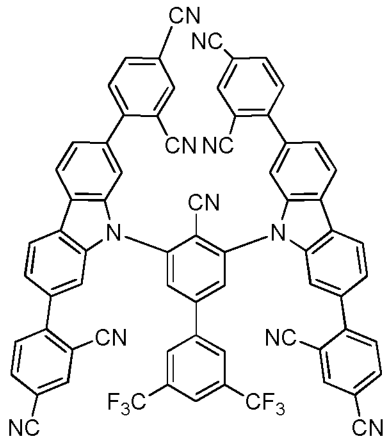

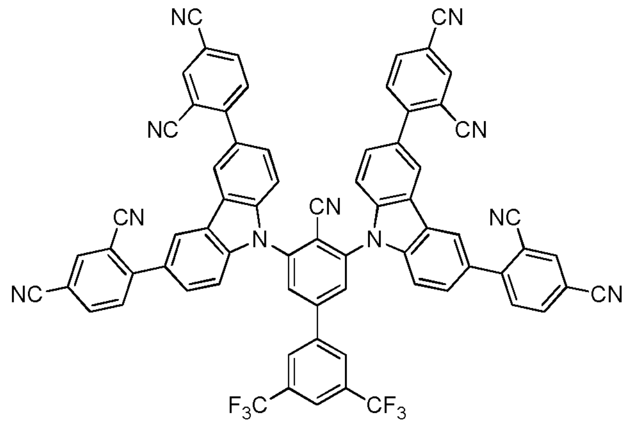

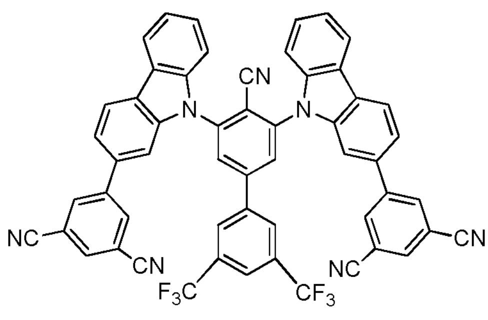

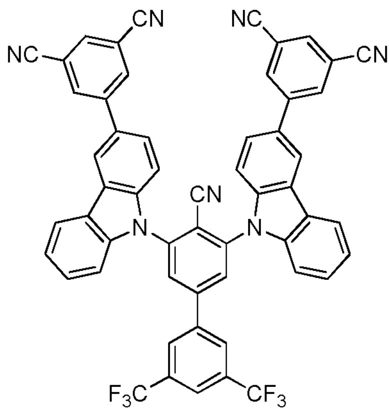

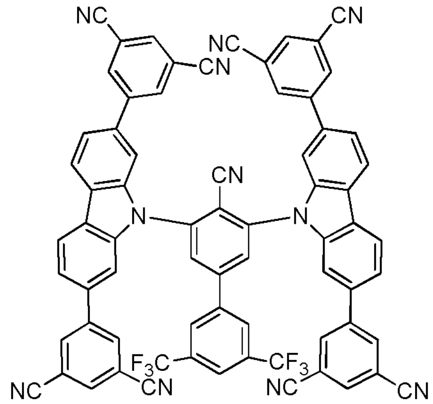

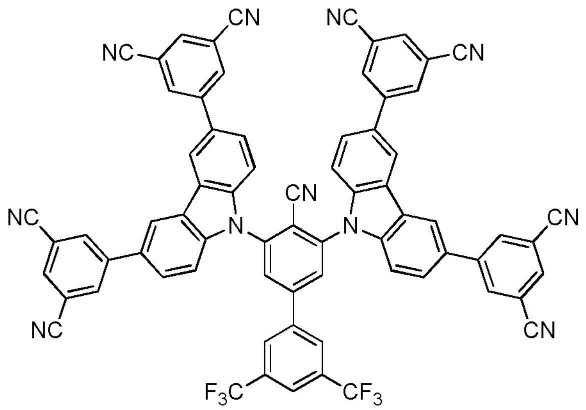

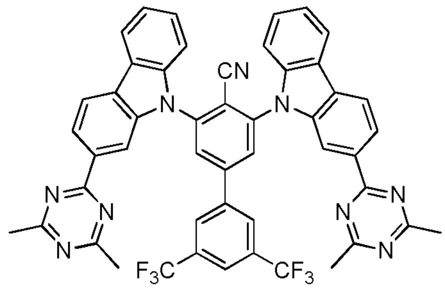

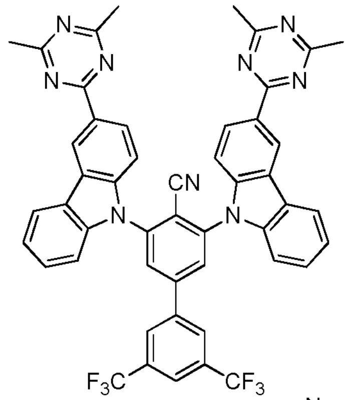

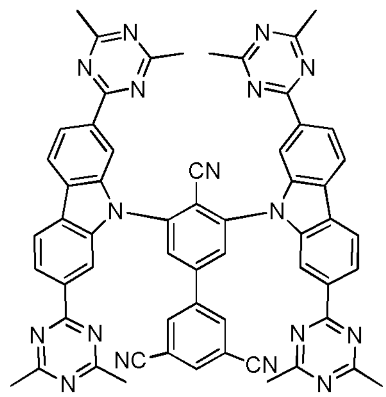

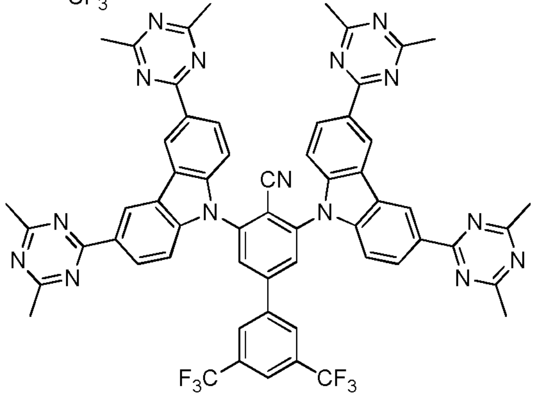

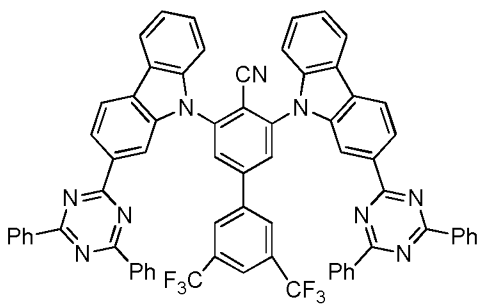

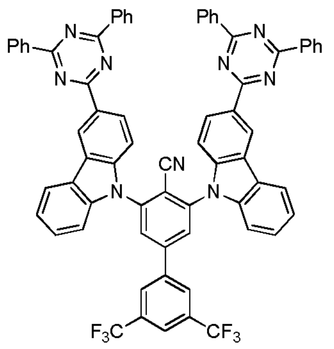

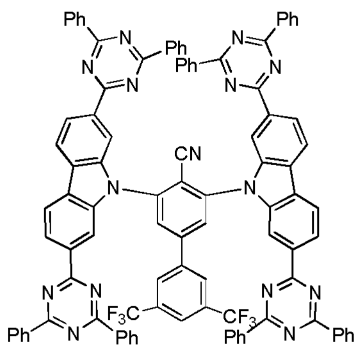

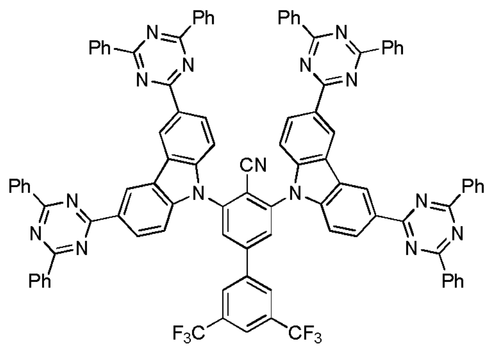

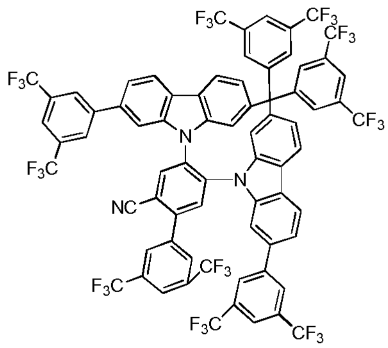

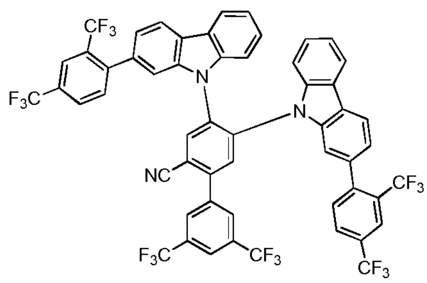

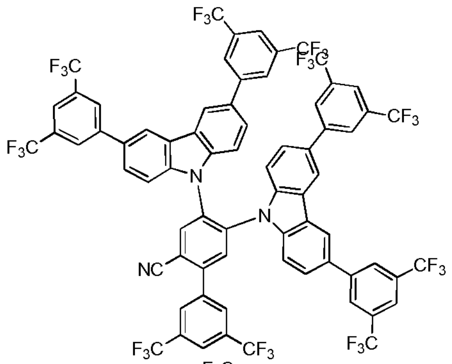

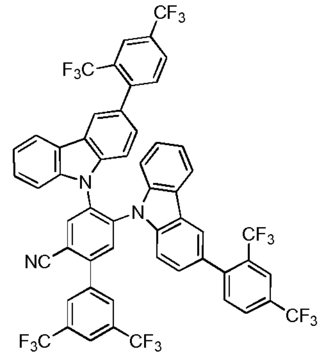

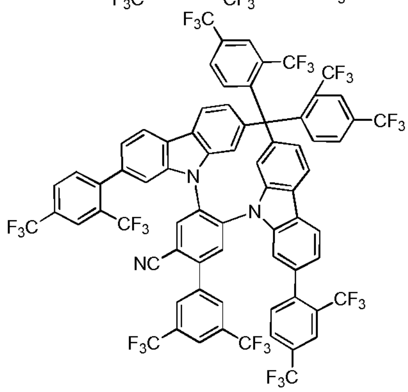

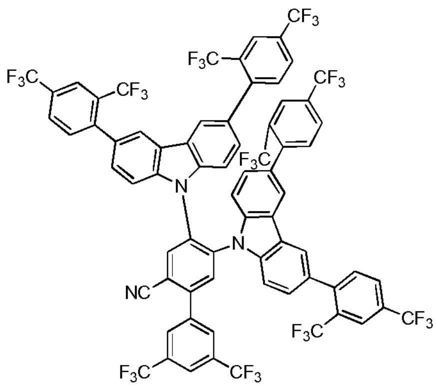

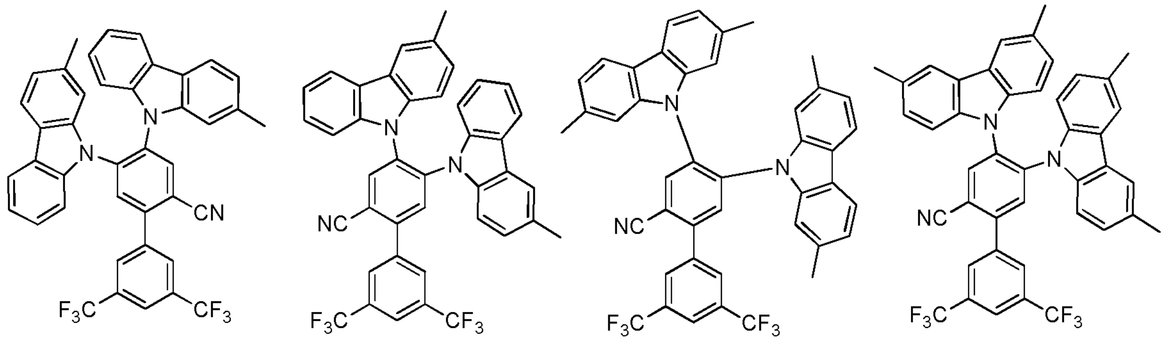

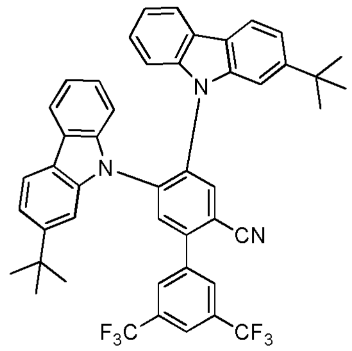

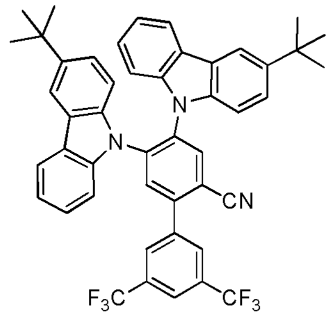

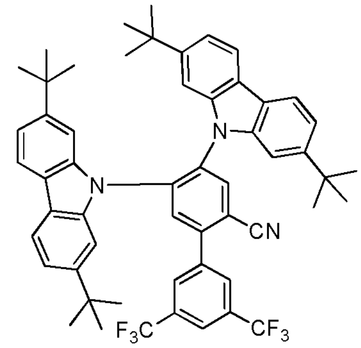

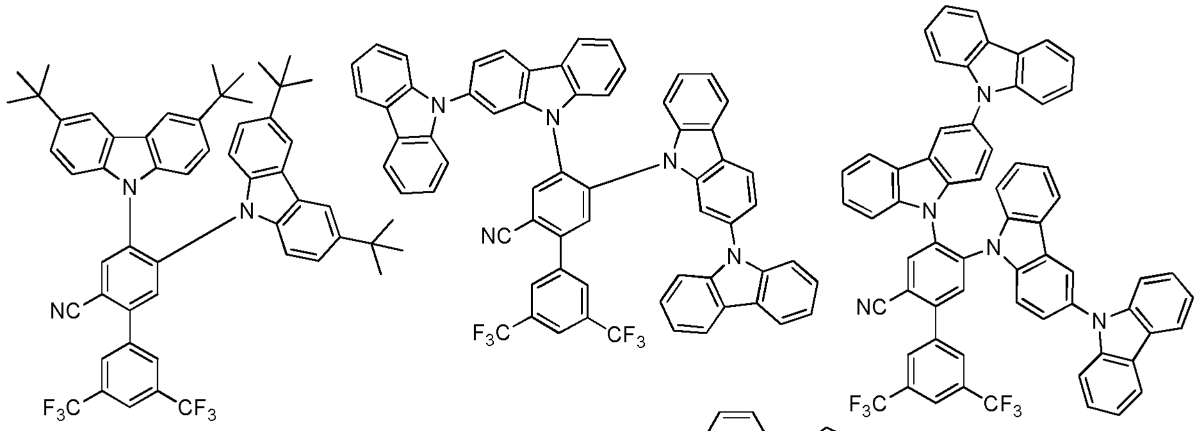

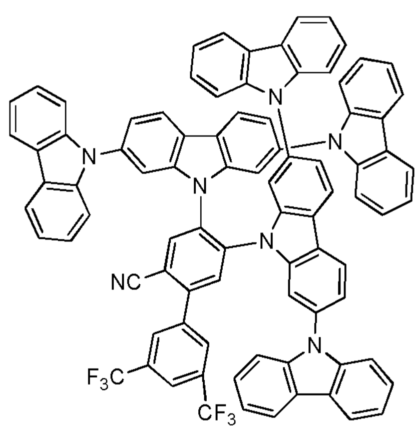

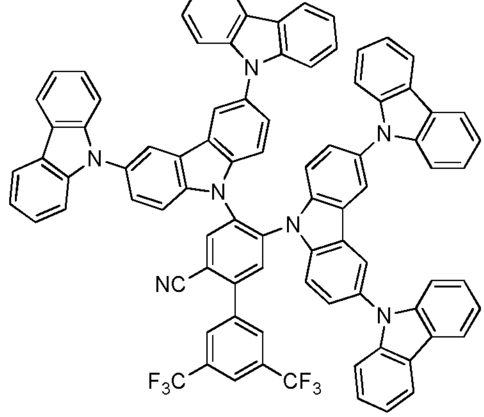

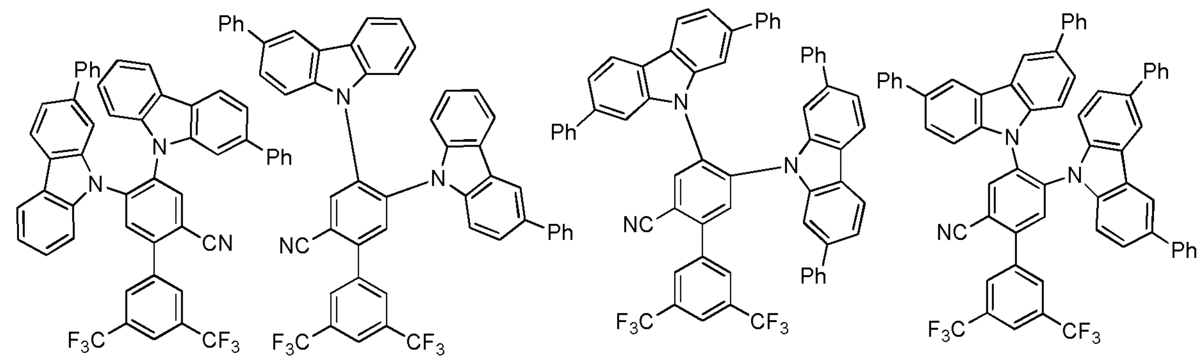

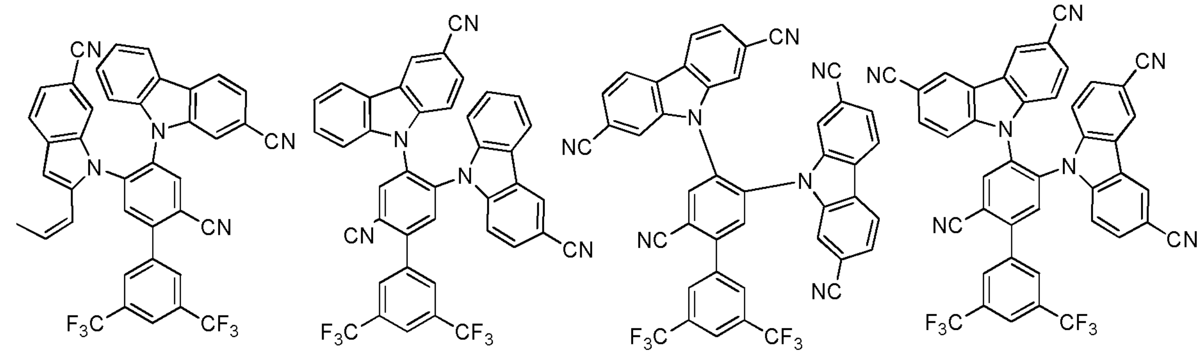

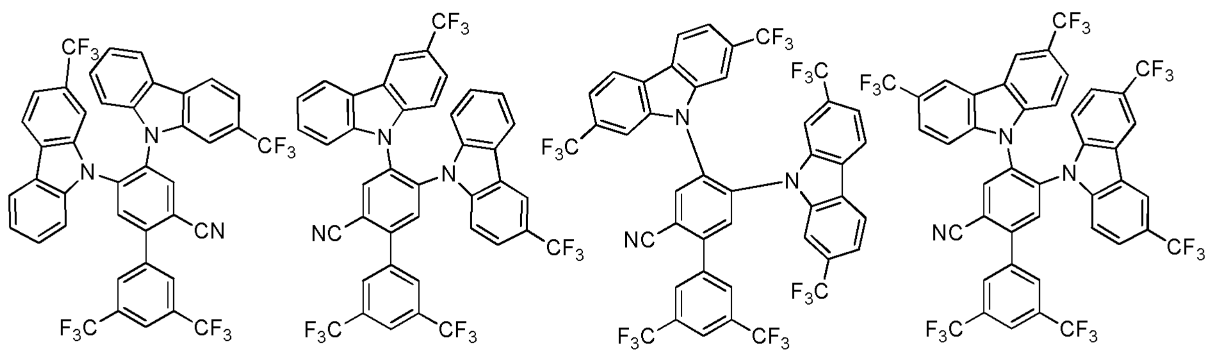

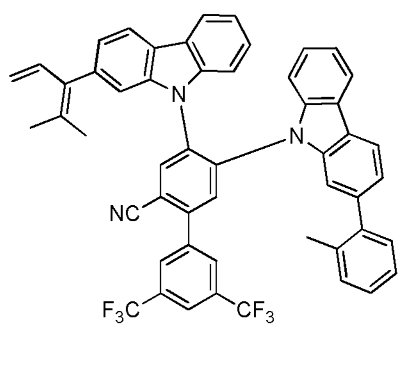 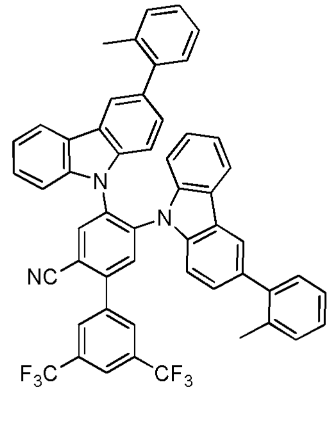 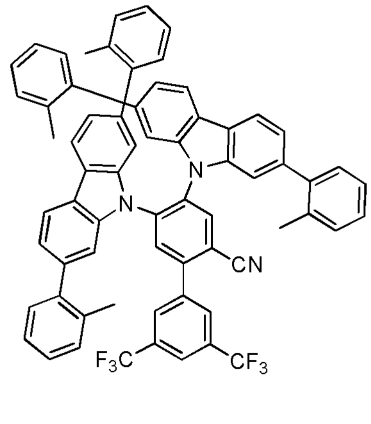

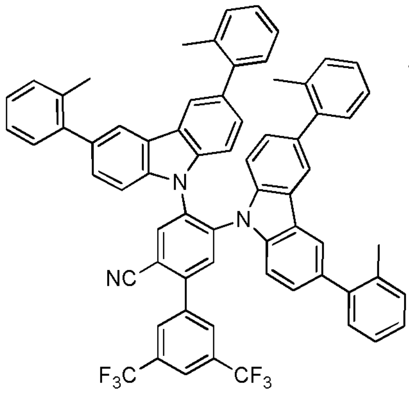

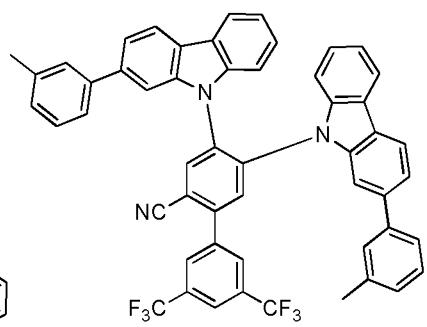

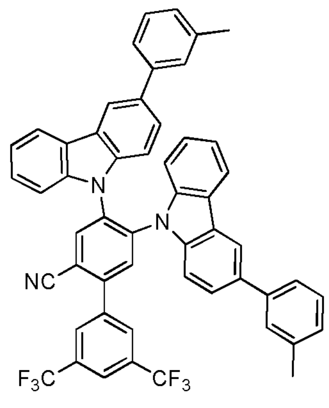

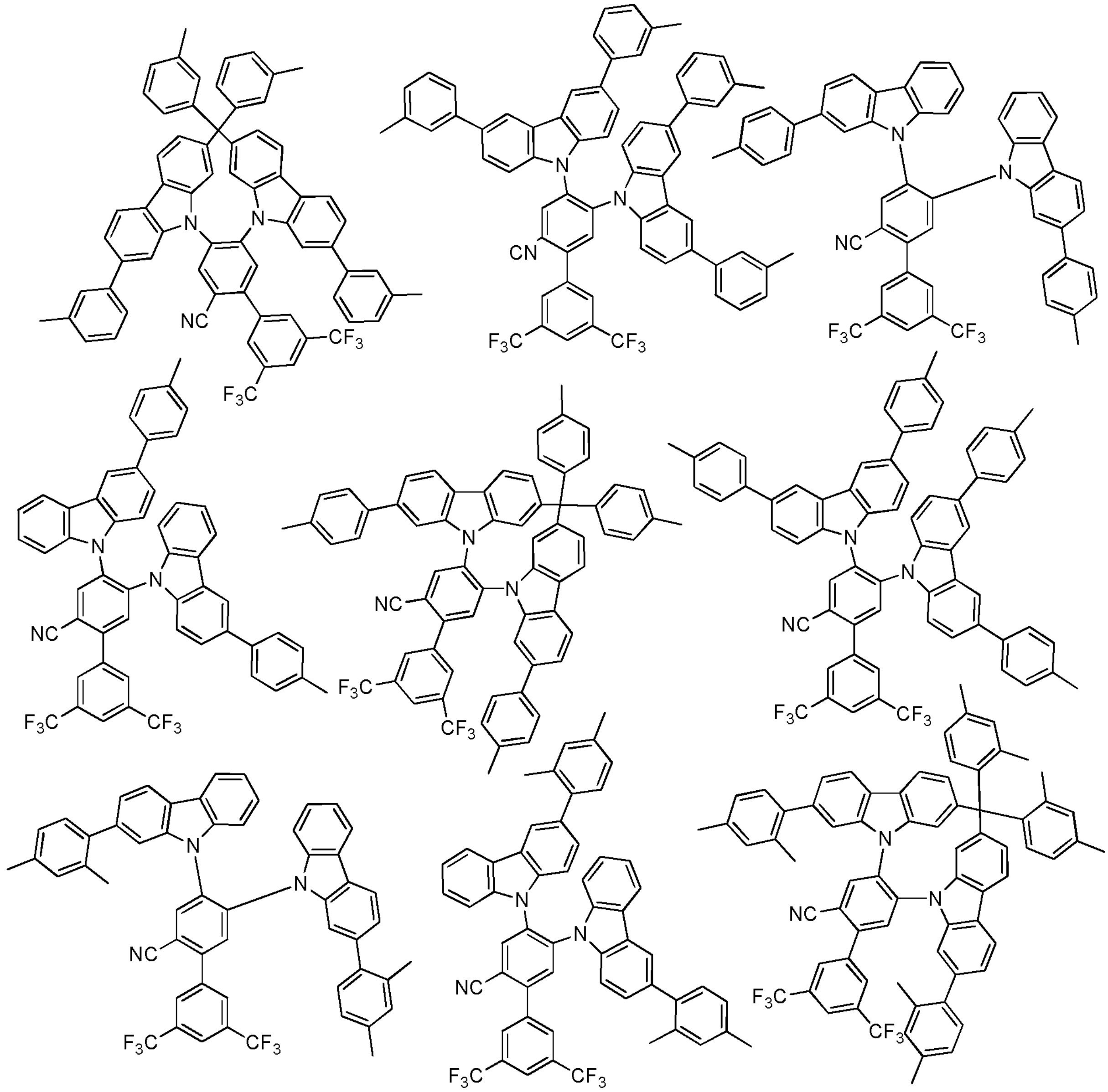

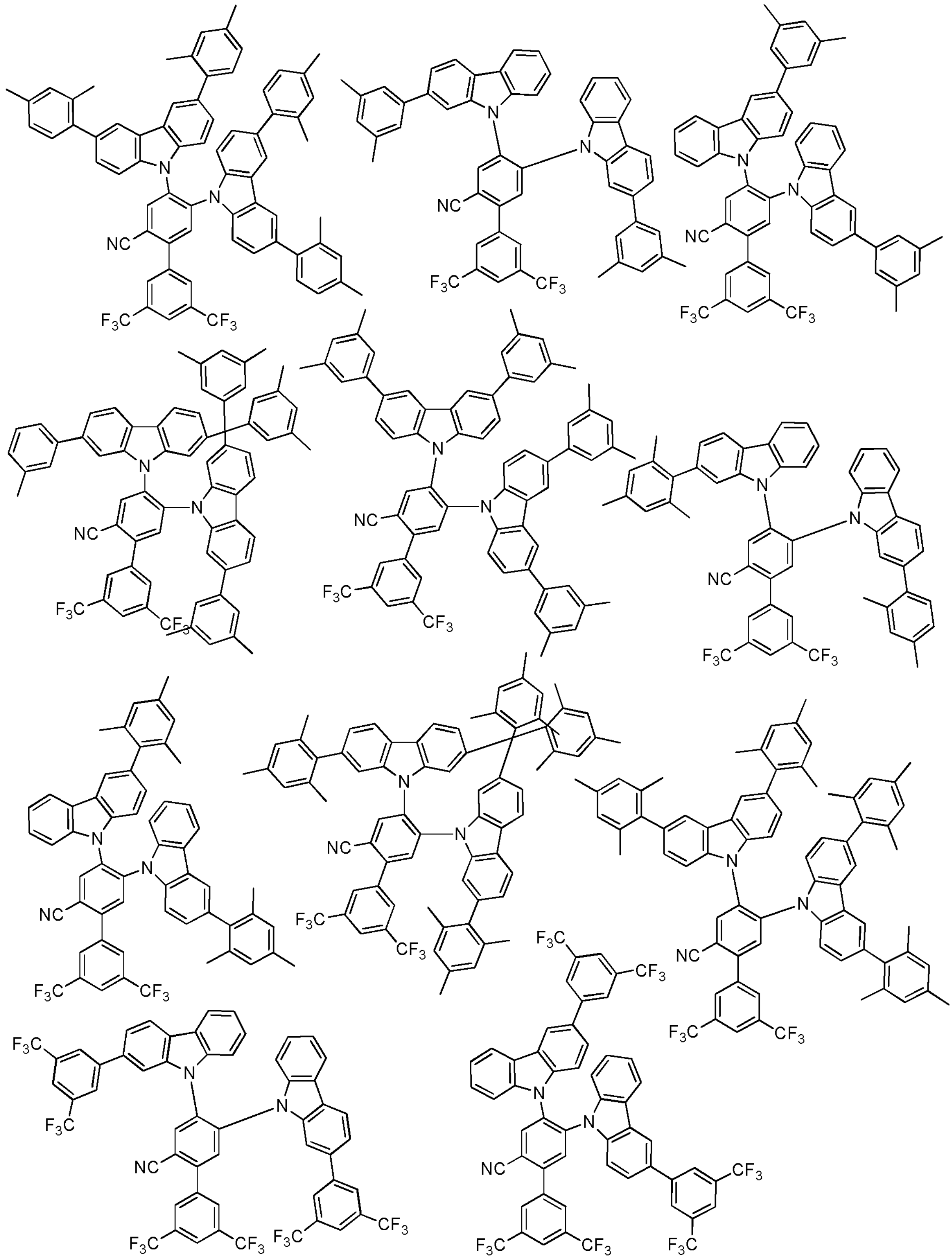

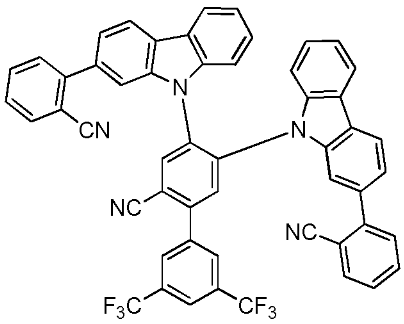

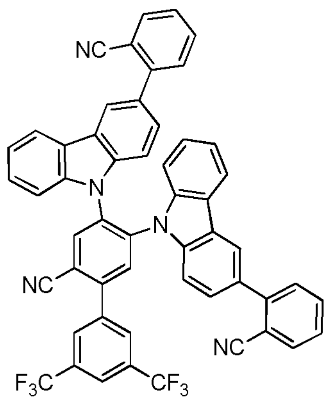

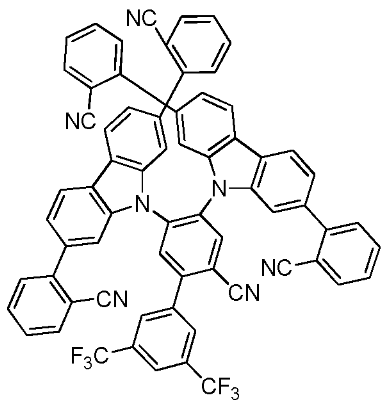

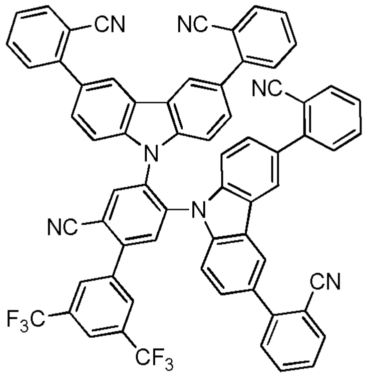

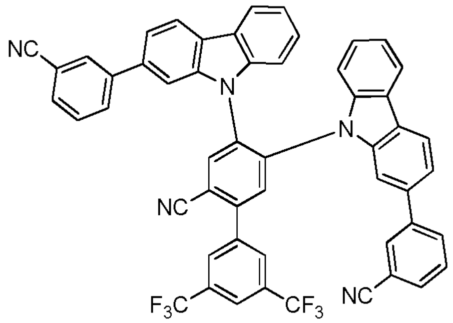

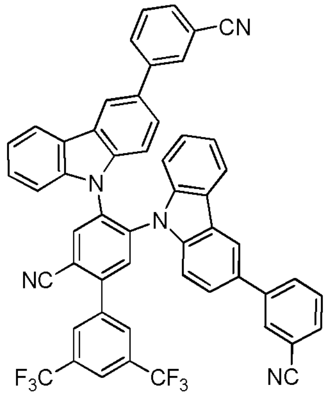

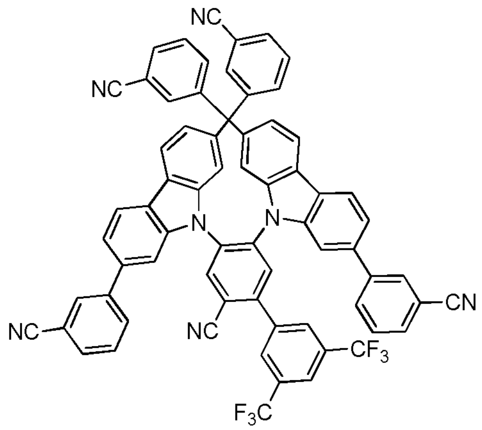

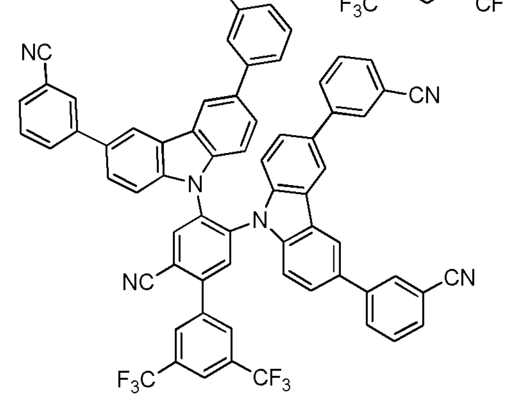

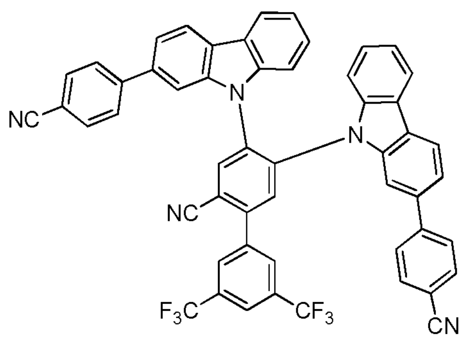

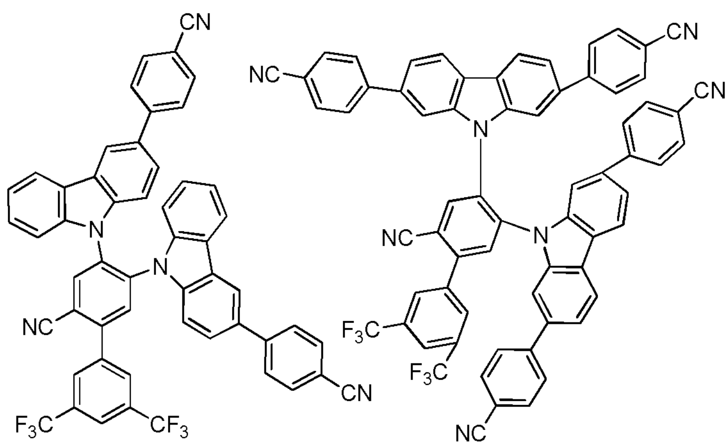

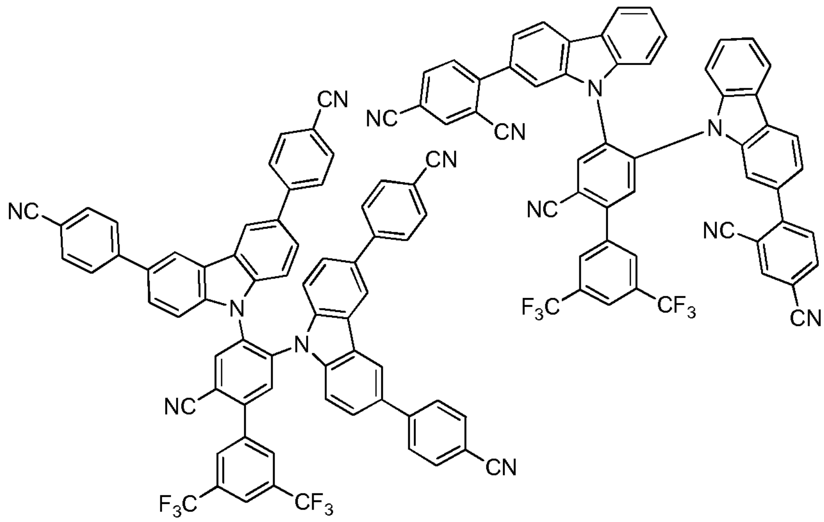

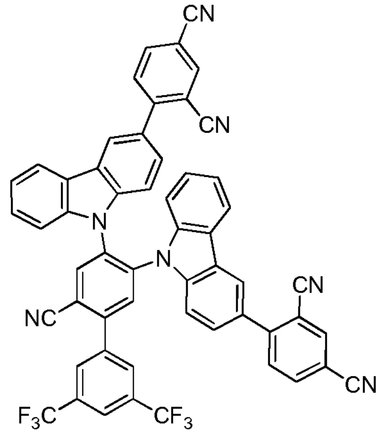

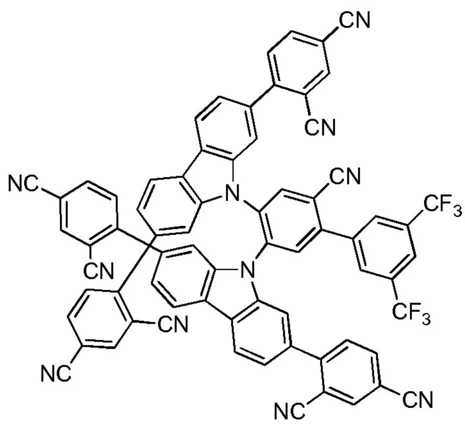

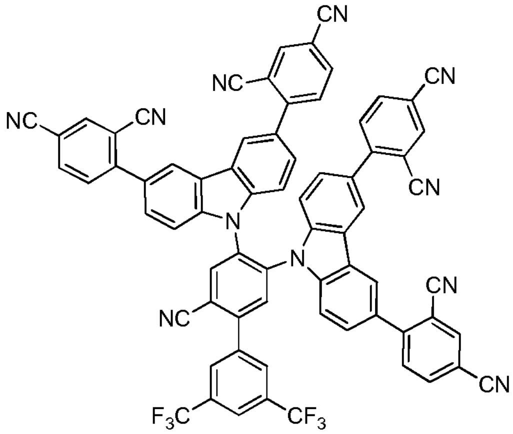

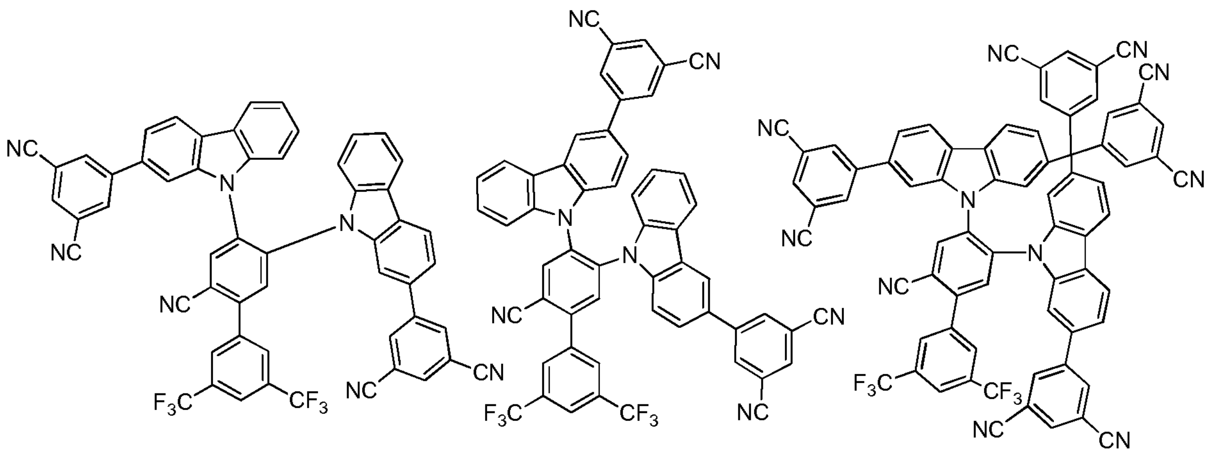

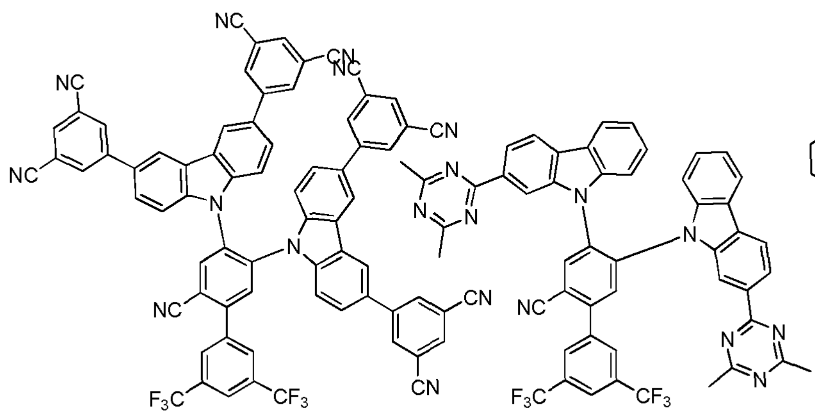

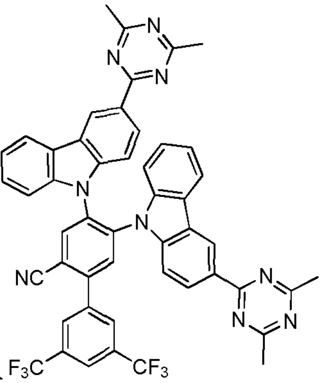

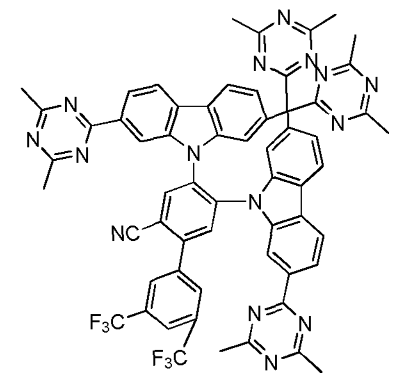

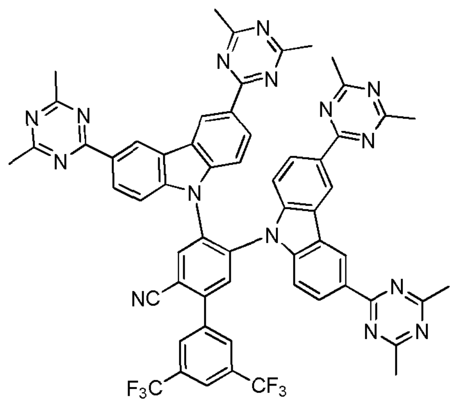

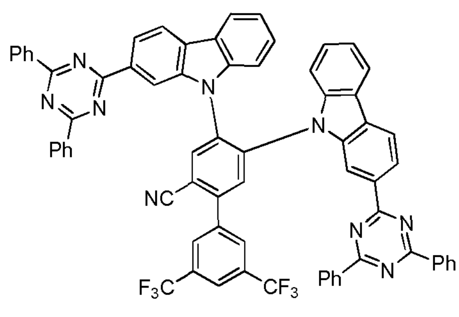

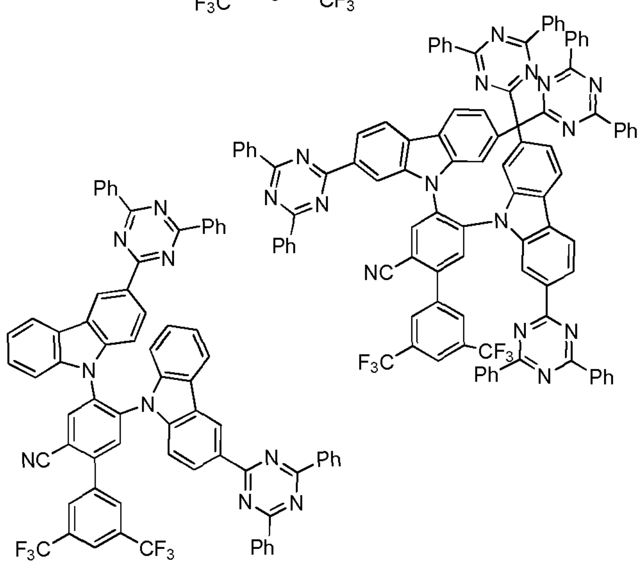

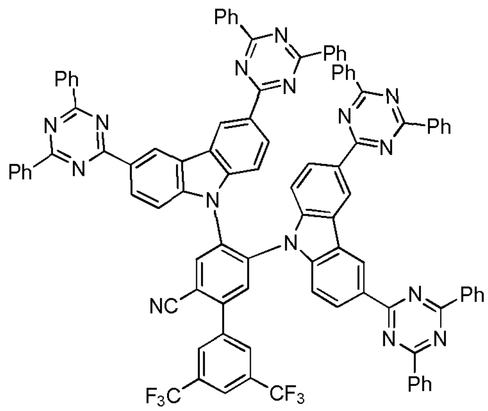

## Figuren

Es zeigen:

Figur 1 Emissionsspektrum von Beispiel **1** (10 % in PMMA)..
Figur 2 Emissionsspektrum von Beispiel **2** (10 % in PMMA).
Figur 3 Emissionsspektrum von Beispiel **3** (10 % in PMMA).
Figur 4 Emissionsspektrum von Beispiel **4** (10 % in PMMA).
Figur 5 Emissionsspektrum von Beispiel **5** (10 % in PMMA).
Figur 6 Emissionsspektrum von Beispiel **6** (10 % in PMMA).
Figur 7 Emissionsspektrum von Beispiel **7** (10 % in PMMA).
Figur 8 Emissionsspektrum von Beispiel **8** (10 % in PMMA).
Figur 9 Emissionsspektrum von Beispiel **9** (10 % in PMMA).
Figur 10 Emissionsspektrum von Beispiel **10** (10 % in PMMA).
Figur 11 Emissionsspektrum von Beispiel **11** (10 % in PMMA).
Figur 12 Emissionsspektrum von Beispiel **12** (10 % in PMMA).
Figur 13 Emissionsspektrum von Beispiel **13** (10 % in PMMA).
Figur 14 Emissionsspektrum von Beispiel **14** (10 % in PMMA).
Figur 15 Emissionsspektrum von Beispiel **15** (10 % in PMMA).
Figur 16 Emissionsspektrum von Beispiel **16** (10 % in PMMA).
Figur 17 Emissionsspektrum von Beispiel **17** (10 % in PMMA).
Figur 18 Emissionsspektrum von Beispiel **18** (10 % in PMMA).
Figur 19 Emissionsspektrum von Beispiel **19** (10 % in PMMA).
Figur 20 Emissionsspektrum von Beispiel **20** (10 % in PMMA).
Figur 21 Emissionsspektrum von Beispiel **21** (10 % in PMMA).
Figur 22 Emissionsspektrum von Beispiel **22** (10 % in PMMA).
Figur 23 Emissionsspektrum von Beispiel **23** (10 % in PMMA).
Figur 24 Emissionsspektrum von Beispiel **24** (10 % in PMMA).
Figur 25 Emissionsspektrum von Beispiel **25** (10 % in PMMA).
Figur 26 Emissionsspektrum von Beispiel **26** (10 % in PMMA).
Figur 27 Emissionsspektrum von Beispiel **27** (10 % in PMMA).
Figur 28 Emissionsspektrum von Beispiel **28** (10 % in PMMA).
Figur 29 Emissionsspektrum von Beispiel **29** (10 % in PMMA).
Figur 30 Emissionsspektrum von Beispiel **30** (10 % in PMMA).
Figur 31 Emissionsspektrum von Beispiel **31** (10 % in PMMA).
Figur 32 Emissionsspektrum von Beispiel **32** (10 % in PMMA).
Figur 33 Emissionsspektrum von Beispiel **33** (10 % in PMMA).
Figur 34 Emissionsspektrum von Beispiel **34** (10 % in PMMA).
Figur 35 Emissionsspektrum von Beispiel **35** (10 % in PMMA).
Figur 36 Emissionsspektrum von Beispiel **36** (10 % in PMMA).
Figur 37 Emissionsspektrum von Beispiel **37** (10 % in PMMA).
Figur 38 Emissionsspektrum von Beispiel **38** (10 % in PMMA).
Figur 39 Emissionsspektrum von Beispiel **39** (10 % in PMMA).
Figur 40 Emissionsspektrum von Beispiel **40** (10 % in PMMA).

Figur 41 Emissionsspektrum von Beispiel **41** (10 % in PMMA).
Figur 42 Emissionsspektrum von Beispiel **42** (10 % in PMMA).

## Patentansprüche

**1.** Organisches Molekül, aufweisend

- eine erste chemische Einheit aufweisend eine oder bestehend aus einer Struktur gemäß Formel I

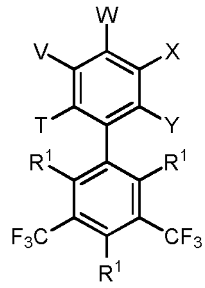

Formel I

und
- zwei zweite chemische Einheiten jeweils bei jedem Auftreten gleich oder verschieden aufweisend eine oder bestehend aus einer Struktur gemäß Formel II,

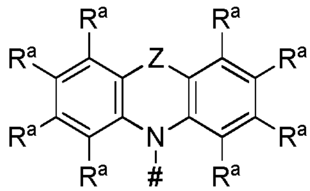

Formel II

wobei die erste chemische Einheit jeweils über eine Einfachbindung mit den zwei zweiten chemischen Einheiten verknüpft ist; mit

T ist Anknüpfungspunkt der Einfachbindung zwischen der ersten chemischen Einheit und einer zweiten chemischen Einheit oder ist H;
V ist Anknüpfungspunkt der Einfachbindung zwischen der ersten chemischen Einheit und einer zweiten chemischen Einheit oder ist H;
W ist Anknüpfungspunkt der Einfachbindung zwischen der ersten chemischen Einheit und einer zweiten chemischen Einheit oder ist ausgewählt aus der Gruppe bestehend aus H, CN und $CF_3$;
X ist Anknüpfungspunkt der Einfachbindung zwischen der ersten chemischen Einheit und einer zweiten chemischen Einheit oder ist ausgewählt aus der Gruppe bestehend aus H, CN und $CF_3$;
Y ist Anknüpfungspunkt der Einfachbindung zwischen der ersten chemischen Einheit und einer zweiten chemischen Einheit oder ist ausgewählt aus der Gruppe bestehend aus H, CN und $CF_3$;
# kennzeichnet den Anknüpfungspunkt der Einfachbindung zwischen einer zweiten chemischen Einheit und der ersten chemischen Einheit;
Z ist bei jedem Auftreten gleich oder verschieden eine direkte Bindung oder ausgewählt aus der Gruppe bestehend aus $CR^3R^4$, $C=CR^3R^4$, C=O, $C=NR^3$, $NR^3$, O, $SiR^3R^4$, S, S(O) und $S(O)_2$;
$R^1$ ist bei jedem Auftreten gleich oder verschieden ausgewählt aus der Gruppe bestehend aus:

- H, Deuterium;
- einer linearen Alkylgruppe mit 1 bis 5 C-Atomen, einer linearen Alkenyl- oder Alkinylgruppe mit 2 bis 8 C-Atomen, einer verzweigten oder cyclischen Alkyl-, Alkenyl- oder Alkinylgruppe mit 3 bis 10 C-Atomen, wobei ein oder mehrere H-Atome durch Deuterium ersetzt sein können; und

- einem aromatischen oder heteroaromatischen Ringsystem mit 5 bis 15 aromatischen Ringatomen, das jeweils mit einem oder mehreren Reste $R^6$ substituiert sein kann;

$R^a$, $R^3$ und $R^4$ ist bei jedem Auftreten gleich oder verschieden ausgewählt aus der Gruppe bestehend aus:

- H, Deuterium, $N(R^5)_2$, OH, $Si(R^5)_3$, $B(OR^5)_2$, $OSO_2R^5$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^5$ substituiert sein kann;
- einer Aryloxy- oder Heteroaryloxygruppe mit 5 bis 60 aromatischen Ringatomen, die jeweils mit einem oder mehreren Resten $R^5$ substituiert sein kann; und
- eine Diarylaminogruppe, Diheteroarylaminogruppe oder Arylheteroarylaminogruppe mit 10 bis 40 aromatischen Ringatomen, welche jeweils mit einem oder mehreren Resten $R^5$ substituiert sein kann;

$R^5$ ist bei jedem Auftreten gleich oder verschieden ausgewählt aus der Gruppe bestehend aus:

- H, Deuterium, $N(R^6)_2$, OH, $Si(R^6)_3$, $B(OR^6)_2$, $OSO_2R^6$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^6$ substituiert sein kann,
- einer Aryloxy- oder Heteroaryloxygruppe mit 5 bis 60 aromatischen Ringatomen, die jeweils mit einem oder mehreren Resten $R^6$ substituiert sein kann; und
- einer Diarylaminogruppe, Diheteroarylaminogruppe oder Arylheteroarylaminogruppe mit 10 bis 40 aromatischen Ringatomen, welche jeweils mit einem oder mehreren Resten $R^6$ substituiert sein kann;

$R^6$ ist bei jedem Auftreten gleich oder verschieden ausgewählt aus der Gruppe bestehend aus:

- H, Deuterium, OH, $CF_3$, CN, F;
- einer linearen Alkyl-, Alkoxy- oder Thioalkoxygruppe mit 1 bis 5 C-Atomen; wobei ein oder mehrere H-Atome durch Deuterium, CN, $CF_3$ oder $NO_2$ ersetzt sein können;

- einer linearen Alkenyl- oder Alkinylgruppe mit 2 bis 5 C-Atomen, wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 5 C-Atomen, wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen;
- einer Aryloxy- oder Heteroaryloxygruppe mit 5 bis 60 aromatischen Ringatomen; und
- einer Diarylaminogruppe, Diheteroarylaminogruppe oder Arylheteroarylaminogruppe mit 10 bis 40 aromatischen Ringatomen;

wobei jeder der Reste $R^a$, $R^3$, $R^4$ oder $R^5$ auch mit einem oder mehreren weiteren Resten $R^a$, $R^3$, $R^4$ oder $R^5$ ein mono- oder polycyclisches, aliphatisches, aromatisches und/oder benzoanelliertes Ringsystem bilden kann;
wobei
genau ein Rest ausgewählt aus der Gruppe bestehend aus W, X und Y gleich CN oder gleich $CF_3$ ist und genau zwei Reste ausgewählt aus der Gruppe bestehend aus T, V, W, X und Y gleich einem Anknüpfungspunkt der Einfachbindung zwischen der ersten chemischen Einheit und einer zweiten chemischen Einheit sind.

**2.** Organisches Molekül nach Anspruch 1, wobei $R^1$ bei jedem Auftreten gleich oder verschieden H, Methyl oder Phenyl ist.

**3.** Organisches Molekül nach Anspruch 1 oder 2, wobei W gleich CN ist.

**4.** Organisches Molekül nach Anspruch 1 bis 3, wobei die zweite chemische Einheit bei jedem Auftreten gleich oder verschieden eine Struktur der Formel IIa aufweist oder aus einer derartigen Struktur besteht:

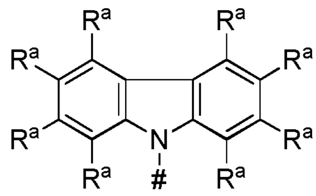

Formel IIa

wobei für # und $R^a$ die in Anspruch 1 genannten Definitionen gelten.

**5.** Organisches Molekül nach Anspruch 1 bis 4, wobei die zweite chemische Einheit jeweils eine Struktur der Formel IIb aufweist oder aus einer derartigen Struktur besteht:

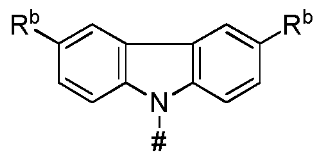

Formel IIb

wobei

$R^b$ bei jedem Auftreten gleich oder verschieden ausgewählt ist aus der Gruppe bestehend aus:

- $N(R^5)_2$, OH, $Si(R^5)_3$, $B(OR^5)_2$, $OSO_2R^5$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C{=}CR^5$, C=C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C{=}CR^5$,

C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^5$ substituiert sein kann;
- einer Aryloxy- oder Heteroaryloxygruppe mit 5 bis 60 aromatischen Ringatomen, die jeweils mit einem oder mehreren Resten $R^5$ substituiert sein kann; und
- einer Diarylaminogruppe, Diheteroarylaminogruppe oder Arylheteroarylaminogruppe mit 10 bis 40 aromatischen Ringatomen, welche jeweils mit einem oder mehreren Resten $R^5$ substituiert sein kann;

und für # und $R^5$ die in Anspruch 1 genannten Definitionen gelten.

**6.** Organisches Molekül nach Anspruch 1 bis 4, wobei die zweite chemischen Einheit jeweils eine Struktur der Formel IIc aufweist oder aus einer derartigen Struktur besteht:

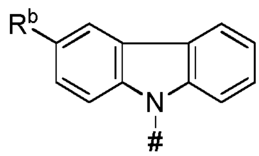

Formel IIc

wobei

$R^b$ bei jedem Auftreten gleich oder verschieden ausgewählt ist aus der Gruppe bestehend aus:

- $N(R^5)_2$, OH, $Si(R^5)_3$, $B(OR^5)_2$, $OSO_2R^5$, $CF_3$, CN, F, Br, I;
- einer linearen Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer linearen Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einer verzweigten oder cyclischen Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
- einem aromatischen oder heteroaromatischen Ringsystem mit 5 bis 60 aromatischen Ringatomen, das jeweils mit einem oder mehreren Resten $R^5$ substituiert sein kann;
- einer Aryloxy- oder Heteroaryloxygruppe mit 5 bis 60 aromatischen Ringatomen, die jeweils mit einem oder mehreren Resten $R^5$ substituiert sein kann; und
- einer Diarylaminogruppe, Diheteroarylaminogruppe oder Arylheteroarylaminogruppe mit 10 bis 40 aromatischen Ringatomen, welche jeweils mit einem oder mehreren Resten $R^5$ substituiert sein kann;

und im Übrigen die in Anspruch 1 genannte Definitionen gelten.

**7.** Organisches Molekül nach Anspruch 5 oder 6, wobei $R^b$ bei jedem Auftreten gleich oder verschieden Me, $^iPr$, $^tBu$,

CN, $CF_3$ oder
Ph, das jeweils mit einem oder mehreren Resten ausgewählt aus Me, $^iPr$, $^tBu$, CN, $CF_3$ oder Ph substituiert sein kann,
Pyridinyl, das jeweils mit einem oder mehreren Resten ausgewählt aus Me, $^iPr$, $^tBu$, CN, $CF_3$ oder Ph substituiert sein kann,
Pyrimidinyl, das jeweils mit einem oder mehreren Resten ausgewählt aus Me, $^iPr$, $^tBu$, CN, $CF_3$ oder Ph substituiert sein kann,
Triazinyl, das jeweils mit einem oder mehreren Resten ausgewählt aus Me, $^iPr$, $^tBu$, CN, $CF_3$ oder Ph substituiert sein kann,
Carbazolyl, das jeweils mit einem oder mehreren Resten ausgewählt aus Me, $^iPr$, $^tBu$, CN, $CF_3$ oder Ph substituiert sein kann,
oder $N(Ph)_2$ ist.

**8.** Verfahren zur Herstellung eines Organischen Moleküls nach Anspruch 1 bis 7, wobei eine in 2,4,6-Position $R^1$-substituierte 3,5-Bis(trifluormethyl)-phenylboronsäure oder ein entsprechender in 2,4,6-Position $R^1$-substituierter 3,5-Bis(trifluormethyl)-phenylboronsäureester als Edukt eingesetzt wird.

**9.** Verwendung eines organischen Moleküls nach Anspruch 1 bis 7 als lumineszierender Emitter und/oder als Hostmaterial und/oder als Elektronentransportmaterial und/oder als Lochinjektionsmaterial und/oder als Lochblockiermaterial in einer organischen optoelektronischen Vorrichtung.

**10.** Verwendung nach Anspruch 9, wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

**11.** Zusammensetzung aufweisend oder bestehend aus:

(a) mindestens einem organischen Molekül nach einem der Ansprüche 1 bis 7, insbesondere in Form eines Emitters und/oder Hosts, und
(b) einem oder mehrerer Emitter- und/oder Hostmaterialien, die von dem Molekül nach Anspruch 1 bis 7 verschiedenen sind und
(c) optional einem oder mehreren Farbstoffen und/oder einem oder mehreren Lösungsmitteln.

**12.** Organische optoelektronische Vorrichtung, aufweisend ein organisches Molekül nach Anspruch 1 bis 7 oder eine Zusammensetzung nach Anspruch 11, insbesondere ausgeformt als eine Vorrichtung ausgewählt aus der Gruppe bestehend ausorganischer lichtemittierender Diode (OLED), lichtemittierender elektrochemischer Zelle, OLED-Sensor, insbesondere nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

**13.** Organische optoelektronische Vorrichtung nach Anspruch 12, aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein organisches Molekül nach Anspruch 1 bis 7 oder eine Zusammensetzung nach Anspruch 11 aufweist.

**14.** Verfahren zur Herstellung eines optoelektronischen Bauelements, wobei ein organisches Molekül nach Anspruch 1 bis 7 verwendet wird, insbesondere umfassend die Verarbeitung des organischen Moleküls mittels eines Vakuumverdampfungsverfahrens oder aus einer Lösung.

## Claims

**1.** Organic molecule comprising

- a first chemical unit comprising or consisting of a structure of formula I

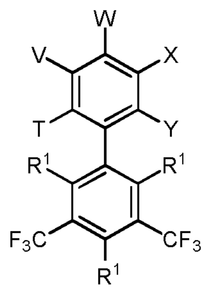

Formula I

and

- two second chemical units each at each instance identically or differently comprising or consisting of a structure of formula II

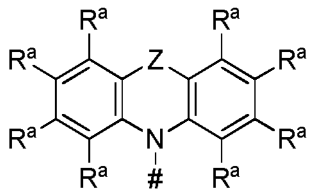

Formula II

where the first chemical unit is joined to each of the two second chemical units via a single bond;
where

T is an attachment point of the single bond between the first chemical unit and a second chemical unit or is H;
V is an attachment point of the single bond between the first chemical unit and a second chemical unit or is H;
W is an attachment point of the single bond between the first chemical unit and a second chemical unit or is selected from the group consisting of H, CN and $CF_3$;
X is an attachment point of the single bond between the first chemical unit and a second chemical unit or is selected from the group consisting of H, CN and $CF_3$;
Y is an attachment point of the single bond between the first chemical unit and a second chemical unit or is selected from the group consisting of H, CN and $CF_3$;
# indicates the attachment point of the single bond between a second chemical unit and the first chemical unit;
Z is the same or different at each instance and is a direct bond or selected from the group consisting of $CR^3R^4$, $C=CR^3R^4$, C=O, $C=NR^3$, $NR^3$, O, $SiR^3R^4$, S, S(O) and $S(O)_2$;
$R^1$ is the same or different at each instance and is selected from the group consisting of:

- H, deuterium;
- a linear alkyl group having 1 to 5 carbon atoms, a linear alkenyl or alkynyl group having 2 to 8 carbon atoms, a branched or cyclic alkyl, alkenyl or alkynyl group having 3 to 10 carbon atoms, where one or more hydrogen atoms may be replaced by deuterium; and
- an aromatic or heteroaromatic ring system which has 5 to 15 aromatic ring atoms and may be substituted in each case by one or more $R^6$ moieties;

$R^a$, $R^3$ and $R^4$ are the same or different at each instance and are selected from the group consisting of:

- H, deuterium, $N(R^5)_2$, OH, $Si(R^5)_3$, $B(OR^5)_2$, $OSO_2R^5$, $CF_3$, CN, F, Br, I;
- a linear alkyl, alkoxy or thioalkoxy group which has 1 to 40 carbon atoms and may be substituted in each case by one or more $R^5$ moieties, where one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and where one or more hydrogen atoms may be replaced by deuterium, CN, $CF_3$ or $NO_2$;
- a linear alkenyl or alkynyl group which has 2 to 40 carbon atoms and may be substituted in each case by one or more $R^5$ moieties, where one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and where one or more hydrogen atoms may be replaced by deuterium, CN, $CF_3$ or $NO_2$;
- a branched or cyclic alkyl, alkenyl, alkynyl, alkoxy or thioalkoxy group which has 3 to 40 carbon atoms and may be substituted in each case by one or more $R^5$ moieties, where one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and where one or more hydrogen atoms may be replaced by deuterium, CN, $CF_3$ or $NO_2$;
- an aromatic or heteroaromatic ring system which has 5 to 60 aromatic ring atoms and may be substituted in each case by one or more $R^5$ moieties;
- an aryloxy or heteroaryloxy group which has 5 to 60 aromatic ring atoms and may be substituted in each case by one or more $R^5$ moieties; and
- a diarylamino group, diheteroarylamino group or arylheteroarylamino group which has 10 to 40 aromatic ring atoms and may be substituted in each case by one or more $R^5$ moieties;

$R^5$ is the same or different at each instance and is selected from the group consisting of:

- H, deuterium, $N(R^6)_2$, OH, $Si(R^6)_3$, $B(OR^6)_2$, $OSO_2R^6$, $CF_3$, CN, F, Br, I;
- a linear alkyl, alkoxy or thioalkoxy group which has 1 to 40 carbon atoms and may be substituted in each case by one or more $R^6$ moieties, where one or more nonadjacent $CH_2$ groups may be replaced by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and where one or more hydrogen atoms may be replaced by deuterium, CN, $CF_3$ or $NO_2$;
- a linear alkenyl or alkynyl group which has 2 to 40 carbon atoms and may be substituted in each case by one or more $R^6$ moieties, where one or more nonadjacent $CH_2$ groups may be replaced by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and where one or more hydrogen atoms may be replaced by deuterium, CN, $CF_3$ or $NO_2$;
- a branched or cyclic alkyl, alkenyl, alkynyl, alkoxy or thioalkoxy group which has 3 to 40 carbon atoms and may be substituted in each case by one or more $R^6$ moieties, where one or more nonadjacent $CH_2$ groups may be replaced by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and where one or more hydrogen atoms may be replaced by deuterium, CN, $CF_3$ or $NO_2$;
- an aromatic or heteroaromatic ring system which has 5 to 60 aromatic ring atoms and may be substituted in each case by one or more $R^6$ moieties;
- an aryloxy or heteroaryloxy group which has 5 to 60 aromatic ring atoms and may be substituted in each case by one or more $R^6$ moieties; and
- a diarylamino group, diheteroarylamino group or arylheteroarylamino group which has 10 to 40 aromatic ring atoms and may be substituted in each case by one or more $R^6$ moieties;

$R^6$ is the same or different at each instance and is selected from the group consisting of:

- H, deuterium, OH, $CF_3$, CN, F;
- a linear alkyl, alkoxy or thioalkoxy group having 1 to 5 carbon atoms, where one or more hydrogen atoms may be replaced by deuterium, CN, $CF_3$ or $NO_2$;
- a linear alkenyl or alkynyl group having 2 to 5 carbon atoms, where one or more hydrogen atoms may be replaced by deuterium, CN, $CF_3$ or $NO_2$;
- a branched or cyclic alkyl, alkenyl, alkynyl, alkoxy or thioalkoxy group having 3 to 5 carbon atoms, where one or more hydrogen atoms may be replaced by deuterium, CN, $CF_3$ or $NO_2$;
- an aromatic or heteroaromatic ring system having 5 to 60 aromatic ring atoms;
- an aryloxy or heteroaryloxy group having 5 to 60 aromatic ring atoms; and
- a diarylamino group, diheteroarylamino group or arylheteroarylamino group having 10 to 40 aromatic ring atoms;

where each of the $R^a$, $R^3$, $R^4$ or $R^5$ moieties together with one or more further $R^a$, $R^3$, $R^4$ or $R^5$ moieties may form a mono- or polycyclic, aliphatic, aromatic and/or benzofused ring system; where exactly one moiety selected from the group consisting of W, X and Y is CN or $CF_3$ and exactly two moieties selected from the group consisting of T, V, W, X and Y are an attachment point of the single bond between the first chemical unit and a second chemical unit.

**2.** Organic molecule according to claim 1, where $R^1$ is the same or different at each instance and is H, methyl or phenyl.

**3.** Organic molecule according to claim 1 or 2, where W is CN.

**4.** Organic molecule according to claims 1 to 3, where the second chemical unit is the same or different at each instance and comprises a structure of the formula IIa or consists of such a structure:

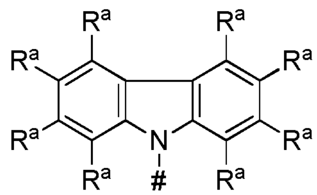

Formula IIa

where the definitions given in claim 1 are applicable to # and $R^a$.

**5.** Organic molecule according to claims 1 to 4, where the second chemical unit in each case comprises a structure of the formula IIb or consists of such a structure:

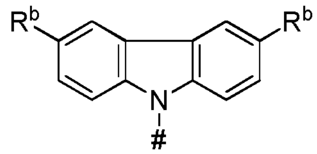

Formula IIb

where

$R^b$ is the same or different at each instance and is selected from the group consisting of:

- $N(R^5)_2$, OH, $Si(R^5)_3$, $B(OR^5)_2$, $OSO_2R^5$, $CF_3$, CN, F, Br, I;
- a linear alkyl, alkoxy or thioalkoxy group which has 1 to 40 carbon atoms and may be substituted in each case by one or more $R^5$ moieties, where one or more nonadjacent $CH_2$ groups may be replaced by $R^5C{=}CR^5$, C=C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S or $CONR^5$ and where one or more hydrogen atoms may be replaced by deuterium, CN, $CF_3$ or $NO_2$;
- a linear alkenyl or alkynyl group which has 2 to 40 carbon atoms and may be substituted in each case by one or more $R^5$ moieties, where one or more nonadjacent $CH_2$ groups may be replaced by $R^5C{=}CR^5$, C=C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S or $CONR^5$ and where one or more hydrogen atoms may be replaced by deuterium, CN, $CF_3$ or $NO_2$;
- a branched or cyclic alkyl, alkenyl, alkynyl, alkoxy or thioalkoxy group which has 3 to 40 carbon atoms and may be substituted in each case by one or more $R^5$ moieties, where one or more nonadjacent $CH_2$ groups may be replaced by $R^5C{=}CR^5$, C=C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S or $CONR^5$ and where one or more hydrogen atoms may be replaced by deuterium, CN, $CF_3$ or $NO_2$;
- an aromatic or heteroaromatic ring system which has 5 to 60 aromatic ring atoms and may be substituted in each case by one or more $R^5$ moieties;
- an aryloxy or heteroaryloxy group which has 5 to 60 aromatic ring atoms and may be substituted in each case by one or more $R^5$ moieties; and
- a diarylamino group, diheteroarylamino group or arylheteroarylamino group which has 10 to 40 aromatic ring atoms and may be substituted in each case by one or more $R^5$ moieties;

and the definitions given in claim 1 are applicable to # and $R^5$.

**6.** Organic molecule according to claims 1 to 4, where the second chemical unit in each case comprises of a structure of the formula IIc or consists of such a structure:

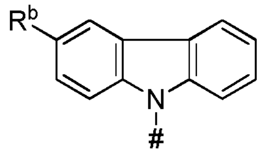

Formula IIc

where

$R^b$ is the same or different at each instance and is selected from the group consisting of:

- $N(R^5)_2$, OH, $Si(R^5)_3$, $B(OR^5)_2$, $OSO_2R^5$, $CF_3$, CN, F, Br, I;
- a linear alkyl, alkoxy or thioalkoxy group which has 1 to 40 carbon atoms and may be substituted in each case by one or more $R^5$ moieties, where one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and where one or more hydrogen atoms may be replaced by deuterium, CN, $CF_3$ or $NO_2$;
- a linear alkenyl or alkynyl group which has 2 to 40 carbon atoms and may be substituted in each case by one or more $R^5$ moieties, where one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and where one or more hydrogen atoms may be replaced by deuterium, CN, $CF_3$ or $NO_2$;
- a branched or cyclic alkyl, alkenyl, alkynyl, alkoxy or thioalkoxy group which has 3 to 40 carbon atoms and may be substituted in each case by one or more $R^5$ moieties, where one or more nonadjacent $CH_2$ groups may be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and where one or more hydrogen atoms may be replaced by deuterium, CN, $CF_3$ or $NO_2$;
- an aromatic or heteroaromatic ring system which has 5 to 60 aromatic ring atoms and may be substituted in each case by one or more $R^5$ moieties;
- an aryloxy or heteroaryloxy group which has 5 to 60 aromatic ring atoms and may be substituted in each case by one or more $R^5$ moieties; and
- a diarylamino group, diheteroarylamino group or arylheteroarylamino group which has 10 to 40 aromatic ring atoms and may be substituted in each case by one or more $R^5$ moieties;

and for the rest the definitions given in claim 1 are applicable.

**7.** Organic molecule according to claim 5 or 6, where $R^b$ is the same or different at each instance and is Me, $^iPr$, $^tBu$, CN, $CF_3$ or
Ph which may be substituted in each case by one or more moieties selected from Me, $^iPr$, $^tBu$, CN, $CF_3$ and Ph,
pyridinyl which may be substituted in each case by one or more moieties selected from Me, $^iPr$, $^tBu$, CN, $CF_3$ and Ph,
pyrimidinyl which may be substituted in each case by one or more moieties selected from Me, $^iPr$, $^tBu$, CN, $CF_3$ and Ph,
triazinyl which may be substituted in each case by one or more moieties selected from Me, $^iPr$, $^tBu$, CN, $CF_3$ and Ph,
carbazolyl which may be substituted in each case by one or more moieties selected from Me, $^iPr$, $^tBu$, CN, $CF_3$ and Ph,
or $N(Ph)_2$.

**8.** Method for preparing an organic molecule according to claims 1 to 7, wherein a 2,4,6-$R^1$-substituted 3,5-bis(trifluoromethyl)phenylboronic acid or a corresponding 2,4,6-$R^1$-substituted 3,5-bis(trifluoromethyl)phenylboronic ester is used as reactant.

**9.** Use of an organic molecule according to claims 1 to 7 as a luminescent emitter and/or as a host material and/or as an electron transport material and/or as a hole injection material and/or as a hole blocker material in an organic optoelectronic device.

**10.** Use according to claim 9, wherein the organic optoelectronic device is selected from the group consisting of:

- organic light-emitting diodes (OLEDs),
- light-emitting electrochemical cells,
- OLED sensors, in particular in gas and vapor sensors not hermetically shielded from the outside,
- organic diodes,
- organic solar cells,
- organic transistors,
- organic field-effect transistors,
- organic lasers and
- down-conversion elements.

**11.** Composition comprising or consisting of:

(a) at least one organic molecule according to any of claims 1 to 7, in particular in the form of an emitter and/or host, and
(b) one or more emitter and/or host materials other than the molecule according to claims 1 to 7 and
(c) optionally, one or more dyes and/or one or more solvents.

**12.** Organic optoelectronic device comprising an organic molecule according to claims 1 to 7 or a composition according to claim 11, in particular in the form of a device selected from the group consisting of organic light-emitting diode (OLED), light-emitting electrochemical cell, OLED sensor, in particular gas and vapor sensors that are not hermetically shielded from the outside, organic diode, organic solar cell, organic transistor, organic field effect transistor, organic laser and down-conversion element.

**13.** Organic optoelectronic device according to claim 12, comprising

- a substrate,
- an anode and
- a cathode, where the anode or cathode has been applied to the substrate, and
- at least one light-emitting layer which is arranged between the anode and the cathode and comprises an organic molecule according to claims 1 to 7 or a composition according to claim 11.

**14.** Method for producing an optoelectronic component, wherein an organic molecule according to claims 1 to 7 is used, in particular comprising the processing of the organic molecule by means of a vacuum evaporation process or from a solution.

**Revendications**

**1.** Molécule organique, comprenant :

- une première unité chimique comprenant ou constituée par une structure selon la formule I

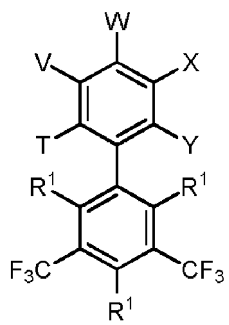

Formule I

et

- deux deuxièmes unités chimiques, à chaque fois à chaque occurrence de manière identique ou différente comprenant ou constituée par une structure selon la formule II

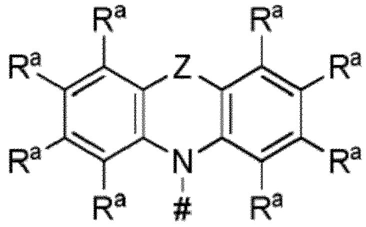

Formule II

la première unité chimique à chaque fois étant reliée par une simple liaison avec les deux deuxièmes unités chimiques ;
T étant point de liaison de la simple liaison entre la première unité chimique et une deuxième unité chimique, ou représentant H ;
V étant point de liaison de la simple liaison entre la première unité chimique et une deuxième unité chimique ou représentant H ;
W étant point de liaison de la simple liaison entre la première unité chimique et une deuxième unité chimique, ou étant choisi dans le groupe constitué par H, CN et $CF_3$ ;
X étant point de liaison de la simple liaison entre la première unité chimique et une deuxième unité chimique ou étant choisi dans le groupe constitué par H, CN et $CF_3$ ;
Y étant point de liaison de la simple liaison entre la première unité chimique et une deuxième unité chimique ou étant choisi dans le groupe constitué par H, CN et $CF_3$ ;
# désignant le point de liaison de la simple liaison entre la deuxième unité chimique et la première unité chimique ;
les Z représentant à chaque occurrence de manière identique ou différente une liaison directe ou étant choisis dans le groupe constitué par $CR^3R^4$, $C=CR^3R^4$, C=O $C=NR^3$, $NR^3$, O, $SiR^3R^4$, S, S(O) et $S(O)_2$ ;
les $R^1$ étant choisis à chaque occurrence de manière identique ou différente dans le groupe constitué par :

- H, le deutérium,
- un groupe alkyle linéaire de 1 à 5 atomes C, un groupe alcényle ou alcynyle linéaire de 2 à 8 atomes C, un groupe alkyle, alcényle ou alcynyle ramifié ou cyclique de 3 à 10 atomes C, un ou plusieurs atomes H pouvant être remplacés par le deutérium ; et
- un système cyclique aromatique ou hétéroaromatique de 5 à 15 atomes de cycle aromatique, qui peut à chaque fois être substitué avec un ou plusieurs radicaux $R^6$ ;

$R^a$, $R^3$ et $R^4$ étant choisis à chaque occurrence de manière identique ou différente dans le groupe constitué par :

- H, le deutérium, $N(R^5)_2$, OH, $Si(R^5)_3$, $B(OR^5)_2$, $OSO_2R^5$, $CF_3$, CN, F, Br, I ;
- un groupe alkyle, alcoxy ou thioalcoxy linéaire de 1 à 40 atomes C, qui peut à chaque fois être substitué avec un ou plusieurs radicaux $R^5$, un ou plusieurs groupes $CH_2$ non voisins pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$ ;
- un groupe alcényle ou alcynyle linéaire de 2 à 40 atomes C, qui peut à chaque fois être substitué avec un ou plusieurs radicaux $R^5$, un ou plusieurs groupes $CH_2$ non voisins pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$ ;
- un groupe alkyle, alcényle, alcynyle, alcoxy ou thioalcoxy ramifié ou cyclique de 3 à 40 atomes C, qui peut à chaque fois être substitué avec un ou plusieurs radicaux $R^5$, un ou plusieurs groupes $CH_2$ non voisins pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$;
- un système cyclique aromatique ou hétéroaromatique de 5 à 60 atomes de cycle aromatique, qui peut à chaque fois être substitué avec un ou plusieurs radicaux $R^5$ ;
- un groupe aryloxy ou hétéroaryloxy de 5 à 60 atomes de cycle aromatique, qui peut à chaque fois être

substitué avec un ou plusieurs radicaux $R^5$ ; et
- un groupe diarylamino, un groupe dihétéroarylamino ou un groupe arylhétéroarylamino de 10 à 40 atomes de cycle aromatique, qui peut à chaque fois être substitué avec un ou plusieurs radicaux $R^5$ ;

les $R^5$ étant choisis à chaque occurrence de manière identique ou différente dans le groupe constitué par :

- H, le deutérium, $N(R^6)_2$, OH, $Si(R^6)_3$, $B(OR^6)_2$, $OSO_2R^6$, $CF_3$, CN, F, Br, I ;
- un groupe alkyle, alcoxy ou thioalcoxy linéaire de 1 à 40 atomes C, qui peut à chaque fois être substitué avec un ou plusieurs radicaux $R^6$, un ou plusieurs groupes $CH_2$ non voisins pouvant être remplacés par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$, et un ou plusieurs atomes H pouvant être remplacés par le deutérium, CN, $CF_3$ ou $NO_2$ ;
- un groupe alcényle ou alcynyle linéaire de 2 à 40 atomes C, qui peut à chaque fois être substitué avec un ou plusieurs radicaux $R^6$, un ou plusieurs groupes $CH_2$ non voisins pouvant être remplacés par $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$, et un ou plusieurs atomes H pouvant être remplacés par le deutérium, CN, $CF_3$ ou $NO_2$ ;
- un groupe alkyle, alcényle, alcynyle, alcoxy ou thioalcoxy ramifié ou cyclique de 3 à 40 atomes C, qui peut à chaque fois être substitué avec un ou plusieurs radicaux $R^6$, un ou plusieurs groupes $CH_2$ non voisins pouvant être remplacés par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$, et un ou plusieurs atomes H pouvant être remplacés par le deutérium, CN, $CF_3$ ou $NO_2$;
- un système cyclique aromatique ou hétéroaromatique de 5 à 60 atomes de cycle aromatique, qui peut à chaque fois être substitué avec un ou plusieurs radicaux $R^6$,
- un groupe aryloxy ou hétéroaryloxy de 5 à 60 atomes de cycle aromatique, qui peut à chaque fois être substitué avec un ou plusieurs radicaux $R^6$ ; et
- un groupe diarylamino, un groupe dihétéroarylamino ou un groupe arylhétéroarylamino de 10 à 40 atomes de cycle aromatique, qui peut à chaque fois être substitué avec un ou plusieurs radicaux $R^6$;

les $R^6$ étant choisis à chaque occurrence de manière identique ou différente dans le groupe constitué par :

- H, le deutérium, OH, $CF_3$, CN, F ;
- un groupe alkyle, alcoxy ou thioalcoxy linéaire de 1 à 5 atomes C, un ou plusieurs atomes H pouvant être remplacés par le deutérium, CN, $CF_3$ ou $NO_2$ ;
- un groupe alcényle ou alcynyle linéaire de 2 à 5 atomes C, un ou plusieurs atomes H pouvant être remplacés par le deutérium, CN, $CF_3$ ou $NO_2$ ;
- un groupe alkyle, alcényle, alcynyle, alcoxy ou thioalcoxy ramifié ou cyclique de 3 à 5 atomes C, un ou plusieurs atomes H pouvant être remplacés par le deutérium, CN, $CF_3$ ou $NO_2$ ;
- un système cyclique aromatique ou hétéroaromatique de 5 à 60 atomes de cycle aromatique ;
- un groupe aryloxy ou hétéroaryloxy de 5 à 60 atomes de cycle aromatique ; et
- un groupe diarylamino, un groupe dihétéroarylamino ou un groupe arylhétéroarylamino de 10 à 40 atomes de cycle aromatique ;

les radicaux $R^a$, $R^3$, $R^4$ ou $R^5$ pouvant également former avec un ou plusieurs autres radicaux $R^a$, $R^3$, $R^4$ ou $R^5$ un système cyclique mono- ou polycyclique, aliphatique, aromatique et/ou benzoannelé ;
exactement un radical choisi dans le groupe constitué par W, X et Y représentant CN ou représentant $CF_3$, et exactement deux radicaux choisis dans le groupe constitué par T, V, W, X et Y représentant un point de liaison de la simple liaison entre la première unité chimique et une deuxième unité chimique.

**2.** Molécule organique selon la revendication 1, dans laquelle les $R^1$ représentent à chaque occurrence de manière identique ou différente H, méthyle ou phényle.

**3.** Molécule organique selon la revendication 1 ou 2, dans laquelle W représente CN.

**4.** Molécule organique selon les revendications 1 à 3, dans laquelle la deuxième unité chimique à chaque occurrence de manière identique ou différente présente une structure de la formule IIa ou est constituée par une telle structure :

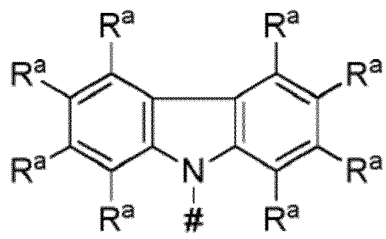

Formule IIa

dans laquelle # et $R^a$ ont les définitions indiquées dans la revendication 1.

**5.** Molécule organique selon les revendications 1 à 4, dans laquelle la deuxième unité chimique à chaque fois présente une structure de formule IIb ou est constituée par une telle structure :

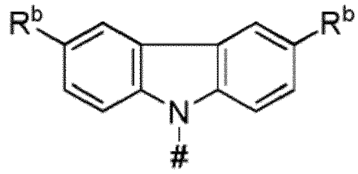

Formule IIb

dans laquelle

les $R^b$ sont choisis à chaque occurrence de manière identique ou différente dans le groupe constitué par :

- $N(R^5)_2$, OH, $Si(R^5)_3$, $B(OR^5)_2$, $OSO_2R^5$, $CF_3$, CN, F, Br, I ;
- un groupe alkyle, alcoxy ou thioalcoxy linéaire de 1 à 40 atomes C, qui peut à chaque fois être substitué avec un ou plusieurs radicaux $R^5$, un ou plusieurs groupes $CH_2$ non voisins pouvant être remplacés par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$ ;
- un groupe alcényle ou alcynyle linéaire de 2 à 40 atomes C, qui peut à chaque fois être substitué avec un ou plusieurs radicaux $R^5$, un ou plusieurs groupes $CH_2$ non voisins pouvant être remplacés par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$ ;
- un groupe alkyle, alcényle, alcynyle, alcoxy ou thioalcoxy ramifié ou cyclique de 3 à 40 atomes C, qui peut à chaque fois être substitué avec un ou plusieurs radicaux $R^5$, un ou plusieurs groupes $CH_2$ non voisins pouvant être remplacés par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$;
- un système cyclique aromatique ou hétéroaromatique de 5 à 60 atomes de cycle aromatique, qui peut à chaque fois être substitué avec un ou plusieurs radicaux $R^5$ ;
- un groupe aryloxy ou hétéroaryloxy de 5 à 60 atomes de cycle aromatique, qui peut à chaque fois être substitué avec un ou plusieurs radicaux $R^5$ ; et
- un groupe diarylamino, un groupe dihétéroarylamino ou un groupe arylhétéroarylamino de 10 à 40 atomes de cycle aromatique, qui peut à chaque fois être substitué avec un ou plusieurs radicaux $R^5$;

# et $R^5$ ayant les définitions indiquées dans la revendication 1.

**6.** Molécule organique selon les revendications 1 à 4, dans laquelle la deuxième unité chimique à chaque fois présente une structure de formule IIc ou est constituée par une telle structure :

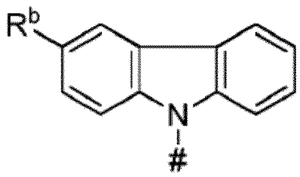

Formule IIc

dans laquelle

les $R^b$ sont choisis à chaque occurrence de manière identique ou différente dans le groupe constitué par :

- $N(R^5)_2$, OH, $Si(R^5)_3$, $B(OR^5)_2$, $OSO_2R^5$, $CF_3$, CN, F, Br, I ;
- un groupe alkyle, alcoxy ou thioalcoxy linéaire de 1 à 40 atomes C, qui peut à chaque fois être substitué avec un ou plusieurs radicaux $R^5$, un ou plusieurs groupes $CH_2$ non voisins pouvant être remplacés par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$ ;
- un groupe alcényle ou alcynyle linéaire de 2 à 40 atomes C, qui peut à chaque fois être substitué avec un ou plusieurs radicaux $R^5$, un ou plusieurs groupes $CH_2$ non voisins pouvant être remplacés par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$ ;
- un groupe alkyle, alcényle, alcynyle, alcoxy ou thioalcoxy ramifié ou cyclique de 3 à 40 atomes C, qui peut à chaque fois être substitué avec un ou plusieurs radicaux $R^5$, un ou plusieurs groupes $CH_2$ non voisins pouvant être remplacés par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$, et un ou plusieurs atomes H pouvant être remplacés par le deutérium, CN, $CF_3$ ou $NO_2$;
- un système cyclique aromatique ou hétéroaromatique de 5 à 60 atomes de cycle aromatique, qui peut à chaque fois être substitué avec un ou plusieurs radicaux $R^5$ ;
- un groupe aryloxy ou hétéroaryloxy de 5 à 60 atomes de cycle aromatique, qui peut à chaque fois être substitué avec un ou plusieurs radicaux $R^5$ ; et
- un groupe diarylamino, un groupe dihétéroarylamino ou un groupe arylhétéroarylamino de 10 à 40 atomes de cycle aromatique, qui peut à chaque fois être substitué avec un ou plusieurs radicaux $R^5$ ;

les définitions indiquées dans la revendication 1 s'appliquant pour le reste.

**7.** Molécule organique selon la revendication 5 ou 6, dans laquelle les $R^b$ représentent à chaque occurrence de manière identique ou différente Me, $^i$Pr, $^t$Bu, CN, $CF_3$, ou
Ph, qui peut à chaque fois être substitué avec un ou plusieurs radicaux choisis parmi Me, $^i$Pr, $^t$Bu, CN, $CF_3$ ou Ph,
pyridinyle, qui peut à chaque fois être substitué avec un ou plusieurs radicaux choisis parmi Me, $^i$Pr, $^t$Bu, CN, $CF_3$ ou Ph,
pyrimidinyle, qui peut à chaque fois être substitué avec un ou plusieurs radicaux choisis parmi Me, $^i$Pr, $^t$Bu, CN, $CF_3$ ou Ph,
triazinyle, qui peut à chaque fois être substitué avec un ou plusieurs radicaux choisis parmi Me, $^i$Pr, $^t$Bu, CN, $CF_3$ ou Ph,
carbazolyle, qui peut à chaque fois être substitué avec un ou plusieurs radicaux choisis parmi Me, $^i$Pr, $^t$Bu, CN, $CF_3$ ou Ph,
ou $N(Ph)_2$.

**8.** Procédé de fabrication d'une molécule organique selon les revendications 1 à 7, selon lequel un acide 3,5-bis(trifluorméthyle)phényl-boronique substitué par $R^1$ en position 2, 4 et 6 ou un ester 3,5-bis(trifluorméthyle)phénylboronique correspondant substitué par $R^1$ en position 2, 4 et 6 est utilisé en tant que réactif.

**9.** Utilisation d'une molécule organique selon les revendications 1 à 7 en tant qu'émetteur luminescent et/ou en tant que matériau hôte et/ou en tant que matériau de transport d'électrons et/ou en tant que matériau d'injection de trous et/ou en tant que matériau de blocage de trous dans un dispositif optoélectronique organique.

**10.** Utilisation selon la revendication 9, dans laquelle le dispositif optoélectronique organique est choisi dans le groupe

constitué par :

- les diodes organiques électroluminescentes (OLED),
- les cellules électrochimiques électroluminescentes,
- les capteurs à OLED, notamment dans des capteurs de gaz et de vapeur non blindés hermétiquement vers l'extérieur,
- les diodes organiques,
- les cellules solaires organiques,
- les transistors organiques,
- les transistors à effet de champ organiques ;
- les lasers organiques et
- les éléments de conversion descendante.

**11.** Composition comprenant ou constituée par :

(a) au moins une molécule organique selon l'une quelconque des revendications 1 à 7, notamment sous la forme d'un émetteur et/ou d'un hôte, et
(b) un ou plusieurs matériaux émetteurs et/ou hôtes, qui sont différents de la molécule selon les revendications 1 à 7 ; et
(c) éventuellement un ou plusieurs colorants et/ou un ou plusieurs solvants.

**12.** Dispositif optoélectronique organique, comprenant une molécule organique selon les revendications 1 à 7 ou une composition selon la revendication 11, notamment configurée sous la forme d'un dispositif choisi dans le groupe constitué par une diode organique électroluminescente (OLED), une cellule électrochimique électroluminescente, un capteur d'OLED, notamment des capteurs de gaz et de vapeur non blindés hermétiquement vers l'extérieur, une diode organique, une cellule solaire organique, un transistor organique, un transistor à effet de champ organique, un laser organique et un élément de conversion descendante.

**13.** Dispositif optoélectronique organique selon la revendication 12, comprenant :

- un substrat,
- une anode et
- une cathode, l'anode ou la cathode étant appliquée sur le substrat et
- au moins une couche électroluminescente, qui est agencée entre l'anode et la cathode, et comprend une molécule organique selon les revendications 1 à 7 ou une composition selon la revendication 11.

**14.** Procédé de fabrication d'un composant optoélectronique, selon lequel une molécule organique selon les revendications 1 à 7 est utilisée, notamment comprenant le traitement de la molécule organique par un procédé d'évaporation sous vide ou à partir d'une solution.

**Figur 1**

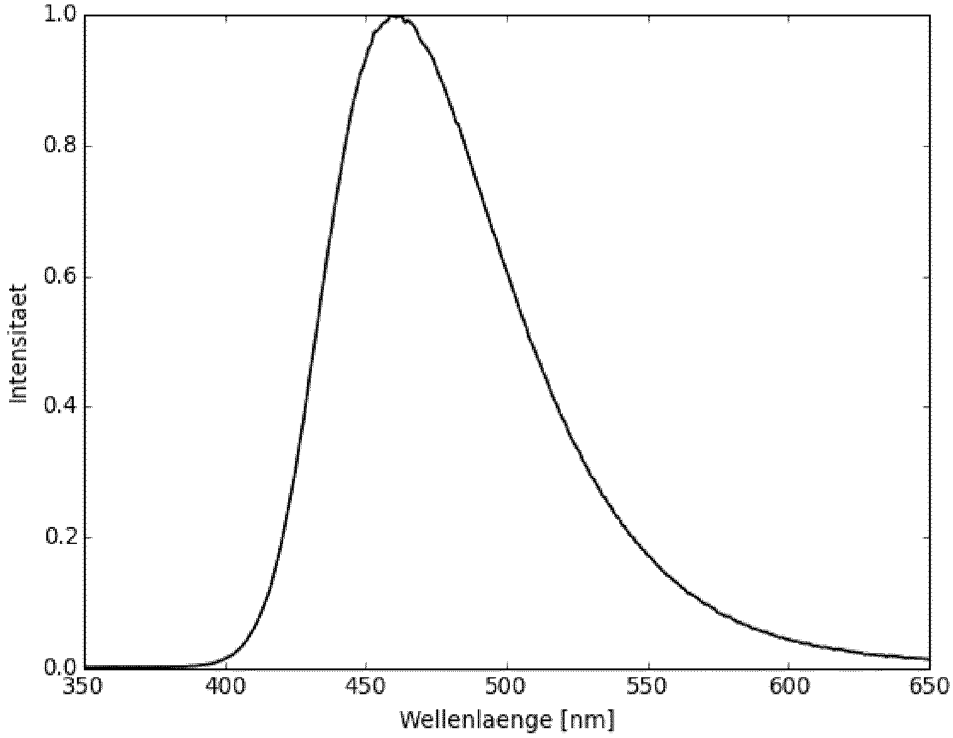


**Figur 2**

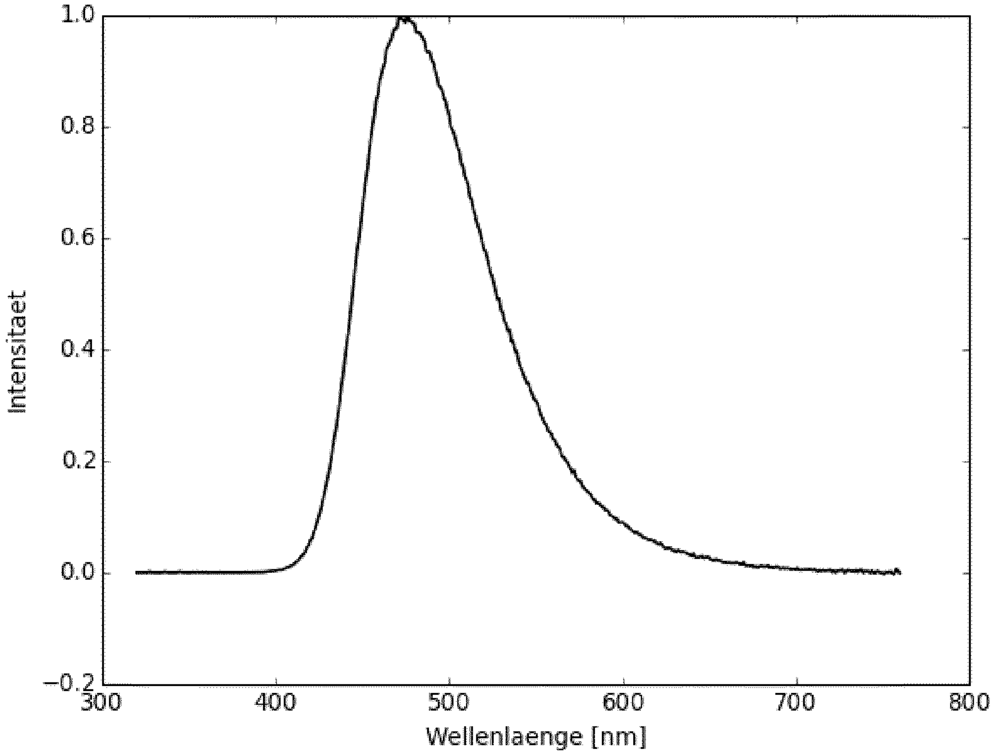

Figur 3

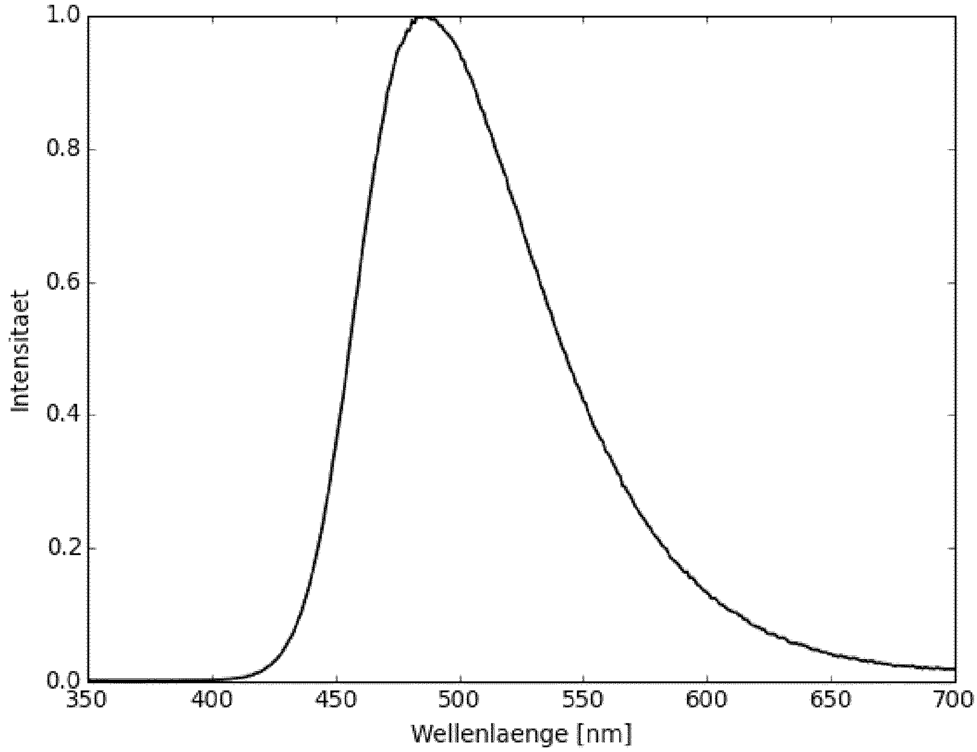


Figur 4

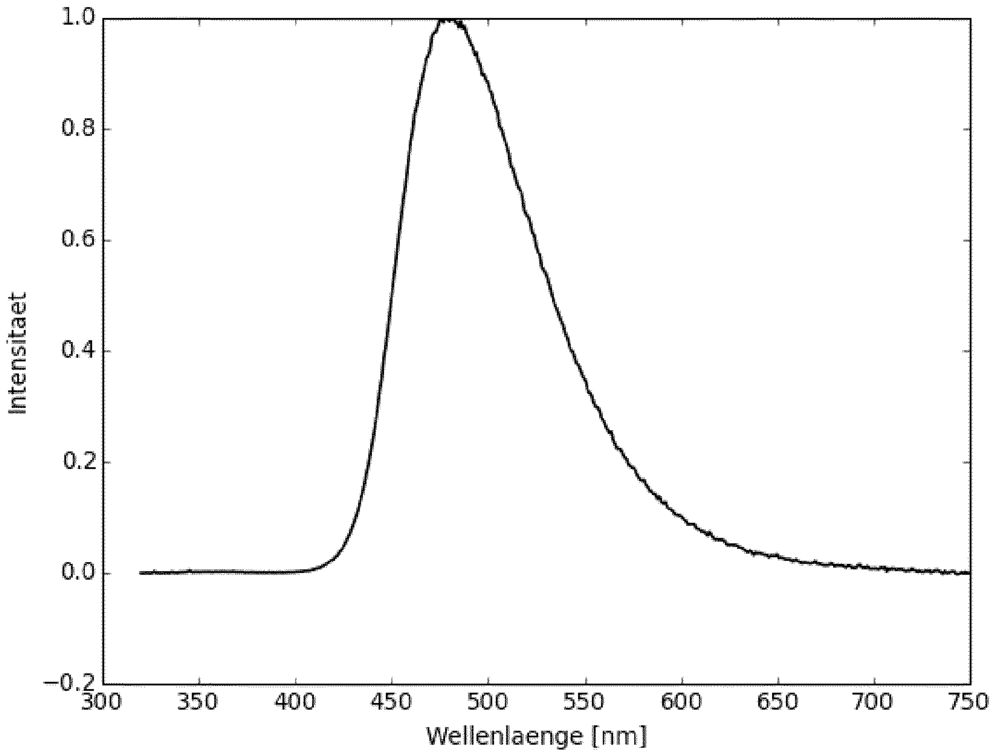

Figur 5

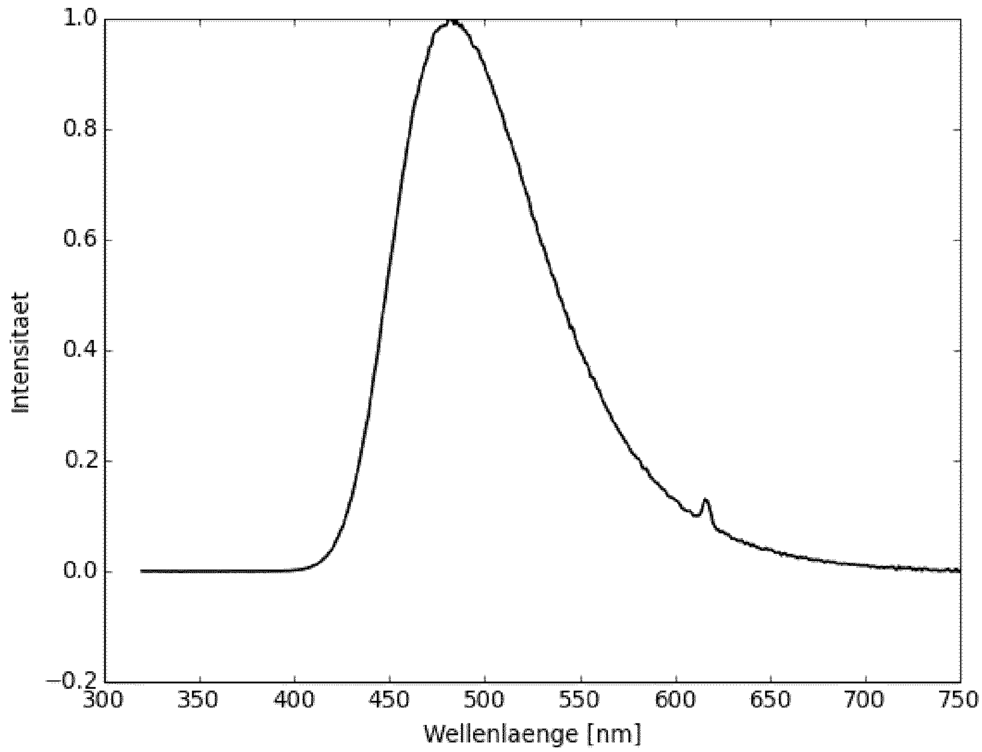


Figur 6

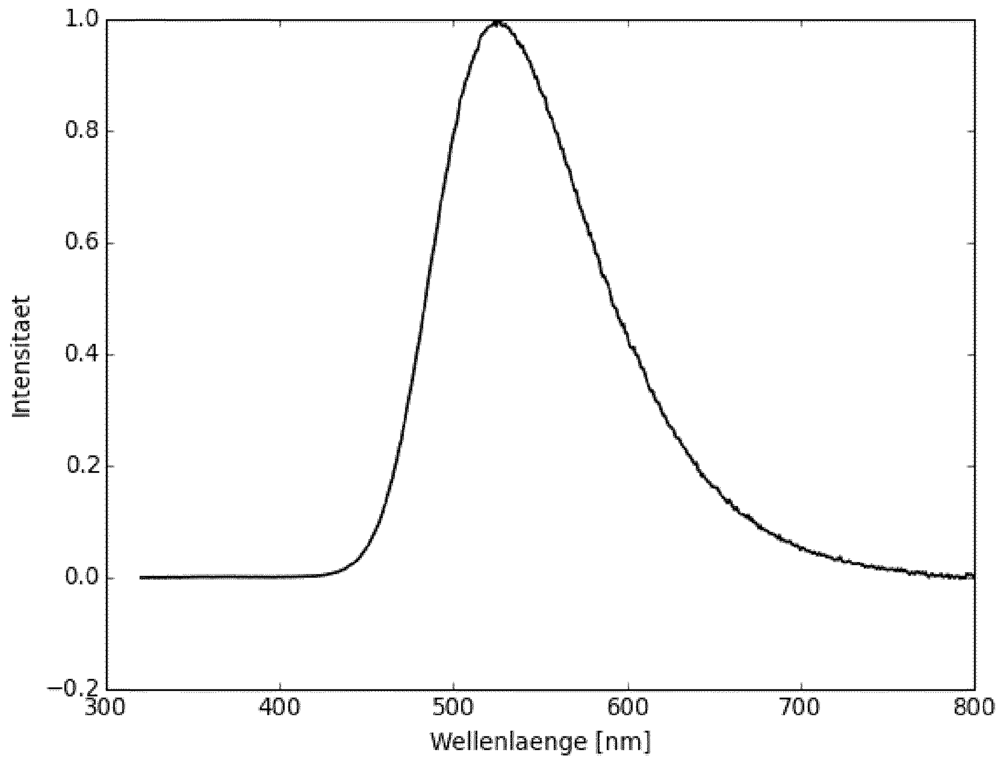

**Figur 7**

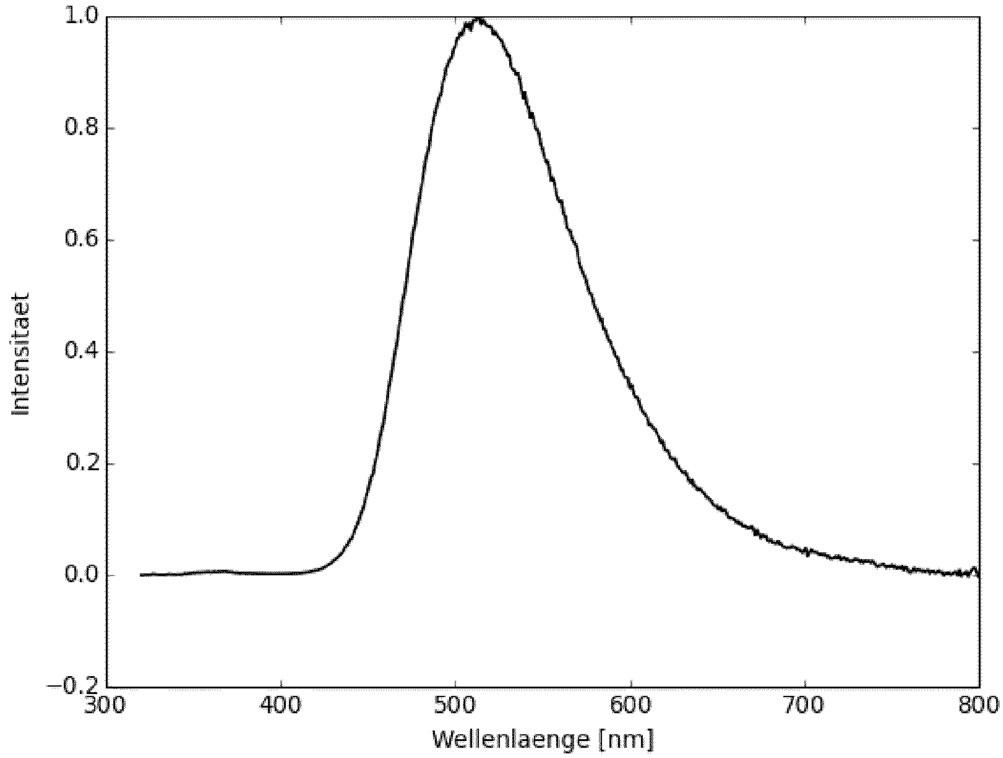


**Figur 8**

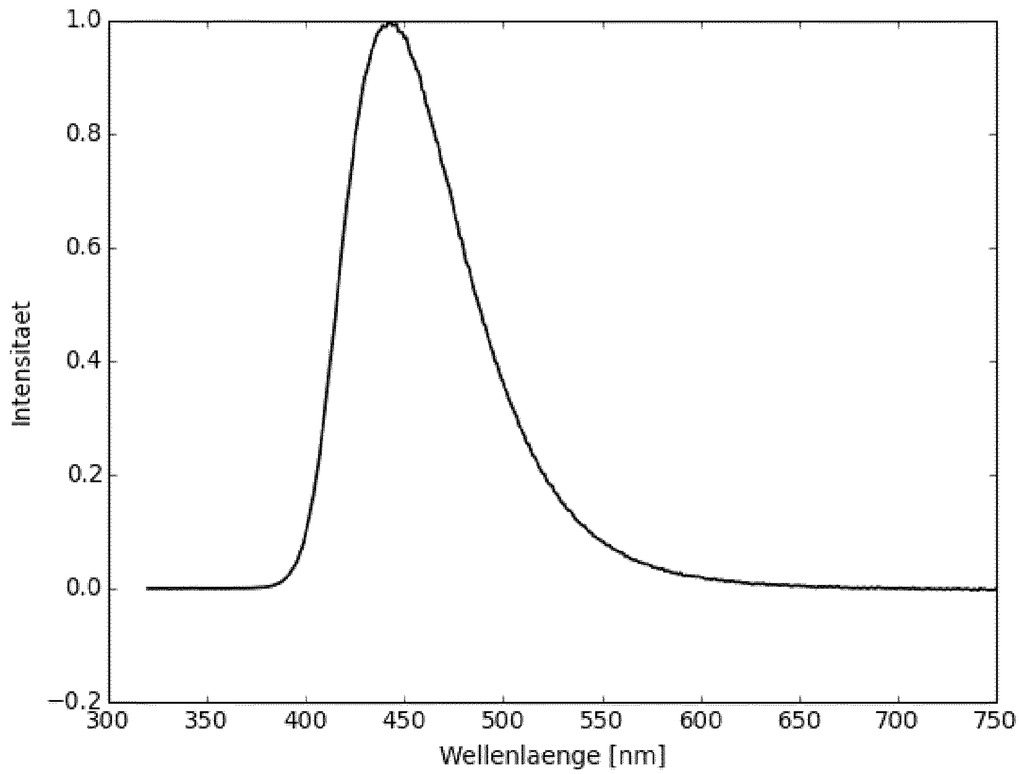

Figur 9

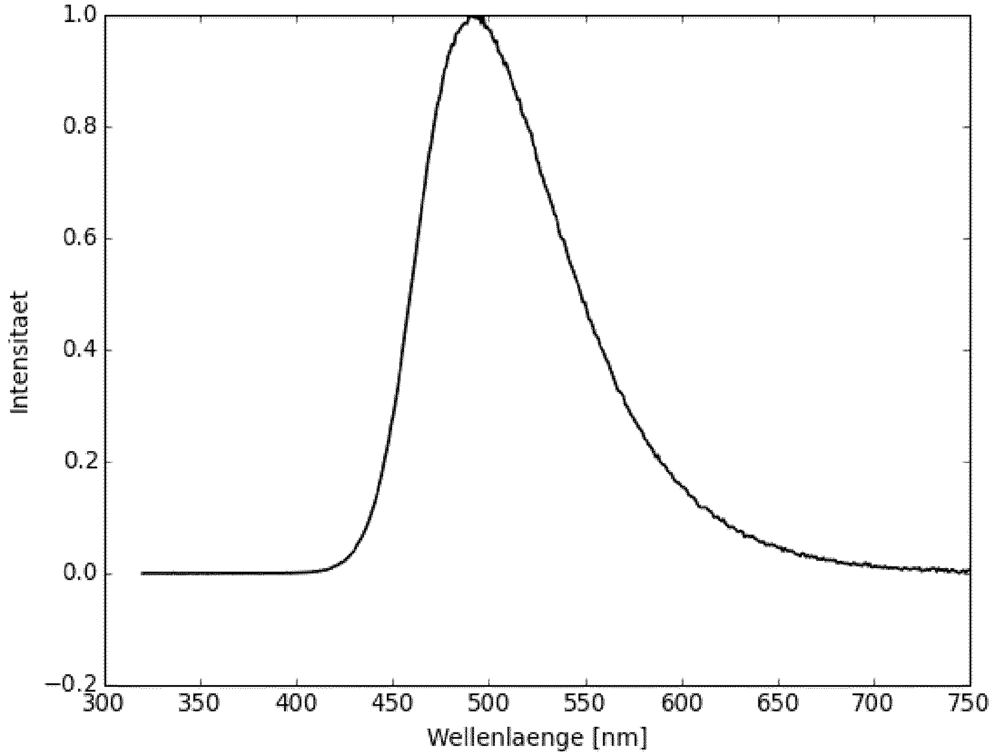


Figur 10

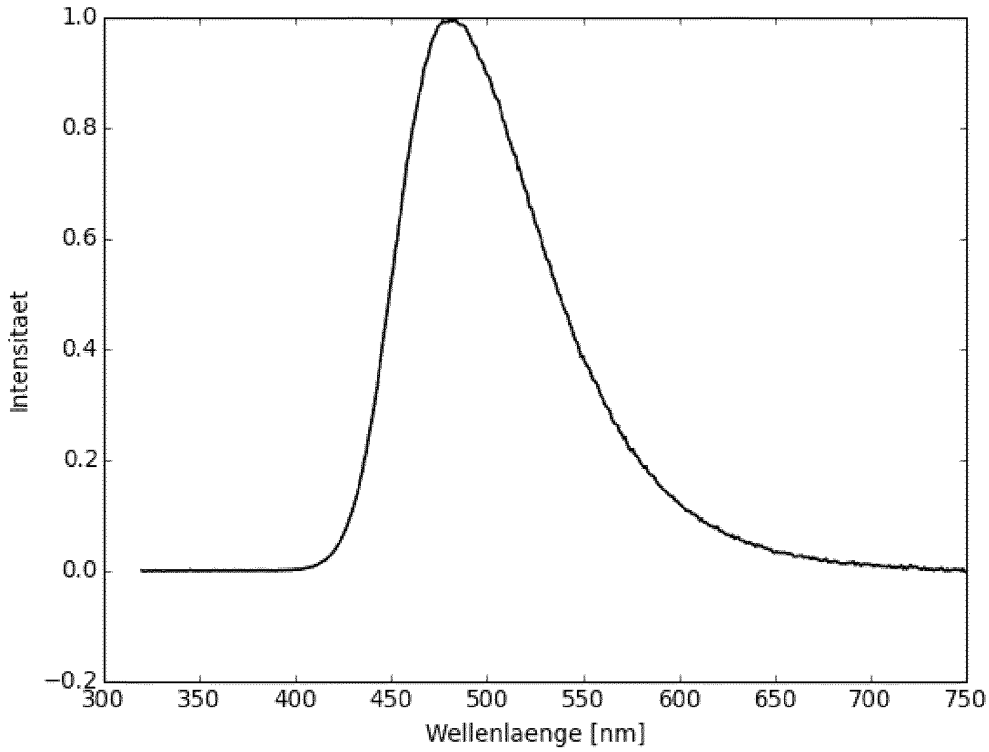

Figur 11

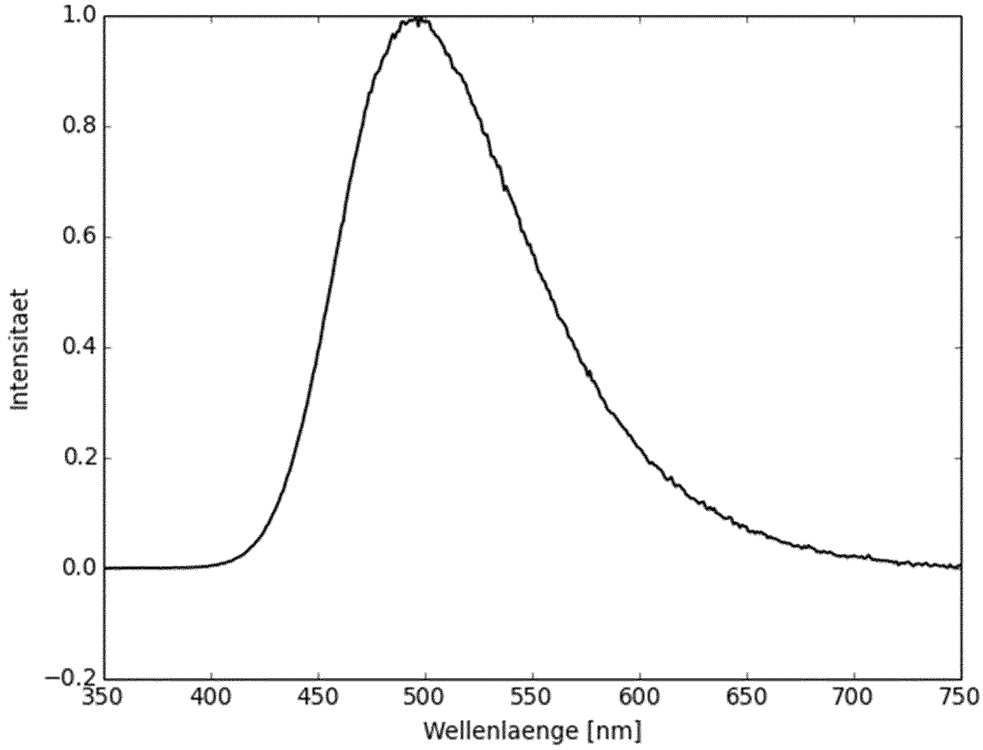


Figur 12

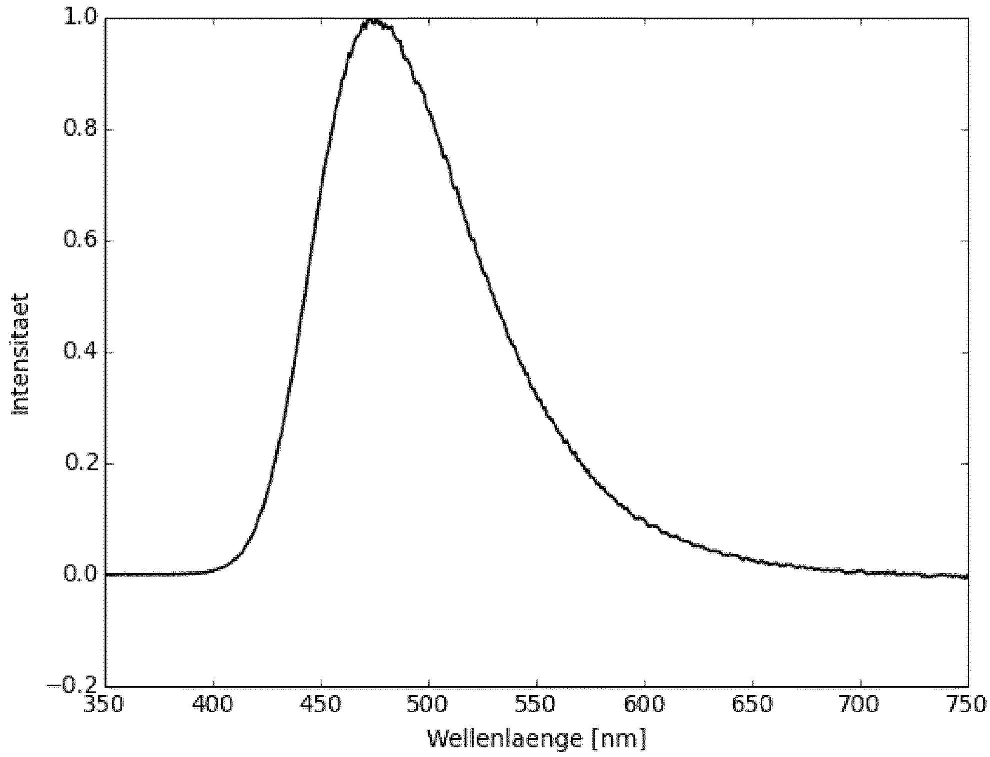

Figur 13

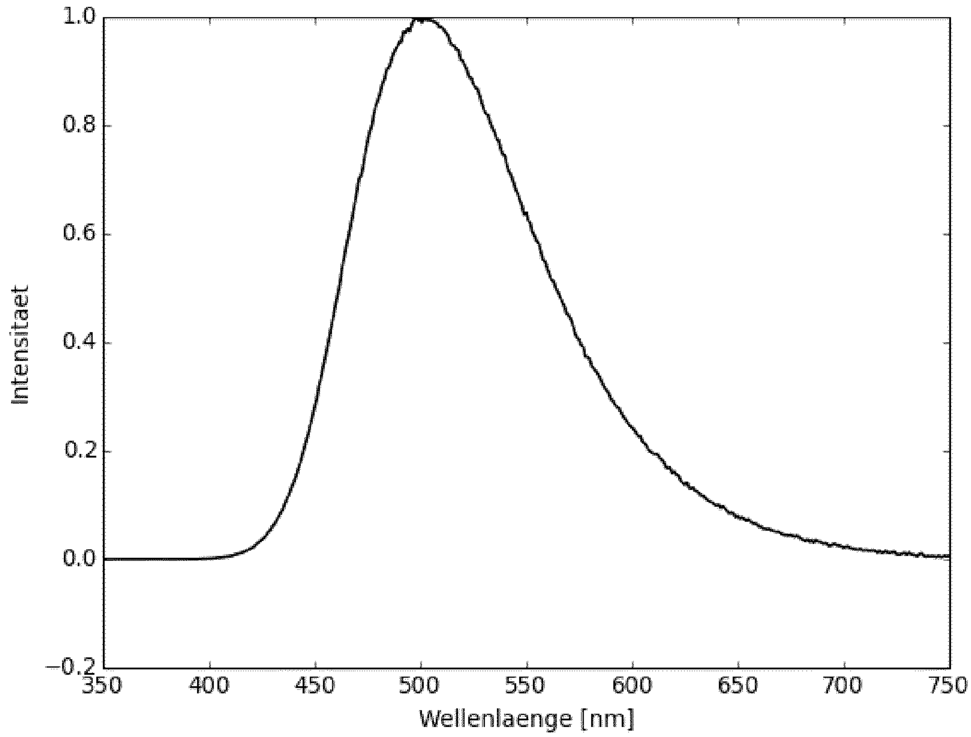


Figur 14

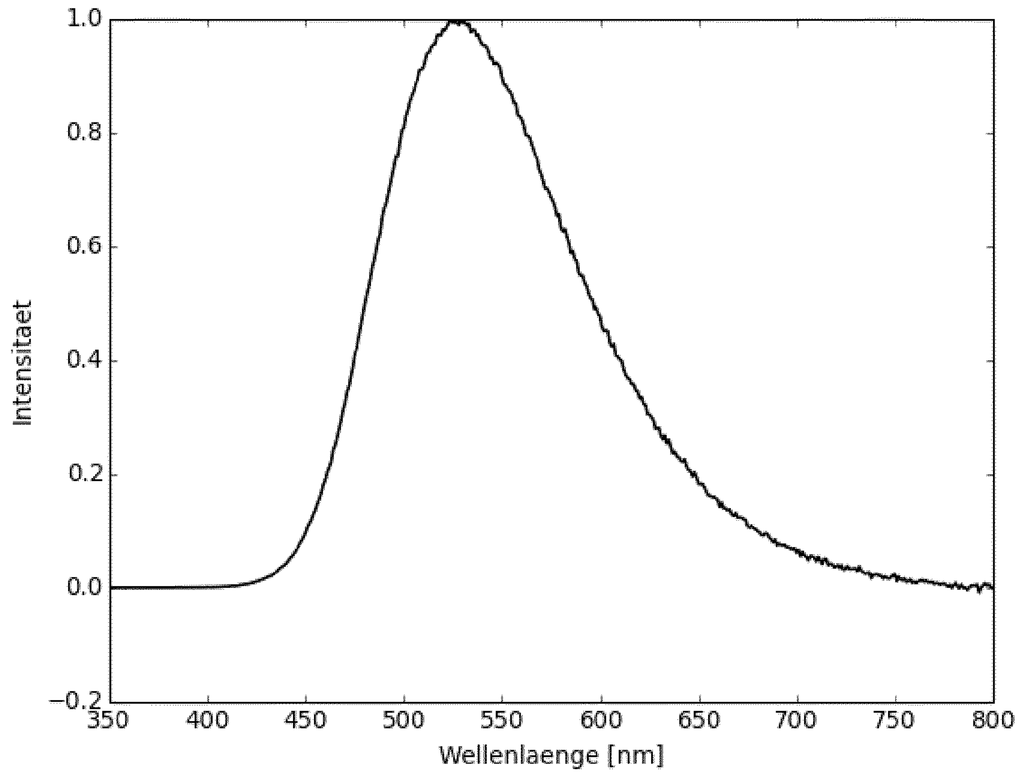

**Figur 15**

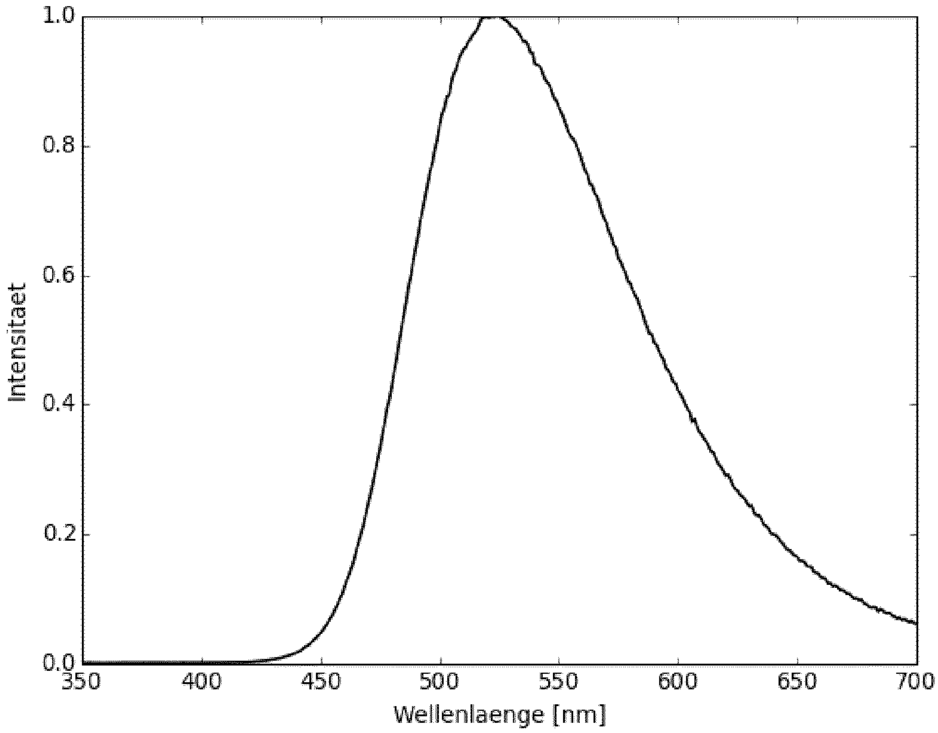


**Figur 16**

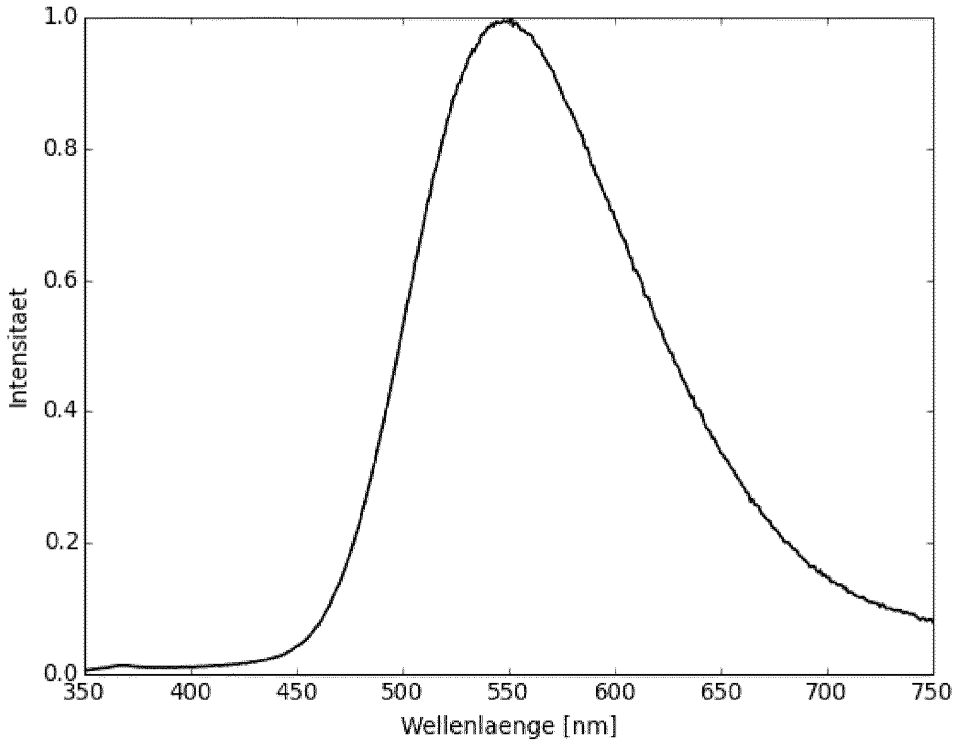

Figur 17

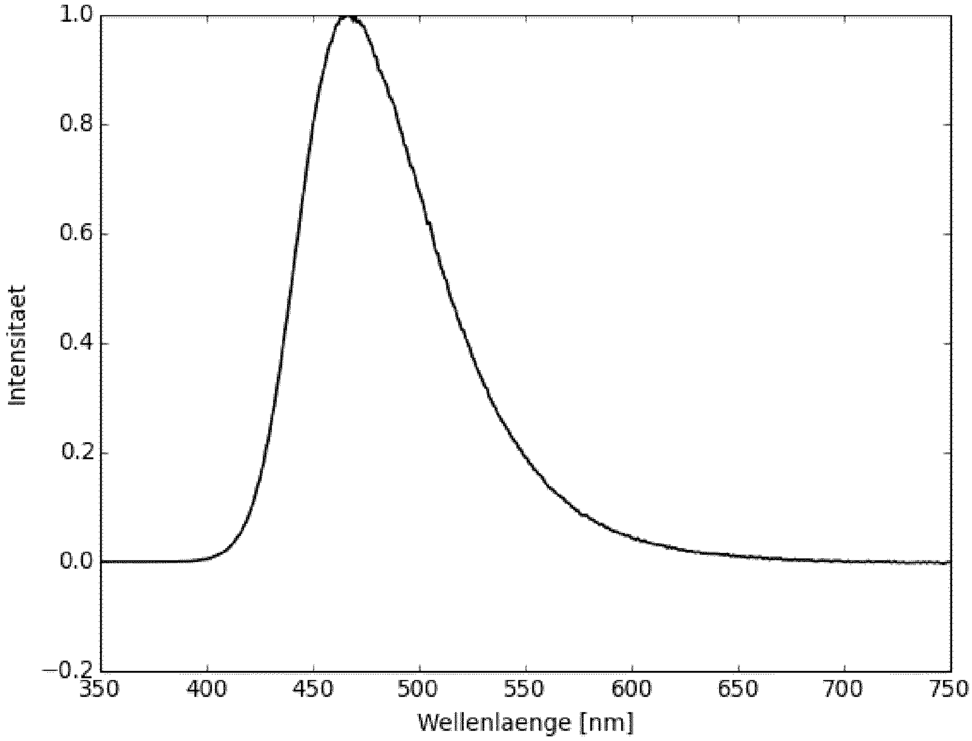


Figur 18

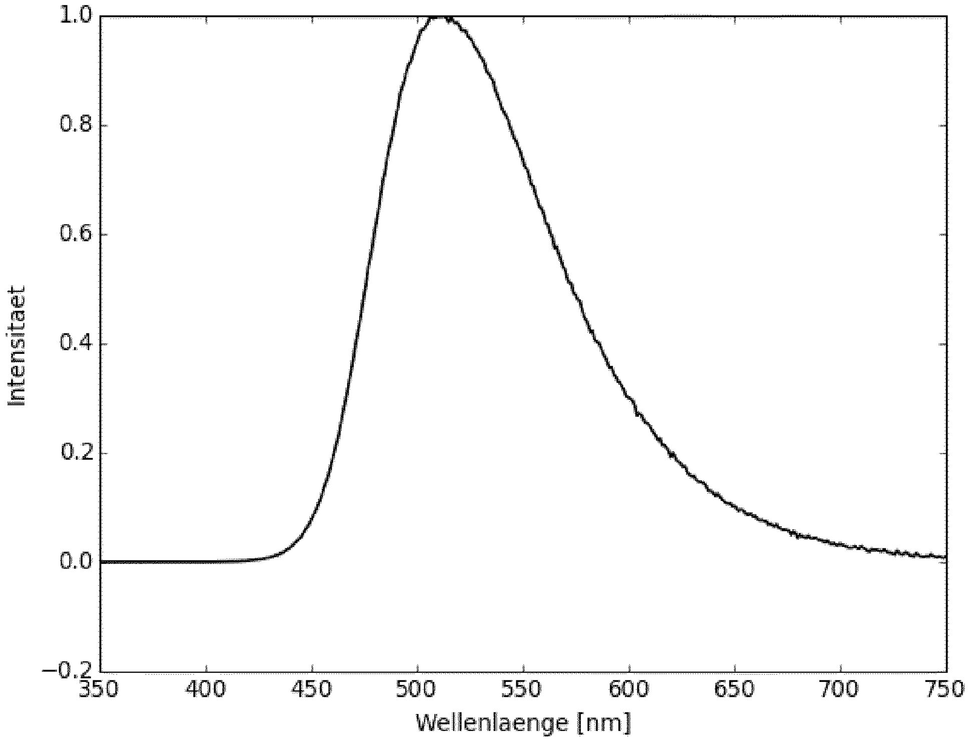

**Figur 19**

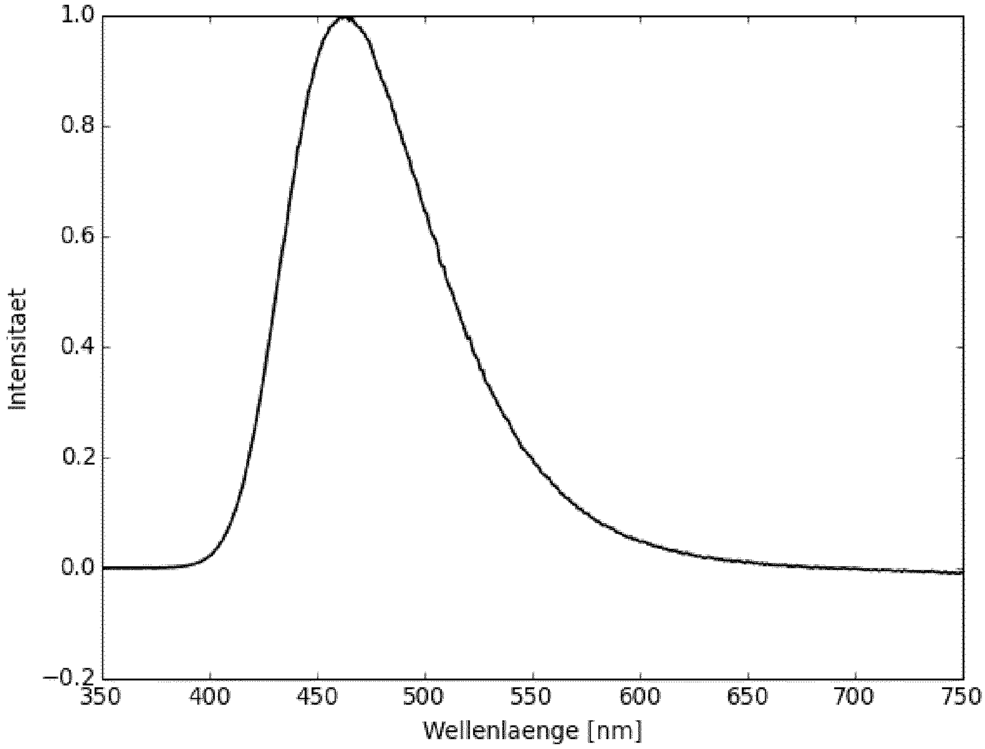


**Figur 20**

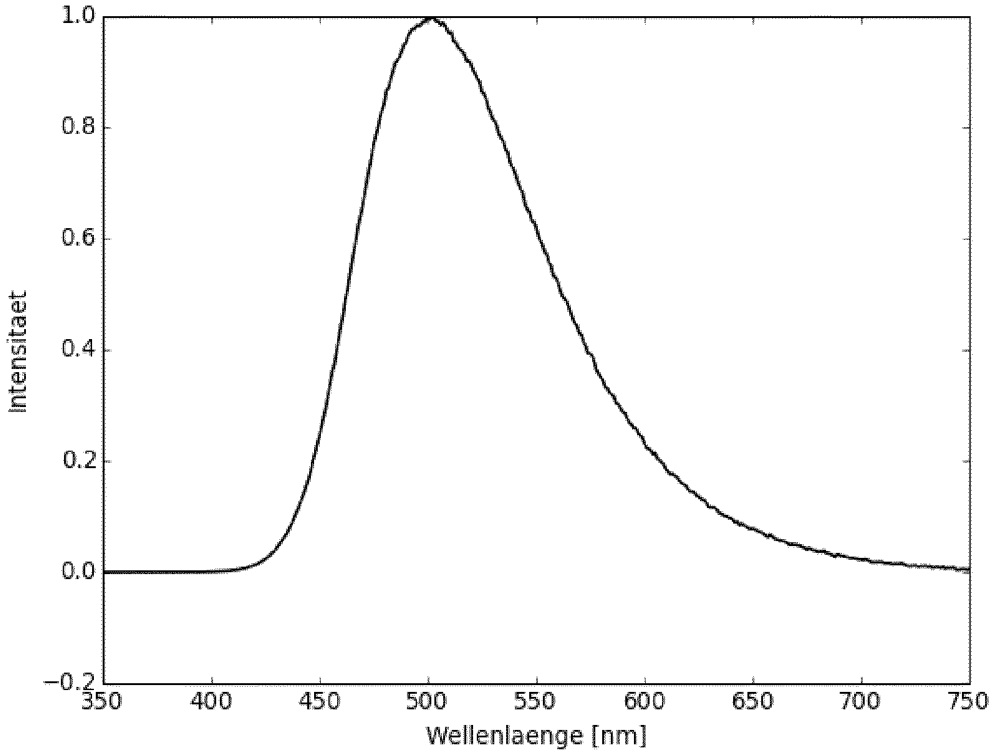

Figur 21

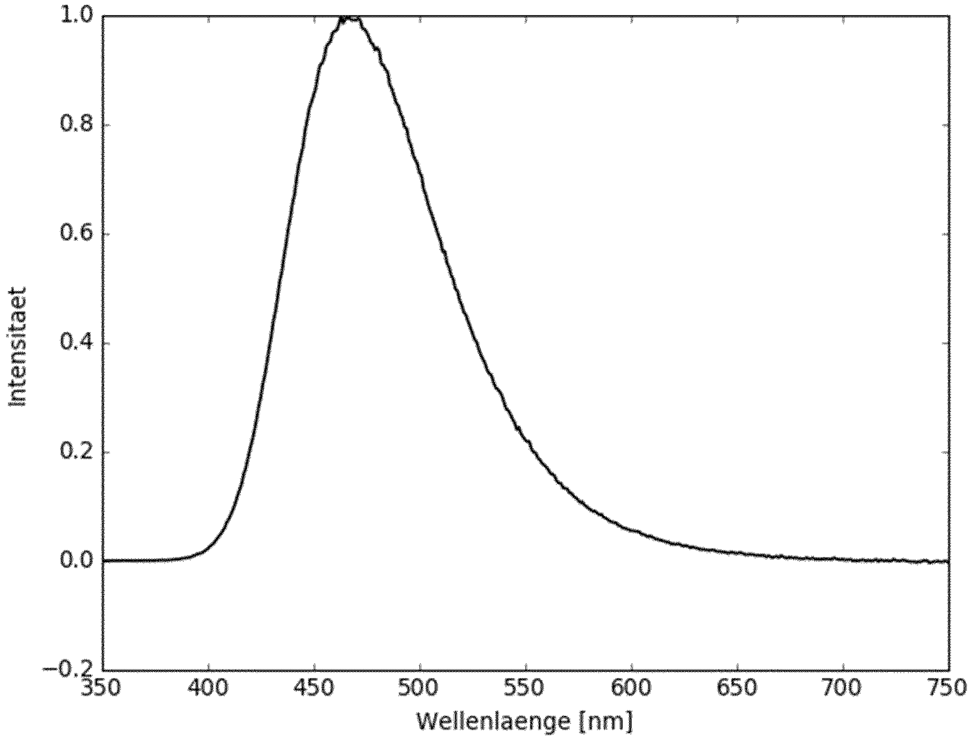


Figur 22

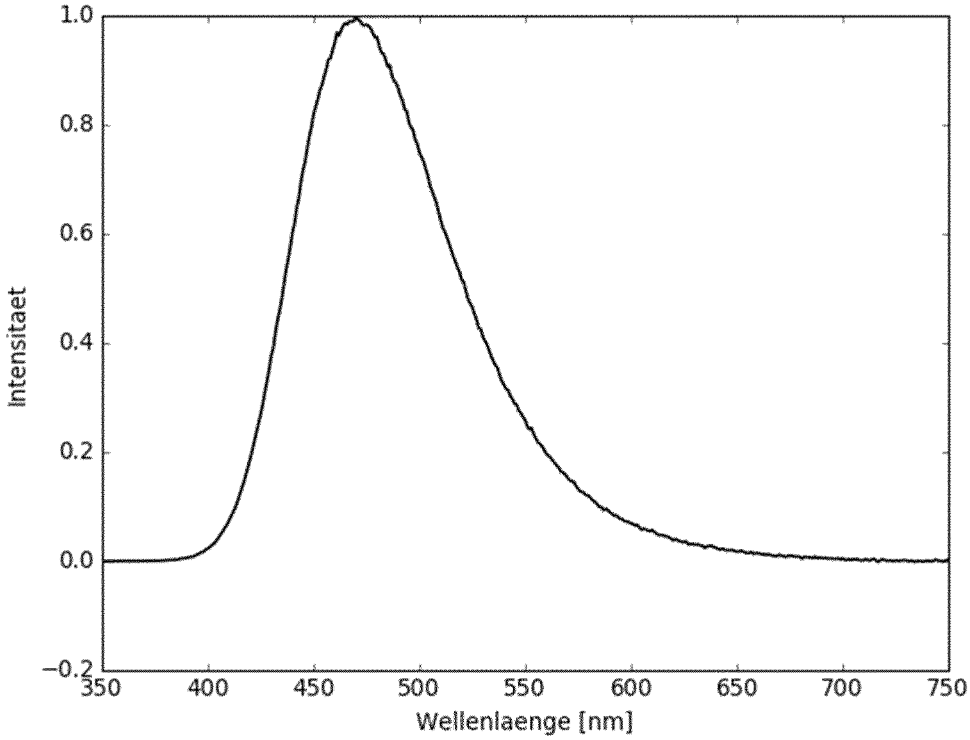

Figur 23

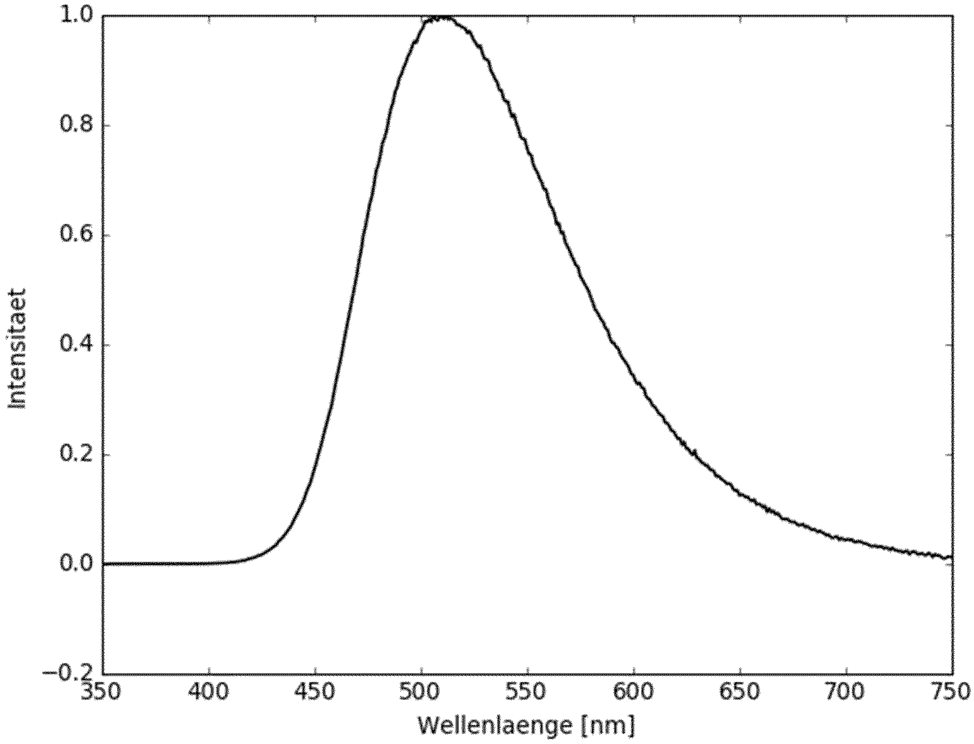


Figur 24

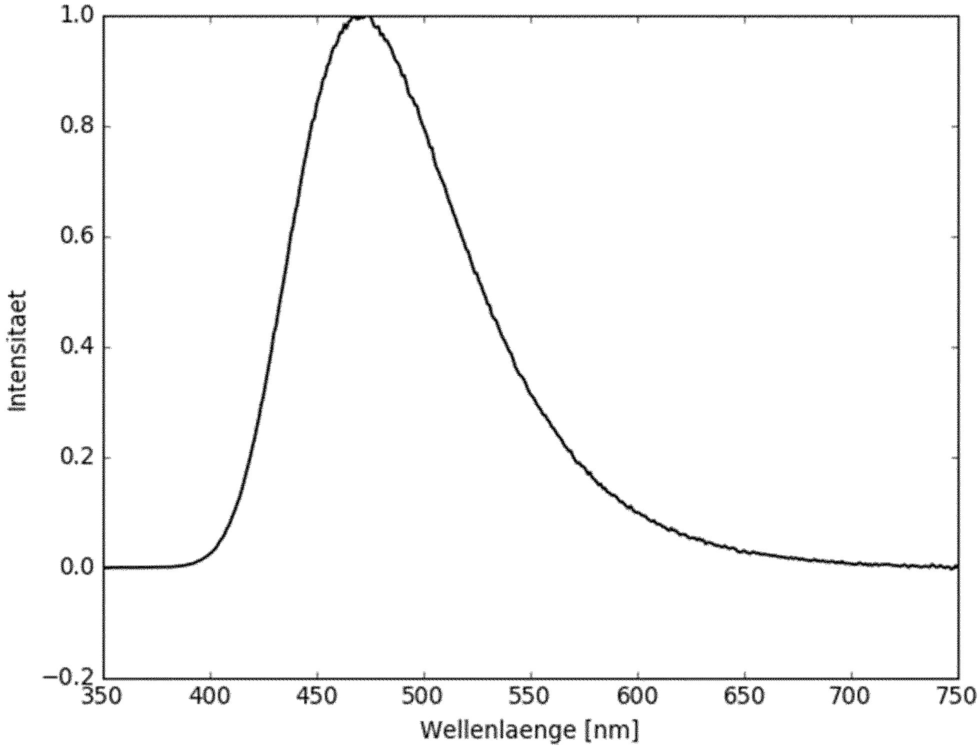

**Figur 25**

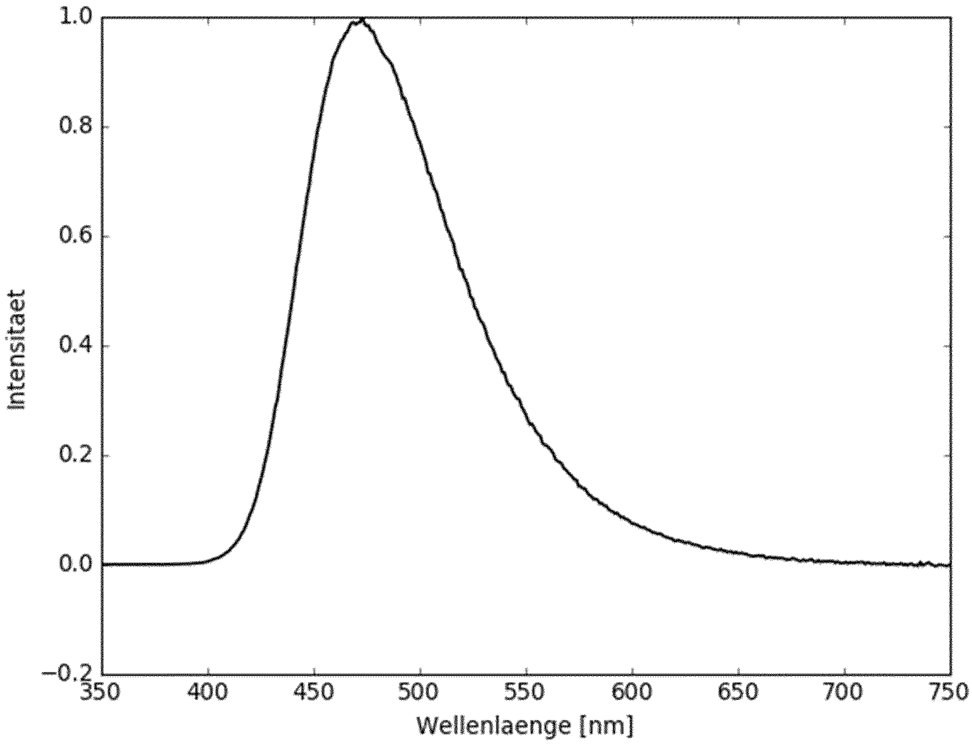


**Figur 26**

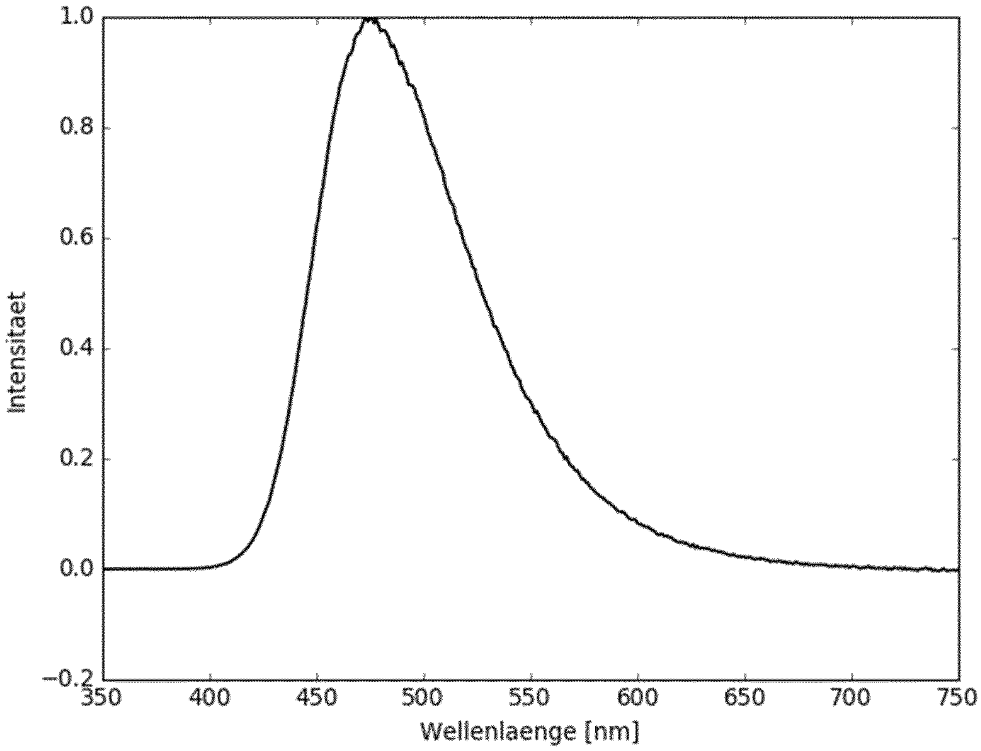

Figur 27

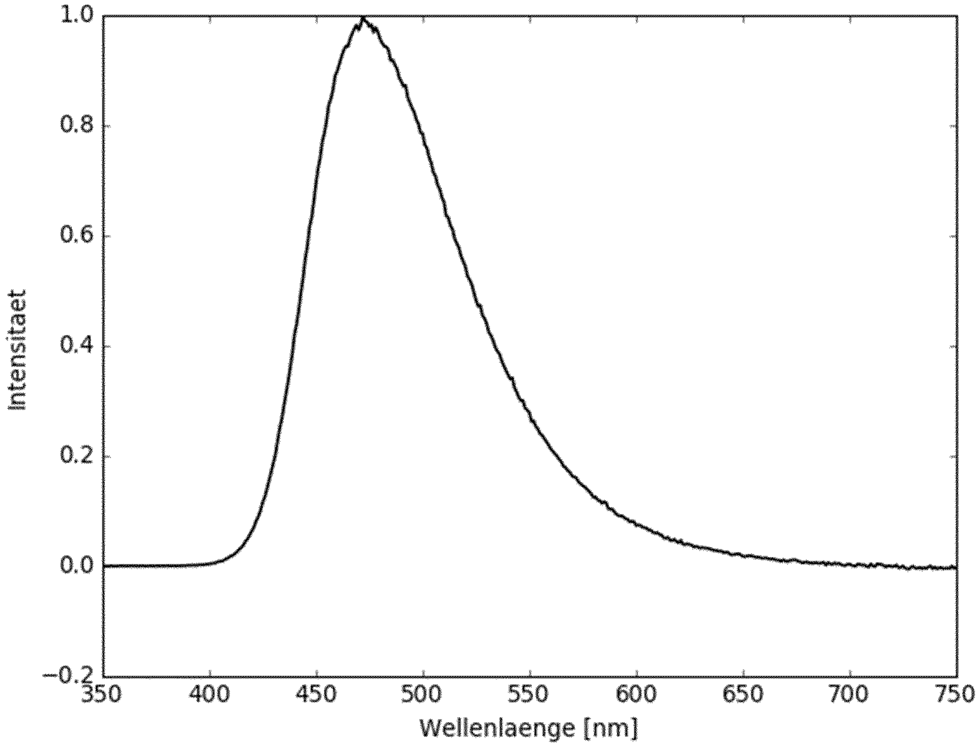


Figur 28

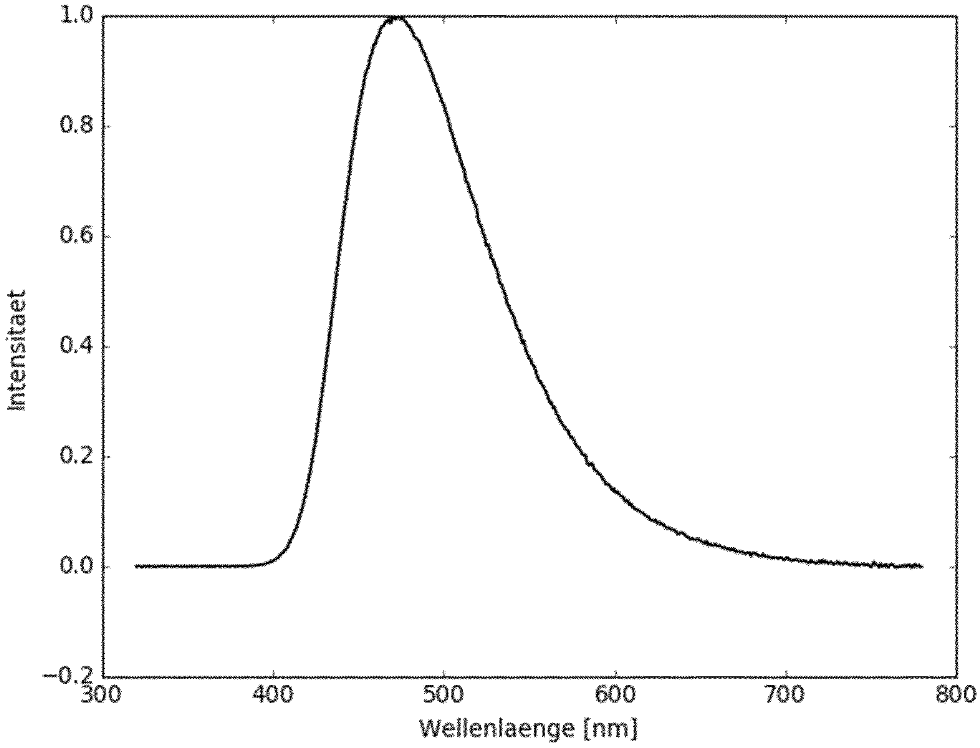

Figur 29

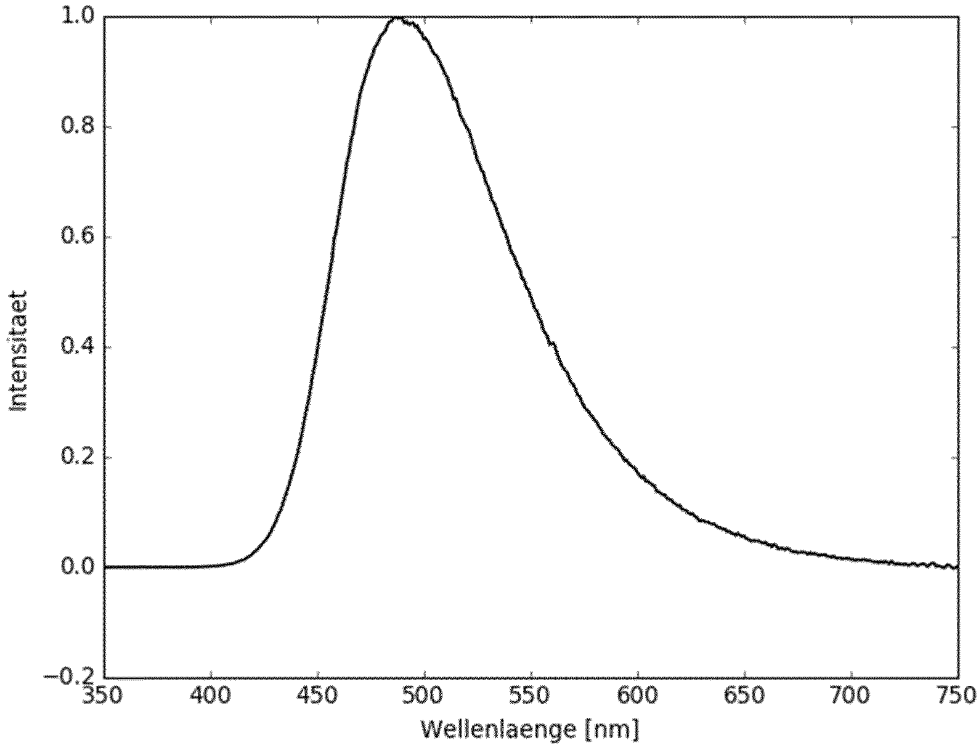


Figur 30

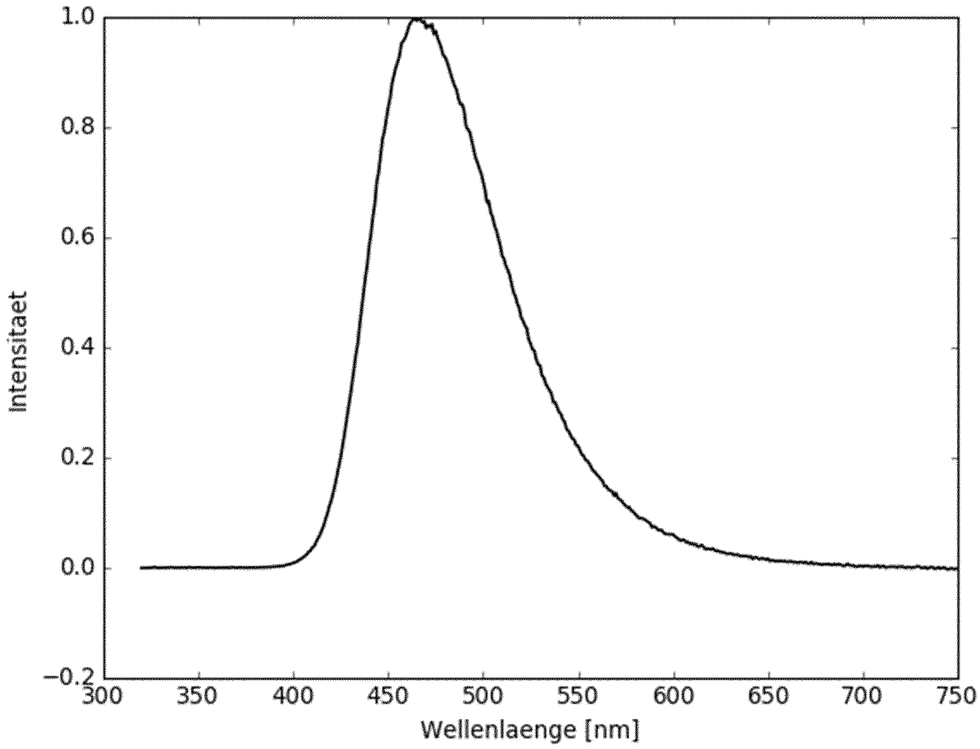

Figur 31

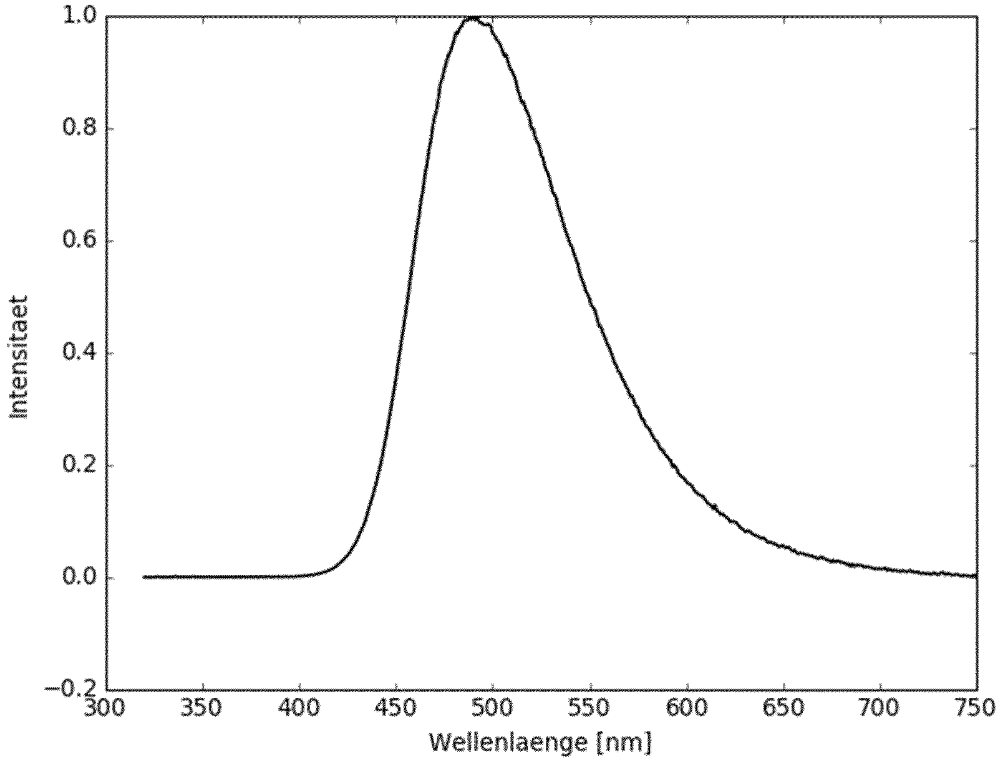


Figur 32

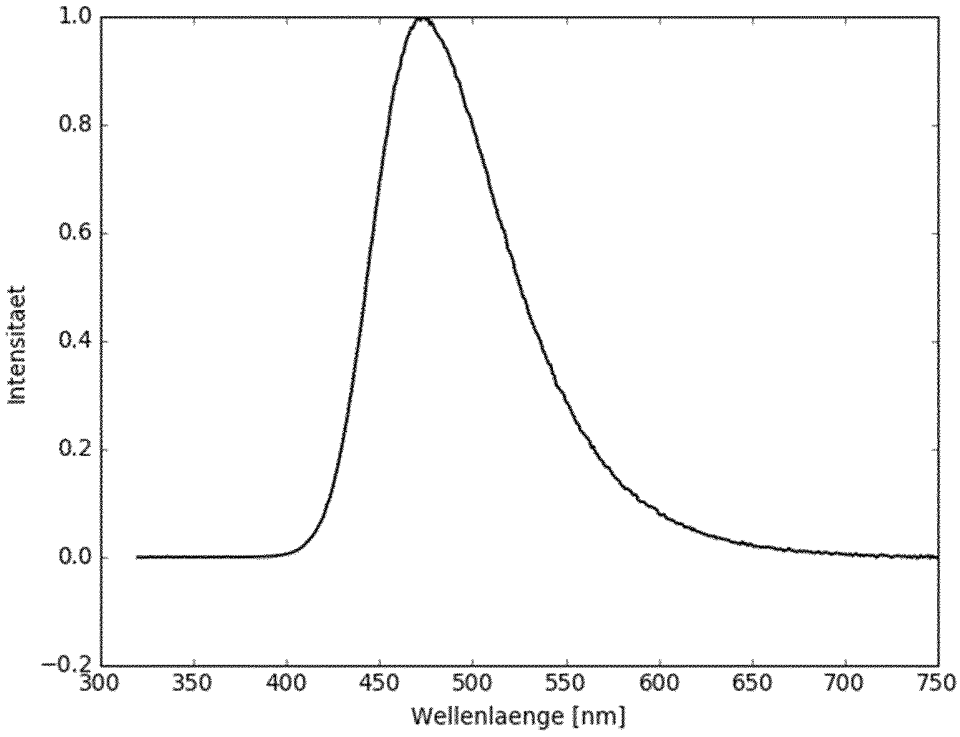

Figur 33

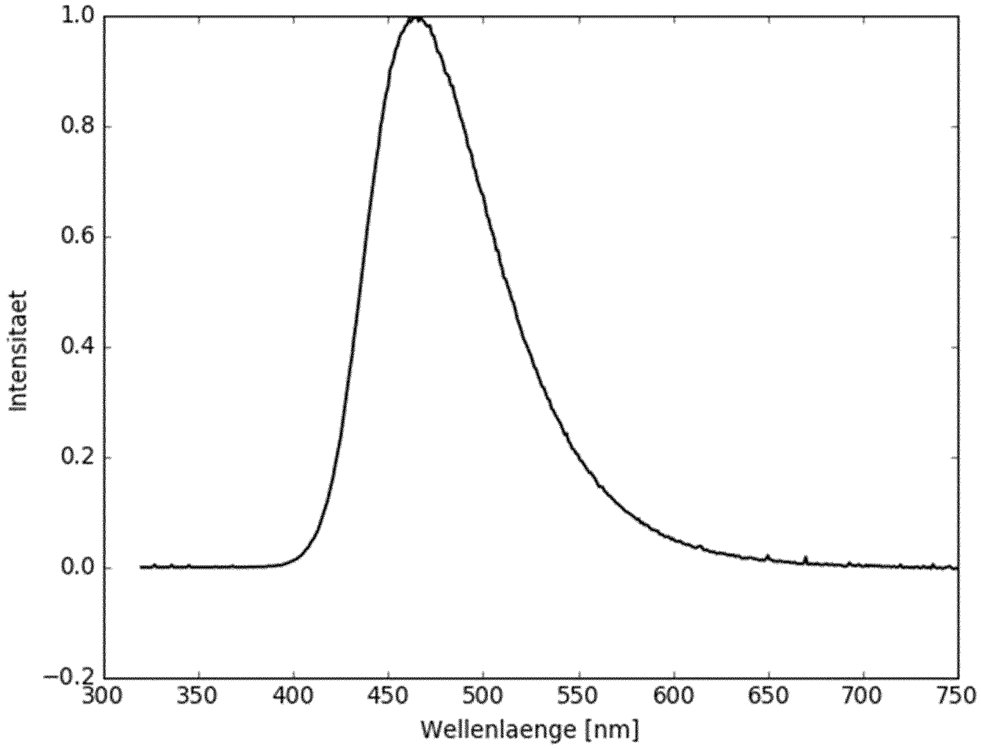


Figur 34

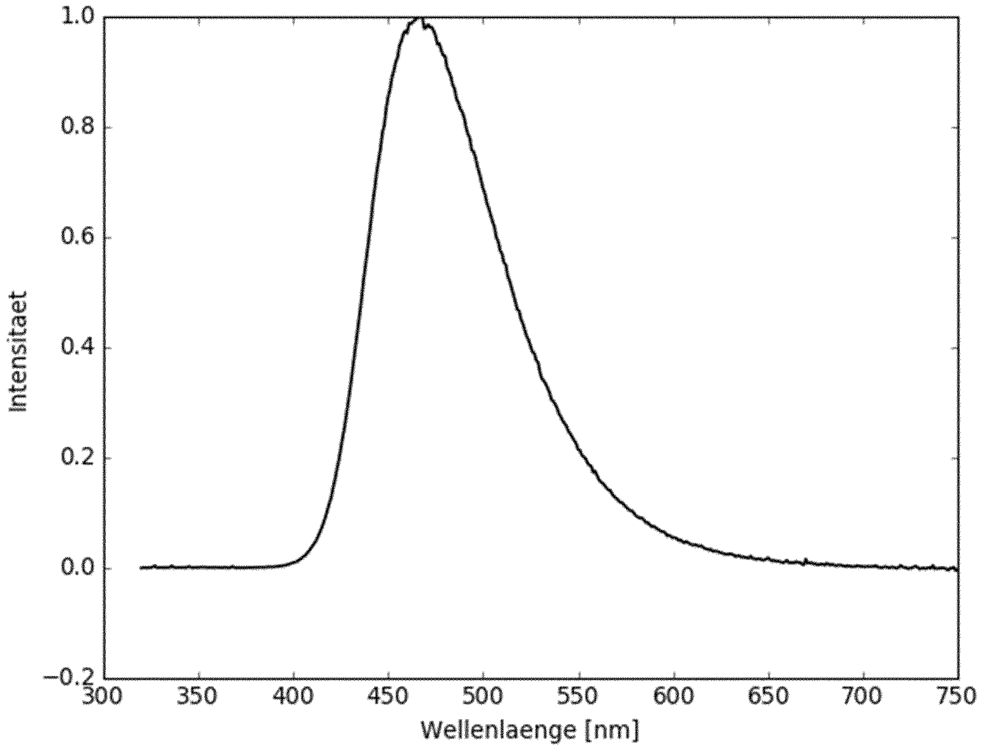

**Figur 35**

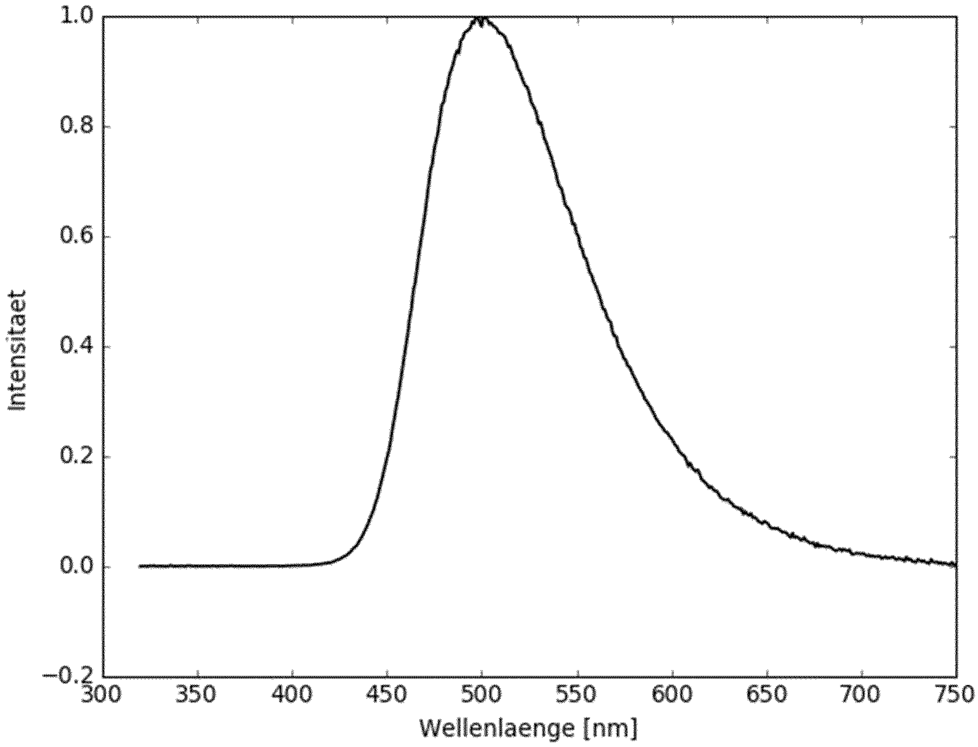


**Figur 36**

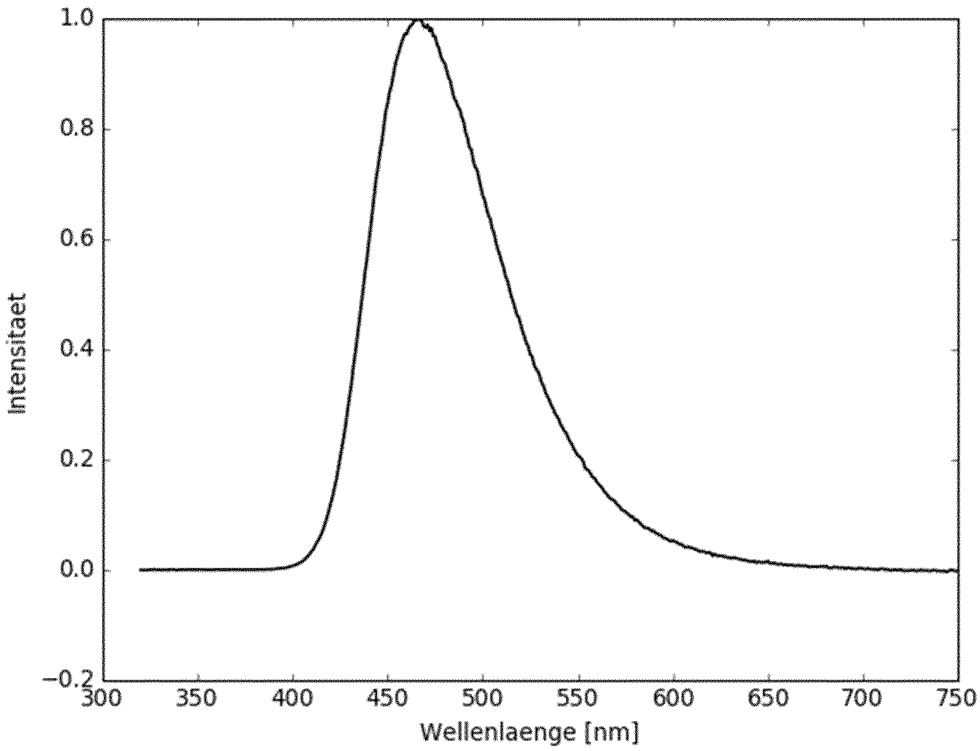

Figur 37

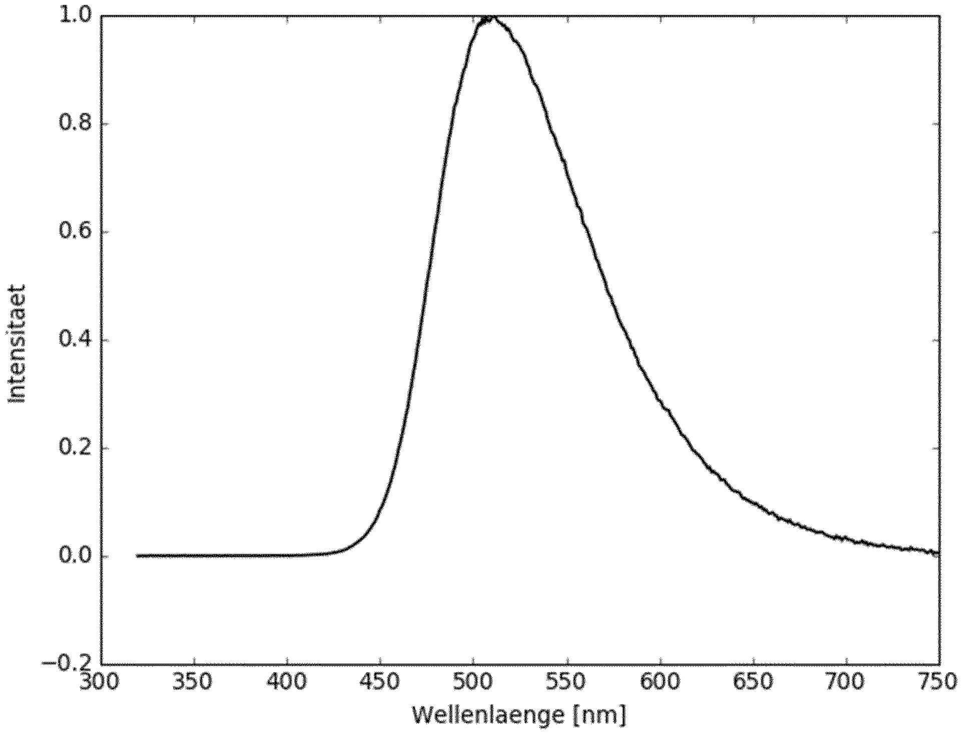


Figur 38

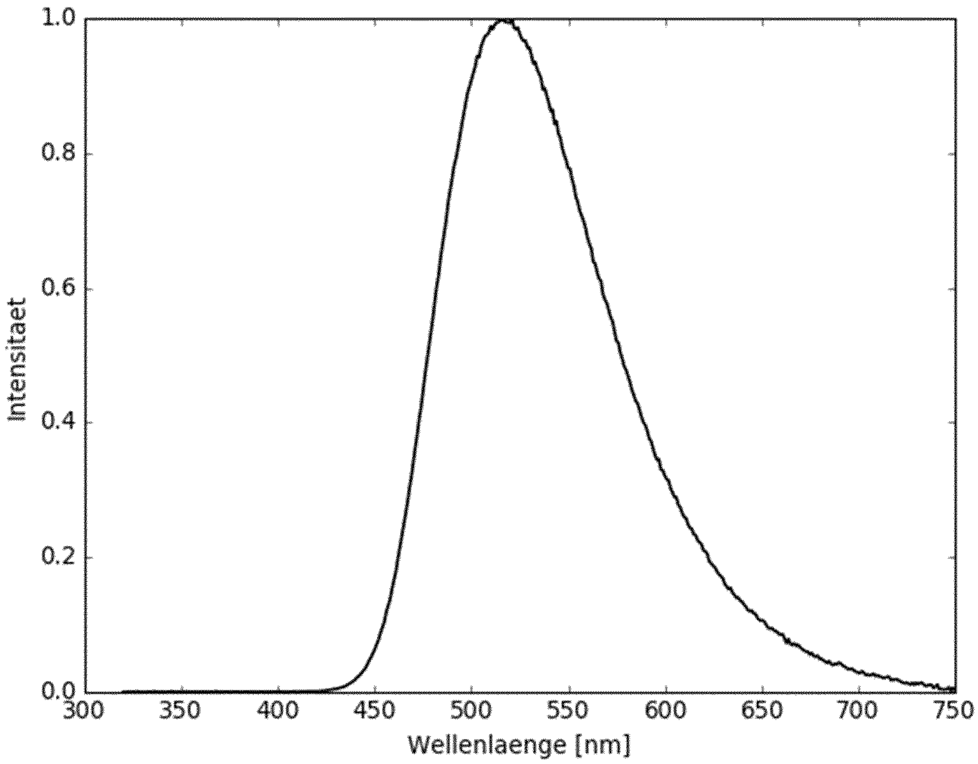

**Figur 39**

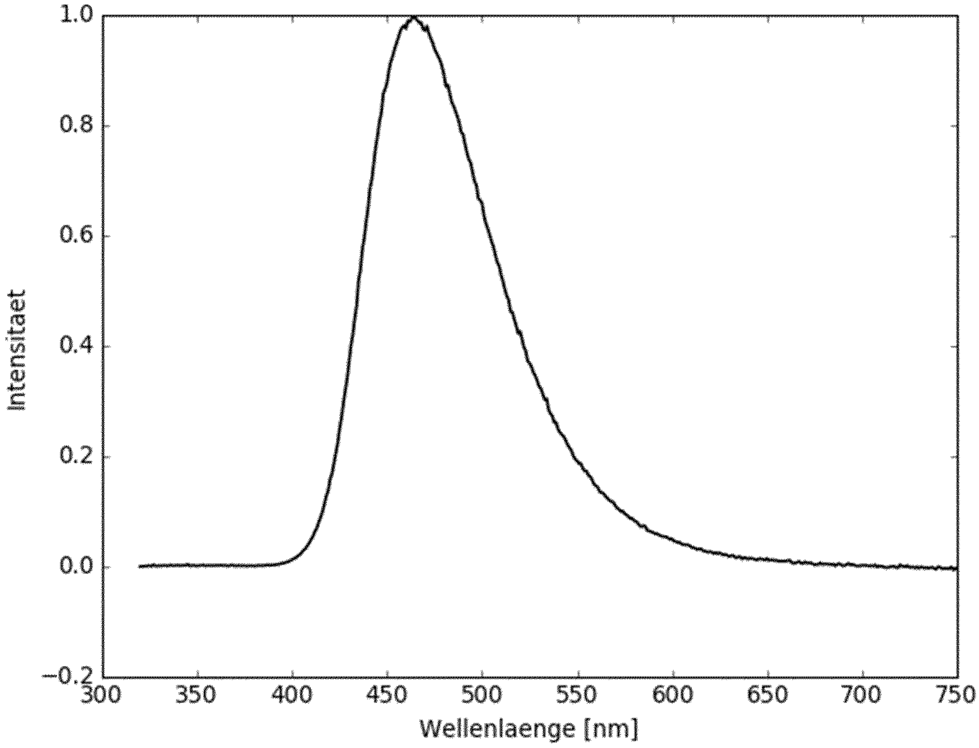


**Figur 40**

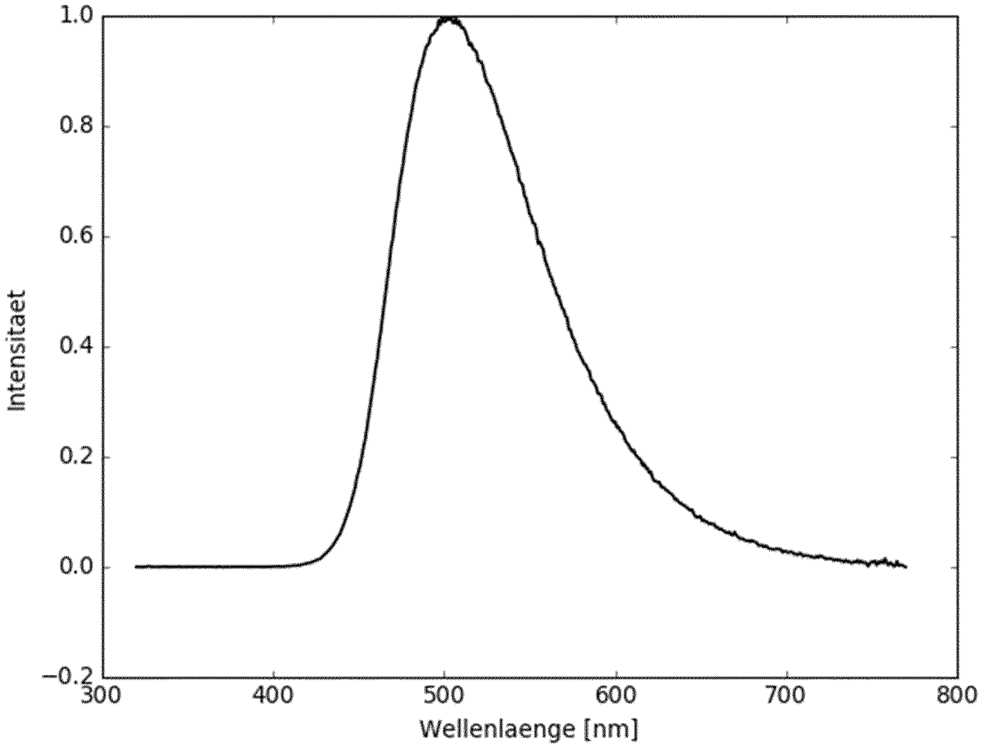

Figur 41

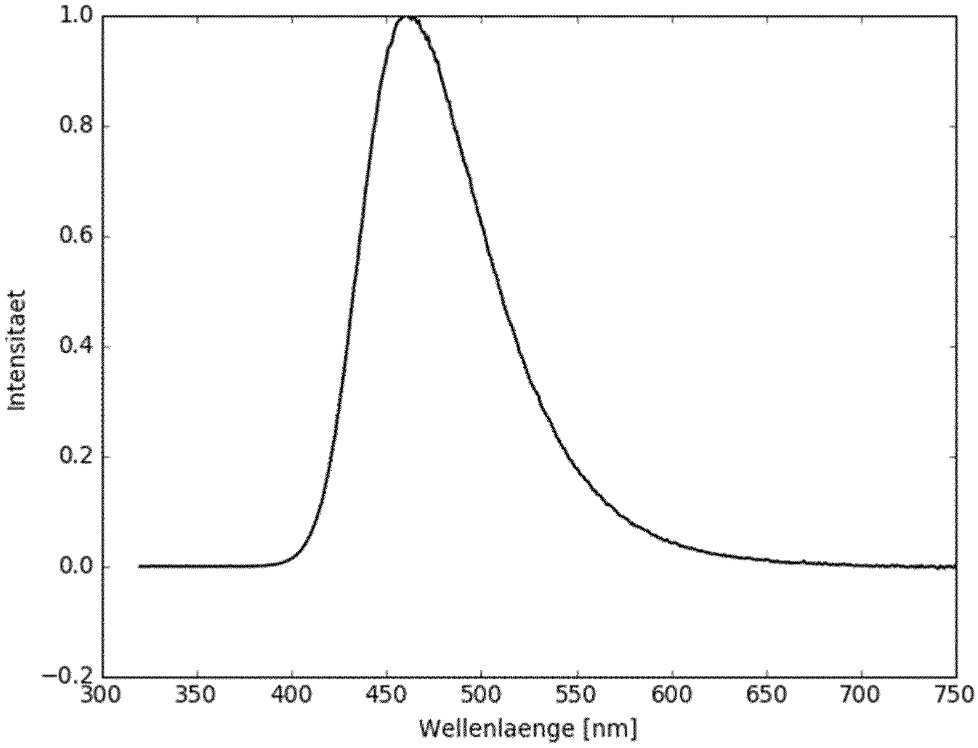


Figur 42

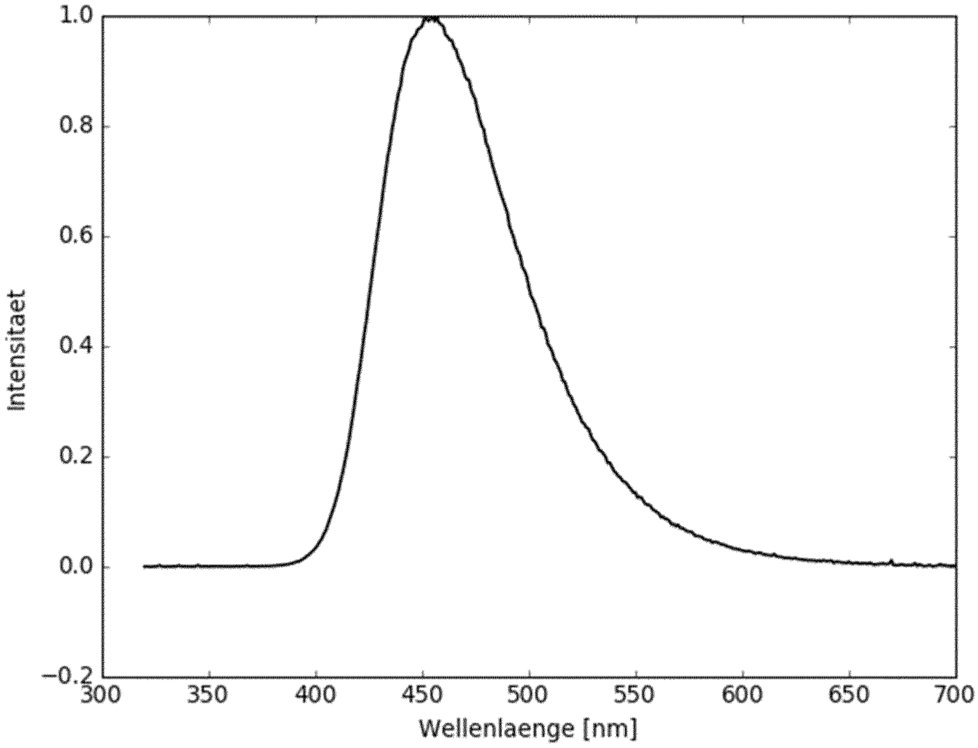

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2015175678 A
- WO 2014183080 A


### In der Beschreibung aufgeführte Nicht-Patentliteratur


- **B. M.E. THOMPSON et al.** *Chem. Mater.,* 2004, vol. 16, 4743